# FASCICULE DE BREVET EUROPEEN

(11) **EP 1 218 370 B1**
(45) Date de publication et mention de la délivrance du brevet: **08.12.2004**
(21) Numéro de dépôt: 00962637.5
(22) Date de dépôt: 14.09.2000
(51) Int. Cl.: C07D 401/06, A61K 31/47, A61P 31/04, C07D 409/14, C07D 417/14, C07D 401/14

(54) **DERIVES DE LA QUINOLYL PROPYL PIPERIDINE ET LEUR UTILISATION EN TANT QUE ANTIBACTERIENS**
CHINOLINPROPYL-PIPERIDINDERIVATE UND DEREN VERWENDUNG ALS ANTIBAKTERIELLE WIRKSTOFFE
QUINOLYL PROPYL PIPERIDINE DERIVATIVES AND THEIR USE AS ANTIBACTERIAL AGENTS

(30) Priorité: 17.09.1999 FR 9911679
(43) Date de publication de la demande: 03.07.2002
(60) Demande divisionnaire: 04019136.3
(73) Titulaire: Aventis Pharma S.A., 92160 Antony (FR)
(72) Inventeur: MALLERON, Jean-Luc, F-91460 Marcoussis (FR); TABART, Michel, F-91290 La Norville (FR); CARRY, Jean-Christophe, F-94100 Saint Maur (FR); EVERS, Michel, F-94510 La Queue En Brie (FR); EL AHMAD, Youssef, F-94000 Creteil (FR); MIGNANI, Serge, F-92290 Chatenay Malabry (FR); VIVIANI, Fabrice, F-95380 Louvres (FR); CHEVE, Michel, F-91450 SOISY SUR SEINE (FR)
(86) Numéro de dépôt international: PCT/FR2000/002541
(87) Numéro de publication internationale: WO 2001/025227

(56) Documents cités:
- WO-A-99/37635

## Description

La présente invention concerne des dérivés de quinolyl propyl pipéridine de formule générale : 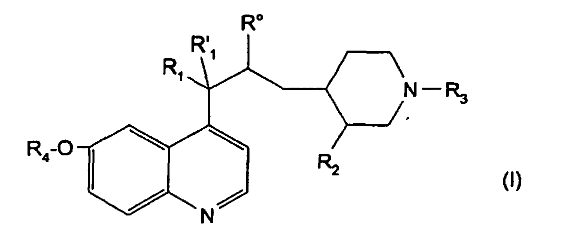 qui sont actifs comme antimicrobiens. L'invention concerne également leur préparation et les compositions les contenant.

Dans la demande de brevet WO 99/37635 ont été décrits des dérivés de quinolyl propyl pipéridine antimicrobiens, de formule générale : 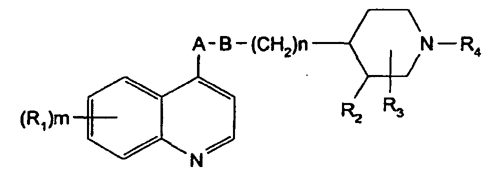 dans laquelle le radical R₁ est notamment alcoxy (C1-6), R₂ est hydrogène, R₃ est en position -2 ou -3 et représente alcoyle (C1-6) pouvant être éventuellement substitué par 1 à 3 substituants choisis parmi thiol, halogène, alcoylthio, trifluorométhyl, alcoyloxycarbonyle, alcoylcarbonyle, alcènyloxycarbonyle, alcènylcarbonyle, hydroxy éventuellement substitué par alcoyle ..., R₄ est un groupe -CH₂-R₅ pour lequel R₅ est selectionné parmi alcoyle hydroxyalcoyle, alcènyle, alcynyle, tétrahydrofuryle, phénylalcoyle éventuellement substitué, phénylalcényle éventuellement substitué, hétéroarylalcoyle éventuellement substitué, hétéroaroyle éventuellement substitué ..., n est 0 à 2, m est 1 ou 2 et A et B sont notamment oxygène, soufre, sulfinyle, sulfonyle, CR₆R₇ pour lequel R₆ et R₇ représentent H, thiol, alcoylthio, halo, trifluorométhyle, alcènyle, alcènylcarbonyle, hydroxy, amino ...

Dans la demande de brevet européen EP30044 ont été décrits des dérivés de quinoléine utiles comme cardiovasculaires, répondant à la formule générale : 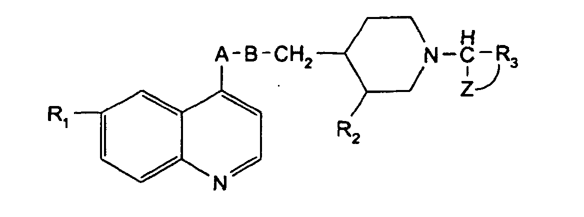 dans laquelle R₁ est notamment alcoyloxy, A-B est -CH₂-CH₂-, -CHOH-CH₂-, -CH₂-CHOH-, -CH₂-CO- ou -CO-CH₂-, R₁ est H, OH ou alcoyloxy, R₂ est éthyle ou vinyle, R₃ est notamment alcoyle, hydroxyalcoyle, cycloalcoyle, hydroxy, alcènyle, alcynyle, tétrahydrofuryle, phénylalcoyle, diphénylalcoyle éventuellement substitué, phénylalcényle éventuellement substitué, benzoyl ou benzoylalcoyle éventuellement substitué, hétéroaryle ou hétéroarylalcoyle éventuellement substitué et Z est H ou alcoyle ou forme avec R₃ un radical cycloalcoyle.

Il a maintenant été trouvé, et c'est ce qui fait l'objet de la présente invention, que les produits de formule générale (I) pour lesquels :
- R₁: est un atome d'hydrogène ou d'halogène, ou un radical hydroxy,
- R'₁: est un atome d'hydrogène, ou peut représenter un atome d'halogène lorsque R₁ est également un atome d'halogène, et
- R°: est un atome d'hydrogène, ou bien
- R₁ et R°: forment ensemble une liaison et
- R'₁: est un atome d'hydrogène,
- R₂: représente un radical carboxy, carboxyméthyle, carboxy-2 éthyle, hydroxyméthyle, alcoyloxycarbonyle, alcoyloxycarbonylméthyle ou alcoyloxycarbonyl-2-éthyle (dont les parties alcoyle contiennent de 1 à 6 C),
- R₃: représente un radical alcoylène (contenant 1 à 6 atomes de carbone) substitué par un atome de soufre portant un substituant phényle pouvant lui-même porter 1 à 4 substituants [choisis parmi halogène, hydroxy, alcoyle (C₁₋₄), alcoyloxy (C₁₋₄), trifluorométhyle, trifluorométhoxy, carboxy, alcoyl (C₁₋₄) oxycarbonyle, cyano et amino], cycloalcoyle de 3 à 7 chaînons, ou hétérocyclyle aromatique de 5 à 6 chaînons, comprenant 1 à 4 hétéroatomes choisis parmi l'azote, l'oxygène et le soufre et étant lui-même éventuellement substitué [par halogène, hydroxy, alcoyle (C₁₋₄), alcoyloxy (C₁₋₄), trifluorométhyle, trifluorométhoxy, oxo, carboxy, alcoyl (C₁₋₄) oxycarbonyle, cyano ou amino],
- et R₄: représente un radical alcoyle (contenant 1 à 6 atomes de carbone), alcényl-CH₂- ou alcynyl-CH₂- dont les parties alcényle ou alcynyle contiennent 2 à 6 atomes de carbone,
étant entendu que les radicaux et portions alcoyle sont en en chaîne droite ou ramifiée,
sous leurs formes diastéréoisomères ou leurs mélanges, ainsi que leurs sels, sont de puissants agents antibactériens.

Il est entendu que les radicaux et portions alcoyle sont en chaîne droite ou ramifiée et contiennent (sauf mention spéciale) 1 à 4 atomes de carbone, et que dans l'alternative où R₁ ou R'₁ repésentent un atome d'halogène ou lorsque R₃ porte un substituant halogène, celui-ci peut être choisi parmi fluor, chlore, brome ou iode, de préférence le fluor.

Dans la formule générale ci-dessus, lorsque R₃ porte un substituant hétérocyclyle aromatique, ce dernier peut être choisi (à titre non limitatif) parmi thiényle, furyle, pyrrolyle, imidazolyle, thiazolyle, oxazolyle, thiadiazolyle, oxadiazolyle, tétrazolyle, pyridyle, pyridazinyle, pyrazinyle, pyrimidinyle. Il est également entendu que dans la définition de R₃ le radical alcoyle substitué ne porte simultanément qu'un seul radical cyclique.

Selon l'invention, les produits de formule générale (I) peuvent être obtenus par condensation de la chaîne R₃ sur le dérivé de quinolyl propyl pipéridine de formule générale : 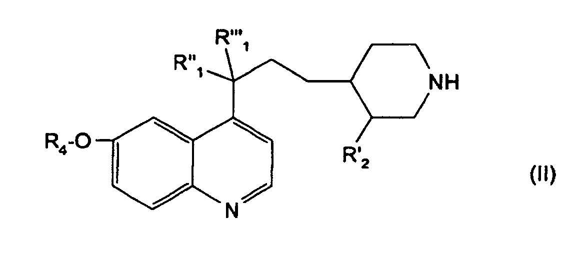 dans laquelle R₄ est défini comme précédemment, R"₁ et R"'₁ représentent des atomes d'hydrogène ou forment ensemble un radical oxo et R'₂ représente un radical carboxy, carboxyméthyle ou carboxy-2 éthyle protégés, ou un radical alcoyloxycarbonyle, alcoyloxycarbonylméthyle ou alcoyloxycarbonyl-2-éthyle, pour obtenir un dérivé de quinolyl propyl pipéridine de formule générale : 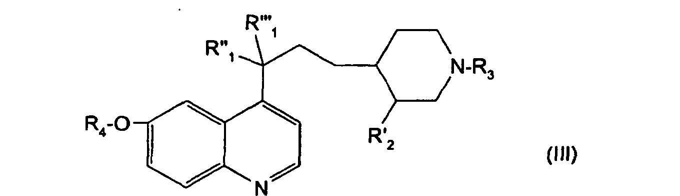 pour lequel R"₁, R"'₁, R'₂ et R₄ sont définis comme ci-dessus et R₃ est défini comme précédemment,
suivie le cas échéant de l'élimination du radical protecteur d'acide,
puis le cas échéant suivie de la réduction du radical oxo représenté par R"₁ et R"'₁ en un alcool pour lequel R₁ représente hydroxy puis éventuellement de l'halogénation si l'on veut obtenir un dérivé de quinolyl propyl pipéridine pour lequel R₁ est un atome d'halogène, et éventuellement de la déhydrohalogénation du dérivé halogéné correspondant, pour obtenir un dérivé de quinolyl propyl pipéridine pour lequel R₁ et R° forment ensemble une liaison, ou bien de la dihalogénation du produit de formule générale (III) pour lequel R"₁ et R"'₁ forment ensemble un radical oxo pour obtenir un dérivé de quinolyl propyl pipéridine pour lequel R₁ et R'₁ sont des atomes d'halogène,
et/ou le cas échéant suivie de la réduction de l'acide protégé sous forme d'un radical R'₂ en position -3 de la pipéridine, en un radical hydroxyméthyle et éventuellement de la transformation en un radical carboxyméthyle ou carboxy-2 éthyle selon les méthodes habituelles,
puis éventuellement suivie de l'élimination du radical protecteur d'acide et éventuellement de la transformation du produit obtenu en un sel.

La condensation de la chaîne R₃ sur la pipéridine s'effectue avantageusement par action d'un dérivé de formule générale :

R₃-X (IV)

dans laquelle R₃ est défini comme précédemment et X représente un atome d'halogène, un radical méthylsulfonyle, un radical trifluorométhylsulfonyle ou p.toluènesulfonyle, en opérant en milieu anhydre, de préférence inerte (azote ou argon par exemple) dans un solvant organique tel qu'un amide (diméthylformamide par exemple), une cétone (acétone par exemple) ou un nitrile (acétonitrile par exemple) en présence d'une base telle qu'une base organique azotée (par exemple triéthylamine) ou une base minérale (carbonate alcalin : carbonate de potassium par exemple) à une température comprise entre 20°C et la température de reflux du solvant.
De préférence, on fait agir un dérivé pour lequel X est un atome de brome ou d'iode.

Il est entendu que, lorsque les radicaux alcoyle représentés par R₃ portent des substituants carboxy ou amino, ces derniers sont préalablement protégés, puis libérés après la réaction. On opère selon les méthodes habituelles qui n'altèrent pas le reste de la molécule, notamment selon les méthodes décrites par T.W. Greene et P.G.M. Wuts, Protective Groups in Organic Synthesis (2^{ème} éd.), A. Wiley - Interscience Publication (1991), ou par Mc Omie, Protective Groups in Organic Chemistry, Plenum Press (1973).

Le radical carboxy protégé représenté par R'₂ peut être choisi parmi les esters facilement hydrolysables. A titre d'exemple peuvent être cités les esters méthylique, benzylique, tertiobutylique, ou bien les esters de phénylpropyle ou de propargyle. Eventuellement la protection du radical carboxy s'effectue simultanément à la réaction. Dans ce cas le produit de formule générale (II) mis en oeuvre porte un radical R'₂ = carboxy.

La réduction du radical oxo en un alcool s'effectue selon les méthodes habituelles qui n'altèrent pas le reste de la molécule, notamment par action d'un agent réducteur comme par exemple un hydrure (borohydrure alcalin : borohydrure de sodium ou de potassium, triacétoxy-borohydrure de sodium, cyanoborohydrure de sodium par exemple, hydrure d'aluminium et de lithium ou hydrure de diisobutyl aluminium) en opérant de préférence dans sous atmosphère inerte, dans un solvant organique comme un alcool (méthanol, éthanol, isopropanol par exemple), ou un éther (par exemple tétrahydrofuranne) ou un solvant chloré (par exemple dichorométhane) à une température comprise entre 20°C et la température de reflux du solvant.

L'halogénation destinée à obtenir un dérivé de quinolyl propyl quinoléine pour lequel R'₁ est un atome d'halogène, à partir du dérivé pour lequel R'₁ est hydroxy, peut être mise en oeuvre en présence d'un trifluorure d'aminosoufre (trifluorure de diéthylamino soufre, trifluorure de bis(2-méthoxyéthyl)amino soufre (Deoxofluor®), trifluorure de morpholino soufre par exemple) ou alternativement en présence de tétrafluorure de soufre, au moyen d'un réactif comme un halogénure de tétra alkylammonium, de tri alkyl benzylammonium où de tri alkyl phénylammonium ou au moyen d'un halogénure de métal alcalin additionné éventuellement d'un éther couronne. La réaction de fluoration peut être également mise en oeuvre par action d'un agent de fluoration comme un fluorure de soufre [par exemple trifluorure de morpholino soufre, tétrafluorure de soufre (J. Org. Chem., 40, 3808 (1975)), trifluorure de diéthylamino soufre (Tetrahedron, 44, 2875 (1988)), trifluorure de bis(2-méthoxyéthyl)amino soufre (Deoxofluor®). Alternativement la réaction de fluoration peut aussi s'effectuer au moyen d'un agent de fluoration comme l'hexafluoropropyl diéthylamine (JP 2 039 546) ou la N-(chloro-2 trifluoro-1,1,2 éthyl) diéthylamine.
Lorsque l'on met en oeuvre un halogénure de tétra alkylammonium, ce dernier peut être choisi, à titre d'exemple, parmi les halogénures de tétra méthylammonium, de tétra éthylammonium, de tétra propylammonium, de tétra butylammonium (tétra n-butylammonium par exemple), de tétra pentylammonium, de tétra cyclohexylammonium, de tri éthyl méthylarnmonium de tri butyl méthylammonium, ou de tri méthyl propylammonium.
On opère dans un solvant organique tel qu'un solvant chloré (par exemple dichlorométhane, dichloréthane, chloroforme) ou dans un éther (tétrahydrofurane, dioxanne par exemple) à une température comprise entre -78 et 40°C (de préférence entre 0 et 30°C). Il est avantageux d'opérer en milieu inerte (argon ou azote notamment).
Il est également possible d'opérer par traitement par un agent d'halogénation comme le chlorure de thionyle ou trichlorure de phosphore dans un solvant organique tel qu'un solvant chloré (dichlorométhane, chloroforme par exemple), à une température comprise entre 0°C et la température de reflux du mélange réactionnel.

La dihalogénation du produit de formule générale (III) pour lequel R"₁ et R"'₁ forment ensemble un radical oxo pour obtenir un dérivé de quinolyl propyl pipéridine pour lequel R₁ et R'₁ sont des atomes d'halogène peut être mise en oeuvre dans des conditions analogues à celles de l'halogénation ci-dessus.

La déhydrohalogénation du dérivé halogéné obtenu à partir du dérivé pour lequel R₁ est hydroxy peut être mise en oeuvre notamment par traitement par le diazabicyclo[5,4,0]undéc-7-ène dans un solvant organique aromatique (toluène par exemple), à une température comprise entre 20°C et la température de reflux du mélange réactionnel.

La réduction de l'acide protégé sous forme d'un radical R'₂ en position -3 de la pipéridine, en un radical hydroxyméthyle s'effectue selon les méthodes habituelles qui n'altèrent pas le reste de la molécule, notamment on opère par action d'un hydrure (hydrure d'aluminium et de lithium ou hydrure de diisobutyl aluminium par exemple) dans un solvant tel qu'un éther (tétrahydrofurane par exemple) à une température comprise entre 20 et 60°C.

La transformation du radical hydroxyméthyle en position -3 de la pipéridine en un radical carboxyméthyle s'effectue selon les méthodes habituelles qui n'altèrent pas le reste de la molécule, notamment elle peut être mise en oeuvre par action d'un agent d'halogénation comme par exemple le chlorure de thionyle ou le trichlorure de phosphore ou le tribromure de phosphore puis d'un cyanure alcalin (cyanure de potassium ou cyanure de sodium par exemple) pour préparer le dérivé cyanométhyle correspondant, suivie de l'hydrolyse du nitrile.
L'halogénation peut être effectuée dans un solvant chloré (dichlorométhane, chloroforme par exemple), à une température comprise entre 0°C et la température de reflux du solvant.
La réaction du cyanure alcalin peut être mise en oeuvre dans un solvant comme le diméthylsulfoxyde, un amide (diméthylformamide par exemple), une cétone (acétone par exemple), un éther comme par exemple le tétrahydrofurane ou un alcool comme par exemple le méthanol ou l'éthanol, à une température comprise entre 20°C et la température de reflux du mélange réactionnel.
L'hydrolyse du nitrile s'effectue selon les méthodes classiques qui n'altèrent pas le reste de la molécule, notamment par action de l'acide chlorhydrique en milieu méthanolique, à une température comprise entre 20 et 70°C, suivi de la saponification de l'ester obtenu (par exemple par l'hydroxyde de sodium dans un mélange de dioxane et d'eau), ou bien directement par action de l'acide sulfurique aqueux à une température comprise entre 50 et 80°C.
La transformation du radical hydroxyméthyle en position -3 de la pipéridine en un radical carboxy-2 éthyle s'effectue par exemple à partir du dérivé halogéné, préparé comme décrit ci-dessus, par condensation du sel de sodium du malonate de diéthyle suivie de l'hydrolyse acide en milieu aqueux du produit obtenu.

L'élimination le cas échéant du radical protecteur d'acide pour obtenir un dérivé de quinolyl propyl pipéridine pour lequel R₂ est un radical carboxy, s'effectue selon les méthodes habituelles, notamment par hydrolyse acide ou saponification de l'ester R'₂. Notamment on fait agir la soude en milieu hydroorganique, par exemple dans un alcool comme le méthanol ou un éther comme le dioxanne, à une température comprise entre 20°C et la température de reflux du mélange réactionnel. On peut également mettre en oeuvre l'hydrolyse en milieu chlorhydrique aqueux à une température comprise entre 20 et 100°C.

Le dérivé de quinolyl propyl pipéridine de formule générale (II) ou l'acide correspondant pour lequel R'₂ représente un radical carboxy, peut être préparé selon ou par analogie avec les méthodes décrites ci-après dans les exemples ou selon ou par analogie avec les méthodes décrites dans la demande de brevet européen EP 30044 ou dans la demande internationale WO 99/37635. Les intermédiaires des dérivés de quinolyl propyl pipéridine pour lesquels R₄ représente alcényl-CH₂O- ou alcynyl-CH₂O- peuvent être obtenus par analogie avec la préparation des intermédiaires pour lesquels R₄ est alcoyloxy, par action du dérivé halogéné correspondant sur le dérivé de quinoléine hydroxylé en position -6.

Le dérivé carboxy-2 éthyle protégé de formule générale (II) peut être obtenu selon ou par analogie avec la méthode décrite dans la demande internationale WO 99/37635 suivie de l'hydrolyse du nitrile et de l'esterification de l'acide ainsi obtenu, ou peut être préparé selon ou par analogie avec les méthodes décrites ci-après dans les exemples.

Il est entendu que les dérivés de formule générale (I), (II), (III), ou leurs intermédiaires de départ peuvent exister sous forme cis ou trans au niveau des substituants en position -3 et -4 de la pipéridine. Les dérivés de configuration trans peuvent être obtenus à partir des dérivés de configuration cis selon ou par analogie avec la méthode décrite dans la demande internationale WO 99/37635.

Les dérivés de quinolyl propyl pipéridine de formule générale (I) peuvent être purifiés le cas échéant par des méthodes physiques telles que la cristallisation ou la chromatographie.

Par ailleurs il est entendu que lorsque R'₁ est un atome d'hydrogène et R₁ est hydroxy ou halogène, il existe des formes diastéréoisomères et que les formes diastéréoisomères et leurs mélanges entrent aussi dans le cadre de la présente invention. Ces derniers peuvent être notamment séparés par chromatographie sur silice ou par Chromatographie Liquide Haute Performance (CLHP).

Les dérivés de quinolyl propyl pipéridine de formule générale (I) peuvent être transformés en sels d'addition avec les acides, par les méthodes connues. Il est entendu que ces sels entrent aussi dans le cadre de la présente invention.

Comme exemples de sels d'addition avec des acides pharmaceutiquement acceptables, peuvent être cités les sels formés avec les acides minéraux (chlorhydrates, bromhydrates, sulfates, nitrates, phosphates) ou avec les acides organiques (succinates, fumarates, tartrates, acétates, propionates, maléates, citrates, méthanesulfonates, éthanesulfonates, phénylsulfonates, p.toluènesulfonates, iséthionates, naphtylsulfonates ou camphorsulfonates, ou avec des dérivés de substitution de ces composés).

Certains des dérivés de quinolyl propyl pipéridine de formule générale (I) portant un radical carboxy peuvent être transformés à l'état de sels métalliques ou en sels d'addition avec les bases azotées selon les méthodes connues en soi. Ces sels entrent également dans le cadre de la présente invention. Les sels peuvent être obtenus par action d'une base métallique (par exemple alcaline ou alcalino terreuse), de l'ammoniac ou d'une amine, sur un produit selon l'invention, dans un solvant approprié tel qu'un alcool, un éther ou l'eau, ou par réaction d'échange avec un sel d'un acide organique. Le sel formé précipite après concentration éventuelle de la solution, il est séparé par filtration, décantation ou lyophilisation. Comme exemples de sels pharmaceutiquement acceptables peuvent être cités les sels avec les métaux alcalins (sodium, potassium, lithium) ou avec les métaux alcalinoterreux (magnésium, calcium), le sel d'ammonium, les sels de bases azotées (éthanolamine, diéthanolamine, triméthylamine, triéthylamine, méthylamine, propylamine, diisopropylamine, NN-diméthyléthanolamine, benzylamine, dicyclohexylamine, N-benzyl-β-phénéthylamine, NN'-dibenzyléthylènediamine, diphénylènediamine, benzhydrylamine, quinine, choline, arginine, lysine, leucine, dibenzylamine).

Les dérivés de quinolyl propyl pipéridine selon l'invention sont des agents antibactériens particulièrement intéressants.

In vitro, sur germes gram positifs les dérivés de quinolyl propyl pipéridine selon l'invention se sont montrés actifs à des concentrations comprises entre 0,015 et 4 µg/ml sur *Staphylococcus a ureus* A S5155 résistante à la méticilline, ainsi que la majorité d'entre eux à des concentrations comprises entre 0,06 et 8 µg/ml sur *Streptococcus pneumoniae* IP53146 et à des concentrations comprises entre 0,12 et 64 µg/ml sur *Enterococcus faecium* ATCC 19434 ou H983401 et sur germes gram négatifs, ils se sont montrés actifs à des concentrations comprises entre 0,12 et 32 µg/ml sur *Moraxella catharrhalis* IPA152 ; in vivo, ils se sont montrés actifs sur les infections expérimentales de la souris à *Staphylococcus aureus* IP8203 à des doses comprises entre 10 et 150 mg/kg par voie sous cutanée (DC₅₀) et pour certains d'entre eux à des doses comprises entre 20 et 150 mg/kg par voie orale.

Enfin, les produits selon l'invention sont particulièrement intéressants du fait de leur faible toxicité. Aucun des produits n'a manifesté de toxicité à la dose de 100 mg/kg par voie sous cutanée chez la souris (2 administrations).

Parmi les produits selon l'invention, plus particulièrement intéressants sont les dérivés de quinolyl propyl quinoléine de formule générale (I) pour lesquels :
- R₁: est un atome d'hydrogène ou d'halogène, ou un radical hydroxy,
- R'₁: est un atome d'hydrogène, et
- R°: est un atome d'hydrogène, ou bien
- R₁ et R°: forment ensemble une liaison et
- R'₁: est un atome d'hydrogène,
- R₂: représente un radical carboxy ou carboxyméthyle, et
- R₃: représente un radical alcoylène (1 à 6 atomes de carbone) substitué par un atome de soufre portant un substituant phényle pouvant lui-même porter 1 à 4 atomes d'halogène, cycloalcoyle dont la partie cyclique contient 3 à 7 chaînons, ou hétérocyclyle aromatique de 5 à 6 chaînons comprenant 1 à 4 hétéroatomes choisis parmi l'azote, l'oxygène et le soufre et éventuellement lui-même substitué par halogène ou bien
lorsque R₂ représente un radical hydroxyméthyle, alcoyloxycarbonyle ou alcoyloxycarbonylméthyle (dont les portions alcoyle contiennent 1 à 6 atomes de carbone) alors
- R₃: représente un radical alcoylène (1 à 6 atomes de carbone) substitué par un atome de soufre portant un substituant hétérocyclyle aromatique de 5 à 6 chaînons comprenant 1 à 4 hétéroatomes choisis parmi l'azote, l'oxygène ou le soufre et éventuellement lui-même substitué par halogène
- et R₄: représente un radical alcoyle (contenant 1 à 6 atomes de carbone),
les radicaux et portions alcoyle étant en en chaîne droite ou ramifiée, ainsi que les formes diastéréoisomères ou leurs mélanges, ainsi que leurs sels,
et parmi ces produits, plus spécialement préférés sont les produits suivants:
- acide (3R,4R)-4-[3-hydroxy-3-(6-méthoxyquinolin-4-yl)propyl]-1-[2-(2-thiénylthio)éthyl]pipéridine-3-acétique,
- acide (3R,4R)-4-[3-(6-méthoxyquinolin-4-yl)propyl]-1-[2-(2-thiénylthio)éthyl] pipéridine-3-acétique ;
- acide (3R,4R)-4-[3-fluoro-3-(6-méthoxyquinolin-4-yl)propyl]-1-[2-(2-thiénylsulfanyl)éthyl]pipéridine-3-acétique ;
- acide (3R,4R)-1-[2-(3-fluorophénylthio)éthyl]-4-[3-hydroxy-3-(6-méthoxyquinolin-4-yl)propyl]pipéridine-3-acétique ;

Les produits cités dans les exemples sont particulièrement préférés ; les dérivés de quinolyl propyl pipéridine ci-après sont également des produits intéressants :
Acide (3R,4R)-4-[3-(6-méthoxyquinolin-4-yl)propyl]-1-[3-phénylthio-propyl]pipéridine-3-carboxylique
Acide (3R,4R)-4-[3-(6-méthoxyquinolin-4-yl)propyl]-1-[3-(2-fluorophénylthio)propyl] pipéridine-3-carboxylique
Acide (3R,4R)-4-[3-(6-méthoxyquinolin-4-yl)propyl]-1-[3-(3-fluorophénylthio)propyl] pipéridine-3-carboxylique
Acide (3R,4R)-4-[3-(6-méthoxyquinolin-4-yl)propyl]-1-[2-(4-fluorophénylthio)éthyl] pipéridine-3-carboxylique
Acide (3R,4R)-4-[3-(6-méthoxyquinolin-4-yl)propyl]-1-[3-(4-fluorophénylthio)propyl] pipéridine-3-carboxylique
Acide (3R,4R)-4-[3-(6-méthoxyquinolin-4-yl)propyl]-1-[2-(2,3-difluorophénylthio)éthyl] pipéridine-3-carboxylique
Acide (3R,4R)-4-[3-(6-méthoxyquinolin-4-yl)propyl]-1-[3-(2,3-difluorophénylthio)propyl] pipéridine-3-carboxylique
Acide (3R,4R)-4-[3-(6-méthoxyquinolin-4-yl)propyl]-1-[2-(2,6-difluorophénylthio)éthyl] pipéridine-3-carboxylique
Acide (3R,4R)-4-[3-(6-méthoxyquinolin-4-yl)propyl]-1-[3-(2,6-difluorophénylthio)propyl] pipéridine-3-carboxylique
Acide (3R,4R)-4-[3-(6-méthoxyquinolin-4-yl)propyl]-1-[2-(2-chlorophénylthio)éthyl] pipéridine-3-carboxylique
Acide (3R,4R)-4-[3-(6-méthoxyquinolin-4-yl)propyl]-1-[3-(2-chlorophénylthio)propyl] pipéridine-3-carboxylique
Acide (3R,4R)-4-[3-(6-méthoxyquinolin-4-yl)propyl]-1-[2-(3-chlorophénylthio)éthyl] pipéridine-3-carboxylique
Acide (3R,4R)-4-[3-(6-méthoxyquinolin-4-yl)propyl]-1-[3-(3-chlorophénylthio)propyl] pipéridine-3-carboxylique
Acide (3R,4R)-4-[3-(6-méthoxyquinolin-4-yl)propyl]-1-[2-(4-chlorophénylthio)éthyl] pipéridine-3-carboxylique
Acide (3R,4R)-4-[3-(6-méthoxyquinolin-4-yl)propyl]-1-[3-(4-chlorophénylthio)propyl] pipéridine-3-carboxylique
Acide (3R,4R)-4-[3-(6-méthoxyquinolin-4-yl)propyl]-1-[2-(2,3-dichlorophénylthio)éthyl] pipéridine-3-carboxylique
Acide (3R,4R)-4-[3-(6-méthoxyquinolin-4-yl)propyl]-1-[3-(2,3-dichlorophénylthio)propyl] pipéridine-3-carboxylique
Acide (3R,4R)-4-[3-(5-méthoxyquinolin-4-yl)propyl]-1-[2-(2,6-dichlorophénylthio)éthyl] pipéridine-3-carboxylique
Acide (3R,4R)-4-[3-(6-méthoxyquinolin-4-yl)propyl]-1-[3-(2,6-dichlorophénylthio)propyl] pipéridine-3-carboxylique
Acide (3R,4R)-4-[3-(6-méthoxyquinolin-4-yl)propyl]-1-[2-(2-méthylphénylthio)éthyl] pipéridine-3-carboxylique
Acide (3R,4R)-4-[3-(6-méthoxyquinolin-4-yl)propyl]-1-[3-(2-méthylphénylthio)propyl] pipéridine-3-carboxylique
Acide (3R,4R)-4-[3-(6-méthoxyquinolin-4-yl)propyl]-1-[2-(3-méthylphénylthio)éthyl] pipéridine-3-carboxylique
Acide (3R,4R)-4-[3-(6-méthoxyquinolin-4-yl)propyl]-1-[3-(3-méthylphénylthio)propyl] pipéridine-3-carboxylique
Acide (3R,4R)-4-[3-(6-méthoxyquinolin-4-yl)propyl]-1-[2-(4-méthylphénylthio)éthyl] pipéridine-3-carboxylique
Acide (3R,4R)-4-[3-(6-méthoxyquinolin-4-yl)propyl]-1-[3-(4-méthylphénylthio)propyl] pipéridine-3-carboxylique
Acide (3R,4R)-4-[3-(6-méthoxyquinolin-4-yl)propyl]-1-[2-(2-trifluorométhylphénylthio)éthyl] pipéridine-3-carboxylique
Acide (3R,4R)-4-[3-(6-méthoxyquinolin-4-yl)propyl]-1-[3-(2-triflurométhylphénylthio) propyl]pipéridine-3-carboxylique
Acide (3R,4R)-4-[3-(6-méthoxyquinolin-4-yl)propyl]-1-[2-(3-trifluorométhylphénylthio) éthyl]pipéridine-3-carboxylique
Acide (3R,4R)-4-[3-(6-méthoxyquinolin-4-yl)propyl]-1-[3-(3-triflurométhylphénylthio) propyl]pipéridine-3-carboxylique
Acide (3R,4R)-4-[3-(6-méthoxyquinolin-4-yl)propyl]-1-[2-(4-trifluorométhylphénylthio) éthyl]pipéridine-3-carboxylique
Acide (3R,4R)-4-[3-(6-méthoxyquinolin-4-yl)propyl]-1-[3-(4-triflurométhylphénylthio) propyl]pipéridine-3-carboxylique
Acide (3R,4R)-4-[3-(6-méthoxyquinolin-4-yl)propyl]-1-[2-(2-méthoxyphénylthio)éthyl] pipéridine-3-carboxylique
Acide (3R,4R)-4-[3-(6-méthoxyquinolin-4-yl)propyl]-1-[3-(2-méthoxyphénylthio)propyl] pipéridine-3-carboxylique
Acide (3R,4R)-4-[3-(6-méthoxyquinolin-4-yl)propyl]-1-[2-(3-méthoxyphénylthio)éthyl] pipéridine-3-carboxylique
Acide (3R,4R)-4-[3-(6-méthoxyquinolin-4-yl)propyl]-1-[3-(3-méthoxyphénylthio)propyl] pipéridine-3-carboxylique
Acide (3R,4R)-4-[3-(6-méthoxyquinolin-4-yl)propyl]-1-[2-(4-méthoxyphénylthio)éthyl] pipéridine-3-carboxylique
Acide (3R,4R)-4-[3-(6-méthoxyquinolin-4-yl)propyl]-1-[3-(4-méthoxyphénylthio)propyl] pipéridine-3-carboxylique
Acide (3R,4R)-4-[3-(6-méthoxyquinolin-4-yl)propyl]-1-[2-(cyclopropylthio)éthyl]pipéridine-3-carboxylique
Acide (3R,4R)-4-[3-(6-méthoxyquinolin-4-yl)propyl]-1-[3-(cyclopropylthio)propyl] pipéridine-3-carboxylique
Acide (3R,4R)-4-[3-(6-méthoxyquinolin-4-yl)propyl]-1-[2-(cyclobutylthio)éthyl]pipéridine-3-carboxylique
Acide (3R,4R)-4-[3-(6-méthoxyquinolin-4-yl)propyl]-1-[3-(cyclobutylthiopropyl]pipéridine-3-carboxylique
Acide (3R,4R)-4-[3-(6-méthoxyquinolin-4-yl)propyl]-1-[2-(cyclopentylthio)éthyl]pipéridine-3-carboxylique
Acide (3R,4R)-4-[3-(6-méthoxyquinolin-4-yl)propyl]-1-[3-(cyclopentylthio)propyl]pipéridine-3-carboxylique
Acide (3R,4R)-4-[3-(6-méthoxyquinolin-4-yl)propyl]-1-[3-(cyclohexylthio)propyl]pipéridine-3-carboxylique
Acide (3R,4R)-4-[3-(6-méthoxyquinolin-4-yl)propyl]-1-[3-(thien-2-yl)thiopropyl]pipéridine-3-carboxylique
Acide (3R,4R)-4-[3-(6-méthoxyquinolin-4-yl)propyl]-1-[2-(5-chloro-thien-2-yl)thioéthyl] pipéridine-3-carboxylique
Acide (3R,4R)-4-[3-(6-méthoxyquinolin-4-yl)propyl]-1-[3-(5-chloro-thien-2-yl)thiopropyl] pipéridine-3-carboxylique
Acide (3R,4R)-4-[3-(6-méthoxyquinolin-4-yl)propyl]-1-[2-(3-chloro-thien-2-yl)thioéthyl] pipéridine-3-carboxylique
Acide (3R,4R)-4-[3-(6-méthoxyquinolin-4-yl)propyl]-1-[3-(3-chloro-thien-2-yl)thiopropyl] pipéridine-3-carboxylique
Acide (3R,4R)-4-[3-(6-méthoxyquinolin-4-yl)propyl]-1-[2-(5-méthyl-thien-2-yl)thioéthyl] pipéridine-3-carboxylique
Acide (3R,4R)-4-[3-(6-méthoxyquinolin-4-yl)propyl]-1-[3-(5-méthyl -thien-2-yl)thiopropyl] pipéridine-3-carboxylique
Acide (3R,4R)-4-[3-(6-méthoxyquinolin-4-yl)propyl]-1-[2-(3-méthyl-thien-2-yl)thioéthyl] pipéridine-3-carboxylique
Acide (3R,4R)-4-[3-(6-méthoxyquinolin-4-yl)propyl]-1-[3-(3-méthyl-thien-2-yl)thiopropyl] pipétidine-3-carboxylique
Acide (3R,4R)-4-[3-(6-méthoxyquinolin-4-yl)propyl]-1-[2-(thien-3-yl)thioéthyl]pipéridine-3-carboxylique
Acide (3R,4R)-4-[3-(6-méthoxyquinolin-4-yl)propyl]-1-[3-(thien-3-yl)thiopropyl]pipéridine-3-carboxylique
Acide (3R,4R)-4-[3-(6-méthoxyquinolin-4-yl)propyl]-1-[2-(fur-2-yl)thioéthyl]pipéridine-3-carboxylique
Acide (3R,4R)-4-[3-(6-méthoxyquinolin-4-yl)propyl]-1-[3-(fur-2-yl)thiopropyl]pipéridine-3-carboxylique
Acide (3R,4R)-4-[3-(6-méthoxyquinolin-4-yl)propyl]-1-[2-(fur-3-yl)thioéthyl]pipéridine-3-carboxylique
Acide (3R,4R)-4-[3-(6-méthoxyquinolin-4-yl)propyl]-1-[3-(fur-3-yl)thiopropyl]pipéridine-3-carboxylique
Acide (3R,4R)-4-[3-(6-méthoxyquinolin-4-yl)propyl]-1-[2-(1-méthyl-pyrrol-2-yl)thioéthyl] pipéridine-3-carboxylique
Acide (3R,4R)-4-[3-(6-méthoxyquinolin-4-yl)propyl]-1-[3-(1-méthyl-pyrrol-2-yl)thiopropyl] pipéridine-3-carboxylique
Acide (3R,4R)-4-[3-(6-méthoxyquinolin-4-yl)propyl]-1-[2-(1-méthyl-pyrrol-3-yl)thioéthyl] pipéridine-3-carboxylique
Acide (3R,4R)-4-[3-(6-méthoxyquinolin-4-yl)propyl]-1-[3-(1-méthyl-pyrrol-3-yl)thiopropyl] pipéridine-3-carboxylique
Acide (3R,4R)-4-[3-(6-méthoxyquinolin-4-yl)propyl]-1-[3-(1,3-thiazol-2-yl)thiopropyl] pipéridine-3-carboxylique
Acide (3R,4R)-4-[3-(6-méthoxyquinolin-4-yl)propyl]-1-[2-(1-méthyl-imidazol-2-yl)thioéthyl] pipéridine-3-carboxylique
Acide (3R,4R)-4-[3-(6-méthoxyquinolin-4-yl)propyl]-1-[3-(1-méthyl-imidazol-2-yl) thiopropyl]pipéridine-3-carboxylique
Acide (3R,4R)-4-[3-(6-méthoxyquinolin-4-yl)propyl]-1-[2-(3-méthyl-imidazol-4-yl)thioéthyl] pipéridine-3-carboxylique
Acide (3R,4R)-4-[3-(6-méthoxyquinolin-4-yl)propyl]-1-[3-(3-méthyl-imidazol-4-yl) thiopropyl]pipéridine-3-carboxylique
Acide (3R,4R)-4-[3-(6-méthoxyquinolin-4-yl)propyl]-1-[2-(3-méthyl-pyrazol-4-yl)thioéthyl] pipéridine-3-carboxylique
Acide (3R,4R)-4-[3-(6-méthoxyquinolin-4-yl)propyl]-1-[3-(3-méthyl-pyrazol-4-yl)thiopropyl] pipéridine-3-carboxylique
Acide (3R,4R)-4-[3-(6-méthoxyquinolin-4-yl)propyl]-1-[2-(oxazol-2-yl)thioéthyl]pipéridine-3-carboxylique
Acide (3R,4R)-4-[3-(6-méthoxyquinolin-4-yl)propyl]-1-[3-(oxazol-2-yl)thiopropyl]pipéridine-3-carboxylique
Acide (3R,4R)-4-[3-(6-méthoxyquinolin-4-yl)propyl]-1-[3-(pyridin-2-yl)thiopropyl] pipéridine-3-carboxylique
Acide (3R,4R)-4-[3-(6-méthoxyquinolin-4-yl)propyl]-1-[2-(pyridin-3-yl)thioéthyl]pipéridine-3-carboxylique
Acide (3R,4R)-4-[3-(6-méthoxyquinolin-4-yl)propyl]-1-[3-(pyridin-3-yl)thiopropyl] pipéridine-3-carboxylique
Acide (3R,4R)-4-[3-(6-méthoxyquinolin-4-yl)propyl]-1-[2-(pyridin-4-yl)thioéthyl]pipéridine-3-carboxylique
Acide (3R,4R)-4-[3-(6-méthoxyquinolin-4-yl)propyl]-1-[3-(pyridin-4-yl)thiopropyl] pipéridine-3-carboxylique
Acide (3R,4R)-4-[3-(6-méthoxyquinolin-4-yl)propyl]-1-[2-(pyrimidin-2-yl)thioéthyl] pipéridine-3-carboxylique
Acide (3R,4R)-4-[3-(6-méthoxyquinolin-4-yl)propyl]-1-[3-(pyrimidin-2-yl)thiopropyl] pipéridine-3-carboxylique
Acide (3R,4R)-4-[3-(6-méthoxyquinolin-4-yl)propyl]-1-[2-(pyrimidin-4-yl)thioéthyl] pipéridine-3-carboxylique
Acide (3R,4R)-4-[3-(6-méthoxyquinolin-4-yl)propyl]-1-[3-(pyrimidin-4-yl)thiopropyl] pipéridine-3-carboxylique
Acide (3R,4R)-4-[3-(6-méthoxyquinolin-4-yl)propyl]-1-[2-(pyrimidin-5-yl)thioéthyl] pipéridine-3-carboxylique
Acide (3R,4R)-4-[3-(6-méthoxyquinolin-4-yl)propyl]-1-[3-(pyrimidin-5-yl)thiopropyl] pipéridine-3-carboxylique
Acide (3R,4R)-4-[3-(6-méthoxyquinolin-4-yl)propyl]-1-[2-(pyrazin-2-yl)thioéthyl]pipéridine-3-carboxylique
Acide (3R,4R)-4-[3-(6-méthoxyquinolin-4-yl)propyl]-1-[3-(pyrazin-2-yl)thiopropyl] pipéridine-3-carboxylique
Acide (3R,4R)-4-[3-(6-méthoxyquinolin-4-yl)propyl]-1-[2-(pyridazin-3-yl)thioéthyl] pipéridine-3-carboxylique
Acide (3R,4R)-4-[3-(6-méthoxyquinolin-4-yl)propyl]-1-[3-(pyridazin-3-yl)thiopropyl] pipéridine-3-carboxylique
Acide (3R,4R)-4-[3-(6-méthoxyquinolin-4-yl)propyl]-1-[2-(pyridazin-4-yl)thioéthyl] pipéridine-3-carboxylique
Acide (3R,4R)-4-[3-(6-méthoxyquinolin-4-yl)propyl]-1-[3-(pyridazin-4-yl)thiopropyl] pipéridine-3-carboxylique
Acide (3R,4R)-4-[3-(R,S)-hydroxy-3-(6-méthoxyquinolin-4-yl)propyl]-1-[3-phénylthioéthyl] pipéridine-3-carboxylique
Acide (3R,4R)-4-[3-(R,S)-hydroxy-3-(6-méthoxyquinolin-4-yl)propyl]-1-[3-phénylthiopropyl]pipéridine-3-carboxylique
Acide (3R,4R)-4-[3-(R,S)-hydroxy-3-(6-méthoxyquinolin-4-yl)propyl]-1-[3-(2-fluorophénylthio)propyl]pipéridine-3-carboxylique
Acide (3R,4R)-4-[3-(R,S)-hydroxy-3-(6-méthoxyquinolin-4-yl)propyl]-1-[3-(3-fluorophénylthio)propyl]pipéridine-3-carboxylique
Acide (3R,4R)-4-[3-(R,S)-hydroxy-3-(6-méthoxyquinolin-4-yl)propyl]-1-[2-(2-fluorophénylthio)éthyl]pipéridine-3-carboxylique
Acide (3R,4R)-4-[3-(R,S)-hydroxy-3-(6-méthoxyquinolin-4-yl)propyl]-1-[2-(4-fluorophénylthio)éthyl]pipéridine-3-carboxylique
Acide (3R,4R)-4-[3-(R,S)-hydroxy-3-(6-méthoxyquinolin-4-yl)propyl]-1-[3-(4-fluorophénylthio)propyl]pipéridine-3-carboxylique
Acide (3R,4R)-4-[3-(R,S)-hydroxy-3-(6-méthoxyquinolin-4-yl)propyl]-1-[2-(2,3-difluorophénylthio)éthyl]pipéridine-3-carboxylique
Acide (3R,4R)-4-[3-(R,S)-hydroxy-3-(6-méthoxyquinolin-4-yl)propyl]-1-[3-(2,3-difluorophénylthio)propyl]pipéridine-3-carboxylique
Acide (3R,4R)-4-[3-(R,S)-hydroxy-3-(6-méthoxyquinolin-4-yl)propyl]-1-[2-(2,6-difluorophénylthio)éthyl]pipéridine-3-carboxylique
Acide (3R,4R)-4-[3-(R,S)-hydroxy-3-(6-méthoxyquinolin-4-yl)propyl]-1-[3-(2,6-difluorophénylthio)propyl]pipéridine-3-carboxylique
Acide (3R,4R)-4-[3-(R,S)-hydroxy-3-(6-méthoxyquinolin-4-yl)propyl]-1-[2-(2-chlorophénylthio)éthyl]pipéridine-3-carboxylique
Acide (3R,4R)-4-[3-(R,S)-hydroxy-3-(6-méthoxyquinolin-4-yl)propyl]-1-[3-(2-chlorophénylthio)propyl]pipéridine-3-carboxylique
Acide (3R,4R)-4-[3-(R,S)-hydroxy-3-(6-méthoxyquinolin-4-yl)propyl]-1-[2-(3-chlorophénylthio)éthyl]pipéridine-3-carboxylique
Acide (3R,4R)-4-[3-(R,S)-hydroxy-3-(6-méthoxyquinolin-4-yl)propyl]-1-[3-(3-chlorophénylthio)propyl]pipéridine-3-carboxylique
Acide (3R,4R)-4-[3-(R,S)-hydroxy-3-(6-méthoxyquinolin-4-yl)propyl]-1-[2-(4-chlorophénylthio)éthyl]pipéridine-3-carboxylique
Acide (3R,4R)-4-[3-(R,S)-hydroxy-3-(6-méthoxyquinolin-4-yl)propyl]-1-[3-(4-chlorophénylthio)propyl]pipéridine-3-carboxylique
Acide (3R,4R)-4-[3-(R,S)-hydroxy-3-(6-méthoxyquinolin-4-yl)propyl]-1-[2-(2,3-dichlorophénylthio)éthyl]pipéridine-3-carboxylique
Acide (3R,4R)-4-[3-(R,S)-hydroxy-3-(6-méthoxyquinolin-4-yl)propyl]-1-[3-(2,3-dichlorophénylthio)propyl]pipéridine-3-carboxylique
Acide (3R,4R)-4-[3-(R,S)-hydroxy-3-(6-méthoxyquinolin-4-yl)propyl]-1-[2-(2,6-dichlorophénylthio)éthyl]pipéridine-3-carboxylique
Acide (3R,4R)-4-[3-(R,S)-hydroxy-3-(6-méthoxyquinolin-4-yl)propyl]-1-[3-(2,6-dichlorophénylthio)propyl]pipéridine-3-carboxylique
Acide (3R,4R)-4-[3-(R,S)-hydroxy-3-(6-méthoxyquinolin-4-yl)pnopyl]-1-[2-(2-méthylphénylthio)éthyl]pipéridine-3-carboxylique
Acide (3R,4R)-4-[3-(R,S)-hydroxy-3-(6-méthoxyquinolin-4-yl)propyl]-1-[3-(2-méthylphénylthio)propyl]pipéridine-3-carboxylique
Acide (3R,4R)-4-[3-(R,S)-hydroxy-3-(6-méthoxyquinolin-4-yl)propyl]-1-[2-(3-méthylphénylthio)éthyl]pipéridine-3-carboxylique
Acide (3R,4R)-4-[3-(R,S)-hydroxy-3-(6-méthoxyquinolin-4-yl)propyl]-1-[3-(3-méthylphénylthio)propyl]pipéridine-3-carboxylique
Acide (3R,4R)-4-[3-(R,S)-hydroxy-3-(6-méthoxyquinolin-4-yl)propyl]-1-[2-(4-méthylphénylthio)éthyl]pipéridine-3-carboxylique
Acide (3R,4R)-4-[3-(R,S)-hydroxy-3-(6-méthoxyquinolin-4-yl)propyl]-1-[3-(4-méthylphénylthio)propyl]pipéridine-3-carboxylique
Acide (3R,4R)-4-[3-(R,S)-hydroxy-3-(6-méthoxyquinolin-4-yl)propyl]-1-[2-(2-trifluorométhyphénylthio)éthyl]pipéridine-3-carboxylique
Acide (3R,4R)-4-[3-(R,S)-hydroxy-3-(6-méthoxyquinolin-4-yl)propyl]-1-[3-(2-triflurométhylphénylthio)propyl]pipéridine-3-carboxylique
Acide (3R,4R)-4-[3-(R,S)-hydroxy-3-(6-méthoxyquinolin-4-yl)propyl]-1-[2-(3-trifluorométhylphénylthio)éthyl]pipéridine-3-carboxylique
Acide (3R,4R)-4-[3-(R,S)-hydroxy-3-(6-méthoxyquinolin-4-yl)propyl]-1-[3-(3-triflurométhylphénylthio)propyl]pipéridine-3-carboxylique
Acide (3R,4R)-4-[3-(R,S)-hydroxy-3-(6-méthoxyquinolin-4-yl)propyl]-1-[2-(4-trifluorométhylphénylthio)éthyl]pipéridine-3-carboxylique
Acide (3R,4R)-4-[3-(R,S)-hydroxy-3-(6-méthoxyquinolin-4-yl)propyl]-1-[3-(4-triflurométhylphénylthio)propyl]pipéridine-3-carboxylique
Acide (3R,4R)-4-[3-(R,S)-hydroxy-3-(6-méthoxyquinolin-4-yl)propyl]-1-[2-(2-méthoxyphénylthio)éthyl]pipéridine-3-carboxylique
Acide (3R,4R)-4-[3-(R,S)-hydroxy-3-(6-méthoxyquinolin-4-yl)propyl]-1-[3-(2-méthoxyphénylthio)propyl]pipéridine-3-carboxylique
Acide (3R,4R)-4-[3-(R,S)-hydroxy-3-(6-méthoxyquinolin-4-yl)propyl]-1-[2-(3-méthoxyphénylthio)éthyl]pipéridine-3-carboxylique
Acide (3R,4R)-4-[3-(R,S)-hydroxy-3-(6-méthoxyquinolin-4-yl)propyl]-1-[3-(3-méthoxyphénylthio)propyl]pipéridine-3-carboxylique
Acide (3R,4R)-4-[3-(R,S)-hydroxy-3-(6-méthoxyquinolin-4-yl)propyl]-1-[2-(4-méthoxyphénylthio)éthyl]pipéridine-3-carboxylique
Acide (3R,4R)-4-[3-(R,S)-hydroxy-3-(6-méthoxyquinolin-4-yl)propyl]-1-[3-(4-méthoxyphénylthio)propyl]pipéridine-3-carboxylique
Acide (3R,4R)-4-[3-(R,S)-hydroxy-3-(6-méthoxyquinolin-4-yl)propyl]-1-[2-(cyclopropylthio) éthyl]pipéridine-3-carboxylique
Acide (3R,4R)-4-[3-(R,S)-hydroxy-3-(6-méthoxyquinolin-4-yl)propyl]-1-[3-(cyclopropylthio)propyl]pipéridine-3-carboxylique
Acide (3R,4R)-4-[3-(R,S)-hydroxy-3-(6-méthoxyquinolin-4-yl)propyl]-1-[2-(cyclobutylthio) éthyl]pipéridine-3-carboxylique
Acide (3R,4R)-4-[3-(R,S)-hydroxy-3-(6-méthoxyquinolin-4-yl)propyl]-1-[3-(cyclobutylthiopropyl]pipéridine-3-carboxylique
Acide (3R,4R)-4-[3-(R,S)-hydroxy-3-(6-méthoxyquinolin-4-yl)propyl]-1-[2-(cyclopentylthio)éthyl]pipéridine-3-carboxylique
Acide (3R,4R)-4-[3-(R,S)-hydroxy-3-(6-méthoxyquinolin-4-yl)propyl]-1-[3-(cyclopentylthio)propyl]pipéridine-3-carboxylique
Acide (3R,4R)-4-[3-(R,S)-hydroxy-3-(6-méthoxyquinolin-4-yl)propyl]-1-[2-(cyclohexylthio) éthyl]pipéridine-3-carboxylique
Acide (3R,4R)-4-[3-(R,S)-hydroxy-3-(6-méthoxyquinolin-4-yl)propyl]-1-[3-(cyclohexylthio) propyl]pipéridine-3-carboxylique
Acide (3R,4R)-4-[3-(R,S)-hydroxy-3-(6-méthoxyquinolin-4-yl)propyl]-1-[3-(thien-2-yl) thiopropyl]pipéridine-3-carboxylique
Acide (3R,4R)-4-[3-(R,S)-hydroxy-3-(6-méthoxyquinolin-4-yl)propyl]-1-[2-(5-chloro-thien-2-yl)thioéthyl]pipéridine-3-carboxylique
Acide (3R,4R)-4-[3-(R,S)-hydroxy-3-(6-méthoxyquinolin-4-yl)propyl]-1-[3-(5-chloro-thien-2-yl)thiopropyl]pipéridine-3-carboxylique
Acide (3R,4R)-4-[3-(R,S)-hydroxy-3-(6-méthoxyquinolin-4-yl)propyl]-1-[2-(3-chloro-thien-2-yl)thioéthyl]pipéridine-3-carboxylique
Acide (3R,4R)-4-[3-(R,S)-hydroxy-3-(6-méthoxyquinolin-4-yl)propyl]-1-[3-(3-chloro-thien-2-yl)thiopropyl]pipéridine-3-carboxylique
Acide (3R,4R)-4-[3-(R,S)-hydroxy-3-(6-méthoxyquinolin-4-yl)propyl]-1-[2-(5-méthylthien-2-yl)thioéthyl]pipéridine-3-carboxylique
Acide (3R,4R)-4-[3-(R,S)-hydroxy-3-(6-méthoxyquinolin-4-yl)propyl]-1-[3-(5-méthylthien-2-yl)thiopropyl]pipéridine-3-carboxylique
Acide (3R,4R)-4-[3-(R,S)-hydroxy-3-(6-méthoxyquinolin-4-yl)propyl]-1-[2-(3-méthylthien-2-yl)thioéthyl]pipéridine-3-carboxylique
Acide (3R,4R)-4-[3-(R,S)-hydroxy-3-(6-méthoxyquinolin-4-yl)propyl]-1-[3-(3-méthylthien-2-yl)thiopropyl]pipéridine-3-carboxylique
Acide (3R,4R)-4-[3-(R,S)-hydroxy-3-(6-méthoxyquinolin-4-yl)propyl]-1-[2-(thien-3-yl)thioéthyl]pipéridine-3-carboxylique
Acide (3R,4R)-4-[3-(R,S)-hydroxy-3-(6-méthoxyquinolin-4-yl)propyl]-1-[3-(thien-3-yl)thiopropyl]pipéridine-3-carboxylique
Acide (3R,4R)-4-[3-(R,S)-hydroxy-3-(6-méthoxyquinolin-4-yl)propyl]-1-[2-(fur-2-yl)thioéthyl]pipéridine-3-carboxylique
Acide (3R,4R)-4-[3-(R,S)-hydroxy-3-(6-méthoxyquinolin-4-yl)propyl]-1-[3-(fur-2-yl)thiopropyl]pipéridine-3-carboxylique
Acide (3R,4R)-4-[3-(R,S)-hydroxy-3-(6-méthoxyquinolin-4-yl)propyl]-1-[2-(fur-3-yl)thioéthyl]pipéridine-3-carboxylique
Acide (3R,4R)-4-[3-(R,S)-hydroxy-3-(6-méthoxyquinolin-4-yl)propyl]-1-[3-(fur-3-yl)thiopropyl]pipéridine-3-carboxylique
Acide (3R,4R)-4-[3-(R,S)-hydroxy-3-(6-méthoxyquinolin-4-yl)propyl]-1-[2-(1-méthylpyrrol-2-yl)thioéthyl]pipéridine-3-carboxylique
Acide (3R,4R)-4-[3-(R,S)-hydroxy-3-(6-méthoxyquinolin-4-yl)propyl]-1-[3-(1-méthylpyrrol-2-yl)thiopropyl]pipéridine-3-carboxylique
Acide (3R,4R)-4-[3-(R,S)-hydroxy-3-(6-méthoxyquinolin-4-yl)propyl]-1-[2-(1-méthylpyrrol-3-yl)thioéthyl]pipéridine-3-carboxylique
Acide (3R,4R)-4-[3-(R,S)-hydroxy-3-(6-méthoxyquinolin-4-yl)propyl]-1-[3-(1-méthylpyrrol-3-yl)thiopropyl]pipéridine-3-carboxylique
Acide (3R,4R)-4-[3-(R,S)-hydroxy-3-(6-méthoxyquinolin-4-yl)propyl]-1-[3-(1,3-thiazol-2-yl)thiopropyl]pipéridine-3-carboxylique
Acide (3R,4R)-4-[3-(R,S)-hydroxy-3-(6-méthoxyquinolin-4-yl)propyl]-1-[2-(1-méthylimidazol-2-yl)thioéthyl]pipéridine-3-carboxylique
Acide (3R,4R)-4-[3-(R,S)-hydroxy-3-(6-méthoxyquinolin-4-yl)propyl]-1-[3-(1-méthylimidazol-2-yl)thiopropyl]pipéridine-3-carboxylique
Acide (3R,4R)-4-[3-(R,S)-hydroxy-3-(6-méthoxyquinolin-4-yl)propyl]-1-[2-(3-methylimidazol-4-yl)thioéthyl]pipéridine-3-carboxylique
Acide (3R,4R)-4-[3-(R,S)-hydroxy-3-(6-méthoxyquinolin-4-yl)propyl]-1-[3-(3-méthylimidazol-4-yl)thiopropyl]pipéridine-3-carboxylique
Acide (3R,4R)-4-[3-(R,S)-hydroxy-3-(6-méthoxyquinolin-4-yl)propyl]-1-[2-(3-méthylpyrazol-4-yl)thioéthyl]pipéridine-3-carboxylique
Acide (3R,4R)-4-[3-(R,S)-hydroxy-3-(6-méthoxyquinolin-4-yl)propyl]-1-[3-(3-méthylpyrazol-4-yl)thiopropyl]pipéridine-3-carboxylique
Acide (3R,4R)-4-[3-(R,S)-hydroxy-3-(6-méthoxyquinolin-4-yl)propyl]-1-[2-(oxazol-2-yl)thioéthyl]pipéridine-3-carboxylique
Acide (3R,4R)-4-[3-(R,S)-hydroxy-3-(6-méthoxyquinolin-4-yl)propyl]-1-[3-(oxazol-2-yl)thiopropyl]pipéridine-3-carboxylique
Acide (3R,4R)-4-[3-(R,S)-hydroxy-3-(6-méthoxyquinolin-4-yl)propyl]-1-[3-(pyridin-2-yl)thiopropyl]pipéridine-3-carboxylique
Acide (3R,4R)-4-[3-(R,S)-hydroxy-3-(6-méthoxyquinolin-4-yl)propyl]-1-[2-(pyridin-3-yl)thioéthyl]pipéridine-3-carboxylique
Acide (3R,4R)-4-[3-(R,S)-hydroxy-3-(6-méthoxyquinolin-4-yl)propyl]-1-[3-(pyridin-3-yl)thiopropyl]pipéridine-3-carboxylique
Acide (3R,4R)-4-[3-(R,S)-hydroxy-3-(6-méthoxyquinolin-4-yl)propyl]-1-[2-(pyridin-2-yl)thioéthyl]pipéridine-3-carboxylique
Acide (3R,4R)-4-[3-(R,S)-hydroxy-3-(6-méthoxyquinolin-4-yl)propyl]-1-[2-(pyridin-4-yl)thioéthyl]pipéridine-3-carboxylique
Acide (3R,4R)-4-[3-(R,S)-hydroxy-3-(6-méthoxyquinolin-4-yl)propyl]-1-[3-(pyridin-4-yl)thiopropyl]pipéridine-3-carboxylique
Acide (3R,4R)-4-[3-(R,S)-hydroxy-3-(6-méthoxyquinolin-4-yl)propyl]-1-[2-(pyrimidin-2-yl)thioéthyl]pipéridine-3-carboxylique
Acide (3R,4R)-4-[3-(R,S)-hydroxy-3-(6-méthoxyquinolin-4-yl)propyl]-1-[3-(pyrimidin-2-yl)thiopropyl]pipéridine-3-carboxylique
Acide (3R,4R)-4-[3-(R,S)-hydroxy-3-(6-méthoxyquinolin-4-yl)propyl]-1-[2-(pyrimidin-4-yl)thioéthyl]pipéridine-3-carboxylique
Acide (3R,4R)-4-[3-(R,S)-hydroxy-3-(6-méthoxyquinolin-4-yl)propyl]-1-[3-(pyrimidin-4-yl)thiopropyl]pipéridine-3-carboxylique
Acide (3R,4R)-4-[3-(R,S)-hydroxy-3-(6-méthoxyquinolin-4-yl)propyl]-1-[2-(pyrimidin-5-yl)thioéthyl]pipéridine-3-carboxylique
Acide (3R,4R)-4-[3-(R,S)-hydroxy-3-(6-méthoxyquinolin-4-yl)propyl]-1-[3-(pyrimidin-5-yl)thiopropyl]pipéridine-3-carboxylique
Acide (3R,4R)-4-[3-(R,S)-hydroxy-3-(6-méthoxyquinolin-4-yl)propyl]-1-[2-(pyrazin-2-yl)thioéthyl]pipéridine-3-carboxylique
Acide (3R,4R)-4-[3-(R,S)-hydroxy-3-(6-méthoxyquinolin-4-yl)propyl]-1-[3-(pyrazin-2-yl)thiopropyl]pipéridine-3-carboxylique
Acide (3R,4R)-4-[3-(R,S)-hydroxy-3-(6-méthoxyquinolin-4-yl)propyl]-1-[2-(pyridazin-3-yl)thioéthyl]pipéridine-3-carboxylique
Acide (3R,4R)-4-[3-(R,S)-hydroxy-3-(6-méthoxyquinolin-4-yl)propyl]-1-[3-(pyridazin-3-yl)thiopropyl]pipéridine-3-carboxylique
Acide (3R,4R)-4-[3-(R,S)-hydroxy-3-(6-méthoxyquinolin-4-yl)propyl]-1-[2-(pyridazin-4-yl)thioéthyl]pipéridine-3-carboxylique
Acide (3R,4R)-4-[3-(R,S)-hydroxy-3-(6-méthoxyquinolin-4-yl)propyl]-1-[3-(pyridazin-4-yl)thiopropyl]pipéridine-3-carboxylique
Acide (3R,4R)-4-[3-(R,S)-fluoro-3-(6-méthoxyquinolin-4-yl)propyl]-1-[3-phénylthioéthyl]pipéridine-3-carboxylique
Acide (3R,4R)-4-[3-(R,S)-fluoro-3-(6-méthoxyquinolin-4-yl)propyl]-1-[3-phénylthiopropyl]pipéridine-3-carboxylique
Acide (3R,4R)-4-[3-(R,S)-fluoro-3-(6-méthoxyquinolin-4-yl)propyl]-1-[3-(2-fluorophénylthio)propyl]pipéridine-3-carboxylique
Acide (3R,4R)-4-[3-(R,S)-fluoro-3-(6-méthoxyquinolin-4-yl)propyl]-1-[3-(3-fluorophénylthio)propyl]pipéridine-3-carboxylique
Acide (3R,4R)-4-[3-(R,S)-fluoro-3-(6-méthoxyquinolin-4-yl)propyl]-1-[2-(2-fluorophénylthio)éthyl]pipéridine-3-carboxylique
Acide (3R,4R)-4-[3-(R,S)-fluoro-3-(6-méthoxyquinolin-4-yl)propyl]-1-[2-(4-fluorophénylthio)éthyl]pipéridine-3-carboxylique
Acide (3R,4R)-4-[3-(R,S)-fluoro-3-(6-méthoxyquinolin-4-yl)propyl]-1-[3-(4-fluorophénylthio)propyl]pipéridine-3-carboxylique
Acide (3R,4R)-4-[3-(R,S)-fluoro-3-(6-méthoxyquinolin-4-yl)propyl]-1-[2-(2,3-difluorophénylthio)éthyl]pipéridine-3-carboxylique
Acide (3R,4R)-4-[3-(R,S)-fluoro-3-(6-méthoxyquinolin-4-yl)propyl]-1-[3-(2,3-difluorophénylthio)propyl]pipéridine-3-carboxylique
Acide (3R,4R)-4-[3-(R,S)-fluoro-3-(6-méthoxyquinolin-4-yl)propyl]-1-[2-(2,6-difluorophénylthio)éthyl]pipéridine-3-carboxylique
Acide (3R,4R)-4-[3-(R,S)-fluoro-3-(6-méthoxyquinolin-4-yl)propyl]-1-[3-(2,6-difluorophénylthio)propyl]pipéridine-3-carboxylique
Acide (3R,4R)-4-[3-(R,S)-fluoro-3-(6-méthoxyquinolin-4-yl)propyl]-1-[2-(2-chlorophénylthio)éthyl]pipéridine-3-carboxylique
Acide (3R,4R)-4-[3-(R,S)-fluoro-3-(6-méthoxyquinolin-4-yl)propyl]-1-[3-(2-chlorophénylthio)propyl]pipéridine-3-carboxylique
Acide (3R,4R)-4-[3-(R,S)-fluoro-3-(6-méthoxyquinolin-4-yl)propyl]-1-[2-(3-chlorophénylthio)éthyl]pipéridine-3-carboxylique
Acide (3R,4R)-4-[3-(R,S)-fluoro-3-(6-méthoxyquinolin-4-yl)propyl]-1-[3-(3-chlorophénylthio)propyl]pipéridine-3-carboxylique
Acide (3R,4R)-4-[3-(R,S)-fluoro-3-(6-méthoxyquinolin-4-yl)propyl]-1-[2-(4-chlorophénylthio)éthyl]pipéridine-3-carboxylique
Acide (3R,4R)-4-[3-(R,S)-fluoro-3-(6-méthoxyquinolin-4-yl)propyl]-1-[3-(4-chlorophénylthio)propyl]pipéridine-3-carboxylique
Acide (3R,4R)-4-[3-(R,S)-fluoro-3-(6-méthoxyquinolin-4-yl)propyl]-1-[2-(2,3-dichlorophénylthio)éthyl]pipéridine-3-carboxylique
Acide (3R,4R)-4-[3-(R,S)-fluoro-3-(6-méthoxyquinolin-4-yl)propyl]-1-[3-(2,3-dichlorophénylthio)propyl]pipéridine-3-carboxylique
Acide (3R,4R)-4-[3-(R,S)-fluoro-3-(6-méthoxyquinolin-4-yl)propyl]-1-[2-(2,6-dichlorophénylthio)éthyl]pipéridine-3-carboxylique
Acide (3R,4R)-4-[3-(R,S)-fluoro-3-(6-méthoxyquinolin-4-yl)propyl]-1-[3-(2,6-dichlorophénylthio)propyl]pipéridine-3-carboxylique
Acide (3R,4R)-4-[3-(R,S)-fluoro-3-(6-méthoxyquinolin-4-yl)propyl]-1-[2-(2-méthylphénylthio)éthyl]pipéridine-3-carboxylique
Acide (3R,4R)-4-[3-(R,S)-fluoro-3-(6-méthoxyquinolin-4-yl)propyl]-1-[3-(2-méthylphénylthio)propyl]pipéridine-3-carboxylique
Acide (3R,4R)-4-[3-(R,S)-fluoro-3-(6-méthoxyquinolin-4-yl)propyl]-1-[2-(3-méthylphénylthio)éthyl]pipéridine-3-carboxylique
Acide (3R,4R)-4-[3-(R,S)-fluoro-3-(6-méthoxyquinolin-4-yl)propyl]-1-[3-(3-méthylphénylthio)propyl]pipéridine-3-carboxylique
Acide (3R,4R)-4-[3-(R,S)-fluoro-3-(6-méthoxyquinolin-4-yl)propyl]-1-[2-(4-méthylphénylthio)éthyl]pipéridine-3-carboxylique
Acide (3R,4R)-4-[3-(R,S)-fluoro-3-(6-méthoxyquinolin-4-yl)propyl]-1-[3-(4-méthylphénylthio)propyl]pipéridine-3-carboxylique
Acide (3R,4R)-4-[3-(R,S)-fluoro-3-(6-méthoxyquinolin-4-yl)propyl]-1-[2-(2-trifluorométhylphénylthio)éthyl]pipéridine-3-carboxylique
Acide (3R,4R)-4-[3-(R,S)-fluoro-3-(6-méthoxyquinolin-4-yl)propyl]-1-[3-(2-triflurométhylphénylthio)propyl]pipéridine-3-carboxylique
Acide (3R,4R)-4-[3-(R,S)-fluoro-3-(6-méthoxyquinolin-4-yl)propyl]-1-[2-(3-trifluorométhylphénylthio)éthyl]pipéridine-3-carboxylique
Acide (3R,4R)-4-[3-(R,S)-fluoro-3-(6-méthoxyquinolin-4-yl)propyl]-1-[3-(3-triflurométhylphénylthio)propyl]pipéridine-3-carboxylique
Acide (3R,4R)-4-[3-(R,S)-fluoro-3-(6-méthoxyquinolin-4-yl)propyl]-1-[2-(4-trifluorométhylphénylthio)éthyl]pipéridine-3-carboxylique
Acide (3R,4R)-4-[3-(R,S)-fluoro-3-(6-méthoxyquinolin-4-yl)propyl]-1-[3-(4-triflurométhylphénylthio)propyl]pipéridine-3-carboxylique
Acide (3R,4R)-4-[3-(R,S)-fluoro-3-(6-méthoxyquinolin-4-yl)propyl]-1-[2-(2-méthoxyphénylthio)éthyl]pipéridine-3-carboxylique
Acide (3R,4R)-4-[3-(R,S)-fluoro-3-(6-méthoxyquinolin-4-yl)propyl]-1-[3-(2-méthoxyphénylthio)propyl]pipéridine-3-carboxylique
Acide (3R,4R)-4-[3-(R,S)-fluoro-3-(6-méthoxyquinolin-4-yl)propyl]-1-[2-(3-méthoxyphénylthio)éthyl]pipéridine-3-carboxylique
Acide (3R,4R)-4-[3-(R,S)-fluoro-3-(6-méthoxyquinolin-4-yl)propyl]-1-[3-(3-méthoxyphénylthio)propyl]pipéridine-3-carboxylique
Acide (3R,4R)-4-[3-(R,S)-fluoro-3-(6-méthoxyquinolin-4-yl)propyl]-1-[2-(4-méthoxyphénylthio)éthyl]pipéridine-3-carboxylique
Acide (3R,4R)-4-[3-(R,S)-fluoro-3-(6-méthoxyquinolin-4-yl)propyl]-1-[3-(4-méthoxyphénylthio)propyl]pipéridine-3-carboxylique
Acide (3R,4R)-4-[3-(R,S)-fluoro-3-(6-méthoxyquinolin-4-yl)propyl]-1-[2-(cyclopropylthio)éthyl]pipéridine-3-carboxylique
Acide (3R,4R)-4-[3-(R,S)-fluoro-3-(6-méthoxyquinolin-4-yl)propyl]-1-[3-(cyclopropylthio)propyl]pipéridine-3-carboxylique
Acide (3R,4R)-4-[3-(R,S)-fluoro-3-(6-méthoxyquinolin-4-yl)propyl]-1-[2-(cyclobutylthio)éthyl]pipéridine-3-carboxylique
Acide (3R,4R)-4-[3-(R,S)-fluoro-3-(6-méthoxyquinolin-4-yl)propyl]-1-[3-(cyclobutylthiopropyl]pipéridine-3-carboxylique
Acide (3R,4R)-4-[3-(R,S)-fluoro-3-(6-méthoxyquinolin-4-yl)propyl]-1-[2-(cyclopentylthio)éthyl]pipéridine-3-carboxylique
Acide (3R,4R)-4-[3-(R,S)-fluoro-3-(6-méthoxyquinolin-4-yl)propyl]-1-[3-(cyclopentylthio)propyl]pipéridine-3-carboxylique
Acide (3R,4R)-4-[3-(R,S)-fluoro-3-(6-méthoxyquinolin-4-yl)propyl]-1-[2-(cyclohexylthio)éthyl]pipéridine-3-carboxylique
Acide (3R,4R)-4-[3-(R,S)-fluoro-3-(6-méthoxyquinolin-4-yl)propyl]-1-[3-(cyclohexylthio)propyl]pipéridine-3-carboxylique
Acide (3R,4R)-4-[3-(R,S)-fluoro-3-(6-méthoxyquinolin-4-yl)propyl]-1-[3-(thien-2-yl)thiopropyl]pipéridine-3-carboxylique
Acide (3R,4R)-4-[3-(R,S)-fluoro-3-(6-méthoxyquinolin-4-yl)propyl]-1-[2-(5-chloro-thien-2-yl)thioéthyl]pipéridine-3-carboxylique
Acide (3R,4R)-4-[3-(R,S)-fluoro-3-(6-méthoxyquinolin-4-yl)propyl]-1-[3-(5-chloro-thien-2-yl)thiopropyl]pipéridine-3-carboxylique
Acide (3R,4R)-4-[3-(R,S)-fluoro-3-(6-méthoxyquinolin-4-yl)propyl]-1-[2-(3-chloro-thien-2-yl)thioéthyl]pipéridine-3-carboxylique
Acide (3R,4R)-4-[3-(R,S)-fluoro-3-(6-méthoxyquinolin-4-yl)propyl]-1-[3-(3-chloro-thien-2-yl)thiopropyl]pipéridine-3-carboxylique
Acide (3R,4R)-4-[3-(R,S)-fluoro-3-(6-méthoxyquinolin-4-yl)propyl]-1-[2-(5-méthyl-thien-2-yl)thioéthyl]pipéridine-3-carboxylique
Acide (3R,4R)-4-[3-(R,S)-fluoro-3-(6-méthoxyquinolin-4-yl)propyl]-1-[3-(5-méthyl-thien-2-yl)thiopropyl]pipéridine-3-carboxylique
Acide (3R,4R)-4-[3-(R,S)-fluoro-3-(6-méthoxyquinolin-4-yl)propyl]-1-[2-(3-méthyl-thien-2-yl)thioéthyl]pipéridine-3-carboxylique
Acide (3R,4R)-4-[3-(R,S)-fluoro-3-(6-méthoxyquinolin-4-yl)propyl]-1-[3-(3-méthyl-thien-2-yl)thiopropyl]pipéridine-3-carboxylique
Acide (3R,4R)-4-[3-(R,S)-fluoro-3-(6-méthoxyquinolin-4-yl)propyl]-1-[2-(thien-3-yl)thioéthyl]pipéridine-3-carboxylique
Acide (3R,4R)-4-[3-(R,S)-fluoro-3-(6-méthoxyquinolin-4-yl)propyl]-1-[3-(thien-3-yl)thiopropyl]pipéridine-3-carboxylique
Acide (3R,4R)-4-[3-(R,S)-fluoro-3-(6-méthoxyquinolin-4-yl)propyl]-1-[2-(fur-2-yl)thioéthyl]pipéridine-3-carboxylique
Acide (3R,4R)-4-[3-(R,S)-fluoro-3-(6-méthoxyquinolin-4-yl)propyl]-1-[3-(fur-2-yl)thiopropyl]pipéridine-3-carboxylique
Acide (3R,4R)-4-[3-(R,S)-fluoro-3-(6-méthoxyquinolin-4-yl)propyl]-1-[2-(fur-3-yl)thioéthyl]pipéridine-3-carboxylique
Acide (3R,4R)-4-[3-(R,S)-fluoro-3-(6-méthoxyquinolin-4-yl)propyl]-1-[3-(fur-3-yl)thiopropyl]pipéridine-3-carboxylique
Acide (3R,4R)-4-[3-(R,S)-fluoro-3-(6-méthoxyquinolin-4-yl)propyl]-1-[2-(1-méthyl-pyrrol-2-yl)thioéthyl]pipéridine-3-carboxylique
Acide (3R,4R)-4-[3-(R,S)-fluoro-3-(6-méthoxyquinolin-4-yl)propyl]-1-[3-(1-méthyl-pyrrol-2-yl)thiopropyl]pipéridine-3-carboxylique
Acide (3R,4R)-4-[3-(R,S)-fluoro-3-(6-méthoxyquinolin-4-yl)propyl]-1-[2-(1-méthyl-pyrrol-3-yl)thioéthyl]pipéridine-3-carboxylique
Acide (3R,4R)-4-[3-(R,S)-fluoro-3-(6-méthoxyquinolin-4-yl)propyl]-1-[3-(1-méthyl-pyrrol-3-yl)thiopropyl]pipéridine-3-carboxylique
Acide (3R,4R)-4-[3-(R,S)-fluoro-3-(6-méthoxyquinolin-4-yl)propyl]-1-[2-(1,3-thiazol-2-yl)thioéthyl]pipéridine-3-carboxylique
Acide (3R,4R)-4-[3-(R,S)-fluoro-3-(6-méthoxyquinolin-4-yl)propyl]-1-[3-(1,3-thiazol-2-yl)thiopropyl]pipéridine-3-carboxylique
Acide (3R,4R)-4-[3-(R,S)-fluoro-3-(6-méthoxyquinolin-4-yl)propyl]-1-[2-(1-méthylimidazol-2-yl)thioéthyl]pipénidine-3-carboxylique
Acide (3R,4R)-4-[3-(R,S)-fluoro-3-(6-méthoxyquinolin-4-yl)propyl]-1-[3-(1-méthylimidazol-2-yl)thiopropyl]pipéridine-3-carboxylique
Acide (3R,4R)-4-[3-(R,S)-fluoro-3-(6-méthoxyquinolin-4-yl)propyl]-1-[2-(3-méthylimidazol-4-yl)thioéthyl]pipéridine-3-carboxylique
Acide (3R,4R)-4-[3-(R,S)-fluoro-3-(6-méthoxyquinolin-4-yl)propyl]-1-[3-(3-méthylimidazol-4-yl)thiopropyl]pipéridine-3-carboxylique
Acide (3R,4R)-4-[3-(R,S)-fluoro-3-(6-méthoxyquinolin-4-yl)propyl]-1-[2-(3-méthylpyrazol-4-yl)thioéthyl]pipéridine-3-carboxylique
Acide (3R,4R)-4-[3-(R,S)-fluoro-3-(6-méthoxyquinolin-4-yl)propyl]-1-[3-(3-méthylpyrazol-4-yl)thiopropyl]pipéridine-3-carboxylique
Acide (3R,4R)-4-[3-(R,S)-fluoro-3-(6-méthoxyquinolin-4-yl)propyl]-1-[2-(oxazol-2-yl)thioéthyl]pipéridine-3-carboxylique
Acide (3R,4R)-4-[3-(R,S)-fluoro-3-(6-méthoxyquinolin-4-yl)propyl]-1-[3-(oxazol-2-yl)thiopropyl]pipéridine-3-carboxylique
Acide (3R,4R)-4-[3-(R,S)-fluoro-3-(6-méthoxyquinolin-4-yl)propyl]-1-[3-(pyridin-2-yl)thiopropyl]pipéridine-3-carboxylique
Acide (3R,4R)-4-[3-(R,S)-fluoro-3-(6-méthoxyquinolin-4-yl)propyl]-1-[2-(pyridin-3yl)thioéthyl]pipéridine-3-carboxylique
Acide (3R,4R)-4-[3-(R,S)-fluoro-3-(6-méthoxyquinolin-4-yl)propyl]-1-[3-(pyridin-3-yl)thiopropyl]pipéridine-3-carboxylique
Acide (3R,4R)-4-[3-(R,S)-fluoro-3-(6-méthoxyquinolin-4-yl)propyl]-1-[2-(pyridin-2-yl)thioéthyl]pipéridine-3-carboxylique
Acide (3R,4R)-4-[3-(R,S)-fluoro-3-(6-méthoxyquinolin-4-yl)propyl]-1-[2-(pyridin-4-yl)thioéthyl]pipéridine-3-carboxylique
Acide (3R,4R)-4-[3-(R,S)-fluoro-3-(6-méthoxyquinolin-4-yl)propyl]-1-[3-(pyridin-4-yl)thiopropyl]pipéridine-3-carboxylique
Acide (3R,4R)-4-[3-(R,S)-fluoro-3-(6-méthoxyquinolin-4-yl)propyl]-1-[2-(pyrimidin-2-yl)thioéthyl]pipéridine-3-carboxylique
Acide (3R,4R)-4-[3-(R,S)-fluoro-3-(6-méthoxyquinolin-4-yl)propyl]-1-[3-(pyrimidin-2-yl)thiopropyl]pipéridine-3-carboxylique
Acide (3R,4R)-4-[3-(R,S)-fluoro-3-(6-méthoxyquinolin-4-yl)propyl]-1-[2-(pyrimidin-4-yl)thioéthyl]pipéridine-3-carboxylique
Acide (3R,4R)-4-[3-(R,S)-fluoro-3-(6-méthoxyquinolin-4-yl)propyl]-1-[3-(pyrimidin-4-yl)thiopropyl]pipéridine-3-carboxylique
Acide (3R,4R)-4-[3-(R,S)-fluoro-3-(6-méthoxyquinolin-4-yl)propyl]-1-[2-(pyrimidin-5-yl)thioéthyl]pipéridine-3-carboxylique
Acide (3R,4R)-4-[3-(R,S)-fluoro-3-(6-méthoxyquinolin-4-yl)propyl]-1-[3-(pyrimidin-5-yl)thiopropyl]pipéridine-3-carboxylique
Acide (3R,4R)-4-[3-(R,S)-fluoro-3-(6-méthoxyquinolin-4-yl)propyl]-1-[2-(pyrazin-2-yl)thioéthyl]pipéridine-3-carboxylique
Acide (3R,4R)-4-[3-(R,S)-fluoro-3-(6-méthoxyquinolin-4-yl)propyl]-1-[3-(pyrazin-2-yl)thiopropyl]pipéridine-3-carboxylique
Acide (3R,4R)-4-[3-(R,S)-fluoro-3-(6-méthoxyquinolin-4-yl)propyl]-1-[2-(pyridazin-3-yl)thioéthyl]pipéridine-3-carboxylique
Acide (3R,4R)-4-[3-(R,S)-fluoro-3-(6-méthoxyquinolin-4-yl)propyl]-1-[3-(pyridazin-3-yl)thiopropyl]pipéridine-3-carboxylique
Acide (3R,4R)-4-[3-(R,S)-fluoro-3-(6-méthoxyquinolin-4-yl)propyl]-1-[2-(pyridazin-4-yl)thioéthyl]pipéridine-3-carboxylique
Acide (3R,4R)-4-[3-(R,S)-fluoro-3-(6-méthoxyquinolin-4-yl)propyl]-1-[3-(pyridazin-4-yl)thiopropyl]pipéridine-3-carboxylique
Acide (3R,4R)-4-[3-(6-méthoxyquinolin-4-yl)propyl]-1-[3-phénylthio-propyl]pipéridine-3-acétique
Acide (3R,4R)-4-[3-(6-méthoxyquinolin-4-yl)propyl]-1-[2-(2-fluorophénylthio)éthyl] pipéridine-3-acétique
Acide (3R,4R)-4-[3-(6-méthoxyquinolin-4-yl)propyl]-1-[3-(2-fluorophénylthio)propyl] pipéridine-3-acétique
Acide (3R,4R)-4-[3-(6-méthoxyquinolin-4-yl)propyl]-1-[2-(3-fluorophénylthio)éthyl] pipéridine-3-acétique
Acide (3R,4R)-4-[3-(6-méthoxyquinolin-4-yl)propyl]-1-[3-(3-fluorophénylthio)propyl] pipéridine-3-acétique
Acide (3R,4R)-4-[3-(6-méthoxyquinolin-4-yl)propyl]-1-[2-(4-fluorophénylthio)éthyl] pipéridine-3-acétique
Acide (3R,4R)-4-[3-(6-méthoxyquinolin-4-yl)propyl]-1-[3-(4-fluorophénylthio)propyl] pipéridine-3-acétique
Acide (3R,4R)-4-[3-(6-méthoxyquinolin-4-yl)propyl]-1-[2-(2,3-difluorophénylthio)éthyl] pipéridine-3-acétique
Acide (3R,4R)-4-[3-(6-méthoxyquinolin-4-yl)propyl]-1-[3-(2,3-difluorophénylthio)propyl] pipéridine-3-acétique
Acide (3R,4R)-4-[3-(6-méthoxyquinolin-4-yl)propyl]-1-[2-(2,6-difluorophénylthio)éthyl] pipéridine-3-acétique
Acide (3R,4R)-4-[3-(6-méthoxyquinolin-4-yl)propyl]-1-[3-(2,6-difluorophénylthio)propyl] pipéridine-3-acétique
Acide (3R,4R)-4-[3-(6-méthoxyquinolin-4-yl)propyl]-1-[2-(2-chlorophénylthio)éthyl] pipéridine-3-acétique
Acide (3R,4R)-4-[3-(6-méthoxyquinolin-4-yl)propyl]-1-[3-(2-chlorophénylthio)propyl] pipéridine-3-acétique
Acide (3R,4R)-4-[3-(6-méthoxyquinolin-4-yl)propyl]-1-[2-(3-chlorophénylthio)éthyl] pipéridine-3-acétique
Acide (3R,4R)-4-[3-(6-méthoxyquinolin-4-yl)propyl]-1-[3-(3-chlorophénylthio)propyl] pipéridine-3-acétique
Acide (3R,4R)-4-[3-(6-méthoxyquinolin-4-yl)propyl]-1-[2-(4-chlorophénylthio)éthyl] pipéridine-3-acétique
Acide (3R,4R)-4-[3-(6-méthoxyquinolin-4-yl)propyl]-1-[3-(4-chlorophénylthio)propyl] pipéridine-3-acétique
Acide (3R,4R)-4-[3-(6-méthoxyquinolin-4-yl)propyl]-1-[2-(2,3-dichlorophénylthio)éthyl] pipéridine-3-acétique
Acide (3R,4R)-4-[3-(6-méthoxyquinolin-4-yl)propyl]-1-[3-(2,3-dichlorophénylthio)propyl] pipéridine-3-acétique
Acide (3R,4R)-4-[3-(6-méthoxyquinolin-4-yl)propyl]-1-[2-(2,6-dichlorophénylthio)éthyl] pipéridine-3-acétique
Acide (3R,4R)-4-[3-(6-méthoxyquinolin-4-yl)propyl]-1-[3-(2,6-dichlorophénylthio)propyl] pipéridine-3-acétique
Acide (3R,4R)-4-[3-(6-méthoxyquinolin-4-yl)propyl]-1-[2-(2-méthylphénylthio)éthyl] pipéridine-3-acétique
Acide (3R,4R)-4-[3-(6-méthoxyquinolin-4-yl)propyl]-1-[3-(2-méthylphénylthio)propyl] pipéridine-3-acétique
Acide (3R,4R)-4-[3-(6-méthoxyquinolin-4-yl)propyl]-1-[2-(3-méthylphénylthio)éthyl] pipéridine-3-acétique
Acide (3R,4R)-4-[3-(6-méthoxyquinolin-4-yl)propyl]-1-[3-(3-méthylphénylthio)propyl] pipéridine-3-acétique
Acide (3R,4R)-4-[3-(6-méthoxyquinolin-4-yl)propyl]-1-[2-(4-méthylphénylthio)éthyl] pipéridine-3-acétique
Acide (3R,4R)-4-[3-(6-méthoxyquinolin-4-yl)propyl]-1-[3-(4-méthylphénylthio)propyl] pipéridine-3-acétique
Acide (3R,4R)-4-[3-(6-méthoxyquinolin-4-yl)propyl]-1-[2-(2-trifluorométhylphénylthio)éthyl]pipéridine-3-acétique
Acide (3R,4R)-4-[3-(6-méthoxyquinolin-4-yl)propyl]-1-[3-(2-triflurométhylphénylthio) propyl]pipéridine-3-acétique
Acide (3R,4R)-4-[3-(6-méthoxyquinolin-4-yl)propyl]-1-[2-(3-trifluorométhylhénylthio) éthyl]pipéridine-3-acétique
Acide (3R,4R)-4-[3-(6-méthoxyquinolin-4-yl)propyl]-1-[3-(3-triflurométhylphénylthio) propyl]pipéridine-3-acétique
Acide (3R,4R)-4-[3-(6-méthoxyquinolin-4-yl)propyl]-1-[2-(4-trifluorométhylhénylthio) éthyl]pipéridine-3-acétique
Acide (3R,4R)-4-[3-(6-méthoxyquinolin-4-yl)propyl]-1-[3-(4-triflurométhylphénylthio) propyl]pipéridine-3-acétique
Acide (3R,4R)-4-[3-(6-méthoxyquinolin-4-yl)propyl]-1-[2-(2-méthoxyphénylthio)éthyl] pipéridine-3-acétique
Acide (3R,4R)-4-[3-(6-méthoxyquinolin-4-yl)propyl]-1-[3-(2-méthoxyphénylthio)propyl] pipéridine-3-acétique
Acide (3R,4R)-4-[3-(6-méthoxyquinolin-4-yl)propyl]-1-[2-(3-méthoxyphénylthio)éthyl] pipéridine-3-acétique
Acide (3R,4R)-4-[3-(6-méthoxyquinolin-4-yl)propyl]-1-[3-(3-méthoxyphénylthio)propyl] pipéridine-3-acétique
Acide (3R,4R)-4-[3-(6-méthoxyquinolin-4-yl)propyl]-1-[2-(4-méthoxyphénylthio)éthyl] pipéridine-3-acétique
Acide (3R,4R)-4-[3-(6-méthoxyquinolin-4-yl)propyl]-1-[3-(4-méthoxyphénylthio)propyl] pipéridine-3-acétique
Acide (3R,4R)-4-[3-(6-méthoxyquinolin-4-yl)propyl]-1-[2-(cyclopropylthio)éthyl]pipéridine-3-acétique
Acide (3R,4R)-4-[3-(6-méthoxyquinolin-4-yl)propyl]-1-[3-(cyclopropylthio)propyl] pipéridine-3-acétique
Acide (3R,4R)-4-[3-(6-méthoxyquinolin-4-yl)propyl]-1-[2-(cyclobutylthio)éthyl]pipéridine-3-acétique
Acide (3R,4R)-4-[3-(6-méthoxyquinolin-4-yl)propyl]-1-[3-(cyclobutylthiopropyl]pipéridine-3-acétique
Acide (3R,4R)-4-[3-(6-méthoxyquinolin-4-yl)propyl]-1-[2-(cyclopentylthio)éthyl]pipéridine-3-acétique
Acide (3R,4R)-4-[3-(6-méthoxyquinolin-4-yl)propyl]-1-[3-(cyclopentylthio)propyl]pipéridine-3-acétique
Acide (3R,4R)-4-[3-(6-méthoxyquinolin-4-yl)propyl]-1-[2-(cyclohexylthio)éthyl]pipéridine-3-acétique
Acide (3R,4R)-4-[3-(6-méthoxyquinolin-4-yl)propyl]-1-[3-(cyclohexylthio)propyl]pipéridine-3-acétique
Acide (3R,4R)-4-[3-(6-méthoxyquinolin-4-yl)propyl]-1-[2-(thien-2-yl)thioéthyl]pipéridine-3-acétique
Acide (3R,4R)-4-[3-(6-méthoxyquinolin-4-yl)propyl]-1-[3-(thien-2-yl)thiopropyl]pipéridine-3-acétique
Acide (3R,4R)-4-[3-(6-méthoxyquinolin-4-yl)propyl]-1-[2-(5-chloro-thien-2-yl)thioéthyl] pipéridine-3-acétique
Acide (3R,4R)-4-[3-(6-méthoxyquinolin-4-yl)propyl]-1-[3-(5-chloro-thien-2-yl)thiopropyl] pipéridine-3-acétique
Acide (3R,4R)-4-[3-(6-méthoxyquinolin-4-yl)propyl]-1-[2-(3-chloro-thien-2-yl)thioéthyl] pipéridine-3-acétique
Acide (3R,4R)-4-[3-(6-méthoxyquinolin-4-yl)propyl]-1-[3-(3-chloro-thien-2-yl)thiopropy] pipéridine-3-acétique
Acide (3R,4R)-4-[3-(6-méthoxyquinolin-4-yl)propyl]-1-[2-(5-méthyl-thien-2-yl)thioéthyl] pipéridine-3-acétique
Acide (3R,4R)-4-[3-(6-méthoxyquinolin-4-yl)propyl]-1-[3-(5-méthyl-thien-2-yl)thiopropyl] pipéridine-3-acétique
Acide (3R,4R)-4-[3-(6-méthoxyquinolin-4-yl)propyl]-1-[2-(3-méthyl-thien-2-yl)thiopropyl] pipéridine-3-acétique
Acide (3R,4R)-4-[3-(6-méthoxyquinolin-4-yl)propyl]-1-[3-(3-méthyl-thien-2-yl)thiopropyl] pipéridine-3-acétique
Acide (3R,4R)-4-[3-(6-méthoxyquinolin-4-yl)propyl]-1-[2-(thien-3-yl)thioéthyl]pipéridine-3-acétique
Acide (3R,4R)-4-[3-(6-méthoxyquinolin-4-yl)propyl]-1-[3-(thien-3-yl)thiopropyl]pipéridine-3-acétique
Acide (3R,4R)-4-[3-(6-méthoxyquinolin-4-yl)propyl]-1-[2-(fur-2-yl)thioéthyl]pipéridine-3-acétique
Acide (3R,4R)-4-[3-(6-méthoxyquinolin-4-yl)propyl]-1-[3-(fur-2-yl)thiopropyl]pipéridine-3-acétique
Acide (3R,4R)-4-[3-(6-méthoxyquinolin-4-yl)propyl]-1-[2-(fur-3-yl)thioéthyl]pipéridine-3-acétique
Acide (3R,4R)-4-[3-(6-méthoxyquinolin-4-yl)propyl]-1-[3-(fur-3-yl)thiopropyl]pipéridine-3-acétique
Acide (3R,4R)-4-[3-(6-méthoxyquinolin-4-yl)propyl]-1-[2-(1-méthyl-pyrrol-2-yl)thioéthyl] pipéridine-3-acétique
Acide (3R,4R)-4-[3-(6-méthoxyquinolin-4-yl)propyl]-1-[3-(1-méthyl-pyrrol-2-yl)thiopropyl] pipéridine-3-acétique
Acide (3R,4R)-4-[3-(6-méthoxyquinolin-4-yl)propyl]-1-[2-(1-méthyl-pyrrol-3-yl)thioéthyl] pipéridine-3-acétique
Acide (3R,4R)-4-[3-(6-méthoxyquinolin-4-yl)propyl]-1-[3-(1-méthyl-pyrrol-3-yl)thiopropyl] pipéridine-3-acétique
Acide (3R,4R)-4-[3-(6-méthoxyquinolin-4-yl)propyl]-1-[2-(thiazol-2-yl)thioéthyl]pipéridine-3-acétique
Acide (3R,4R)-4-[3-(6-méthoxyquinolin-4-yl)propyl]-1-[3-(thiazol-2-yl)thiopropyl]pipéridine-3-acétique
Acide (3R,4R)-4-[3-(6-méthoxyquinolin-4-yl)propyl]-1-[2-(1-méthyl-imidazol-2-yl)thioéthyl] pipéridine-3-acétique
Acide (3R,4R)-4-[3-(6-méthoxyquinolin-4-yl)propyl]-1-[3-(1-méthyl-imidazol-2-yl) thiopropyl]pipéridine-3-acétique
Acide (3R,4R)-4-[3-(6-méthoxyquinolin-4-yl)propyl]-1-[2-(3-méthyl-imidazol-4-yl)thioéthyl]pipéridine-3-acétique
Acide (3R,4R)-4-[3-(6-méthoxyquinolin-4-yl)propyl]-1-[3-(3-méthyl-imidazol-4-yl) thiopropyl]pipéridine-3-acétique
Acide (3R,4R)-4-[3-(6-méthoxyquinolin-4-yl)propyl]-1-[2-(3-méthyl-pyrazol-4-yl)thioéthyl] pipéridine-3-acétique
Acide (3R,4R)-4-[3-(6-méthoxyquinolin-4-yl)propyl]-1-[3-(3-méthyl-pyrazol-4-yl)thiopropyl] pipéridine-3-acétique
Acide(3R,4R)-4-[3-(6-méthoxyquinolin-4-yl)propyl]-1-[2-(oxazol-2-yl)thioéthyl]pipéridine-3-acétique
Acide (3R,4R)-4-[3-(6-méthoxyquinolin-4-yl)propyl)-1-[3-(oxazol-2-yl)thiopropyl]pipéridine-3-acétique
Acide (3R,4R)-4-[3-(6-méthoxyquinolin-4-yl)propyl]-1-[2-(pyridin-2-yl)thioéthyl]pipéridine-3-acétique
Acide (3R,4R)-4-[3-(6-méthoxyquinolin-4-yl)propyl]-1-[3-(pyridin-2-yl)thiopropyl] pipéridine-3-acétique
Acide (3R,4R)-4-[3-(6-méthoxyquinolin-4-yl)propyl]-1-[2-(pyridin-3-yl)thioéthyl]pipéridine-3-acétique
Acide (3R,4R)-4-[3-(6-méthoxyquinolin-4-yl)propyl]-1-[3-(pyridin-3-yl)thiopropyl] pipéridine-3-acétique
Acide (3R,4R)-4-[3-(6-méthoxyquinolin-4-yl)propyl]-1-[2-(pyridin-4-yl)thioéthyl]pipéridine-3-acétique
Acide (3R,4R)-4-[3-(6-méthoxyquinolin-4-yl)propyl]-1-[3-(pyridin-4-yl)thiopropyl] pipéridine-3-acétique
Acide (3R,4R)-4-[3-(6-méthoxyquinolin-4-yl)propyl]-1-[2-(pyrimidin-2-yl)thioéthyl] pipéridine-3-acétique
Acide (3R,4R)-4-[3-(6-méthoxyquinolin-4-yl)propyl]-1-[3-(pyrimidin-2-yl)thiopropyl] pipéridine-3-acétique
Acide (3R,4R)-4-[3-(6-méthoxyquinolin-4-yl)propyl]-1-[2-(pyrimidin-4-yl)thioéthyl] pipéridine-3-acétique
Acide (3R,4R)-4-[3-(6-méthoxyquinolin-4-yl)propyl]-1-[3-(pyrimidin-4-yl)thiopropyl] pipéridine-3-acétique
Acide (3R,4R)-4-[3-(6-méthoxyquinolin-4-yl)propyl]-1-[2-(pyrimidin-5-yl)thioéthyl] pipéridine-3-acétique
Acide (3R,4R)-4-[3-(6-méthoxyquinolin-4-yl)propyl]-1-[3-(pyrimidin-5-yl)thiopropyl] pipéridine-3-acétique
Acide (3R,4R)-4-[3-(6-méthoxyquinolin-4-yl)propyl]-1-[2-(pyrazin-2-yl)thioéthyl]pipéridine-3-acétique
Acide (3R,4R)-4-[3-(6-méthoxyquinolin-4-yl)propyl]-1-[3-(pyrazin-2-yl)thiopropyl] pipéridine-3-acétique
Acide (3R,4R)-4-[3-(6-méthoxyquinolin-4-yl)propyl]-1-[2-(pyridazin-3-yl)thioéthyl] pipéridine-3-acétique
Acide (3R,4R)-4-[3-(6-méthoxyquinolin-4-yl)propyl]-1-[3-(pyridazin-3-yl)thiopropyl] pipéridine-3-acétique
Acide (3R,4R)-4-[3-(6-méthoxyquinolin-4-yl)propyl]-1-[2-(pyridazin-4-yl)thioéthyl] pipéridine-3-acétique
Acide (3R,4R)-4-[3-(6-méthoxyquinolin-4-yl)propyl]-1-[3-(pyridazin-4-yl)thiopropyl] pipéridine-3-acétique
Acide (3R,4R)-4-[3-(R,S)-hydroxy-3-(6-méthoxyquinolin-4-yl)propyl]-1-[2-phénylthioéthyl]pipéridine-3-acétique
Acide (3R,4R)-4-[3-(R,S)-hydroxy-3-(6-méthoxyquinolin-4-yl)propyl]-1-[3-phénylthiopropyl]pipéridine-3-acétique
Acide (3R,4R)-4-[3-(R,S)-hydroxy-3-(6-méthoxyquinolin-4-yl)propyl]-1-[2-(2-fluorophénylthio)éthyl]pipéridine-3-acétique
Acide (3R,4R)-4-[3-(R,S)-hydroxy-3-(6-méthoxyquinolin-4-yl)propyl]-1-[3-(2-fluorophénylthio)propyl]pipéridine-3-acétique
Acide (3R,4R)-4-[3-(R,S)-hydroxy-3-(6-méthoxyquinolin-4-yl)propyl]-1-[2-(3-fluorophénylthio)éthyl]pipéridine-3-acétique
Acide (3R,4R)-4-[3-(R,S)-hydroxy-3-(6-méthoxyquinolin-4-yl)propyl]-1-[3-(3-fluorophénylthio)propyl]pipéridine-3-acétique
Acide (3R,4R)-4-[3-(R,S)-hydroxy-3-(6-méthoxyquinolin-4-yl)propyl]-1-[2-(4-fluorophénylthio)éthyl]pipéridine-3-acétique
Acide (3R,4R)-4-[3-(R,S)-hydroxy-3-(6-méthoxyquinolin-4-yl)propyl]-1-[3-(4-fluorophénylthio)propyl]pipéridine-3-acétique
Acide (3R,4R)-4-[3-(R,S)-hydroxy-3-(6-méthoxyquinolin-4-yl)propyl]-1-[2-(2,3-difluorophénylthio)éthyl]pipéridine-3-acétique
Acide (3R,4R)-4-[3-(R,S)-hydroxy-3-(6-méthoxyquinolin-4-yl)propyl]-1-[3-(2,3-difluorophénylthio)propyl]pipéridine-3-acétique
Acide (3R,4R)-4-[3-(R,S)-hydroxy-3-(6-methoxyquinolin-4-yl)propyl]-1-[2-(2,6-difluorophénylthio)éthyl]pipéridine-3-acétique
Acide (3R,4R)-4-[3-(R,S)-hydroxy-3-(6-méthoxyquinolin-4-yl)propyl]-1-[3-(2,6-difluorophénylthio)propyl]pipéridine-3-acétique
Acide (3R,4R)-4-[3-(R,S)-hydroxy-3-(6-méthoxyquinolin-4-yl)propyl]-1-[2-(2-chlorophényl-thio)éthyl]pipéridine-3-acétique
Acide (3R,4R)-4-[3-(R,S)-hydroxy-3-(6-méthoxyquinolin-4-yl)propyl]-1-[3-(2-chlorophényl-thio)propyl]pipéridine-3-acétique
Acide (3R,4R)-4-[3-(R,S)-hydroxy-3-(6-méthoxyquinolin-4-yl)propyl]-1-[2-(3-chlorophényl-thio)éthyl]pipéridine-3-acétique
Acide (3R,4R)-4-[3-(R,S)-hydroxy-3-(6-méthoxyquinolin-4-yl)propyl]-1-[3-(3-chlorophényl-thio)propyl]pipéridine-3-acétique
Acide (3R,4R)-4-[3-(R,S)-hydroxy-3-(6-méthoxyquinolin-4-yl)propyl]-1-[2-(4-chlorophényl-thio)éthyl]pipéridine-3-acétique
Acide (3R,4R)-4-[3-(R,S)-hydroxy-3-(6-méthoxyquinolin-4-yl)propyl]-1-[3-(4-chlorophényl-thio)propyl]pipéridine-3-acétique
Acide (3R,4R)-4-[3-(R,S)-hydroxy-3-(6-méthoxyquinolin-4-yl)propyl]-1-[2-(2,3-dichlorophénylthio)éthyl]pipéridine-3-acétique
Acide (3R,4R)-4-[3-(R,S)-hydroxy-3-(6-méthoxyquinolin-4-yl)propyl]-1-[3-(2,3-dichlorophénylthio)propyl]pipéridine-3-acétique
Acide (3R,4R)-4-[3-(R,S)-hydroxy-3-(6-méthoxyquinolin-4-yl)propyl]-1-[2-(2,6-dichlorophénylthio)éthyl]pipéridine-3-acétique
Acide (3R,4R)-4-[3-(R,S)-hydroxy-3-(6-méthoxyquinolin-4-yl)propyl]-1-[3-(2,6-dichlorophénylthio)propyl]pipéridine-3-acétique
Acide (3R,4R)-4-[3-(R,S)-hydroxy-3-(6-méthoxyquinolin-4-yl)propyl]-1-[2-(2-méthylphénylthio)éthyl]pipéridine-3-acétique
Acide (3R,4R)-4-[3-(R,S)-hydroxy-3-(6-méthoxyquinolin-4-yl)propyl]-1-[3-(2-méthylphénylthio)propyl]pipéridine-3-acétique
Acide (3R,4R)-4-[3-(R,S)-hydroxy-3-(6-méthoxyquinolin-4-yl)propyl]-1-[2-(3-méthylphénylthio)éthyl]pipéridine-3-acétique
Acide (3R,4R)-4-[3-(R,S)-hydroxy-3-(6-méthoxyquinolin-4-yl)propyl]-1-[3-(3-méthylphénylthio)propyl]pipéridine-3-acétique
Acide (3R,4R)-4-[3-(R,S)-hydroxy-3-(6-méthoxyquinolin-4-yl)propyl]-1-[2-(4-méthylphénylthio)éthyl]pipéridine-3-acétique
Acide (3R,4R)-4-[3-(R,S)-hydroxy-3-(6-méthoxyquinolin-4-yl)propyl]-1-[3-(4-méthylphénylthio)propyl]pipéridine-3-acétique
Acide (3R,4R)-4-[3-(R,S)-hydroxy-3-(6-méthoxyquinolin-4-yl)propyl]-1-[2-(2-trifluorométhylphénylthio)éthyl]pipéridine-3-acétique
Acide (3R,4R)-4-[3-(R,S)-hydroxy-3-(6-méthoxyquinolin-4-yl)propyl]-1-[3-(2-triflurométhylphénylthio)propyl]pipéridine-3-acétique
Acide (3R,4R)-4-[3-(R,S)-hydroxy-3-(6-méthoxyquinolin-4-yl)propyl]-1-[2-(3-trifluorométhylphénylthio)éthyl]pipéridine-3-acétique
Acide (3R,4R)-4-[3-(R,S)-hydroxy-3-(6-méthoxyquinolin-4-yl)propyl]-1-[3-(3-triflurométhylphénylthio)propyl]pipéridine-3-acétique
Acide (3R,4R)-4-[3-(R,S)-hydroxy-3-(6-méthoxyquinolin-4-yl)propyl]-1-[2-(4-trifluorométhylphénylthio)éthyl]pipéridine-3-acétique
Acide (3R,4R)-4-[3-(R,S)-hydroxy-3-(6-méthoxyquinolin-4-yl)propyl]-1-[3-(4-triflurométhylphénylthio)propyl]pipéridine-3-acétique
Acide (3R,4R)-4-[3-(R,S)-hydroxy-3-(6-méthoxyquinolin-4-yl)propyl]-1-[2-(2-méthoxyphénylthio)éthyl]pipéridine-3-acétique
Acide (3R,4R)-4-[3-(R,S)-hydroxy-3-(6-méthoxyquinolin-4-yl)propyl]-1-[3-(2-méthoxyphénylthio)propyl]pipéridine-3-acétique
Acide (3R,4R)-4-[3-(R,S)-hydroxy-3-(6-méthoxyquinolin-4-yl)propyl]-1-[2-(3-méthoxyphénylthio)éthyl]pipéridine-3-acétique
Acide (3R,4R)-4-[3-(R,S)-hydroxy-3-(6-méthoxyquinolin-4-yl)propyl]-1-[3-(3-méthoxyphénylthio)propyl]pipéridine-3-acétique
Acide (3R,4R)-4-[3-(R,S)-hydroxy-3-(6-méthoxyquinolin-4-yl)propyl]-1-[2-(4-méthoxyphénylthio)éthyl]pipéridine-3-acétique
Acide (3R,4R)-4-[3-(R,S)-hydroxy-3-(6-méthoxyquinolin-4-yl)propyl]-1-[3-(4-méthoxyphénylthio)propyl]pipéridine-3-acétique
Acide (3R,4R)-4-[3-(R,S)-hydroxy-3-(6-méthoxyquinolin-4-yl)propyl]-1-[2-(cyclopropylthio)éthyl]pipéridine-3-acétique
Acide (3R,4R)-4-[3-(R,S)-hydroxy-3-(6-méthoxyquinolin-4-yl)propyl]-1-[3-(cyclopropylthio)propyl]pipéridine-3-acétique
Acide (3R,4R)-4-[3-(R,S)-hydroxy-3-(6-méthoxyquinolin-4-yl)propyl]-1-[2-(cyclobutylthio) éthyl]pipéridine-3-acétique
Acide (3R,4R)-4-[3-(R,S)-hydroxy-3-(6-méthoxyquinolin-4-yl)propyl]-1-[3-(cyclobutylthiopropyl]pipéridine-3-acétique
Acide (3R,4R)-4-[3-(R,S)-hydroxy-3-(6-méthoxyquinolin-4-yl)propyl]-1-[2-(cyclopentylthio)éthyl]pipéridine-3-acétique
Acide (3R,4R)-4-[3-(R,S)-hydroxy-3-(6-méthoxyquinolin-4-yl)propyl]-1-[3-(cyclopentylthio)propyl]pipéridine-3-acétique
Acide (3R,4R)-4-[3-(R,S)-hydroxy-3-(6-méthoxyquinolin-4-yl)propyl]-1-[2-(cyclohexylthio) éthyl]pipéridine-3-acétique
Acide (3R,4R)-4-[3-(R,S)-hydroxy-3-(6-méthoxyquinolin-4-yl)propyl]-1-[3-(cyclohexylthio) propyl]pipéridine-3-acétique
Acide (3R,4R)-4-[3-(R,S)-hydroxy-3-(6-méthoxyquinolin-4-yl)propyl]-1-[2-(thien-2-yl) thioéthyl]pipéridine-3-acétique
Acide (3R,4R)-4-[3-(R,S)-hydroxy-3-(6-méthoxyquinolin-4-yl)propyl]-1-[3-(thien-2-yl)thiopropyl]pipéridine-3-acétique
Acide (3R,4R)-4-[3-(R,S)-hydroxy-3-(6-méthoxyquinolin-4-yl)propyl]-1-[2-(5-chloro-thien-2-yl)thioéthyl]pipéridine-3-acétique
Acide (3R,4R)-4-[3-(R,S)-hydroxy-3-(6-méthoxyquinolin-4-yl)propyl]-1-[3-(5-chloro-thien2-yl)thiopropyl]pipéridine-3-acétique
Acide (3R,4R)-4-[3-(R,S)-hydroxy-3-(6-méthoxyquinolin-4-yl)propyl]-1-[2-(3-chloro-thien-2-yl)thioéthyl]pipéridine-3-acétique
Acide (3R,4R)-4-[3-(R,S)-hydroxy-3-(6-méthoxyquinolin-4-yl)propyl]-1-[3-(3-chloro-thien-2-yl)thiopropyl]pipéridine-3-acétique
Acide (3R,4R)-4-[3-(R,S)-hydroxy-3-(6-méthoxyquinolin-4-yl)propyl]-1-[2-(5-méthylthien-2-yl)thioéthyl]pipéridine-3-acétique
Acide (3R,4R)-4-[3-(R,S)-hydroxy-3-(6-méthoxyquinolin-4-yl)propyl]-1-[3-(5-méthylthien-2-yl)thiopropyl]pipéridine-3-acétique
Acide (3R,4R)-4-[3-(R,S)-hydroxy-3-(6-méthoxyquinolin-4-yl)propyl]-1-[2-(3-méthylthien-2-yl)thioéthyl]pipéridine-3-acétique
Acide (3R,4R)-4-[3-(R,S)-hydroxy-3-(6-méthoxyquinolin-4-yl)propyl]-1-[3-(3-méthylthien-2-yl)thiopropyl]pipéridine-3-acétique
Acide (3R,4R)-4-[3-(R,S)-hydroxy-3-(6-méthoxyquinolin-4-yl)propyl]-1-[2-(thien-3-yl) thioéthyl]pipéridine-3-acétique
Acide (3R,4R)-4-[3-(R,S)-hydroxy-3-(6-méthoxyquinolin-4-yl)propyl]-1-[3-(thien-3-yl) thiopropyl]pipéridine-3-acétique
Acide (3R,4R)-4-[3-(R,S)-hydroxy-3-(6-méthoxyquinolin-4-yl)propyl]-1-[2-(fur-2-yl) thioéthyl]pipéridine-3-acétique
Acide (3R,4R)-4-[3-(R,S)-hydroxy-3-(6-méthoxyquinolin-4-yl)propyl]-1-[3-(fur-2-yl) thiopropyl]pipéridine-3-acétique
Acide (3R,4R)-4-[3-(R,S)-hydroxy-3-(6-méthoxyquinolin-4-yl)propyl]-1-[2-(fur-3-yl) thioéthyl]pipéridine-3-acétique
Acide (3R,4R)-4-[3-(R,S)-hydroxy-3-(6-méthoxyquinolin-4-yl)propyl]-1-[3-(fur-3-yl) thiopropyl]pipéridine-3-acétique
Acide (3R,4R)-4-[3-(R,S)-hydroxy-3-(6-méthoxyquinolin-4-yl)propyl]-1-[2-(1-méthylpyrrol-2-yl)thioéthyl]pipéridine-3-acétique
Acide (3R,4R)-4-[3-(R,S)-hydroxy-3-(6-méthoxyquinolin-4-yl)propyl]-1-[3-(1-méthylpyrrol-2-yl)thiopropyl]pipéridine-3-acétique
Acide (3R,4R)-4-[3-(R,S)-hydroxy-3-(6-méthoxyquinolin-4-yl)propyl]-1-[2-(1-méthylpyrrol-3-yl)thioéthyl]pipéridine-3-acétique
Acide (3R,4R)-4-[3-(R,S)-hydroxy-3-(6-méthoxyquinolin-4-yl)propyl]-1-[3-(1-méthylpyrrol-3-yl)thiopropyl]pipéridine-3-acétique
Acide (3R,4R)-4-[3-(R,S)-hydroxy-3-(6-méthoxyquinolin-4-yl)propyl]-1-[2-(thiazol-2-yl) thioéthyl]pipéridine-3-acétique
Acide (3R,4R)-4-[3-(R,S)-hydroxy-3-(6-méthoxyquinolin-4-yl)propyl]-1-[3-(thiazol-2-yl) thiopropyl]pipéridine-3-acétique
Acide (3R,4R)-4-[3-(R,S)-hydroxy-3-(6-méthoxyquinolin-4-yl)propyl]-1-[2-(1-méthylimidazol-2-yl)thioéthyl]pipéridine-3-acétique
Acide (3R,4R)-4-[3-(R,S)-hydroxy-3-(6-méthoxyquinolin-4-yl)propyl]-1-[3-(1-méthylimidazol-2-yl)thiopropyl]pipéridine-3-acétique
Acide (3R,4R)-4-[3-(R,S)-hydroxy-3-(6-méthoxyquinolin-4-yl)propyl]-1-[2-(3-méthylimidazol-4-yl)thioéthyl]pipéridine-3-acétique
Acide (3R,4R)-4-[3-(R,S)-hydroxy-3-(6-méthoxyquinolin-4-yl)propyl]-1-[3-(3-méthylimidazol-4-yl)thiopropyl]pipéridine-3-acétique
Acide (3R,4R)-4-[3-(R,S)-hydroxy-3-(6-méthoxyquinolin-4-yl)propyl]-1-[2-(3-méthylpyrazol-4-yl)thioéthyl]pipéridine-3-acétique
Acide (3R,4R)-4-[3-(R,S)-hydroxy-3-(6-méthoxyquinolin-4-yl)propyl]-1-[3-(3-méthylpyrazol-4-yl)thiopropyl]pipéridine-3-acétique
Acide (3R,4R)-4-[3-(R,S)-hydroxy-3-(6-méthoxyquinolin-4-yl)propyl]-1-[2-(oxazol-2-yl)thioéthyl]pipéridine-3-acétique
Acide (3R,4R)-4-[3-(R,S)-hydroxy-3-(6-méthoxyquinolin-4-yl)propyl]-1-[3-(oxazol-2-yl)thiopropyl]pipéridine-3-acétique
Acide (3R,4R)-4-[3-(R,S)-hydroxy-3-(6-méthoxyquinolin-4-yl)propyl]-1-[2-(pyridin-2-yl) thioéthyl]pipéridine-3-acétique
Acide (3R,4R)-4-[3-(R,S)-hydroxy-3-(6-méthoxyquinolin-4-yl)propyl]-1-[3-(pyridin-2-yl) thiopropyl]pipéridine-3-acétique
Acide (3R,4R)-4-[3-(R,S)-hydroxy-3-(6-méthoxyquinolin-4-yl)propyl]-1-[2-(pyridin-3-yl) thioéthyl]pipéridine-3-acétique
Acide (3R,4R)-4-[3-(R,S)-hydroxy-3-(6-méthoxyquinolin-4-yl)propyl]-1-[3-(pyridin-3-yl) thiopropyl]pipéridine-3-acétique
Acide (3R,4R)-4-[3-(R,S)-hydroxy-3-(6-méthoxyquinolin-4-yl)propyl]-1-[2-(pyridin-4-yl) thioéthyl]pipéridine-3-acétique
Acide (3R,4R)-4-[3-(R,S)-hydroxy-3-(6-méthoxyquinolin-4-yl)propyl]-1-[3-(pyridin-4-yl) thiopropyl]pipéridine-3-acétique
Acide (3R,4R)-4-[3-(R,S)-hydroxy-3-(6-méthoxyquinolin-4-yl)propyl]-1-[2-(pyrimidin-2-yl) thioéthyl]pipéridine-3-acétique
Acide (3R,4R)-4-[3-(R,S)-hydroxy-3-(6-méthoxyquinolin-4-yl)propyl]-1-[3-(pyrimidin-2-yl) thiopropyl]pipéridine-3-acétique
Acide (3R,4R)-4-[3-(R,S)-hydroxy-3-(6-méthoxyquinolin-4-yl)propyl]-1-[2-(pyrimidin-4-yl) thioéthyl]pipéridine-3-acétique
Acide (3R,4R)-4-[3-(R,S)-hydroxy-3-(6-méthoxyquinolin-4-yl)propyl]-1-[3-(pyrimidin-4-yl) thiopropyl]pipéridine-3-acétique
Acide (3R,4R)-4-[3-(R,S)-hydroxy-3-(6-méthoxyquinolin-4-yl)propyl]-1-[2-(pyrimidin-5-yl) thioéthyl]pipéridine-3-acétique
Acide (3R,4R)-4-[3-(R,S)-hydroxy-3-(6-méthoxyquinolin-4-yl)propyl]-1-[3-(pyrimidin-5-yl) thiopropyl]pipéridine-3-acétique
Acide (3R,4R)-4-[3-(R,S)-hydroxy-3-(6-méthoxyquinolin-4-yl]propyl]-1-[2-(pyrazin-2-yl) thioéthyl]pipéridine-3-acétique
Acide (3R,4R)-4-[3-(R,S)-hydroxy-3-(6-méthoxyquinolin-4-yl)propyl]-1-[3-(pyrazin-2-yl) thiopropyl]pipéridine-3-acétique
Acide (3R,4R)-4-[3-(R,S)-hydroxy-3-(6-méthoxyquinolin-4-yl)propyl]-1-[2-(pyridazin-3-yl) thioéthyl]pipéridine-3-acétique
Acide (3R,4R)-4-[3-(R,S)-hydroxy-3-(6-méthoxyquinolin-4-yl)propyl]-1-[3-(pyridazin-3-yl) thiopropyl]pipéridine-3-acétique
Acide (3R,4R)-4-[3-(R,S)-hydroxy-3-(6-méthoxyquinolin-4-yl)propyl]-1-[2-(pyridazin-4-yl) thioéthyl]pipéridine-3-acétique
Acide (3R,4R)-4-[3-(R,S)-hydroxy-3-(6-méthoxyquinolin-4-yl)propyl]-1-[3-(pyridazin-4-yl) thiopropyl]pipéridine-3-acétique
Acide (3R,4R)-4-[3-(R,S)-fluoro-3-(6-méthoxyquinolin-4-yl)propyl]-1-[2-phénylthio-éthyl] pipéridine-3-acétique
Acide (3R,4R)-4-[3-(R,S)-fluoro-3-(6-méthoxyquinolin-4-yl)propyl]-1-[3-phénylthiopropyl] pipéridine-3-acétique
Acide (3R,4R)-4-[3-(R,S)-fluoro-3-(6-méthoxyquinolin-4-yl)propyl]-1-[2-(2-fluorophénylthio)éthyl]pipéridine-3-acétique
Acide (3R,4R)-4-[3-(R,S)-fluoro-3-(6-méthoxyquinolin-4-yl)propyl]-1-[3-(2-fluorophénylthio)propyl]pipéridine-3-acétique
Acide (3R,4R)-4-[3-(R,S)-fluoro-3-(6-méthoxyquinolin-4-yl)propyl]-1-[2-(3-fluorophénylthio)éthyl]pipéridine-3-acétique
Acide (3R,4R)-4-[3-(R,S)-fluoro-3-(6-méthoxyquinolin-4-yl)propyl]-1-[3-(3-fluorophénylthio)propyl]pipéridine-3-acétique
Acide (3R,4R)-4-[3-(R,S)-fluoro-3-(6-méthoxyquinolin-4-yl)propyl]-1-[2-(4-fluorophénylthio)éthyl]pipéridine-3-acétique
Acide (3R,4R)-4-[3-(R,S)-fluoro-3-(6-méthoxyquinolin-4-yl)propyl]-1-[3-(4-fluorophénylthio)propyl]pipéridine-3-acétique
Acide (3R,4R)-4-[3-(R,S)-fluoro-3-(6-méthoxyquinolin-4-yl)propyl]-1-[2-(2,3-difluorophénylthio)éthyl]pipéridine-3-acétique
Acide (3R,4R)-4-[3-(R,S)-fluoro-3-(6-méthoxyquinolin-4-yl)propyl]-1-[3-(2,3-difluorophénylthio)propyl]pipéridine-3-acétique
Acide (3R,4R)-4-[3-(R,S)-fluoro-3-(6-méthoxyquinolin-4-yl)propyl]-1-[2-(2,6-difluorophénylthio)éthyl]pipéridine-3-acétique
Acide (3R,4R)-4-[3-(R,S)-fluoro-3-(6-méthoxyquinolin-4-yl)propyl]-1-[3-(2,6-difluorophénylthio)propyl]pipéridine-3-acétique
Acide (3R,4R)-4-[3-(R,S)-fluoro-3-(6-méthoxyquinolin-4-yl)propyl]-1-[2-(2-chlorophénylthio)éthyl]pipéridine-3-acétique
Acide (3R,4R)-4-[3-(R,S)-fluoro-3-(6-méthoxyquinolin-4-yl)propyl]-1-[3-(2-chlorophénylthio)propyl]pipéridine-3-acétique
Acide (3R,4R)-4-[3-(R,S)-fluoro-3-(6-méthoxyquinolin-4-yl)propyl]-1-[2-(3-chlorophénylthio)éthyl]pipéridine-3-acétique
Acide (3R,4R)-4-[3-(R,S)-fluoro-3-(6-méthoxyquinolin-4-yl)propyl]-1-[3-(3-chlorophénylthio)propyl]pipéridine-3-acétique
Acide (3R,4R)-4-[3-(R,S)-fluoro-3-(6-méthoxyquinolin-4-yl)propyl]-1-[2-(4-chlorophénylthio)éthyl]pipéridine-3-acétique
Acide (3R,4R)-4-[3-(R,S)-fluoro-3-(6-méthoxyquinolin-4-yl)propyl]-1-[3-(4-chlorophénylthio)propyl]pipéridine-3-acétique
Acide (3R,4R)-4-[3-(R,S)-fluoro-3-(6-méthoxyquinolin-4-yl)propyl]-1-[2-(2,3-dichlorophénylthio)éthyl]pipéridine-3-acétique
Acide (3R,4R)-4-[3-(R,S)-fluoro-3-(6-méthoxyquinolin-4-yl)propyl]-1-[3-(2,3-dichlorophénylthio)propyl]pipéridine-3-acétique
Acide (3R,4R)-4-[3-(R,S)-fluoro-3-(6-méthoxyquinolin-4-yl)propyl]-1-[2-(2,6-dichlorophénylthio)éthyl]pipéridine-3-acétique
Acide (3R,4R)-4-[3-(R,S)-fluoro-3-(6-méthoxyquinolin-4-yl)propyl]-1-[3-(2,6-dichlorophénylthio)propyl]pipéridine-3-acétique
Acide (3R,4R)-4-[3-(R,S)-fluoro-3-(6-méthoxyquinolin-4-yl)propyl]-1-[2-(2-méthylphénylthio)éthyl]pipéridine-3-acétique
Acide (3R,4R)-4-[3-(R,S)-fluoro-3-(6-méthoxyquinolin-4-yl)propyl]-1-[3-(2-méthylphénylthio)propyl]pipéridine-3-acétique
Acide (3R,4R)-4-[3-(R,S)-fluoro-3-(6-méthoxyquinolin-4-yl)propyl]-1-[2-(3-méthylphénylthio)éthyl]pipéridine-3-acétique
Acide (3R,4R)-4-[3-(R,S)-fluoro-3-(6-méthoxyquinolin-4-yl)propyl]-1-[3-(3-méthylphénylthio)propyl]pipéridine-3-acétique
Acide (3R,4R)-4-[3-(R,S)-fluoro-3-(6-méthoxyquinolin-4-yl)propyl]-1-[2-(4-méthylphénylthio)éthyl]pipéridine-3-acétique
Acide (3R,4R)-4-[3-(R,S)-fluoro-3-(6-méthoxyquinolin-4-yl)propyl]-1-[3-(4-méthylphénylthio)propyl]pipéridine-3-acétique
Acide (3R,4R)-4-[3-(R,S)-fluoro-3-(6-méthoxyquinolin-4-yl)propyl]-1-[2-(2-trifluorométhyl-phénylthio)éthyl]pipéridine-3-acétique
Acide (3R,4R)-4-[3-(R,S)-fluoro-3-(6-méthoxyquinolin-4-yl)propyl]-1-[3-(2-triflurométhylphénylthio)propyl]pipéridine-3-acétique
Acide (3R,4R)-4-[3-(R,S)-fluoro-3-(6-méthoxyquinolin-4-yl)propyl]-1-[2-(3-trifluorométhyl-phénylthio)éthyl]pipéridine-3-acétique
Acide (3R,4R)-4-[3-(R,S)-fluoro-3-(6-méthoxyquinolin-4-yl)propyl]-1-[3-(3-triflurométhylphénylthio)propyl]pipéridine-3-acétique
Acide (3R,4R)-4-[3-(R,S)-fluoro-3-(6-méthoxyquinolin-4-yl)propyl]-1-[2-(4-trifluorométhyl-phénylthio)éthyl]pipéridine-3-acétique
Acide (3R,4R)-4-[3-(R,S)-fluoro-3-(6-méthoxyquinolin-4-yl)propyl]-1-[3-(4-triflurométhylphénylthio)propyl]pipéridine-3-acétique
Acide (3R,4R)-4-[3-(R,S)-fluoro-3-(6-méthoxyquinolin-4-yl)propyl]-1-[2-(2-méthoxyphényl-thio)éthyl]pipéridine-3-acétique
Acide (3R,4R)-4-[3-(R,S)-fluoro-3-(6-méthoxyquinolin-4-yl)propyl]-1-[3-(2-méthoxyphényl-thio)propyl]pipéridine-3-acétique
Acide (3R,4R)-4-[3-(R,S)-fluoro-3-(6-méthoxyquinolin-4-yl)propyl]-1-[2-(3-méthoxyphényl-thio)éthyl]pipéridine-3-acétique
Acide (3R,4R)-4-[3-(R,S)-fluoro-3-(6-méthoxyquinolin-4-yl)propyl]-1-[3-(3-méthoxyphényl-thio)propyl]pipéridine-3-acétique
Acide (3R,4R)-4-[3-(R,S)-fluoro-3-(6-méthoxyquinolin-4-yl)propyl]-1-[2-(4-méthoxyphényl-thio)éthyl]pipéridine-3-acétique
Acide (3R,4R)-4-[3-(R,S)-fluoro-3-(6-méthoxyquinolin-4-yl)propyl]-1-[3-(4-méthoxyphényl-thio)propyl]pipéndine-3-acétique
Acide (3R,4R)-4-[3-(R,S)-fluoro-3-(6-méthoxyquinolin-4-yl)propyl]-1-[2-(cyclopropylthio) éthyl]pipéridine-3-acétique
Acide (3R,4R)-4-[3-(R,S)-fluoro-3-(6-méthoxyquinolin-4-yl)propyl]-1-[3-(cyclopropylthio) propyl]pipéridine-3-acétique
Acide (3R,4R)-4-[3-(R,S)-fluoro-3-(6-méthoxyquinolin-4-yl)propyl]-1-[2-(cyclobutylthio) éthyl]pipéridine-3-acétique
Acide (3R,4R)-4-[3-(R,S)-fluoro-3-(6-méthoxyquinolin-4-yl)propyl]-1-[3-(cyclobutylthiopropyl]pipéridine-3-acétique
Acide (3R,4R)-4-[3-(R,S)-fluoro-3-(6-méthoxyquinolin-4-yl)propyl]-1-[2-(cyclopentylthio) éthyl]pipéridine-3-acétique
Acide (3R,4R)-4-[3-(R,S)-fluoro-3-(6-méthoxyquinolin-4-yl)propyl]-1-[3-(cyclopentylthio) propyl]pipéridine-3-acétique
Acide (3R,4R)-4-[3-(R,S)-fluoro-3-(6-méthoxyquinolin-4-yl)propyl]-1-[2-(cyclohexylthio) éthyl]pipéridine-3-acétique
Acide (3R,4R)-4-[3-(R,S)-fluoro-3-(6-méthoxyquinolin-4-yl)propyl]-1-[3-(cyclohexylthio) propyl]pipéridine-3-acétique
Acide (3R,4R)-4-[3-(R,S)-fluoro-3-(6-méthoxyquinolin-4-yl)propyl]-1-[2-(thien-2-yl)thioéthyl]pipéridine-3-acétique
Acide (3R,4R)-4-[3-(R,S)-fluoro-3-(6-méthoxyquinolin-4-yl)propyl]-1-[3-(thien-2-yl)thiopropyl]pipéridine-3-acétique
Acide (3R,4R)-4-[3-(R,S)-fluoro-3-(6-méthoxyquinolin-4-yl)propyl]-1-[2-(5-chloro-thien-2-yl)thioéthyl]pipéridine-3-acétique
Acide (3R,4R)-4-[3-(R,S)-fluoro-3-(6-méthoxyquinolin-4-yl)propyl]-1-[3-(5-chloro-thien-2-yl)thiopropyl]pipéridine-3-acétique
Acide (3R,4R)-4-[3-(R,S)-fluoro-3-(6-méthoxyquinolin-4-yl)propyl]-1-[2-(3-chloro-thien-2-yl)thioéthyl]pipéridine-3-acétique
Acide (3R,4R)-4-[3-(R,S)-fluoro-3-(6-méthoxyquinolin-4-yl)propyl]-1-[3-(3-chloro-thien-2-yl)thiopropyl]pipéridine-3-acétique
Acide (3R,4R)-4-[3-(R,S)-fluoro-3-(6-méthoxyquinolin-4-yl)propyl]-1-[2-(5-méthyl-thien-2-yl)thioéthyl]pipéridine-3-acétique
Acide (3R,4R)-4-[3-(R,S)-fluoro-3-(6-méthoxyquinolin-4-yl)propyl]-1-[3-(5-méthyl-thien-2-yl)thiopropyl]pipéridine-3-acétique
Acide (3R,4R)-4-[3-(R,S)-fluoro-3-(6-méthoxyquinolin-4-yl)propyl]-1-[2-(3-méthyl-thien-2-yl)thioéthyl]pipéridine-3-acétique
Acide (3R,4R)-4-[3-(R,S)-fluoro-3-(6-méthoxyquinolin-4-yl)propyl]-1-[3-(3-méthyl-thien-2-yl)thiopropyl]pipéridine-3-acétique
Acide (3R,4R)-4-[3-(R,S)-fluoro-3-(6-méthoxyquinolin-4-yl)propyl]-1-[2-(thien-3-yl)thioéthyl]pipéridine-3-acétique
Acide (3R,4R)-4-[3-(R,S)-fluoro-3-(6-méthoxyquinolin-4-yl)propyl]-1-[3-(thien-3-yl)thiopropyl]pipéridine-3-acétique
Acide (3R,4R)-4-[3-(R,S)-fluoro-3-(6-méthoxyquinolin-4-yl)propyl]-1-[2-(fur-2-yl)thioéthyl] pipéridine-3-acétique
Acide (3R,4R)-4-[3-(R,S)-fluoro-3-(6-méthoxyquinolin-4-yl)propyl]-1-[3-(fur-2-yl)thiopropyl]pipéridine-3-acétique
Acide (3R,4R)-4-[3-(R,S)-fluoro-3-(6-méthoxyquinolin-4-yl)propyl]-1-[2-(fur-3-yl)thioéthyl] pipéridine-3-acétique
Acide (3R,4R)-4-[3-(R,S)-fluoro-3-(6-méthoxyquinolin-4-yl)propyl]-1-[3-(fur-3-yl)thiopropyl]pipéridine-3-acétique
Acide (3R,4R)-4-[3-(R,S)-fluoro-3-(6-méthoxyquinolin-4-yl)propyl]-1-[2-(1-méthyl-pyrrol-2-yl)thioéthyl]pipéridine-3-acétique
Acide (3R,4R)-4-[3-(R,S)-fluoro-3-(6-méthoxyquinolin-4-yl)propyl]-1-[3-(1-méthyl-pyrrol-2-yl)thiopropyl]pipéridine-3-acétique
Acide (3R,4R)-4-[3-(R,S)-fluoro-3-(6-méthoxyquinolin-4-yl)propyl]-1-[2-(1-méthyl-pyrrol-3-yl)thioéthyl]pipéridine-3-acétique
Acide (3R,4R)-4-[3-(R,S)-fluoro-3-(6-méthoxyquinolin-4-yl)propyl]-1-[3-(1-méthyl-pyrrol-3-yl)thiopropyl]pipéridine-3-acétique
Acide (3R,4R)-4-[3-(R,S)-fluoro-3-(6-méthoxyquinolin-4-yl)propyl]-1-[2-(thiazol-2-yl)thioéthyl]pipéridine-3-acétique
Acide (3R,4R)-4-[3-(R,S)-fluoro-3-(6-méthoxyquinolin-4-yl)propyl]-1-[3-(thiazol-2-yl)thiopropyl]pipéridine-3-acétique
Acide (3R,4R)-4-[3-(R,S)-fluoro-3-(6-méthoxyquinolin-4-yl)propyl]-1-[2-(1-méthylimidazol-2-yl)thioéthyl]pipéridine-3-acétique
Acide (3R,4R)-4-[3-(R,S)-fluoro-3-(6-méthoxyquinolin-4-yl)propyl]-1-[3-(1-méthylimidazol-2-yl)thiopropyl]pipéridine-3-acétique
Acide (3R,4R)-4-[3-(R,S)-fluoro-3-(6-méthoxyquinolin-4-yl)propyl]-1-[2-(3-méthylimidazol-4-yl)thioéthyl]pipéridine-3-acétique
Acide (3R,4R)-4-[3-(R,S)-fluoro-3-(6-méthoxyquinolin-4-yl)propyl]-1-[3-(3-méthylimidazol-4-yl)thiopropyl]pipéridine-3-acétique
Acide (3R,4R)-4-[3-(R,S)-fluoro-3-(6-méthoxyquinolin-4-yl)propyl]-1-[2-(3-méthylpyrazol-4-yl)thioéthyl]pipéridine-3-acétique
Acide (3R,4R)-4-[3-(R,S)-fluoro-3-(6-méthoxyquinolin-4-yl)propyl]-1-[3-(3-méthylpyrazol-4-yl)thiopropyl]pipéridine-3-acétique
Acide (3R,4R)-4-[3-(R,S)-fluoro-3-(6-méthoxyquinolin-4-yl)propyl]-1-[2-(oxazol-2-yl) thioéthyl]pipéridine-3-acétique
Acide (3R,4R)-4-[3-(R,S)-fluoro-3-(6-méthoxyquinolin-4-yl)propyl]-1-[3-(oxazol-2-yl) thiopropyl]pipéridine-3-acétique
Acide (3R,4R)-4-[3-(R,S)-fluoro-3-(6-méthoxyquinolin-4-yl)propyl]-1-[2-(pyridin-2-yl) thioéthyl]pipéridine-3-acétique
Acide (3R,4R)-4-[3-(R,S)-fluoro-3-(6-méthoxyquinolin-4-yl)propyl]-1-[3-(pyridin-2-yl) thiopropyl]pipéridine-3-acétique
Acide (3R,4R)-4-[3-(R,S)-fluoro-3-(6-méthoxyquinolin-4-yl)propyl]-1-[2-(pyridin-3-yl) thioéthyl]pipéridine-3-acétique
Acide (3R,4R)-4-[3-(R,S)-fluoro-3-(6-méthoxyquinolin-4-yl)propyl]-1-[3-(pyridin-3-yl) thiopropyl]pipéridine-3-acétique
Acide (3R,4R)-4-[3-(R,S)-fluoro-3-(6-méthoxyquinolin-4-yl)propyl]-1-[2-(pyridin-4-yl) thioéthyl]pipéridine-3-acétique
Acide (3R,4R)-4-[3-(R,S)-fluoro-3-(6-méthoxyquinolin-4-yl)propyl]-1-[3-(pyridin-4-yl) thiopropyl]pipéridine-3-acétique
Acide (3R,4R)-4-[3-(R,S)-fluoro-3-(6-méthoxyquinolin-4-yl)propyl]-1-[2-(pyrimidin-2-yl) thioéthyl]pipéridine-3-acétique
Acide (3R,4R)-4-[3-(R,S)-fluoro-3-(6-méthoxyquinolin-4-yl)propyl]-1-[3-(pyrimidin-2-yl) thiopropyl]pipéridine-3-acétique
Acide (3R,4R)-4-[3-(R,S)-fluoro-3-(6-méthoxyquinolin-4-yl)propyl]-1-[2-(pyrimidin-4-yl) thioéthyl]pipéridine-3-acétique
Acide (3R,4R)-4-[3-(R,S)-fluoro-3-(6-méthoxyquinolin-4-yl)propyl]-1-[3-(pyrimidin-4-yl) thiopropyl]pipéridine-3-acétique
Acide (3R,4R)-4-[3-(R,S)-fluoro-3-(6-méthoxyquinolin-4-yl)propyl]-1-[2-(pyrimidin-5-yl) thioéthyl]pipéridine-3-acétique
Acide (3R,4R)-4-[3-(R,S)-fluoro-3-(6-méthoxyquinolin-4-yl)propyl]-1-[3-(pyrimidin-5-yl) thiopropyl]pipéridine-3-acétique
Acide (3R,4R)-4-[3-(R,S)-fluoro-3-(6-méthoxyquinolin-4-yl)propyl]-1-[2-(pyrazin-2-yl) thioéthyl]pipéridine-3-acétique
Acide (3R,4R)-4-[3-(R,S)-fluoro-3-(6-méthoxyquinolin-4-yl)propyl]-1-[3-(pyrazin-2-yl) thiopropyl]pipéridine-3-acétique
Acide (3R,4R)-4-[3-(R,S)-fluoro-3-(6-méthoxyquinolin-4-yl)propyl]-1-[2-(pyridazin-3-yl) thioéthyl]pipéridine-3-acétique
Acide (3R,4R)-4-[3-(R,S)-fluoro-3-(6-méthoxyquinolin-4-yl)propyl]-1-[3-(pyridazin-3-yl) thiopropyl]pipéridine-3-acétique
Acide (3R,4R)-4-[3-(R,S)-fluoro-3-(6-méthoxyquinolin-4-yl)propyl]-1-[2-(pyridazin-4-yl) thioéthyl]pipéridine-3-acétique
Acide (3R,4R)-4-[3-(R,S)-fluoro-3-(6-méthoxyquinolin-4-yl)propyl]-1-[3-(pyridazin-4-yl) thiopropyl]pipéridine-3-acétique
Acide (3R,4R)-3-{4-[3-(6-méthoxyquinolin-4-yl)propyl]-1-[2-phénylthio-éthyl]piperidin-3-yl}propan-1-oique
Acide (3R,4R)-3-{4-[3-(6-méthoxyquinolin-4-yl)propyl]-1-[3-phénylthio-propyl]piperidin-3-yl}propan-1-oique
Acide (3R,4R)-3-{4-[3-(6-méthoxyquinolin-4-yl)propyl]-1-[2-(2-fluorophénylthio)éthyl] piperidin-3-yl}propan-1-oique
Acide (3R,4R)-3-{4-[3-(6-méthoxyquinolin-4-yl)propyl]-1-[3-(2-fluorophénylthio)propyl] piperidin-3-yl}propan-1-oique
Acide (3R,4R)-3-{4-[3-(6-méthoxyquinolin-4-yl)propyl]-1-[2-(3-fluorophénylthio)éthyl] piperidin-3-yl}propan-1-oique
Acide (3R,4R)-3-{4-[3-(6-méthoxyquinolin-4-yl)propyl]-1-[3-(3-fluorophénylthio)propyl] piperidin-3-yl}propan-1-oique
Acide (3R,4R)-3-{4-[3-(6-méthoxyquinolin-4-yl)propyl]-1-[2-(4-fluorophénylthio)éthyl] piperidin-3-yl}propan-1-oique
Acide (3R,4R)-3-{4-[3-(6-méthoxyquinolin-4-yl)propyl]-1-[3-(4-fluorophénylthio)propyl] piperidin-3-yl}propan-1-oique
Acide (3R,4R)-3-{4-[3-(6-méthoxyquinolin-4-yl)propyl]-1-[2-(2,3-difluorophénylthio)éthyl] piperidin-3-yl}propan-1-oique
Acide (3R,4R)-3-{4-[3-(6-méthoxyquinolin-4-yl)propyl]-1-[3-(2,3-difluorophénylthio)propyl] piperidin-3-yl}propan-1-oique
Acide (3R,4R)-3-{4-[3-(6-méthoxyquinolin-4-yl)propyl]-1-[2-(2,6-difluorophénylthio)éthyl] piperidin-3-yl}propan-1-oique
Acide (3R,4R)-3-{4-[3-(6-méthoxyquinolin-4-yl)propyl]-1-[3-(2,6-difluorophénylthio)propyl] piperidin-3-yl}propan-1-oique
Acide (3R,4R)-3-{4-[3-(6-méthoxyquinolin-4-yl)propyl]-1-[2-(2-chlorophénylthio)éthyl] piperidin-3-yl}propan-1-oique
Acide (3R,4R)-3-{4-[3-(6-méthoxyquinolin-4-yl)propyl]-1-[3-(2-chlorophénylthio)propyl] piperidin-3-yl}propan-1-oique
Acide (3R,4R)-3-{4-[3-(6-méthoxyquinolin-4-yl)propyl]-1-[2-(3-chlorophénylthio)éthyl] piperidin-3-yl}propan-1-oique
Acide (3R,4R)-3-{4-[3-(6-méthoxyquinolin-4-yl)propyl]-1-[3-(3-chlorophénylthio)propyl] piperidin-3-yl}propan-1-oique
Acide (3R,4R)-3-{4-[3-(6-méthoxyquinolin-4-yl)propyl]-1-[2-(4-chlorophénylthio)éthyl] piperidin-3-yl}propan-1-oique
Acide (3R,4R)-3-{4-[3-(6-méthoxyquinolin-4-yl)propyl]-1-[3-(4-chlorophénylthio)propyl] piperidin-3-yl}propan-1-oique
Acide (3R,4R)-3-{4-[3-(6-méthoxyquinolin-4-yl)propyl]-1-[2-(2,3-dichlorophénylthio)éthyl] piperidin-3-yl}propan-1-oique
Acide (3R,4R)-3-{4-[3-(6-méthoxyquinolin-4-yl)propyl]-1-[3-(2,3-dichlorophénylthio)propyl] piperidin-3-yl}propan-1-oique
Acide (3R,4R)-3-{4-[3-(6-méthoxyquinolin-4-yl)propyl]-1-[2-(2,6-dichlorophénylthio)éthyl] piperidin-3-yl}propan-1-oique
Acide (3R,4R)-3-{4-[3-(6-méthoxyquinolin-4-yl)propyl]-1-[3-(2,6-dichlorophénylthio)propyl] piperidin-3-yl}propan-1-oique
Acide (3R,4R)-3-{4-[3-(6-méthoxyquinolin-4-yl)propyl]-1-[2-(2-méthylphénylthio)éthyl] piperidin-3-yl}propan-1-oique
Acide (3R,4R)-3-{4-[3-(6-méthoxyquinolin-4-yl)propyl]-1-[3-(2-méthylphénylthio)propyl] piperidin-3-yl}propan-1-oique
Acide (3R,4R)-3-{4-[3-(6-méthoxyquinolin-4-yl)propyl]-1-[2-(3-méthylphénylthio)éthyl] piperidin-3-yl}propan-1-oique
Acide (3R,4R)-3-{4-[3-(6-méthoxyquinolin-4-yl)propyl]-1-[3-(3-méthylphénylthio)propyl] piperidin-3-yl}propan-1-oique
Acide (3R,4R)-3-{4-[3-(6-méthoxyquinolin-4-yl)propyl]-1-[2-(4-méthylphénylthio)éthyl] piperidin-3-yl}propan-1-oique
Acide (3R,4R)-3-{4-[3-(6-méthoxyquinolin-4-yl)propyl]-1-[3-(4-méthylphénylthio)propyl] piperidin-3-yl}propan-1-oique
Acide (3R,4R)-3-{4-[3-(6-méthoxyquinolin-4-yl)propyl]-1-[2-(2-trifluorométhylphénylthio) éthyl]piperidin-3-yl}propan-1-oique
Acide (3R,4R)-3-{4-[3-(6-méthoxyquinolin-4-yl)propyl]-1-[3-(2-triflurométhylphénylthio) propyl]piperidin-3-yl}propan-1-oique
Acide (3R,4R)-3-{4-[3-(6-méthoxyquinolin-4-yl)propyl]-1-[2-(3-trifluorométhylphénylthio) éthyl]piperidin-3-yl}propan-1-oique
Acide (3R,4R)-3-{4-[3-(6-méthoxyquinolin-4-yl)propyl]-1-[3-(3-triflurométhylphénylthio) propyl]piperidin-3-yl}propan-1-oique
Acide (3R,4R)-3-{4-[3-(6-méthoxyquinolin-4-yl)propyl]-1-[2-(4-trifluorométhylphénylthio) éthyl]piperidin-3-yl}propan-1-oique
Acide (3R,4R)-3-{4-[3-(6-méthoxyquinolin-4-yl)propyl]-1-[3-(4-triflurométhylphénylthio) propyl]piperidin-3-yl}propan-1-oique
Acide (3R,4R)-3-{4-[3-(6-méthoxyquinolin-4-yl)propyl]-1-[2-(2-méthoxyphénylthio)éthyl] piperidin-3-yl}propan-1-oique
Acide (3R,4R)-3-{4-[3-(6-méthoxyquinolin-4-yl)propyl]-1-[3-(2-méthoxyphénylthio)propyl] piperidin-3-yl}propan-1-oique
Acide (3R,4R)-3-{4-[3-(6-méthoxyquinolin-4-yl)propyl]-1-[2-(3-méthoxyphénylthio)éthyl] piperidin-3-yl}propan-1-oique
Acide (3R,4R)-3-{4-[3-(6-méthoxyquinolin-4-yl)propyl]-1-[3-(3-méthoxyphénylthio)propyl] piperidin-3-yl}propan-1-oique
Acide (3R,4R)-3-{4-[3-(6-méthoxyquinolin-4-yl)propyl]-1-[2-(4-méthoxyphénylthio)éthyl] piperidin-3-yl}propan-1-oique
Acide (3R,4R)-3-{4-[3-(6-méthoxyquinolin-4-yl)propyl]-1-[3-(4-méthoxyphénylthio)propyl] piperidin-3-yl}propan-1-oique
Acide (3R,4R)-3-{4-[3-(6-méthoxyquinolin-4-yl)propyl]-1-[2-(cyclopropylthio)éthyl] piperidin-3-yl}propan-1-oique
Acide (3R,4R)-3-{4-[3-(6-méthoxyquinolin-4-yl)propyl]-1-[3-(cyclopropylthio)propyl] piperidin-3-yl}propan-1-oique
Acide (3R,4R)-3-{4-[3-(6-méthoxyquinolin-4-yl)propyl]-1-[2-(cyclobutylthio)éthyl]piperidin-3-yl}propan-1-oique
Acide (3R,4R)-3-{4-[3-(6-métlioxyquinolin-4-yl)propyl]-1-[3-(cyclobutylthiopropyl] piperidin-3-yl}propan-1-oique
Acide (3R,4R)-3-{4-[3-(6-méthoxyquinolin-4-yl)propyl]-1-[2-(cyclopentylthio)éthyl] piperidin-3-yl}propan-1-oique
Acide (3R,4R)-3-{4-[3-(6-méthoxyquinolin-4-yl)propyl]-1-[3-(cyclopentylthio)propyl] piperidin-3-yl}propan-1-oique
Acide (3R,4R)-3-{4-[3-(6-méthoxyquinolin-4-yl)propyl]-1-[2-(cyclohexylthio)éthyl]piperidin-3-yl}propan-1-oique
Acide (3R,4R)-3-{4-[3-(6-méthoxyquinolin-4-yl)propyl]-1-[3-(cyclohexylthio)propyl] piperidin-3-yl}propan-1-oique
Acide (3R,4R)-3-{4-[3-(6-méthoxyquinolin-4-yl)propyl]-1-[2-(thien-2-yl)thioéthyl]piperidin-3-yl}propan-1-oique
Acide (3R,4R)-3-{4-[3-(6-méthoxyquinolin-4-yl)propyl]-1-[3-(thien-2-yl)thiopropyl] piperidin-3-yl}propan-1-oique
Acide (3R,4R)-3-{4-[3-(6-méthoxyquinolin-4-yl)propyl]-1-[2-(5-chloro-thien-2-yl)thioéthyl] piperidin-3-yl}propan-1-oique
Acide (3R,4R)-3-{4-[3-(6-méthoxyquinolin-4-yl)propyl]-1-[3-(5-chloro-thien-2-yl)thiopropyl] piperidin-3-yl}propan-1-oique
Acide (3R,4R)-3-{4-[3-(6-méthoxyquinolin-4-yl)propyl]-1-[2-(3-chloro-thien-2-yl)thioéthyl] piperidin-3-yl}propan-1-oique
Acide (3R,4R)-3-{4-[3-(6-méthoxyquinolin-4-yl)propyl]-1-[3-(3-chloro-thien-2-yl)thiopropyl] pipendin-3-yl}propan-1-oique
Acide (3R,4R)-3-{4-[3-(6-méthoxyquinolin-4-yl)propyl]-1-[2-(5-méthyl -thien-2-yl)thioéthyl] piperidin-3-yl}propan-1-oique
Acide (3R,4R)-3-{4-[3-(6-méthoxyquinolin-4-yl)propyl]-1-[3-(5-méthyl-thien-2-yl)thiopropyl]piperidin-3-yl}propan-1-oique
Acide (3R,4R)-3-{4-[3-(6-méthoxyquinolin-4-yl)propyl]-1-[2-(3-méthyl -thien-2-yl)thioéthyl] piperidin-3-yl}propan-1-oique
Acide (3R,4R)-3-{4-[3-(6-méthoxyquinolin-4-yl)propyl]-1-[3-(3-méthyl -thien-2-yl)thiopropyl]piperidin-3-yl}propan-1-oique
Acide (3R,4R)-3-{4-[3-(6-méthoxyquinolin-4-yl)propyl]-1-[2-(thien-3-yl)thioéthyl]piperidin-3-yl}propan-1-oique
Acide (3R,4R)-3-{4-[3-(6-méthoxyquinolin-4-yl)propyl]-1-[3-(thien-3-yl)thiopropyl] piperidin-3-yl}propan-1-oique
Acide (3R,4R)-3-{4-[3-(6-méthoxyquinolin-4-yl)propyl]-1-[2-(fur-2-yl)thioéthyl]piperidin-3-yl}propan-1-oique
Acide (3R,4R)-3-{4-[3-(6-méthoxyquinolin-4-yl)propyl]-1-[3-(fur-2-yl)thiopropyl]piperidin-3-yl}propan-1-oique
Acide (3R,4R)-3-{4-[3-(6-méthoxyquinolin-4-yl)propyl]-1-[2-(fur-3-yl)thioéthyl]piperidin-3-yl}propan-1-oique
Acide (3R,4R)-3-{4-[3-(6-méthoxyquinolin-4-yl)propyl]-1-[fur-3-yl)thiopropyl]piperidin-3-yl}propan-1-oique
Acide (3R,4R)-3-{4-[3-(6-méthoxyquinolin-4-yl)propyl]-1-[2-(1-méthyl-pyrrol-2-yl)thioéthyl]piperidin-3-yl}propan-1-oique
Acide (3R,4R)-3-{4-[3-(6-méthoxyquinolin-4-yl)propyl]-1-[3-(1-méthyl-pyrrol-2-yl)thiopropyl]piperidin-3-yl}propan-1-oique
Acide (3R,4R)-3-{4-[3-(6-méthoxyquinolin-4-yl)propyl]-1-[2-(1-méthyl-pyrrol-3-yl)thioéthyl]piperidin-3-yl}propan-1-oique
Acide (3R,4R)-3-{4-[3-(6-méthoxyquinolin-4-yl)propyl]-1-[3-(1-méthyl-pyrrol-3-yl)thiopropyl]piperidin-3-yl}propan-1-oique
Acide (3R,4R)-3-{4-[3-(6-méthoxyquinolin-4-yl)propyl]-1-[2-(thiazol-2-yl)thioéthyl] piperidin-3-yl}propan-1-oique
Acide (3R,4R)-3-{4-[3-(6-méthoxyquinolin-4-yl)propyl]-1-[3-(thiazol-2-yl)thiopropyl] piperidin-3-yl}propan-1-oique
Acide (3R,4R)-3-{4-[3-(6-méthoxyquinolin-4-yl)propyl]-1-[2-(1-méthyl-imidazol-2-yl)thioéthyl]piperidin-3-yl}propan-1-oique
Acide (3R,4R)-3-{4-[3-(6-méthoxyquinolin-4-yl)propyl]-1-[3-(1-méthyl-imidazol-2-yl)thiopropyl]piperidin-3-yl}propan-1-oique
Acide (3R,4R)-3-{4-[3-(6-méthoxyquinolin-4-yl)propyl]-1-[2-(3-méthyl-imidazol-4-yl) tlxioéthyl]piperidin-3-yl}propan-1-oique
Acide (3R,4R)-3-{4-[3-(6-méthoxyquinolin-4-yl)propyl]-1-[3-(3-méthyl-imidazol-4-yl) thiopropyl]piperidin-3-yl}propan-1-oique
Acide (3R,4R)-3-{4-[3-(6-méthoxyquinolin-4-yl)propyl]-1-[2-(3-méthyl-pyrazol-4-yl) thioéthyl]piperidin-3-yl}propan-1-oique
Acide (3R,4R)-3-{4-[3-(6-méthoxyquinolin-4-yl)propyl]-1-[3-(3-méthyl-pyrazol-4-yl) thiopropyl]piperidin-3-yl}propan-1-oique
Acide (3R,4R)-3-{4-[3-(6-méthoxyquinolin-4-yl)propyl]-1-[2-(oxazol-2-yl)thioéthyl] piperidin-3-yl}propan-1-oique
Acide (3R,4R)-3-{4-[3-(6-méthoxyquinolin-4-yl)propyl]-1-[3-(oxazol-2-yl)thiopropyl] piperidin-3-yl}propan-1-oique
Acide (3R,4R)-3-{4-[3-(6-méthoxyquinolin-4-yl)propyl]-1-[2-(pyridin-2-yl)thioéthyl] piperidin-3-yl}propan-1-oique
Acide (3R,4R)-3-{4-[3-(6-méthoxyquinolin-4-yl)propyl]-1-[3-(pyridin-2-yl)thiopropyl] piperidin-3-yl}propan-1-oique
Acide (3R,4R)-3-{4-[3-(6-méthoxyquinolin-4-yl)propyl]-1-[2-(pyridin-3-yl)thioéthyl] piperidin-3-yl}propan-1-oique
Acide (3R,4R)-3-{4-[3-(6-méthoxyquinolin-4-yl)propyl]-1-[3-(pyridin-3-yl)thiopropyl] piperidin-3-yl}propan-1-oique
Acide (3R,4R)-3-{4-[3-(6-méthoxyquinolin-4-yl)propyl]-1-[2-(pyridin-4-yl)thioéthyl] piperidin-3-yl}propan-1-oique
Acide (3R,4R)-3-{4-[3-(6-méthoxyquinolin-4-yl)propyl]-1-[3-(pyridin-4-yl)thiopropyl] piperidin-3-yl}propan-1-oique
Acide (3R,4R)-3-{4-[3-(6-méthoxyquinolin-4-yl)propyl]-1-[2-(pyrimidin-2-yl)thioéthyl] piperidin-3-yl}propan-1-oique
Acide (3R,4R)-3-{4-[3-(6-méthoxyquinolin-4-yl)propyl]-1-[3-(pyrimidin-2-yl)thiopropyl] piperidin-3-yl}propan-1-oique
Acide (3R,4R)-3-{4-[3-(6-méthoxyquinolin-4-yl)propyl]-1-[2-(pyrimidin-4-yl)thioéthyl] piperidin-3-yl}propan-1-oique
Acide (3R,4R)-3-{4-[3-(6-méthoxyquinolin-4-yl)propyl]-1-[3-(pyrimidin-4-yl)thiopropyl] piperidin-3-yl}propan-1-oique
Acide (3R,4R)-3-{4-[3-(6-méthoxyquinolin-4-yl)propyl]-1-[2-(pyrimidin-5-yl)thioéthyl] piperidin-3-yl}propan-1-oique
Acide (3R,4R)-3-{4-[3-(6-méthoxyquinolin-4-yl)propyl]-1-[3-(pyrimidin-5-yl)thiopropyl] piperidin-3-yl}propan-1-oique
Acide (3R,4R)-3-{4-[3-(6-méthoxyquinolin-4-yl)propyl]-1-[2-(pyrazin -2-yl)thioéthyl] piperidin-3-yl}propan-1-oique
Acide (3R,4R)-3-{4-[3-(6-méthoxyquinolin-4-yl)propyl]-1-[3-(pyrazin-2-yl)thiopropyl] piperidin-3-yl}propan-1-oique
Acide (3R,4R)-3-{4-[3-(6-méthoxyquinolin-4-yl)propyl]-1-[2-(pyridazin-3-yl)thioéthyl] piperidin-3-yl}propan-1-oique
Acide (3R,4R)-3-{4-[3-(6-méthoxyquinolin-4-yl)propyl]-1-[3-(pyridazin-3-yl)thiopropyl] piperidin-3-yl}propan-1-oique
Acide (3R,4R)-3-{4-[3-(6-méthoxyquinolin-4-yl)propyl]-1-[2-(pyridazin-4-yl)thioéthyl] piperidin-3-yl}propan-1-oique
Acide (3R,4R)-3-{4-[3-(6-méthoxyquinolin-4-yl)propyl]-1-[3-(pyridazin-4-yl)thiopropyl] piperidin-3-yl}propan-1-oique
Acide (3R,4R)-3-{4-[3-(R,S)-hydroxy-3-(6-méthoxyquinolin-4-yl)propyl]-1-[2-phénylthioéthyl]piperidin-3-yl}propan-1-oique
Acide (3R,4R)-3-{4-[3-(R,S)-hydroxy-3-(6-méthoxyquinolin-4-yl)propyl]-1-[3-phénylthiopropyl]piperidin-3-yl}propan-1-oique
Acide (3R,4R)-3-{4-[3-(R,S)-hydroxy-3-(6-méthoxyquinolin-4-yl)propyl]-1-[2-(2-fluorophénylthio)éthyl]piperidin-3-yl}propan-1-oique
Acide (3R,4R)-3-{4-[3-(R,S)-hydroxy-3-(6-méthoxyquinolin-4-yl)propyl]-1-[3-(2-fluorophénylthio)propyl]piperidin-3-yl}propan-1-oique
Acide (3R,4R)-3-{4-[3-(R,S)-hydroxy-3-(6-méthoxyquinolin-4-yl)propyl]-1-[2-(3-fluorophénylthio)éthyl]piperidin-3-yl}propan-1-oique
Acide (3R,4R)-3-{4-[3-(R,S)-hydroxy-3-(6-méthoxyquinolin-4-yl)propyl]-1-[3-(3-fluorophénylthio)propyl]piperidin-3-yl}propan-1-oique
Acide (3R,4R)-3-{4-[3-(R,S)-hydroxy-3-(6-méthoxyquinolin-4-yl)propyl]-1-[2-(4-fluorophénylthio)éthyl]piperidin-3-yl}propan-1-oique
Acide (3R,4R)-3-{4-[3-(R,S)-hydroxy-3-(6-méthoxyquinolin-4-yl)propyl]-1-[3-(4-fluorophénylthio)propyl]piperidin-3-yl}propan-1-oique
Acide (3R,4R)-3-{4-[3-(R,S)-hydroxy-3-(6-méthoxyquinolin-4-yl)propyl]-1-[2-(2,3-difluoro-phénylthio)éthyl]piperidin-3-yl}propan-1-oique
Acide (3R,4R)-3-{4-[3-(R,S)-hydroxy-3-(6-méthoxyquinolin-4-yl)propyl]-1-[3-(2,3-difluoro-phénylthio)propyl]piperidin-3-yl}propan-1-oique
Acide (3R,4R)-3-{4-[3-(R,S)-hydroxy-3-(6-méthoxyquinolin-4-yl)propyl]-1-[2-(2,6-difluoro-phénylthio)éthyl]piperidin-3-yl}propan-1-oique
Acide (3R,4R)-3-{4-[3-(R,S)-hydroxy-3-(6-méthoxyquinolin-4-yl)propyl]-1-[3-(2,6-difluoro-phénylthio)propyl]piperidin-3-yl}propan-1-oique
Acide (3R,4R)-3-{4-[3-(R,S)-hydroxy-3-(6-méthoxyquinolin-4-yl)propyl]-1-[2-(2-chlorophénylthio)éthyl]piperidin-3-yl}propan-1-oique
Acide (3R,4R)-3-{4-[3-(R,S)-hydroxy-3-(6-méthoxyquinolin-4-yl)propyl]-1-[3-(2-chlorophénylthio)propyl]piperidin-3-yl}propan-1-oique
Acide (3R,4R)-3-{4-[3-(R,S)-hydroxy-3-(6-méthoxyquinolin-4-yl)propyl]-1-[2-(3-chlorophénylthio)éthyl]piperidin-3-yl}propan-1-oique
Acide (3R,4R)-3-{4-[3-(R,S)-hydroxy-3-(6-méthoxyquinolin-4-yl)propyl]-1-[3-(3-chlorophénylthio)propyl]piperidin-3-yl}propan-1-oique
Acide (3R,4R)-3-{4-[3-(R,S)-hydroxy-3-(6-méthoxyquinolin-4-yl)propyl]-1-[2-(4-chlorophénylthio)éthyl]piperidin-3-yl}propan-1-oique
Acide (3R,4R)-3-{4-[3-(R,S)-hydroxy-3-(6-méthoxyquinolin-4-yl)propyl]-1-[3-(4-chlorophénylthio)propyl]piperidin-3-yl}propan-1-oique
Acide (3R,4R)-3-{4-[3-(R,S)-hydroxy-3-(6-méthoxyquinolin-4-yl)propyl]-1-[2-(2,3-dichloro-phénylthio)éthyl]piperidin-3-yl}propan-1-oique
Acide (3R,4R)-3-{4-[3-(R,S)-hydroxy-3-(6-méthoxyquinolin-4-yl)propyl]-1-[3-(2,3-dichloro-phénylthio)propyl]piperidin-3-yl}propan-1-oique
Acide (3R,4R)-3-{4-[3-(R,S)-hydroxy-3-(6-méthoxyquinolin-4-yl)propyl]-1-[2-(2,6-dichloro-phénylthio)éthyl]piperidin-3-yl}propan-1-oique
Acide (3R,4R)-3-{4-[3-(R,S)-hydroxy-3-(6-méthoxyquinolin-4-yl)propyl]-1-[3-(2,6-dichloro-phénylthio)propyl]piperidin-3-yl}propan-1-oique
Acide (3R,4R)-3-{4-[3-(R,S)-hydroxy-3-(6-méthoxyquinolin-4-yl)propyl]-1-[2-(2-méthylphénylthio)éthyl]piperidin-3-yl}propan-1-oique
Acide (3R,4R)-3-{4-[3-(R,S)-hydroxy-3-(6-méthoxyquinolin-4-yl)propyl]-1-[3-(2-méthylphénylthio)propyl]piperidin-3-yl}propan-1-oique
Acide (3R,4R)-3-{4-[3-(R,S)-hydroxy-3-(6-méthoxyquinolin-4-yl)propyl]-1-[2-(3-méthylphénylthio)éthyl]piperidin-3-yl}propan-1-oique
Acide (3R,4R)-3-{4-[3-(R,S)-hydroxy-3-(6-méthoxyquinolin-4-yl)propyl]-1-[3-(3-méthylphénylthio)propyl]piperidin-3-yl}propan-1-oique
Acide (3R,4R)-3-{4-[3-(R,S)-hydroxy-3-(6-méthoxyquinolin-4-yl)propyl]-1-[2-(4-méthylphénylthio)éthyl]piperidin-3-yl}propan-1-oique
Acide (3R,4R)-3-{4-[3-(R,S)-hydroxy-3-(6-méthoxyquinolin-4-yl)propyl]-1-[3-(4-méthylphénylthio)propyl]piperidin-3-yl}propan-1-oique
Acide (3R,4R)-3-{4-[3-(R,S)-hydroxy-3-(6-méthoxyquinolin-4-yl)propyl]-1-[2-(2-trifluorométhylphénylthio)éthyl]piperidin-3-yl}propan-1-oique
Acide (3R,4R)-3-{4-[3-(R,S)-hydroxy-3-(6-méthoxyquinolin-4-yl)propyl]-1-[3-(2-triflurométhylphénylthio)propyl]piperidin-3-yl}propan-1-oique
Acide (3R,4R)-3-{4-[3-(R,S)-hydroxy-3-(6-méthoxyquinolin-4-yl)propyl]-1-[2-(3-trifluorométhylphénylthio)éthyl]piperidin-3-yl}propan-1-oique
Acide (3R,4R)-3-{4-[3-(R,S)-hydroxy-3-(6-méthoxyquinolin-4-yl)propyl]-1-[3-(3-triflurométhylphénylthio)propyl]piperidin-3-yl}propan-1-oique
Acide (3R,4R)-3-{4-[3-(R,S)-hydroxy-3-(6-méthoxyquinolin-4-yl)propyl]-1-[2-(4-trifluorométhylphénylthio)éthyl]piperidin-3-yl}propan-1-oique
Acide (3R,4R)-3-{4-[3-(R,S)-hydroxy-3-(6-méthoxyquinolin-4-yl)propyl]-1-[3-(4-triflurométhylphénylthio)propyl]piperidin-3-yl}propan-1-oique
Acide (3R,4R)-3-{4-[3-(R,S)-hydroxy-3-(6-méthoxyquinolin-4-yl)propyl]-1-[2-(2-méthoxyphénylthio)éthyl]piperidin-3-yl}propan-1-oique
Acide (3R,4R)-3-{4-[3-(R,S)-hydroxy-3-(6-méthoxyquinolin-4-yl)propyl]-1-[3-(2-méthoxyphénylthio)propyl]piperidin-3-yl}propan-1-oique
Acide (3R,4R)-3-{4-[3-(R,S)-hydroxy-3-(6-méthoxyquinolin-4-yl)propyl]-1-[2-(3-méthoxyphénylthio)éthyl]piperidin-3-yl}propan-1-oique
Acide (3R,4R)-3-{4-[3-(R,S)-hydroxy-3-(6-méthoxyquinolin-4-yl)propyl]-1-[3-(3-méthoxyphénylthio)propyl]piperidin-3-yl}propan-1-oique
Acide (3R,4R)-3-{4-[3-(R,S)-hydroxy-3-(6-méthoxyquinolin-4-yl)propyl]-1-[2-(4-méthoxyphénylthio)éthyl]piperidin-3-yl}propan-1-oique
Acide (3R,4R)-3-{4-[3-(R,S)-hydroxy-3-(6-méthoxyquinolin-4-yl)propyl]-1-[3-(4-méthoxy phénylthio)propyl]piperidin-3-yl}propan-1-oique
Acide (3R,4R)-3-{4-[3-(R,S)-hydroxy-3-(6-méthoxyquinolin-4-yl)propyl]-1-[2-(cyclopropyl-thio)éthyl]piperidin-3-yl}propan-1-oique
Acide (3R,4R)-3-{4-[3-(R,S)-hydroxy-3-(6-méthoxyquinolin-4-yl)propyl]-1-[3-(cyclopropyl-thio)propyl]piperidin-3-yl}propan-1-oique
Acide (3R,4R)-3-{4-[3-(R,S)-hydroxy-3-(6-méthoxyquinolin-4-yl)propyl]-1-[2-(cyclobutylthio)éthyl]piperidin-3-yl}propan-1-oique
Acide (3R,4R)-3-{4-[3-(R,S)-hydroxy-3-(6-méthoxyquinolin-4-yl)propyl]-1-[3-(cyclobutylthiopropyl]piperidin-3-yl}propan-1-oique
Acide (3R,4R)-3-{4-[3-(R,S)-hydroxy-3-(6-méthoxyquinolin-4-yl)propyl]-1-[2-(cyclopentylthio)éthyl]piperidin-3-yl}propan-1-oique
Acide (3R,4R)-3-{4-[3-(R,S)-hydroxy-3-(6-méthoxyquinolin-4-yl)propyl]-1-[3-(cyclopentylthio)propyl]piperidin-3-yl}propan-1-oique
Acide (3R,4R)-3-{4-[3-(R,S)-hydroxy-3-(6-méthoxyquinolin-4-yl)propyl]-1-[2-(cyclohexylthio)éthyl]piperidin-3-yl}propan-1-oique
Acide (3R,4R)-3-{4-[3-(R,S)-hydroxy-3-(6-méthoxyquinolin-4-yl)propyl]-1-[3-(cyclohexylthio)propyl]piperidin-3-yl}propan-1-oique
Acide (3R,4R)-3-{4-[3-(R,S)-hydroxy-3-(6-méthoxyquinolin-4-yl)propyl]-1-[2-(thien-2-yl) thioéthyl]piperidin-3-yl}propan-1-oique
Acide (3R,4R)-3-{4-[3-(R,S)-hydroxy-3-(6-méthoxyquinolin-4-yl)propyl]-1-[3-(thien-2-yl) thiopropyl)piperidin-3-yl}propan-1-oique
Acide (3R,4R)-3-{4-[3-(R,S)-hydroxy-3-(6-méthoxyquinolin-4-yl)propyl]-1-[2-(5-chlorothien-2-yl)thioéthyl]piperidin-3-yl}propan-1-oique
Acide (3R,4R)-3-{4-[3-(R,S)-hydroxy-3-(6-méthoxyquinolin-4-yl)propyl]-1-[3-(5-chlorothien-2-yl)thiopropyl]piperidin-3-yl}propan-1-oique
Acide (3R,4R)-3-{4-[3-(R,S)-hydroxy-3-(6-méthoxyquinolin-4-yl)propyl]-1-[2-(3-chlorothien-2-yl)thioéthyl]piperidin-3-yl}propan-1-oique
Acide (3R,4R)-3-{4-[3-(R,S)-hydroxy-3-(6-méthoxyquinolin-4-yl)propyl]-1-[3-(3-chlorothien-2-yl)thiopropyl]piperidin-3-yl}propan-1-oique
Acide (3R,4R)-3-{4-[3-(R,S)-hydroxy-3-(6-méthoxyquinolin-4-yl)propyl]-1-[2-(5-méthylthien-2-yl)thioéthyl]piperidin-3-yl}propan-1-oique
Acide (3R,4R)-3-{4-[3-(R,S)-hydroxy-3-(6-méthoxyquinolin-4-yl)propyl]-1-[3-(5-méthylthien-2-yl)thiopropyl]piperidin-3-yl}propan-1-oique
Acide (3R,4R)-3-{4-[3-(R,S)-hydroxy-3-(6-méthoxyquinolin-4-yl)propyl]-1-[2-(3-méthylthien-2-yl)thioéthyl]piperidin-3-yl}propan-1-oique
Acide (3R,4R)-3-{4-[3-(R,S)-hydroxy-3-(6-méthoxyquinolin-4-yl)propyl]-1-[3-(3-méthylthien-2-yl)thiopropyl]piperidin-3-yl}propan-1-oique
Acide (3R,4R)-3-{4-[3-(R,S)-hydroxy-3-(6-méthoxyquinolin-4-yl)propyl]-1-[2-(thien-3-yl)thioéthyl]piperidin-3-yl}propan-1-oique
Acide (3R,4R)-3-{4-(3-(R,S)-hydroxy-3-(6-méthoxyquinolin-4-yl)propyl]-1-[3-(thien-3-yl)thiopropyl]piperidin-3-yl}propan-1-oique
Acide (3R,4R)-3-{4-[3-(R,S)-hydroxy-3-(6-méthoxyquinolin-4-yl)propyl]-1-[2-(fur-2-yl)thioéthyl]piperidin-3-yl}propan-1-oique
Acide (3R,4R)-3-{4-[3-(R,S)-hydroxy-3-(6-méthoxyquinolin-4-yl)propyl]-1-[3-(fur-2-yl)thiopropyl]piperidin-3-yl}propan-1-oique
Acide (3R,4R)-3-{4-[3-(R,S)-hydroxy-3-(6-méthoxyquinolin-4-yl)propyl]-1-[2-(fur-3-yl)thioéthyl]piperidin-3-yl}propan-1-oique
Acide (3R,4R)-3-{4-[3-(R,S)-hydroxy-3-(6-méthoxyquinolin-4-yl)propyl]-1-[3-(fur-3-yl)thiopropyl]piperidin-3-yl}propan-1-oique
Acide (3R,4R)-3-{4-[3-(R,S)-hydroxy-3-(6-méthoxyquinolin-4-yl)propyl]-1-[2-(1-méthylpyrrol-2-yl)thioéthyl]piperidin-3-yl}propan-1-oique
Acide (3R,4R)-3-{4-[3-(R,S)-hydroxy-3-(6-méthoxyquinolin-4-yl)propyl]-1-[3-(1-méthylpyrrol-2-yl)thiopropyl]piperidin-3-yl}propan-1-oique
Acide (3R,4R)-3-{4-[3-(R,S)-hydroxy-3-(6-méthoxyquinolin-4-yl)propyl]-1-[2-(1-méthylpyrrol-3-yl)thioéthyl]piperidin-3-yl}propan-1-oique
Acide (3R,4R)-3-{4-[3-(R,S)-hydroxy-3-(6-méthoxyquinolin-4-yl)propyl]-1-[3-(1-méthylpyrrol-3-yl)thiopropyl]piperidin-3-yl}propan-1-oique
Acide (3R,4R)-3-{4-[3-(R,S)-hydroxy-3-(6-méthoxyquinolin-4-yl)propyl]-1-[2-(thiazol-2-yl)thioéthyl]piperidin-3-yl}propan-1-oique
Acide (3R,4R)-3-{4-[3-(R,S)-hydroxy-3-(6-méthoxyquinolin-4-yl)propyl]-1-[3-(thiazol-2-yl)thiopropyl]piperidin-3-yl}propan-1-oique
Acide (3R,4R)-3-{4-[3-(R,S)-hydroxy-3-(6-méthoxyquinolin-4-yl)propyl]-1-[2-(1-méthylimidazol-2-yl)thioéthyl]piperidin-3-yl}propan-1-oique
Acide (3R,4R)-3-{4-[3-(R,S)-hydroxy-3-(6-méthoxyquinolin-4-yl)propyl]-1-[3-(1-méthylimidazol-2-yl)thiopropyl]piperidin-3-yl}propan-1-oique
Acide (3R,4R)-3-{4-[3-(R,S)-hydroxy-3-(6-méthoxyquinolin-4-yl)propyl]-1-[2-(3-méthylimidazol-4-yl)thioéthyl]pipexidin-3-yl}propan-1-oique
Acide (3R,4R)-3-{4-[3-(R,S)-hydroxy-3-(6-méthoxyquinolin-4-yl)propyl]-1-[3-(3-méthylimidazol-4-yl)thiopropyl]piperidin-3-yl}propan-1-oique
Acide (3R,4R)-3-{4-[3-(R,S)-hydroxy-3-(6-méthoxyquinolin-4-yl)propyl]-1-[2-(3-méthylpyrazol-4-yl)thioéthyl]piperidin-3-yl}propan-1-oique
Acide (3R,4R)-3-{4-[3-(R,S)-hydroxy-3-(6-méthoxyquinolin-4-yl)propyl]-1-[3-(3-méthylpyrazol-4-yl)thiopropyl]piperidin-3-yl}propan-1-oique
Acide (3R,4R)-3-{4-[3-(R,S)-hydroxy-3-(6-méthoxyquinolin-4-yl)propyl]-1-[2-(oxazol-2-yl)thioéthyl]piperidin-3-yl}propan-1-oique
Acide (3R,4R)-3-{4-[3-(R,S)-hydroxy-3-(6-méthoxyquinolin-4-yl)propyl]-1-[3-(oxazol-2-yl)thiopropyl]piperidin-3-yl}propan-1-oique
Acide (3R,4R)-3-{4-[3-(R,S)-hydroxy-3-(6-méthoxyquinolin-4-yl)propyl]-1-[2-(pyridin-2-yl)thioéthyl]piperidin-3-yl}propan-1-oique
Acide (3R,4R)-3-{4-[3-(R,S)-hydroxy-3-(6-méthoxyquinolin-4-yl)propyl]-1-[3-(pyridin-2-yl)thiopropyl]piperidin-3-yl}propan-1-oique
Acide (3R,4R)-3-{4-[3-(R,S)-hydroxy-3-(6-méthoxyquinolin-4-yl)propyl]-1-[2-(pyridin-3-yl)thioéthyl]piperidin-3-yl}propan-1-oique
Acide (3R,4R)-3-{4-[3-(R,S)-hydroxy-3-(6-méthoxyquinolin-4-yl)propyl]-1-[3-(pyridin-3-yl)thiopropyl]piperidin-3-yl}propan-1-oique
Acide (3R,4R)-3-{4-[3-(R,S)-hydroxy-3-(6-méthoxyquinolin-4-yl)propyl]-1-[2-(pyridin-4-yl)thioéthyl]piperidin-3-yl}propan-1-oique
Acide (3R,4R)-3-{4-[3-(R,S)-hydroxy-3-(6-méthoxyquinolin-4-yl)propyl]-1-[3-(pyridin-4-yl)thiopropyl]piperidin-3-yl}propan-1-oique
Acide (3R,4R)-3-{4-[3-(R,S)-hydroxy-3-(6-méthoxyquinolin-4-yl)propyl]-1-[2-(pyrimidin-2-yl)thioéthyl]piperidin-3-yl}propan-1-oique
Acide (3R,4R)-3-{4-[3-(R,S)-hydroxy-3-(6-méthoxyquinolin-4-yl)propyl]-1-[3-(pyrimidin-2-yl)thiopropyl]piperidin-3-yl}propan-1-oique
Acide (3R,4R)-3-{4-[3-(R,S)-hydroxy-3-(6-méthoxyquinolin-4-yl)propyl]-1-[2-(pyrimidin-4-yl)thioéthyl]piperidin-3-yl}propan-1-oique
Acide (3R,4R)-3-{4-[3-(R,S)-hydroxy-3-(6-méthoxyquinolin-4-yl)propyl]-1-[3-(pyrimidin-4-yl)thiopropyl]piperidin-3-yl}propan-1-oique
Acide (3R,4R)-3-{4-[3-(R,S)-hydroxy-3-(6-méthoxyquinolin-4-yl)propyl]-1-[2-(pyrimidin-5-yl)thioéthyl]piperidin-3-yl}propan-1-oique
Acide (3R,4R)-3-{4-[3-(R,S)-hydroxy-3-(6-méthoxyquinolin-4-yl)propyl]-1-[3-(pyrimidin-5-yl)thiopropyl]piperidin-3-yl}propan-1-oique
Acide (3R,4R)-3-{4-[3-(R,S)-hydroxy-3-(6-méthoxyquinolin-4-yl)propyl]-1-[2-(pyrazin-2-yl)thioéthyl]piperidin-3-yl}propan-1-oique
Acide (3R,4R)-3-{4-[3-(R,S)-hydroxy-3-(6-méthoxyquinolin-4-yl)propyl]-1-[3-(pyrazin-2-yl)thiopropyl]piperidin-3-yl}propan-1-oique
Acide (3R,4R)-3-{4-[3-(R,S)-hydroxy-3-(6-méthoxyquinolin-4-yl)propyl]-1-[2-(pyridazin-3-yl)thioéthyl]piperidin-3-yl}propan-1-oique
Acide (3R,4R)-3-{4-[3-(R,S)-hydroxy-3-(6-méthoxyquinolin-4-yl)propyl]-1-[3-(pyridazin-3-yl)thiopropyl]piperidin-3-yl}propan-1-oique
Acide (3R,4R)-3-{4-[3-(R,S)-hydroxy-3-(6-méthoxyquinolin-4-yl)propyl]-1-[2-(pyridazin-4-yl)thioéthyl]piperidin-3-yl}propan-1-oique
Acide (3R,4R)-3-{4-[3-(R,S)-hydroxy-3-(6-méthoxyquinolin-4-yl)propyl]-1-[3-(pyridazin-4-yl)thiopropyl]piperidin-3-yl}propan-1-oique
Acide (3R,4R)-3-{4-[3-(R,S)-fluoro-3-(6-méthoxyquinolin-4-yl)propyl]-1-[2-phénylthioéthyl]piperidin-3-yl}propan-1-oique
Acide (3R,4R)-3-{4-[3-(R,S)-fluoro-3-(6-méthoxyquinolin-4-yl)propyl]-1-[3-phénylthiopropyl]piperidin-3-yl}propan-1-oique
Acide (3R,4R)-3-{4-[3-(R,S)-fluoro-3-(6-méthoxyquinolin-4-yl)propyl]-1-[2-(2-fluorophénylthio)éthyl]piperidin-3-yl}propan-1-oique
Acide (3R,4R)-3-{4-[3-(R,S)-fluoro-3-(6-méthoxyquinolin-4-yl)propyl]-1-[3-(2-fluorophénylthio)propyl]piperidin-3-yl}propan-1-oique
Acide (3R,4R)-3-{4-[3-(R,S)-fluoro-3-(6-méthoxyquinolin-4-yl)propyl]-1-[2-(3-fluorophénylthio)éthyl]piperidin-3-yl}propan-1-oique
Acide (3R,4R)-3-{4-[3-(R,S)-fluoro-3-(6-méthoxyquinolin-4-yl)propyl]-1-[3-(3-fluorophénylthio)propyl]piperidin-3-yl}propan-1-oique
Acide (3R,4R)-3-{4-[3-(R,S)-fluoro-3-(6-méthoxyquinolin-4-yl)propyl]-1-[2-(4-fluorophénylthio)éthyl]piperidin-3-yl}propan-1-oique
Acide (3R,4R)-3-{4-[3-(R,S)-fluoro-3-(6-méthoxyquinolin-4-yl)propyl]-1-[3-(4-fluorophénylthio)propyl]piperidin-3-yl}propan-1-oique
Acide (3R,4R)-3-{4-[3-(R,S)-fluoro-3-(6-méthoxyquinolin-4-yl)propyl]-1-[2-(2,3-difluorophénylthio)éthyl]piperidin-3-yl}propan-1-oique
Acide (3R,4R)-3-{4-[3-(R,S)-fluoro-3-(6-méthoxyquinolin-4-yl)propyl]-1-[3-(2,3-difluorophénylthio)propyl]piperidin-3-yl}propan-1-oique
Acide (3R,4R)-3-{4-[3-(R,S)-fluoro-3-(6-méthoxyquinolin-4-yl)propyl]-1-[2-(2,6-difluorophénylthio)éthyl]piperidin-3-yl}propan-1-oique
Acide (3R,4R)-3-{4-[3-(R,S)-fluoro-3-(6-méthoxyquinolin-4-yl)propyl]-1-[3-(2,6-difluorophénylthio)propyl]piperidin-3-yl}propan-1-oique
Acide (3R,4R)-3-{4-[3-(R,S)-fluoro-3-(6-méthoxyquinolin-4-yl)propyl]-1-[2-(2-chlorophénylthio)éthyl]piperidin-3-yl}propan-1-oique
Acide (3R,4R)-3-{4-[3-(R,S)-fluoro-3-(6-méthoxyquinolin-4-yl)propyl]-1-[3-(2-chlorophénylthio)propyl]piperidin-3-yl}propan-1-oique
Acide (3R,4R)-3-{4-[3-(R,S)-fluoro-3-(6-méthoxyquinolin-4-yl)propyl]-1-[2-(3-chlorophénylthio)éthyl]piperidin-3-yl}propan-1-oique
Acide (3R,4R)-3-{4-[3-(R,S)-fluoro-3-(6-méthoxyquinolin-4-yl)propyl]-1-[3-(3-chlorophénylthio)propyl]piperidin-3-yl}propan-1-oique
Acide (3R,4R)-3-{4-[3-(R,S)-fluoro-3-(6-méthoxyquinolin-4-yl)propyl]-1-[2-(4-chlorophénylthio)éthyl]piperidin-3-yl}propan-1-oique
Acide (3R,4R)-3-{4-[3-(R,S)-fluoro-3-(6-méthoxyquinolin-4-yl)propyl]-1-[3-(4-chlorophénylthio)propyl]piperidin-3-yl}propan-1-oique
Acide (3R,4R)-3-{4-[3-(R,S)-fluoro-3-(6-méthoxyquinolin-4-yl)propyl]-1-[2-(2,3-dichlorophénylthio)éthyl]piperidin-3-yl}propan-1-oique
Acide (3R,4R)-3-{4-[3-(R,S)-fluoro-3-(6-méthoxyquinolin-4-yl)propyl]-1-[3-(2,3-dichlorophénylthio)propyl]piperidin-3-yl}propan-1-oique
Acide (3R,4R)-3-{4-[3-(R,S)-fluoro-3-(6-méthoxyquinolin-4-yl)propyl]-1-[2-(2,6-dichlorophénylthio)éthyl]piperidin-3-yl}propan-1-oique
Acide (3R,4R)-3-{4-[3-(R,S)-fluoro-3-(6-méthoxyquinolin-4-yl)propyl]-1-[3-(2,6-dichlorophénylthio)propyl]piperidin-3-yl}propan-1-oique
Acide (3R,4R)-3-{4-[3-(R,S)-fluoro-3-(6-méthoxyquinolin-4-yl)propyl]-1-[2-(2-méthylphénylthio)éthyl]piperidin-3-yl}propan-1-oique
Acide (3R,4R)-3-{4-[3-(R,S)-fluoro-3-(6-méthoxyquinolin-4-yl)propyl]-1-[3-(2-méthylphénylthio)propyl]piperidin-3-yl}propan-1-oique
Acide (3R,4R)-3-{4-[3-(R,S)-fluoro-3-(6-méthoxyquinolin-4-yl)propyl]-1-[2-(3-méthylphénylthio)éthyl]piperidin-3-yl}propan-1-oique
Acide (3R,4R)-3-{4-[3-(R,S)-fluoro-3-(6-méthoxyquinolin-4-yl)propyl]-1-[3-(3-méthylphénylthio)propyl]piperidin-3-yl}propan-1-oique
Acide (3R,4R)-3-{4-[3-(R,S)-fluoro-3-(6-méthoxyquinolin-4-yl)propyl]-1-[2-(4-méthylphénylthio)éthyl]piperidin-3-yl}propan-1-oique
Acide (3R,4R)-3-{4-[3-(R,S)-fluoro-3-(6-méthoxyquinolin-4-yl)propyl]-1-[3-(4-méthylphénylthio)propyl]piperidin-3-yl}propan-1-oique
Acide (3R,4R)-3-{4-[3-(R,S)-fluoro-3-6-méthoxyquinolin-4-yl)propyl]-1-[2-(2-trifluorométhylphénylthio)éthyl]piperidin-3-yl}propan-1-oique
Acide (3R,4R)-3-{4-[3-(R,S)-fluoro-3-(6-méthoxyquinolin-4-yl)propyl]-1-[3-(2-triflurométhylphénylthio)propyl]piperidin-3-yl}propan-1-oique
Acide (3R,4R)-3-{4-[3-(R,S)-fluoro-3-(6-méthoxyquinolin-4-yl)propyl]-1-[2-(3-trifluorométhylphénylthio)éthyl]piperidin-3-yl}propan-1-oique
Acide (3R,4R)-3-{4-[3-(R,S)-fluoro-3-(6-méthoxyquinolin-4-yl)propyl]-1-[3-(3-triflurométhylphénylthio)propyl]piperidin-3-yl}propan-1-oique
Acide (3R,4R)-3-{4-[3-(R,S)-fluoro-3-(6-méthoxyquinolin-4-yl)propyl]-1-[2-(4-trifluorométhylphénylthio)éthyl]piperidin-3-yl}propan-1-oique
Acide (3R,4R)-3-{4-[3-(R,S)-fluoro-3-(6-méthoxyquinolin-4-yl)propyl]-1-[3-(4-triflurométhylphénylthio)propyl]piperidin-3-yl}propan-1-oique
Acide (3R,4R)-3-{4-[3-(R,S)-fluoro-3-(6-méthoxyquinolin-4-yl)propyl]-1-[2-(2-méthoxyphénylthio)éthyl]piperidin-3-yl}propan-1-oique
Acide (3R,4R)-3-{4-[3-(R,S)-fluoro-3-(6-méthoxyquinolin-4-yl)propyl]-1-[3-(2-méthoxyphénylthio)propyl]piperidin-3-yl}propan-1-oique
Acide (3R,4R)-3-{4-[3-(R,S)-fluoro-3-(6-méthoxyquinolin-4-yl)propyl]-1-[2-(3-méthoxyphénylthio)éthyl]piperidin-3-yl}propan-1-oique
Acide (3R,4R)-3-{4-[3-(R,S)-fluoro-3-(6-méthoxyquinolin-4-yl)propyl]-1-[3-(3-méthoxyphénylthio)propyl]piperidin-3-yl}propan-1-oique
Acide (3R,4R)-3-{4-[3-(R,S)-fluoro-3-(6-méthoxyquinolin-4-yl)propyl]-1-[2-(4-méthoxyphénylthio)éthyl]piperidin-3-yl}propan-1-oique
Acide (3R,4R)-3-{4-[3-(R,S)-fluoro-3-(6-méthoxyquinolin-4-yl)propyl]-1-[3-(4-méthoxy phénylthio)propyl]piperidin-3-yl}propan-1-oique
Acide (3R,4R)-3-{4-[3-(R,S)-fluoro-3-(6-méthoxyquinolin-4-yl)propyl]-1-[2-(cyclopropylthio)éthyl]piperidin-3-yl}propan-1-oique
Acide (3R,4R)-3-{4-[3-(R,S)-fluoro-3-(6-méthoxyquinolin-4-yl)propyl]-1-[3-(cyclopropylthio)propyl]piperidin-3-yl}propan-1-oique
Acide (3R,4R)-3-{4-[3-(R,S)-fluoro-3-(6-méthoxyquinolin-4-yl)propyl]-1-[2-(cyclobutylthio)éthyl]piperidin-3-yl}propan-1-oique
Acide (3R,4R)-3-{4-[3-(R,S)-fluoro-3-(6-méthoxyquinolin-4-yl)propyl]-1-[3-(cyclobutylthiopropyl]piperidin-3-yl}propan-1-oique
Acide (3R,4R)-3-{4-[3-(R,S)-fluoro-3-(6-méthoxyquinolin-4-yl)propyl]-1-[2-(cyclopentylthio)éthyl]piperidin-3-yl}propan-1-oique
Acide (3R,4R)-3-{4-[3-(R,S)-fluoro-3-(6-méthoxyquinolin-4-yl)propyl]-1-[3-(cyclopentylthio)propyl]piperidin-3-yl}propan-1-oique
Acide (3R,4R)-3-{4-[3-(R,S)-fluoro-3-(6-méthoxyquinolin-4-yl)propyl]-1-[2-(cyclohexylthio)éthyl]piperidin-3-yl}propan-1-oique
Acide (3R,4R)-3-{4-[3-(R,S)-fluoro-3-(6-méthoxyquinolin-4-yl)propyl]-1-[3-(cyclohexylthio)propyl]piperidin-3-yl}propan-1-oique
Acide (3R,4R)-3-{4-[3-(R,S)-fluoro-3-(6-méthoxyquinolin-4-yl)propyl]-1-[2-(thien-2-yl) thioéthyl]piperidin-3-yl}propan-1-oique
Acide (3R,4R)-3-{4-[3-(R,S)-fluoro-3-(6-méthoxyquinolin-4-yl)propyl]-1-[3-(thien-2-yl) thiopropyl]piperidin-3-yl}propan-1-oique
Acide (3R,4R)-3-{4-[3-(R,S)-fluoro-3-(6-méthoxyquinolin-4-yl)propyl]-1-[2-(5-chlorothien-2-yl)thioéthyl]piperidin-3-yl}propan-1-oique
Acide (3R,4R)-3-{4-[3-(R,S)-fluoro-3-(6-méthoxyquinolin-4-yl)propyl]-1-[3-(5-chlorothien-2-yl)thiopropyl]piperidin-3-yl}propan-1-oique
Acide (3R,4R)-3-{4-[3-(R,S)-fluoro-3-(6-méthoxyquinolin-4-yl)propyl]-1-[2-(3-chlorothien-2-yl)thioéthyl]piperidin-3-yl}propan-1-oique
Acide (3R,4R)-3-{4-[3-(R,S)-fluoro-3-(6-méthoxyquinolin-4-yl)propyl]-1-[3-(3-chlorothien-2-yl)thiopropyl]piperidin-3-yl}propan-1-oique
Acide (3R,4R)-3-{4-[3-(R,S)-fluoro-3-(6-méthoxyquinolin-4-yl)propyl]-1-[2-(5-méthylthien-2-yl)thioéthyl]piperidin-3-yl}propan-1-oique
Acide (3R,4R)-3-{4-[3-(R,S)-fluoro-3-(6-méthoxyquinolin-4-yl)propyl]-1-[3-(5-méthylthien-2-yl)thiopropyl]piperidin-3-yl}propan-1-oique
Acide (3R,4R)-3-{4-[3-(R,S)-fluoro-3-(6-méthoxyquinolin-4-yl)propyl]-1-[2-(3-méthylthien-2-yl)thioéthyl]piperidin-3-yl}propan-1-oique
Acide (3R,4R)-3-{4-[3-(R,S)-fluoro-3-(6-méthoxyquinolin-4-yl)propyl]-1-[3-(3-méthylthien-2-yl)thiopropyl]piperidin-3-yl}propan-1-oique
Acide (3R,4R)-3-{4-[3-(R,S)-fluoro-3-(6-méthoxyquinolin-4-yl)propyl]-1-[2-(thien-3-yl) thioéthyl]piperidin-3-yl}propan-1-oique
Acide (3R,4R)-3-{4-[3-(R,S)-fluoro-3-(6-méthoxyquinolin-4-yl)propyl]-1-[3-(thien-3-yl) thiopropyl]piperidin-3-yl}propan-1-oique
Acide (3R,4R)-3-{4-[3-(R,S)-fluoro-3-(6-méthoxyquinolin-4-yl)propyl]-1-[2-(fur-2-yl) thioéthyl]piperidin-3-yl}propan-1-oique
Acide (3R,4R)-3-{4-[3-(R,S)-fluoro-3-(6-méthoxyquinolin-4-yl)propyl]-1-[3-(fur-2-yl) thiopropyl]piperidin-3-yl}propan-1-oique
Acide (3R,4R)-3-{4-[3-(R,S)-fluoro-3-(6-méthoxyquinolin-4-yl)propyl]-1-[2-(fur-3-yl) thioéthyl]piperidin-3-yl}propan-1-oique
Acide (3R,4R)-3-{4-[3-(R,S)-fluoro-3-(6-méthoxyquinolin-4-yl)propyl]-1-[3-(fur-3-yl) thiopropyl]piperidin-3-yl}propan-1-oique
Acide (3R,4R)-3-{4-[3-(R,S)-fluoro-3-(6-méthoxyquinolin-4-yl)propyl]-1-[2-(1-méthylpyrrol-2-yl)thioéthyl]piperidin-3-yl}propan-1-oique
Acide (3R,4R)-3-{4-[3-(R,S)-fluoro-3-(6-méthoxyquinolin-4-yl)propyl]-1-[3-(1-méthylpyrrol-2-yl)thiopropyl]piperidin-3-yl}propan-1-oique
Acide (3R,4R)-3-{4-[3-(R,S)-fluoro-3-(6-méthoxyquinolin-4-yl)propyl]-1-[2-(1-méthylpyrrol-3-yl)thioéthyl]piperidin-3-yl}propan-1-oique
Acide (3R,4R)-3-{4-[3-(R,S)-fluoro-3-(6-méthoxyquinolin-4-yl)propyl]-1-[3-(1-méthylpyrrol-3-yl)thiopropyl]piperidin-3-yl}propan-1-oique
Acide (3R,4R)-3-{4-[3-(R,S)-fluoro-3-(6-méthoxyquinolin-4-yl)propyl]-1-[2-(thiazol-2-yl) thioéthyl]piperidin-3-yl}propan-1-oique
Acide (3R,4R)-3-{4-[3-(R,S)-fluoro-3-(6-méthoxyquinolin-4-yl)propyl]-1-[3-(thiazol-2-yl) thiopropyl]piperidin-3-yl}propan-1-oique
Acide (3R,4R)-3-{4-[3-(R,S)-fluoro-3-(6-méthoxyquinolin-4-yl)propyl]-1-[2-(1-méthylimidazol-2-yl)thioéthyl]piperidin-3-yl}propan-1-oique
Acide (3R,4R)-3-{4-[3-(R,S)-fluoro-3-(6-méthoxyquinolin-4-yl)propyl]-1-[3-(1-méthylimidazol-2-yl)thiopropyl]piperidin-3-yl}propan-1-oique
Acide (3R,4R)-3-{4-[3-(R,S)-fluoro-3-(6-méthoxyquinolin-4-yl)propyl]-1-[2-(3-méthylimidazol-4-yl)thioéthyl]piperidin-3-yl}propan-1-oique
Acide (3R,4R)-3-{4-[3-(R,S)-fluoro-3-(6-méthoxyquinolin-4-yl)propyl]-1-[3-(3-méthylimidazol-4-yl)thiopropyl]piperidin-3-yl}propan-1-oique
Acide (3R,4R)-3-{4-[3-(R,S)-fluoro-3-(6-méthoxyquinolin-4-yl)propyl]-1-[2-(3-méthylpyrazol-4-yl)thioéthyl]piperidin-3-yl}propan-1-oique
Acide (3R,4R)-3-{4-[3-(R,S)-fluoro-3-(6-méthoxyquinolin-4-yl)propyl]-1-[3-(3-méthylpyrazol-4-yl)thiopropyl]piperidin-3-yl}propan-1-oique
Acide (3R,4R)-3-{4-[3-(R,S)-fluoro-3-(6-méthoxyquinolin-4-yl)propyl]-1-[2-(oxazol-2-yl) thioéthyl]piperidin-3-yl}propan-1-oique
Acide (3R,4R)-3-{4-[3-(R,S)-fluoro-3-(6-méthoxyquinolin-4-yl)propyl]-1-[3-(oxazol-2-yl) thiopropyl]piperidin-3-yl}propan-1-oique
Acide (3R,4R)-3-{4-[3-(R,S)-fluoro-3-(6-méthoxyquinolin-4-yl)propyl]-1-[2-(pyridin-2-yl) thioéthyl]piperidin-3-yl}propan-1-oique
Acide (3R,4R)-3-{4-[3-(R,S)-fluoro-3-(6-méthoxyquinolin-4-yl)propyl]-1-[3-(pyridin-2-yl) thiopropyl]piperidin-3-yl}propan-1-oique
Acide (3R,4R)-3-{4-[3-(R,S)-fluoro-3-(6-méthoxyquinolin-4-yl)propyl]-1-[2-(pyridin-3-yl) thioéthyl]piperidin-3-yl}propan-1-oique
Acide (3R,4R)-3-{4-[3-(R,S)-fluoro-3-(6-méthoxyquinolin-4-yl)propyl]-1-[3-(pyridin-3-yl) thiopropyl]piperidin-3-yl}propan-1-oique
Acide (3R,4R)-3-{4-[3-(R,S)-fluoro-3-(6-méthoxyquinolin-4-yl)propyl]-1-[2-(pyridin-4-yl) thioéthyl]piperidin-3-yl}propan-1-oique
Acide (3R,4R)-3-{4-[3-(R,S)-fluoro-3-(6-méthoxyquinolin-4-yl)propyl]-1-[3-(pyridin-4-yl) thiopropyl]piperidin-3-yl}propan-1-oique
Acide (3R,4R)-3-{4-[3-(R,S)-fluoro-3-(6-méthoxyquinolin-4-yl)propyl]-1-[2-(pyrimidin-2-yl) thioéthyl]piperidin-3-yl}propan-1-oique
Acide (3R,4R)-3-{4-[3-(R,S)-fluoro-3-(6-méthoxyquinolin-4-yl)propyl]-1-[3-(pyrimidin-2-yl)thiopropyl]piperidin-3-yl}propan-1-oique
Acide (3R,4R)-3-{4-[3-(R,S)-fluoro-3-(6-méthoxyquinolin-4-yl)propyl]-1-[2-(pyrimidin-4-yl) thioéthyl]piperidin-3-yl}propan-1-oique
Acide (3R,4R)-3-{4-[3-(R,S)-fluoro-3-(6-méthoxyquinolin-4-yl)propyl]-1-[3-(pyrimidin-4-yl) thiopropyl]piperidin-3-yl}propan-1-oique
Acide (3R,4R)-3-{4-[3-(R,S)-fluoro-3-(6-méthoxyquinolin-4-yl)propyl]-1-[2-(pyrimidin-5-yl) thioéthyl]piperidin-3-yl}propan-1-oique
Acide (3R,4R)-3-{4-[3-(R,S)-fluoro-3-(6-méthoxyquinolin-4-yl)propyl]-1-[3-(pyrimidin-5-yl) thiopropyl]piperidin-3-yl}propan-1-oique
Acide (3R,4R)-3-{4-[3-(R,S)-fluoro-3-(6-méthoxyquinolin-4-yl)propyl]-1-[2-(pyrazin-2-yl) thioéthyl]piperidin-3-yl}propan-1-oique
Acide (3R,4R)-3-{4-[3-(R,S)-fluoro-3-(6-méthoxyquinolin-4-yl)propyl]-1-[3-(pyrazin-2-yl) thiopropyl]piperidin-3-yl}propan-1-oique
Acide (3R,4R)-3-{4-[3-(R,S)-fluoro-3-(6-méthoxyquinolin-4-yl)propyl]-1-[2-(pyridazin-3-yl) thioéthyl]piperidin-3-yl}propan-1-oique
Acide (3R,4R)-3-{4-[3-(R,S)-fluoro-3-(6-méthoxyquinolin-4-yl)propyl]-1-[3-(pyridazin-3-yl) thiopropyl]piperidin-3-yl}propan-1-oique
Acide (3R,4R)-3-{4-[3-(R,S)-fluoro-3-(6-méthoxyquinolin-4-yl)propyl]-1-[2-(pyridazin-4-yl) thioéthyl]piperidin-3-yl}propan-1-oique
Acide (3R,4R)-3-{4-[3-(R,S)-fluoro-3-(6-méthoxyquinolin-4-yl)propyl]-1-[3-(pyridazin-4-yl) thiopropyl]piperidin-3-yl}propan-1-oique
(3R,4R)-3-hydroxyméthyl-4-[3-(6-méthoxyquinolin-4-yl)propyl]-1-[2-phénylthio-éthyl] pipéridine
(3R,4R)-3-hydroxyméthyl-4-[3-(6-méthoxyquinolin-4-yl)propyl]-1-[3-phénylthio-propyl] pipéridine
(3R,4R)-3-hydroxyméthyl-4-[3-(6-méthoxyquinolin-4-yl)propyl]-1-[2-(2-fluorophénylthio] éthyl]pipéridine
(3R,4R)-3-hydroxyméthyl-4-[3-(6-méthoxyquinolin-4-yl)propyl]-1-[3-(2-fluorophénylthio) propyl]pipéridine
(3R,4R)-3-hydroxyméthyl-4-[3-(6-méthoxyquinolin-4-yl)propyl]-1-[2-(3-fluorophénylthio) éthyl] ipéridine
(3R,4R)-3-hydroxyméthyl-4-[3-(6-méthoxyquinolin-4-yl)propyl]-1-[3-(3-fluorophénylthio) propyl]pipéridine
(3R,4R)-3-hydroxyméthyl-4-[3-(6-méthoxyquinolin-4-yl)propyl]-1-[2-(4-fluorophénylthio) éthyl]pipéridine
(3R,4R)-3-hydroxyméthyl-4-[3-(6-méthoxyquinolin-4-yl)propyl]-1-[3-(4-fluorophénylthio) propyl]pipéridine
(3R,4R)-3-hydroxyméthyl-4-[3-(6-méthoxyquinolin-4-yl)propyl]-1-[2-(2-chlorophénylthio) éthyl]pipéridine
(3R,4R)-3-hydroxyméthyl-4-[3-(6-méthoxyquinolin-4-yl)propyl]-1-[3-(2-chlorophénylthio) propyl]pipéridine
(3R,4R)-3-hydroxyméthyl-4-[3-(6-méthoxyquinolin-4-yl)propyl]-1-[2-(3-chlorophénylthio) éthyl]pipéridine
(3R,4R)-3-hydroxyméthyl-4-[3-(6-méthoxyquinolin-4-yl)propyl]-1-[3-(3-chlorophénylthio) propyl]pipéridine
(3R,4R)-3-hydroxyméthyl-4-[3-(6-méthoxyquinolin-4-yl)propyl]-1-[2-(4-chlorophénylthio) éthyl]pipéridine
(3R,4R)-3-hydroxyméthyl-4-[3-(6-méthoxyquinolin-4-yl)propyl]-1-[3-(4-chlorophénylthio) propyl]pipéridine
(3R,4R)-3-hydroxyméthyl-4-[3-(6-méthoxyquinolin-4-yl)propyl]-1-[2-(2-méthylphénylthio) éthyl]pipéridine
(3R,4R)-3-hydroxyméthyl-4-[3-(6-méthoxyquinolin-4-yl)propyl]-1-[3-(2-méthylphénylthio) propyl]pipéridine
(3R,4R)-3-hydroxyméthyl-4-[3-(6-méthoxyquinolin-4-yl)propyl]-1-[2-(3-méthylphénylthio) éthyl]pipéridine
(3R,4R)-3-hydroxyméthyl-4-[3-(6-méthoxyquinolin-4-yl)propyl]-1-[3-(3-méthylphénylthio) propyl]pipéridine
(3R,4R)-3-hydroxyméthyl-4-[3-(6-méthoxyquinolin-4-yl)propyl]-1-[2-(4-méthylphénylthio) éthyl]pipéridine
(3R,4R)-3-hydroxyméthyl-4-[3-(6-méthoxyquinolin-4-yl)propyl]-1-[3-(4-méthylphénylthio) propyl]pipéridine
(3R,4R)-3-hydroxyméthyl-4-[3-(6-méthoxyquinolin-4-yl)propyl]-1-[2-(2-trifluorométhylphénylthio)éthyl]pipéridine
(3R,4R)-3-hydroxyméthyl-4-[3-(6-méthoxyquinolin-4-yl)propyl]-1-[3-(2-trifluorométhylphénylthio)propyl]pipéridine
(3R,4R)-3-hydroxyméthyl-4-[3-(6-méthoxyquinolin-4-yl)propyl]-1-[2-(3-trifluorométhylphénylthio)éthyl]pipéridine
(3R,4R)-3-hydroxyméthyl-4-[3-(6-méthoxyquinolin-4-yl)propyl]-1-[3-(3-trifluorométhylphénylthio)propyl]pipéridine
(3R,4R)-3-hydroxyméthyl-4-[3-(6-méthoxyquinolin-4-yl)propyl]-1-[2-(4-trifluorométhylphénylthio)éthyl]pipéridine
(3R,4R)-3-hydroxyméthyl-4-[3-(6-méthoxyquinolin-4-yl)propyl]-1-[3-(4-trifluorométhylphénylthio)propyl]pipéridine
(3R,4R)-3-hydroxyméthyl-4-[3-(6-méthoxyquinolin-4-yl)propyl]-1-[2-(2-méthoxyphénylthio) éthyl]pipéridine
(3R,4R)-3-hydroxyméthyl-4-[3-(6-méthoxyquinolin-4-yl)propyl]-1-[3-(2-méthoxyphénylthio) propyl]pipéridine
(3R,4R)-3-hydroxyméthyl-4-[3-(6-méthoxyquinolin-4-yl)propyl]-1-[2-(3-méthoxyphénylthio) éthyl]pipéridine
(3R,4R)-3-hydroxyméthyl-4-[3-(6-méthoxyquinolin-4-yl)propyl]-1-[3-(3-méthoxyphénylthio) propyl]pipéridine
(3R,4R)-3-hydroxyméthyl-4-[3-(6-méthoxyquinolin-4-yl)propyl]-1-[2-(4-méthoxyphénylthio)éthyl]pipéridine
(3R,4R)-3-hydroxyméthyl-4-[3-(6-méthoxyquinolin-4-yl)propyl]-1-[3-(4-méthoxyphénylthio) propyl]pipéridine
(3R,4R)-3-hydroxyméthyl-4-[3-(6-méthoxyquinolin-4-yl)propyl]-1-[2-(thien-3-yl)thioéthyl] pipéridine
(3R,4R)-3-hydroxyméthyl-4-[3-(6-méthoxyquinolin-4-yl)propyl]-1-[3-(thien-3-yl)thiopropyl] pipéridine
(3R,4R)-3-hydroxyméthyl-4-[3-(6-méthoxyquinolin-4-yl)propyl]-1-[2-(fur-2-yl)thioéthyl] pipéridine
(3R,4R)-3-hydroxyméthyl-4-[3-(6-méthoxyquinolin-4-yl)propyl]-1-[3-(fur-2-yl)thiopropyl] pipéridine
(3R,4R)-3-hydroxyméthyl-4-[3-(6-méthoxyquinolin-4-yl)propyl]-1-[2-(fur-3-yl)thioéthyl] pipéridine
(3R,4R)-3-hydroxyméthyl-4-[3-(6-méthoxyquinolin-4-yl)propyl]-1-[3-(fur-3-yl)thiopropyl] pipéridine
(3R,4R)-3-hydroxyméthyl-4-[3-(6-méthoxyquinolin-4-yl)propyl]-1-[2-(thiazol-2-yl)thioéthyl] pipéridine
(3R,4R)-3-hydroxyméthyl-4-[3-(6-méthoxyquinolin-4-yl)propyl]-1-[3-(thiazol-2-yl)thiopropyl]pipéridine
(3R,4R)-3-hydroxyméthyl-4-[3-(6-méthoxyquinolin-4-yl)propyl]-1-[2-(oxazol-2-yl)thioéthyl] pipéridine
(3R,4R)-3-hydxoxyméthyl-4-[3-(6-méthoxyquinolin-4-yl)propyl]-1-[3-(oxazol-2-yl)thiopropyl]pipéridine
(3R,4R)-3-hydroxyméthyl-4-[3-(6-méthoxyquinolin-4-yl)propyl]-1-[2-(pyridin-2-yl)thioéthyl]pipéridine
(3R,4R)-3-hydroxyméthyl-4-[3-(6-méthoxyquinolin-4-yl)propyl]-1-[3-(pyridin-2-yl)thiopropyl]pipéridine
(3R,4R)-3-hydroxyméthyl-4-[3-(6-méthoxyquinolin-4-yl)propyl]-1-[2-(pyridin-3-yl)thioéthyl]pipéridine
(3R,4R)-3-hydroxyméthyl-4-[3-(6-méthoxyquinolin-4-yl)propyl]-1-[3-(pyridin-3-yl)thiopropyl]pipéridine
(3R,4R)-3-hydroxyméthyl-4-[3-(6-méthoxyquinolin-4-yl)propyl]-1-[2-(pyridin-4-yl)thioéthyl]pipéridine
(3R,4R)-3-hydroxyméthyl-4-[3-(6-méthoxyquinolin-4-yl)propyl]-1-[3-(pyridin-4-yl)thiopropyl]pipéridine
(3R,4R)-3-hydroxyméthyl-4-[3-(6-méthoxyquinolin-4-yl)propyl]-1-[2-(pyrimidin-2-yl)thioéthyl]pipéridine
(3R,4R)-3-hydroxyméthyl-4-[3-(6-méthoxyquinolin-4-yl)propyl]-1-[3-(pyrimidin-2-yl)thiopropyl]pipéridine
(3R,4R)-3-hydroxyméthyl-4-[3-(6-méthoxyquinolin-4-yl)propyl]-1-[2-(pyrazin-2-yl)thioéthyl]pipéridine
(3R,4R)-3-hydroxyméthyl-4-[3-(6-méthoxyquinolin-4-yl)propyl]-1-[3-(pyrazin-2-yl)thiopropyl]pipéridine
(3R,4R)-3-hydroxyméthyl-4-[3-(6-méthoxyquinolin-4-yl)propyl]-1-[2-(pyridazin-3-yl)thioéthyl]pipéridine
(3R,4R)-3-hydroxyméthyl-4-[3-(6-méthoxyquinolin-4-yl)propyl]-1-[3-(pyridazin-3-yl)thiopropyl]pipéridine
(3R,4R)-3-hydroxyméthyl-4-[3-(6-méthoxyquinolin-4-yl)propyl]-1-[2-(pyridazin-4-yl)thioéthyl]pipéridine
(3R,4R)-3-hydroxyméthyl-4-[3-(6-méthoxyquinolin-4-yl)propyl]-1-[3-(pyridazin-4-yl)thiopropyl]pipéridine
(3R,4R)-3-hydroxyméthyl-4-[3-(R,S)-hydroxy-3-(6-méthoxyquinolin-4-yl)propyl]-1-[2-phénylthio-éthyl]pipéridine
(3R,4R)-3-hydroxyméthyl-4-[3-(R,S)-hydroxy-3-(6-méthoxyquinolin-4-yl)propyl]-1-[3-phénylthio-propyl]pipéridine
(3R,4R)-3-hydroxyméthyl-4-[3-(R,S)-hydroxy-3-(6-méthoxyquinolin-4-yl)propyl]-1-[2-(2-fluorophénylthio)éthyl]pipéridine
(3R,4R)-3-hydroxyméthyl-4-[3-(R,S)-hydroxy-3-(6-méthoxyquinolin-4-yl)propyl]-1-[3-(2-fluorophénylthio)propyl]pipéridine
(3R,4R)-3-hydroxyméthyl-4-[3-(R,S)-hydroxy-3-(6-méthoxyquinolin-4-yl)propyl]-1-[2-(3-fluorophénylthio)éthyl]pipéridine
(3R,4R)-3-hydroxyméthyl-4-[3-(R,S)-hydroxy-3-(6-méthoxyquinolin-4-yl)propyl]-1-[3-(3-fluorophénylthio)propyl]pipéridine
(3R,4R)-3-hydroxyméthyl-4-[3-(R,S)-hydroxy-3-(6-méthoxyquinolin-4-yl)propyl]-1-[2-(4-fluorophénylthio)éthyl]pipéridine
(3R,4R)-3-hydroxyméthyl-4-[3-(R,S)-hydroxy-3-(6-méthoxyquinolin-4-yl)propyl]-1-[3-(4-fluorophénylthio)propyl]pipéridine
(3R,4R)-3-hydroxyméthyl-4-[3-(R,S)-hydroxy-3-(6-méthoxyquinolin-4-yl)propyl]-1-[2-(2-chlorophénylthio)éthyl]pipéridine
(3R,4R)-3-hydroxyméthyl-4-[3-(R,S)-hydroxy-3-(6-méthoxyquinolin-4-yl)propyl]-1-[3-(2-chlorophénylthio)propyl]pipéridine
(3R,4R)-3-hydroxyméthyl-4-[3-(R,S)-hydroxy-3-(6-méthoxyquinolin-4-yl)propyl]-1-[2-(3-chlorophénylthio)éthyl]pipéridine
(3R,4R)-3-hydroxyméthyl-4-[3-(R,S)-hydroxy-3-(6-méthoxyquinolin-4-yl)propyl]-1-[3-(3-chlomphénylthio)propyl]pipéridine
(3R,4R)-3-hydroxyméthyl-4-[3-(R,S)-hydroxy-3-(6-méthoxyquinolin-4-yl)propyl]-1-[2-(4-chlorophénylthio)éthyl]pipéridine
(3R,4R)-3-hydroxyméthyl-4-[3-(R,S)-hydroxy-3-(6-méthoxyquinolin-4-yl)propyl]-1-[3-(4-chlorophénylthio)propyl]pipéridine
(3R,4R)-3-hydroxyméthyl-4-[3-(R,S)-hydroxy-3-(6-méthoxyquinolin-4-yl)propyl]-1-[2-(2-méthylphénylthio)éthyl]pipéridine
(3R,4R)-3-hydroxyméthyl-4-[3-(R,S)-hydroxy-3-(6-méthoxyquinolin-4-yl)propyl]-1-[3-(2-méthylphénylthio)propyl]pipéridine
(3R,4R)-3-hydroxyméthyl-4-[3-(R,S)-hydroxy-3-(6-méthoxyquinolin-4-yl)propyl]-1-[2-(3-méthylphénylthio)éthyl]pipéridine
(3R,4R)-3-hydroxyméthyl-4-[3-(R,S)-hydroxy-3-(6-méthoxyquinolin-4-yl)propyl]-1-[3-(3-méthylphénylthio)propyl]pipéridine
(3R,4R)-3-hydroxyméthyl-4-[3-(R,S)-hydroxy-3-(6-méthoxyquinolin-4-yl)propyl]-1-[2-(4-méthylphénylthio)éthyl]pipéridine
(3R,4R)-3-hydroxyméthyl-4-[3-(R,S)-hydroxy-3-(6-méthoxyquinolin-4-yl)propyl]-1-[3-(4-méthylphénylthio)propyl]pipéridine
(3R,4R)-3-hydroxyméthyl-4-[3-(R,S)-hydroxy-3-(6-méthoxyquinolin-4-yl)propyl]-1-[2-(2-trifluorométhylphénylthio)éthyl]pipéridine
(3R,4R)-3-hydroxyméthyl-4-[3-(R,S)-hydroxy-3-(6-méthoxyquinolin-4-yl)propyl]-1-[3-(2-trifluorométhylphénylthio)propyl]pipéridine
(3R,4R)-3-hydroxyméthyl-4-[3-(R,S)-hydroxy-3-(6-méthoxyquinolin-4-yl)propyl]-1-[2-(3-trifluorométhylphénylthio)éthyl]pipéridine
(3R,4R)-3-hydroxyméthyl-4-[3-(R,S)-hydroxy-3-(6-méthoxyquinolin-4-yl)propyl]-1-[3-(3-trifluorométhylphénylthio)propyl]pipéridine
(3R,4R)-3-hydroxyméthyl-4-[3-(R,S)-hydroxy-3-(6-méthoxyquinolin-4-yl)propyl]-1-[2-(4-trifluorométhylphénylthio)éthyl]pipéridine
(3R,4R)-3-hydroxyméthyl-4-[3-(R,S)-hydroxy-3-(6-méthoxyquinolin-4-yl)propyl]-1-[3-(4-trifluorométhylphénylthio)propyl]pipéridine
(3R,4R)-3-hydroxyméthyl-4-[3-(R,S)-hydroxy-3-(6-méthoxyquinolin-4-yl)propyl]-1-[2-(2-méthoxyphénylthio)éthyl]pipéridine
(3R,4R)-3-hydroxyméthyl-4-[3-(R,S)-hydroxy-3-(6-méthoxyquinolin-4-yl)propyl]-1-[3-(2-méthoxyphénylthio)propyl]pipéridine
(3R,4R)-3-hydroxyméthyl-4-[3-(R,S)-hydroxy-3-(6-méthoxyquinolin-4-yl)propyl]-1-[2-(3-méthoxyphénylthio)éthyl]pipéridine
(3R,4R)-3-hydroxyméthyl-4-[3-(R,S)-hydroxy-3-(6-méthoxyquinolin-4-yl)propyl]-1-[3-(3-méthoxyphénylthio)propyl]pipéridine
(3R,4R)-3-hydroxyméthyl-4-[3-(R,S)-hydroxy-3-(6-méthoxyquinolin-4-yl)propyl]-1-[2-(4-méthoxyphénylthio)éthyl]pipéridine
(3R,4R)-3-hydroxyméthyl-4-[3-(R,S)-hydroxy-3-(6-méthoxyquinolin-4-yl)propyl]-1-[3-(4-méthoxyphénylthio)propyl]pipéridine
(3R,4R)-3-hydroxyméthyl-4-[3-(R,S)-hydroxy-3-(6-méthoxyquinolin-4-yl)propyl]-1-[2-(thien-3-yl)thioéthyl]pipéridine
(3R,4R)-3-hydroxyméthyl-4-[3-(R,S)-hydroxy-3-(6-méthoxyquinolin-4-yl)propyl]-1-[3-(thien-3-yl)thiopropyl]pipéridine
(3R,4R)-3-hydroxyméthyl-4-[3-(R,S)-hydroxy-3-(6-méthoxyquinolin-4-yl)propyl]-1-[2-(fur-2-yl)thioéthyl]pipéridine
(3R,4R)-3-hydroxyméthyl-4-[3-(R,S)-hydroxy-3-(6-méthoxyquinolin-4-yl)propyl]-1-[3-(fur-2-yl)thiopropyl]pipéridine
(3R,4R)-3-hydroxyméthyl-4-[3-(R,S)-hydroxy-3-(6-méthoxyquinolin-4-yl)propyl]-1-[2-(fur-3-yl)thioéthyl]pipéridine
(3R,4R)-3-hydroxyméthyl-4-[3-(R,S)-hydroxy-3-(6-méthoxyquinolin-4-yl)propyl]-1-[3-(fur-3-yl)thiopropyl]pipéridine
(3R,4R)-3-hydroxyméthyl-4-[3-(R,S)-hydroxy-3-(6-méthoxyquinolin-4-yl)propyl]-1-[2-(thiazol-2-yl)thioéthyl]pipéridine
(3R,4R)-3-hydroxyméthyl-4-[3-(R,S)-hydroxy-3-(6-méthoxyquinolin-4-yl)propyl]-1-[3-(thiazol-2-yl)thiopropyl]pipéridine
(3R,4R)-3-hydroxyméthyl-4-[3-(R,S)-hydroxy-3-(6-méthoxyquinolin-4-yl)propyl]-1-[2-(oxazol-2-yl)thioéthyl]pipéridine
(3R,4R)-3-hydroxyméthyl-4-[3-(R,S)-hydroxy-3-(6-méthoxyquinolin-4-yl)propyl]-1-[3-(oxazol-2-yl)thiopropyl]pipéridine
(3R,4R)-3-hydroxyméthyl-4-[3-(R,S)-hydroxy-3-(6-méthoxyquinolin-4-yl)propyl]-1-[2-(pyridin-2-yl)thioéthyl]pipéridine
(3R,4R)-3-hydroxyméthyl-4-[3-(R,S)-hydroxy-3-(6-méthoxyquinolin-4-yl)propyl]-1-[3-(pyridin-2-yl)thiopropyl]pipéridine
(3R,4R)-3-hydroxyméthyl-4-[3-(R,S)-hydroxy-3-(6-méthoxyquinolin-4-yl)propyl]-1-[2-(pyridin-3-yl)thioéthyl]pipéridine
(3R,4R)-3-hydroxyméthyl-4-[3-(R,S)-hydroxy-3-(6-méthoxyquinolin-4-yl)propyl]-1-[3-(pyridin-3-yl)thiopropyl]pipéridine
(3R,4R)-3-hydroxyméthyl-4-[3-(R,S)-hydroxy-3-(6-méthoxyquinolin-4-yl)propyl]-1-[2-(pyridin-4-yl)thioéthyl]pipéridine
(3R,4R)-3-hydroxyméthyl-4-[3-(R,S)-hydroxy-3-(6-méthoxyquinolin-4-yl)propyl]-1-[3-(pyridin-4-yl)thiopropyl]pipéridine
(3R,4R)-3-hydroxyméthyl-4-[3-(R,S)-hydroxy-3-(6-méthoxyquinolin-4-yl)propyl]-1-[2-(pyrimidin-2-yl)thioéthyl]pipéridine
(3R,4R)-3-hydroxyméthyl-4-[3-(R,S)-hydroxy-3-(6-méthoxyquinolin-4-yl)propyl]-1-[3-(pyrimidin-2-yl)thiopropyl]pipéridine
(3R,4R)-3-hydroxyméthyl-4-[3-(R,S)-hydroxy-3-(6-méthoxyquinolin-4-yl)propyl]-1-[2-(pyrazin -2-yl)thioéthyl]pipéridine
(3R,4R)-3-hydroxyméthyl-4-(3-(R,S)-hydroxy-3-(6-méthoxyquinolin-4-yl)propyl]-1-[3-(pyrazin-2-yl)thiopropyl]pipéridine
(3R,4R)-3-hydroxyméthyl-4-[3-(R,S)-hydroxy-3-(6-méthoxyquinolin-4-yl)propyl]-1-[2-(pyridazin-3-yl)thioéthyl]pipéridine
(3R,4R)-3-hydroxyméthyl-4-[3-(R,S)-hydroxy-3-(6-méthoxyquinolin-4-yl)propyl]-1-[3-(pyridazin-3-yl)thiopropyl]pipéridine
(3R,4R)-3-hydroxyméthyl-4-[3-(R,S)-hydroxy-3-(6-méthoxyquinolin-4-yl)propyl]-1-[2-(pyridazin-4-yl)thioéthyl]pipéridine
(3R,4R)-3-hydroxyméthyl-4-[3-(R,S)-hydroxy-3-(6-méthoxyquinolin-4-yl)propyl]-1-[3-(pyridazin-4-yl)thiopropyl]pipéridine
(3R,4R)-3-hydroxyméthyl-4-[3-(R,S)-fluoro-3-(6-méthoxyquinolin-4-yl)propyl]-1-[2-phénylthio-éthyl]pipéridine
(3R,4R)-3-hydroxyméthyl-4-[3-(R,S)-fluoro-3-(6-méthoxyquinolin-4-yl)propyl]-1-[3-phénylthio-propyl]pipéridine
(3R,4R)-3-hydroxyméthyl-4-[3-(R,S)-fluoro-3-(6-méthoxyquinolin-4-yl)propyl]-1-[2-(2-fluorophénylthio)éthyl]pipéridine
(3R,4R)-3-hydroxyméthyl-4-[3-(R,S)-fluoro-3-(6-méthoxyquinolin-4-yl)propyl]-1-[3-(2-fluorophénylthio)propyl]pipéridine
(3R,4R)-3-hydroxyméthyl-4-[3-(R,S)-fluoro-3-(6-méthoxyquinolin-4-yl)propyl]-1-[2-(3-fluorophénylthio)éthyl]pipéridine
(3R,4R)-3-hydroxyméthyl-4-[3-(R,S)-fluoro-3-(6-méthoxyquinolin-4-yl)propyl]-1-[3-(3-fluorophénylthio)propyl]pipéridine
(3R,4R)-3-hydroxyméthyl-4-[3-(R,S)-fluoro-3-(6-méthoxyquinolin-4-yl)propyl]-1-[2-(4-fluorophénylthio)éthyl]pipéridine
(3R,4R)-3-hydroxyméthyl-4-[3-(R,S)-fluoro-3-(6-méthoxyquinolin-4-yl)propyl]-1-[3-(4-fluorophénylthio)propyl]pipéridine
(3R,4R)-3-hydroxyméthyl-4-[3-(R,S)-fluoro-3-(6-méthoxyquinolin-4-yl)propyl]-1-[2-(2-chlorophénylthio)éthyl]pipéridine
(3R,4R)-3-hydroxyméthyl-4-[3-(R,S)-fluoro-3-(6-méthoxyquinolin-4-yl)propyl]-1-[3-(2-chlorophénylthio)propyl]pipéridine
(3R,4R)-3-hydroxyméthyl-4-[3-(R,S)-fluoro-3-(6-méthoxyquinolin-4-yl)propyl]-1-[2-(3-chlorophénylthio)éthyl]pipéridine
(3R,4R)-3-hydroxyméthyl-4-[3-(R,S)-fluoro-3-(6-méthoxyquinolin-4-yl)propyl]-1-[3-(3-chlorophénylthio)propyl]pipéridine
(3R,4R)-3-hydroxyméthyl-4-[3-(R,S)-fluoro-3-(6-méthoxyquinolin-4-yl)propyl]-1-[2-(4-chlorophénylthio)éthyl]pipéridine
(3R,4R)-3-hydroxyméthyl-4-[3-(R,S)-fluoro-3-(6-méthoxyquinolin-4-yl)propyl]-1-[3-(4-chlorophénylthio)propyl]pipéridine
(3R,4R)-3-hydroxyméthyl-4-[3-(R,S)-fluoro-3-(6-méthoxyquinolin-4-yl)propyl]-1-[2-(2-méthylphénylthio)éthyl]pipéridine
(3R,4R)-3-hydroxyméthyl-4-[3-(R,S)-fluoro-3-(6-méthoxyquinolin-4-yl)propyl]-1-[3-(2-méthylphénylthio)propyl]pipéridine
(3R,4R)-3-hydroxyméthyl-4-[3-(R,S)-fluoro-3-(6-méthoxyquinolin-4-yl)propyl]-1-[2-(3-méthylphénylthio)éthyl]pipéridine
(3R,4R)-3-hydroxyméthyl-4-[3-(R,S)-fluoro-3-(6-méthoxyquinolin-4-yl)propyl]-1-[3-(3-méthylphénylthio)propyl]pipéridine
(3R,4R)-3-hydroxyméthyl-4-[3-(R,S)-fluoro-3-(6-méthoxyquinolin-4-yl)propyl]-1-[2-(4-méthylphénylthio)éthyl]pipéridine
(3R,4R)-3-hydroxyméthyl-4-[3-(R,S)-fluoro-3-(6-méthoxyquinolin-4-yl)propyl]-1-[3-(4-méthylphénylthio)propyl]pipéridine
(3R,4R)-3-hydroxyméthyl-4-[3-(R,S)-fluoro-3-(6-méthoxyquinolin-4-yl)propyl]-1-[2-(2-trifluorométhylphénylthio)éthyl]pipéridine
(3R,4R)-3-hydroxyméthyl-4-[3-(R,S)-fluoro-3-(6-méthoxyquinolin-4-yl)propyl]-1-[3-(2-trifluorométhylphénylthio)propyl]pipéridine
(3R,4R)-3-hydroxyméthyl-4-[3-(R,S)-fluoro-3-(6-méthoxyquinolin-4-yl)propyl]-1-[2-(3-trifluorométhylphénylthio)éthyl]pipéridine
(3R,4R)-3-hydroxyméthyl-4-[3-(R,S)-fluoro-3-(6-méthoxyquinolin-4-yl)propyl]-1-[3-(3-trifluorométhylphénylthio)propyl]pipéridine
(3R,4R)-3-hydroxyméthyl-4-[3-(R,S)-fluoro-3-(6-méthoxyquinolin-4-yl)propyl]-1-[2-(4-trifluorométhylphénylthio)éthyl]pipéridine
(3R,4R)-3-hydroxyméthyl-4-[3-(R,S)-fluoro-3-(6-méthoxyquinolin-4-yl)propyl]-1-[3-(4-trifluorométhylphénylthio)propyl]pipéridine
(3R,4R)-3-hydroxyméthyl-4-[3-(R,S)-fluoro-3-(6-méthoxyquinolin-4-yl)propyl]-1-[2-(2-méthoxyphénylthio)éthyl]pipéridine
(3R,4R)-3-hydroxyméthyl-4-[3-(R,S)-fluoro-3-(6-méthoxyquinolin-4-yl)propyl]-1-[3-(2-méthoxyphénylthio)propyl]pipéridine
(3R,4R)-3-hydroxyméthyl-4-[3-(R,S)-fluoro-3-(6-méthoxyquinolin-4-yl)propyl]-1-[2-(3-méthoxyphénylthio)éthyl]pipéridine
(3R,4R)-3-hydroxyméthyl-4-[3-(R,S)-fluoro-3-(6-méthoxyquinolin-4-yl)propyl]-1-[3-(3-méthoxyphénylthio)propyl]pipéridine
(3R,4R)-3-hydroxyméthyl-4-[3-(R,S)-fluoro-3-(6-méthoxyquinolin-4-yl)propyl]-1-[2-(4-méthoxyphénylthio)éthyl]pipéridine
(3R,4R)-3-hydroxyméthyl-4-[3-(R,S)-fluoro-3-(6-méthoxyquinolin-4-yl)propyl]-1-[3-(4-méthoxyphénylthio)propyl]pipéridine
(3R,4R)-3-hydroxyméthyl-4-[3-(R,S)-fluoro-3-(6-méthoxyquinolin-4-yl)propyl]-1-[2-(thien-3-yl)thioéthyl]pipéridine
(3R,4R)-3-hydroxyméthyl-4-[3-(R,S)-fluoro-3-(6-méthoxyquinolin-4-yl)propyl]-1-[3-(thien-3-yl)thiopropyl]pipéridine
(3R,4R)-3-hydroxyméthyl-4-[3-(R,S)-fluoro-3-(6-méthoxyquinolin-4-yl)propyl]-1-[2-(fur-2-yl)thioéthyl]pipéridine
(3R,4R)-3-hydroxyméthyl-4-[3-(R,S)-fluoro-3-(6-méthoxyquinolin-4-yl)propyl]-1-[3-(fur-2-yl)thiopropyl]pipéridine
(3R,4R)-3-hydroxyméthyl-4-[3-(R,S)-fluoro-3-(6-méthoxyquinolin-4-yl)propyl]-1-[2-(fur-3-yl)thioéthyl]pipéridine
(3R,4R)-3-hydroxyméthyl-4-[3-(R,S)-fluoro-3-(6-méthoxyquinolin-4-yl)propyl]-1-[3-(fur-3-yl)thiopropyl]pipéridine
(3R,4R)-3-hydroxyméthyl-4-[3-(R,S)-fluoro-3-(6-méthoxyquinolin-4-yl)propyl]-1-[2-(thiazol-2-yl)thioéthyl]pipéridine
(3R,4R)-3-hydroxyméthyl-4-[3-(R,S)-fluoro-3-(6-méthoxyquinolin-4-yl)propyl]-1-[3-(thiazol-2-yl)thiopropyl]pipéridine
(3R,4R)-3-hydroxyméthyl-4-[3-(R,S)-fluoro-3-(6-méthoxyquinolin-4-yl)propyl]-1-[2-(oxazol-2-yl)thioéthyl]pipéridine
(3R,4R)-3-hydroxyméthyl-4-[3-(R,S)-fluoro-3-(6-méthoxyquinolin-4-yl)propyl]-1-[3-(oxazol-2-yl)thiopropyl]pipéridine
(3R,4R)-3-hydroxyméthyl-4-[3-(R,S)-fluoro-3-(6-méthoxyquinolin-4-yl)propyl]-1-[2-(pyridin-2-yl)thioéthyl]pipéridine
(3R,4R)-3-hydroxyméthyl-4-[3-(R,S)-fluoro-3-(6-méthoxyquinolin-4-yl)propyl]-1-[3-(pyridin-2-yl)thiopropyl]pipéridine
(3R,4R)-3-hydroxyméthyl-4-[3-(R,S)-fluoro-3-(6-méthoxyquinolin-4-yl)propyl]-1-[2-(pyridin-3-yl)thioéthyl]pipéridine
(3R,4R)-3-hydroxyméthyl-4-[3-(R,S)-fluoro-3-(6-méthoxyquinolin-4-yl)propyl]-1-[3-(pyridin-3-yl)thiopropyl]pipéridine
(3R,4R)-3-hydroxyméthyl-4-[3-(R,S)-fluoro-3-(6-méthoxyquinolin-4-yl)propyl]-1-[2-(pyridin-4-yl)thioéthyl]pipéridine
(3R,4R)-3-hydroxyméthyl-4-[3-(R,S)-fluoro-3-(6-méthoxyquinolin-4-yl)propyl]-1-[3-(pyridin-4-yl)thiopropyl]pipéridine
(3R,4R)-3-hydroxyméthyl-4-[3-(R,S)-fluoro-3-(6-méthoxyquinolin-4-yl)propyl]-1-[2-(pyrimidin-2-yl)thioéthyl]pipéridine
(3R,4R)-3-hydroxyméthyl-4-[3-(R,S)-fluoro-3-(6-méthoxyquinolin-4-yl)propyl]-1-[3-(pyrimidin-2-yl)thiopropyl]pipéridine
(3R,4R)-3-hydroxyméthyl-4-[3-(R,S)-fluoro-3-(6-méthoxyquinolin-4-yl)propyl]-1-[2-(pyrazin-2-yl)thioéthyl]pipéridine
(3R,4R)-3-hydroxyméthyl-4-[3-(R,S)-fluoro-3-(6-méthoxyquinolin-4-yl)propyl]-1-[3-(pyrazin-2-yl)thiopropyl]pipéridine
(3R,4R)-3-hydroxyméthyl-4-[3-(R,S)-fluoro-3-(6-méthoxyquinolin-4-yl)propyl]-1-[2-(pyridazin-3-yl)thioéthyl]pipéridine
(3R,4R)-3-hydroxyméthyl-4-[3-(R,S)-fluoro-3-(6-méthoxyquinolin-4-yl)propyl]-1-[3-(pyridazin-3-yl)thiopropyl]pipéridine
(3R,4R)-3-hydroxyméthyl-4-[3-(R,S)-fluoro-3-(6-méthoxyquinolin-4-yl)propyl]-1-[2-(pyridazin-4-yl)thioéthyl]pipéridine
(3R,4R)-3-hydroxyméthyl-4-[3-(R,S)-fluoro-3-(6-méthoxyquinolin-4-yl)propyl]-1-[3-(pyridazin-4-yl)thiopropyl]pipéridine
Acide (3R,4R)-4-[3-(6-méthoxyquinolin-4-yl)propyl]-1-[2-(3,5-difluorophénylthio) éthyl]pipéridine-3-carboxylique
Acide (3R,4R)-4-[3-(6-méthoxyquinolin-4-yl)propyl]-1-[3-(3,5-difluorophénylthio) propyl]pipéridine-3-carboxylique
Acide (3R,4R)-4-[3-(6-méthoxyquinolin-4-yl)propyl]-1-[2-(2,5-difluorophénylthio) éthyl]pipéridine-3-carboxylique
Acide (3R,4R)-4-[3-(6-méthoxyquinolin-4-yl)propyl]-1-[3-(2,5-difluorophénylthio) propyl]pipéridine-3-carboxylique
Acide (3R,4R)-4-[3-(6-méthoxyquinolin-4-yl)propyl]-1-[2-(2,3,4,6-tétrafluorophénylthio)éthyl]pipéridine-3-carboxylique
Acide (3R,4R)-4-[3-(6-méthoxyquinolin-4-yl)propyl]-1-[3-(2,3,4,6-tétrafluorophénylthio)propyl]pipéridine-3-carboxylique
Acide (3R,4R)-4-[3-(6-méthoxyquinolin-4-yl)propyl]-1-[2-(3-fluoro-5-chlorophénylthio)éthyl]pipéridine-3-carboxylique
Acide (3R,4R)-4-[3-(6-méthoxyquinolin-4-yl)propyl]-1-[3-(3-fluoro-5-chlorophénylthio)propyl]pipéridine-3-carboxylique
Acide (3R,4R)-4-[3-(6-méthoxyquinolin-4-yl)propyl]-1-[2-(3-trifluorométhoxyphénylthio)éthyl]pipéridine-3-carboxylique
Acide (3R,4R)-4-[3-(6-méthoxyquinolin-4-yl)propyl]-1-[3-(3-trifluorométhoxy phénylthio)propyl]pipéridine-3-carboxylique
Acide (3R,4R)-4-[3-(6-méthoxyquinolin-4-yl)propyl]-1-[2-(3-cyanophénylthio) éthyl] pipéridine-3-carboxylique
Acide (3R,4R)-4-[3-(6-méthoxyquinolin-4-yl)propyl]-1-[3-(3-cyanophénylthio) propyl]pipéridine-3-carboxylique
Acide (3R,4R)-4-[3-(6-méthoxyquinolin-4-yl)propyl]-1-[2-(pyridin-2-yl)thioéthyl] pipéridine-3-carboxylique
Acide (3R,4R)-4-[3-(6-méthoxyquinolin-4-yl)propyl]-1-[3-(pyridin-2-yl)thiopropyl] pipéridine-3-carboxylique
Acide (3R,4R)-4-[3-(6-méthoxyquinolin-4-yl)propyl]-1-[2-(3-fluoropyridin-2-yl) thioéthyl]pipéridine-3-carboxylique
Acide (3R,4R)-4-[3-(6-méthoxyquinolin-4-yl)propyl]-1-[3-(3-fluoropyridin-2-yl) thiopropyl]pipéridine-3-carboxylique
Acide (3R,4R)-4-[3-(6-méthoxyquinolin-4-yl)propyl]-1-[2-(cycloheptylthio)éthyl] pipéridine-3-carboxylique
Acide (3R,4R)-4-[3-(6-méthoxyquinolin-4-yl)propyl]-1-[3-(cycloheptylthio)propyl] pipéridine-3-carboxylique
Acide (3R,4R)-4-[3-(R,S)-hydroxy-3-(6-méthoxyquinolin-4-yl)propyl]-1-[3-(3,5-difluorophénylthio)propyl]pipéridine-3-carboxylique
Acide (3R,4R)-4-[3-(R,S)-hydroxy-3-(6-méthoxyquinolin-4-yl)propyl]-1-[2-(2,5-difluorophénylthio)éthyl]pipéridine-3-carboxylique
Acide (3R,4R)-4-[3-(R,S)-hydroxy-3-(6-méthoxyquinolin-4-yl)propyl]-1-[3-(2,5-difluorophénylthio)propyl]pipéridine-3-carboxylique
Acide (3R,4R)-4-[3-(R,S)-hydroxy-3-(6-méthoxyquinolin-4-yl)propyl]-1-[2-(2,3,4,6-tétrafluorophénylthio)éthyl]pipéridine-3-carboxylique
Acide (3R,4R)-4-[3-(R,S)-hydroxy-3-(6-méthoxyquinolin-4-yl)propyl]-1-[3-(2,3,4,6-tétrafluorophénylthio)propyl]pipéridine-3-carboxylique
Acide (3R,4R)-4-[3-(R,S)-hydroxy-3-(6-méthoxyquinolin-4-yl)propyl]-1-[2-(3-fluoro-5-chlorophénylthio)éthyl]pipéridine-3-carboxylique
Acide (3R,4R)-4-[3-(R,S)-hydroxy-3-(6-méthoxyquinolin-4-yl)propyl]-1-[3-(3-fluoro-5-chlorophénylthio)propyl]pipéridine-3-carboxylique
Acide (3R,4R)-4-[3-(R,S)-hydroxy-3-(6-méthoxyquinolin-4-yl)propyl]-1-[2-(3-trifluorométhoxyphénylthio)éthyl]pipéridine-3-carboxylique
Acide (3R,4R)-4-[3-(R,S)-hydroxy-3-(6-méthoxyquinolin-4-yl)propyl]-1-[3-(3-trifluorométhoxyphénylthio)propyl]pipéridine-3-carboxylique
Acide (3R,4R)-4-[3-(R,S)-hydroxy-3-(6-méthoxyquinolin-4-yl)propyl]-1-[2-(3-cyanophénylthio)éthyl]pipéridine-3-carboxylique
Acide (3R,4R)-4-[3-(R,S)-hydroxy-3-(6-méthoxyquinolin-4-yl)propyl]-1-[3-(3-cyanophénylthio)propyl]pipéridine-3-carboxylique
Acide (3R,4R)-4-[3-(R,S)-hydroxy-3-(6-méthoxyquinolin-4-yl)propyl]-1-[3-(pyridin-2-yl)thiopropyl]pipéridine-3-carboxylique
Acide (3R,4R)-4-[3-(R,S)-hydroxy-3-(6-méthoxyquinolin-4-yl)propyl]-1-[2-(3-fluoropyridin-2-yl)thioéthyl]pipéridine-3-carboxylique
Acide (3R,4R)-4-[3-(R,S)-hydroxy-3-(6-méthoxyquinolin-4-yl)propyl]-1-[3-(3-fluoropyridin-2-yl)thiopropyl]pipéridine-3-carboxylique
Acide (3R,4R)-4-[3-(R,S)-hydroxy-3-(6-méthoxyquinolin-4-yl)propyl]-1-[3-(cycloheptylthio)propyl]pipéridine-3-carboxylique
Acide (3R,4R)-4-[3-(R,S)-fluoro-3-(6-méthoxyquinolin-4-yl)propyl]-1-[2-(3,5-difluorophénylthio)éthyl]pipéridine-3-carboxylique
Acide (3R,4R)-4-[3-(R,S)-fluoro-3-(6-méthoxyquinolin-4-yl)propyl]-1-[3-(3,5-difluorophénylthio)propyl]pipéridine-3-carboxylique
Acide (3R,4R)-4-[3-(R,S)-fluoro-3-(6-méthoxyquinolin-4-yl)propyl]-1-[2-(2,5-difluorophénylthio)éthyl]pipéridine-3-carboxylique
Acide (3R,4R)-4-[3-(R,S)-fluoro-3-(6-méthoxyquinolin-4-yl)propyl]-1-[3-(2,5-difluorophénylthio)propyl]pipéridine-3-carboxylique
Acide (3R,4R)-4-[3-(R,S)-fluoro-3-(6-méthoxyquinolin-4-yl)propyl]-1-[2-(2,3,4,6-tétrafluorophénylthio)éthyl]pipéridine-3-carboxylique
Acide (3R,4R)-4-[3-(R,S)-fluoro-3-(6-méthoxyquinolin-4-yl)propyl]-1-[3-(2,3,4,6-tétrafluorophénylthio)propyl]pipéridine-3-carboxylique
Acide (3R,4R)-4-[3-(R,S)-fluoro-3-(6-méthoxyquinolin-4-yl)propyl]-1-[2-(3-fluoro-5-chlorophénylthio)éthyl]pipéridine-3-carboxylique
Acide (3R,4R)-4-[3-(R,S)-fluoro-3-(6-méthoxyquinolin-4-yl)propyl]-1-[3-(3-fluoro-5-chlorophénylthio)propyl]pipéridine-3-carboxylique
Acide (3R,4R)-4-[3-(R,S)-fluoro-3-(6-méthoxyquinolin-4-yl)propyl]-1-[2-(3-trifluorométhoxyphénylthio)éthyl]pipéridine-3-carboxylique
Acide (3R,4R)-4-[3-(R,S)-fluoro-3-(6-méthoxyquinolin-4-yl)propyl]-1-[3-(3-trifluorométhoxyphénylthio)propyl]pipéridine-3-carboxylique
Acide (3R,4R)-4-[3-(R,S)-fluoro-3-(6-méthoxyquinolin-4-yl)propyl]-1-[2-(3-cyanophénylthio)éthyl]pipéridine-3-carboxylique
Acide (3R,4R)-4-[3-(R,S)-fluoro-3-(6-méthoxyquinolin-4-yl)propyl]-1-[3-(3-cyanophénylthio)propyl]pipéridine-3-carboxylique
Acide (3R,4R)-4-[3-(R,S)-fluoro-3-(6-méthoxyquinolin-4-yl)propyl]-1-[2-(pyridin-2-yl)thioéthyl]pipéridine-3-carboxylique
Acide (3R,4R)-4-[3-(R,S)-fluoro-3-(6-méthoxyquinolin-4-yl)propyl]-1-[3-(pyridin-2-yl)thiopropyl]pipéridine-3-carboxylique
Acide (3R,4R)-4-[3-(R,S)-fluoro-3-(6-méthoxyquinolin-4-yl)propyl]-1-[2-(3-fluoropyridin-2-yl)thioéthyl]pipéridine-3-carboxylique
Acide (3R,4R)-4-[3-(R,S)-fluoro-3-(6-méthoxyquinolin-4-yl)propyl]-1-[3-(3-fluoropyridin-2-yl)thiopropyl]pipéridine-3-carboxylique
Acide (3R,4R)-4-[3-(R,S)-fluoro-3-(6-méthoxyquinolin-4-yl)propyl]-1-[2-(cycloheptylthio)éthyl]pipéridine-3-carboxylique
Acide (3R,4R)-4-[3-(R,S)-fluoro-3-(6-méthoxyquinolin-4-yl)propyl]-1-[3-(cycloheptylthio)propyl]pipéridine-3-carboxylique
Acide (3R,4R)-4-[3-(6-méthoxyquinolin-4-yl)propyl]-1-[2-(3,5-difluorophénylthio)éthyl]pipéridine-3-acétique
Acide (3R,4R)-4-[3-(6-méthoxyquinolin-4-yl)propyl]-1-[3-(3,5-difluorophénylthio)propyl]pipéridine-3-acétique
Acide (3R,4R)-4-[3-(6-méthoxyquinolin-4-yl)propyl]-1-[2-(2,5-difluorophénylthio)éthyl]pipéridine-3-acétique
Acide (3R,4R)-4-[3-(6-méthoxyquinolin-4-yl)propyl]-1-[3-(2,5-difluorophénylthio)propyl]pipéridine-3-acétique
Acide (3R,4R)-4-[3-(6-méthoxyquinolin-4-yl)propyl]-1-[2-(2,3,4,6-tétrafluorophénylthio)éthyl]pipéridine-3-acétique
Acide (3R,4R)-4-[3-(6-méthoxyquinolin-4-yl)propyl]-1-[3-(2,3,4,6-tétrafluorophénylthio)propyl]pipéridine-3-acétique
Acide (3R,4R)-4-[3-(6-méthoxyquinolin-4-yl)propyl]-1-[2-(3-fluoro-5-chlorophénylthio)éthyl]pipéridine-3-acétique
Acide (3R,4R)-4-[3-(6-méthoxyquinolin-4-yl)propyl]-1-[3-(3-fluoro-5-chlorophénylthio)propyl]pipéridine-3-acétique
Acide (3R,4R)-4-[3-(6-méthoxyquinolin-4-yl)propyl]-1-[2-(3-trifluorométhoxyphénylthio)éthyl]pipéridine-3-acétique
Acide (3R,4R)-4-[3-(6-méthoxyquinolin-4-yl)propyl]-1-[3-(3-trifluorométhoxyphénylthio)propyl]pipéridine-3-acétique
Acide (3R,4R)-4-[3-(6-méthoxyquinolin-4-yl)propyl]-1-[2-(3-cyanophénylthio)éthyl]pipéridine-3-acétique
Acide (3R,4R)-4-[3-(6-méthoxyquinolin-4-yl)propyl]-1-[3-(3-cyanophénylthio)propyl]pipéridine-3-acétique
Acide (3R,4R)-4-[3-(6-méthoxyquinolin-4-yl)propyl]-1-[2-(pyridin-2-yl)thioéthyl]pipéridine-3-acétique
Acide (3R,4R)-4-[3-(6-méthoxyquinolin-4-yl)propyl]-1-[3-(pyridin-2-yl)thiopropyl]pipéridine-3-acétique
Acide (3R,4R)-4-[3-(6-méthoxyquinolin-4-yl)propyl]-1-[2-(3-fluoropyridin-2-yl)thio-éthyl]pipéridine-3-acétique
Acide (3R,4R)-4-[3-(6-méthoxyquinolin-4-yl)propyl]-1-[3-(3-fluoropyridin-2-yl)thio-propyl]pipéridine-3-acétique
Acide (3R,4R)-4-[3-(6-méthoxyquinolin-4-yl)propyl]-1-[2-(cycloheptylthio)éthyl]pipéridine-3-acétique
Acide (3R,4R)-4-[3-(6-méthoxyquinolin-4-yl)propyl]-1-[3-(cycloheptylthio)propyl]pipéridine-3-acétique
Acide (3R,4R)-4-[3-(R,S)-hydroxy-3-(6-méthoxyquinolin-4-yl)propyl]-1-[2-(3,5-difluarophénylthio)éthyl]pipéridine-3-acétique
Acide (3R,4R)-4-[3-(R,S)-hydroxy-3-(6-méthoxyquinolin-4-yl)propyl]-1-[3-(3,5-difluorophénylthio)prapyl]pipéridine-3-acétique
Acide (3R,4R)-4-[3-(R,S)-hydroxy-3-(6-méthoxyquinolin-4-yl)propyl]-1-[2-(2,5-difluorophénylthio)éthyl]pipéridine-3-acétique
Acide (3R,4R)-4-[3-(R,S)-hydroxy-3-(6-méthoxyquinolin-4-yl)propyl]-1-[3-(2,5-difluorophénylthio)propyl]pipéridine-3-acétique
Acide (3R,4R)-4-[3-(R,S)-hydroxy-3-(6-méthoxyquinolin-4-yl)propyl]-1-[2-(2,3,4,6-tétrafluorophénylthio)éthyl]pipéridine-3-acétique
Acide (3R,4R)-4-[3-(R,S)-hydroxy-3-(6-méthoxyquinolin-4-yl)propyl]-1-[3-(2,3,4,6-tétrafluorophénylthio)propyl]pipéridine-3-acétique
Acide (3R,4R)-4-[3-(R,S)-hydroxy-3-(6-méthoxyquinolin-4-yl)propyl]-1-[2-(3-fluoro-5-chlorophénylthio)éthyl]pipéridine-3-acétique
Acide (3R,4R)-4-[3-(R,S)-hydroxy-3-(6-méthoxyquinolin-4-yl)propyl]-1-[3-(3-fluoro-5-chlorophénylthio)propyl]pipéridine-3-acétique
Acide (3R,4R)-4-[3-(R,S)-hydroxy-3-(6-méthoxyquinolin-4-yl)propyl]-1-[2-(3-tnfluorométhoxyphénylthio)éthyl]pipéndine-3-acétique
Acide (3R,4R)-4-[3-(R,S)-hydroxy-3-(6-méthoxyquinolin-4-yl)propyl]-1-[3-(3-trifluorométhoxyphénytthio)propyl]pipéndine-3-acétique
Acide (3R,4R)-4-[3-(R,S)-hydroxy-3-(6-méthoxyquinolin-4-yl)propyl]-1-[2-(3-cyanophénylthio)éthyl]pipéridine-3-acétique
Acide (3R,4R)-4-[3-(R,S)-hydroxy-3-(6-méthoxyquinalin-4-yl)propyl]-1-[3-(3-cyanophénylthio)propyl]pipéridine-3-acétique
Acide (3R,4R)-4-[3-(R,S)-hydroxy-3-(6-méthoxyquinolin-4-yl)propyl]-1-[2-(pyridin-2-yl)thioéthyl]pipéridine-3-acétique
Acide (3R,4R)-4-[3-(R,S)-hydroxy-3-(6-méthoxyquinolin-4-yl)propyl]-1-[3-(pyridin-2-yl)thiopropyl]pipéridine-3-acétique
Acide (3R,4R)-4-[3-(R,S)-hydroxy-3-(6-méthoxyquinolin-4-yl)propyl]-1-[2-(3-fluoropyridin-2-yl)thioéthyl]pipéridine-3-acétique
Acide (3R,4R)-4-[3-(R,S)-hydroxy-3-(6-méthoxyquinolin-4-yl)propyl]-1-[3-(3-fluoropyridin-2-yl)thiopropyl]pipéridine-3-acétique
Acide (3R,4R)-4-[3-(R,S)-hydroxy-3-(6-méthoxyquinolin-4-yl)propyl]-1-[2-(cycloheptylthio)éthyl]pipéridine-3-acétique
Acide (3R,4R)-4-[3-(R,S)-hydroxy-3-(6-méthoxyquinolin-4-yl)propyl]-1-[3-(cycloheptylthio)propyl]pipéridine-3-acétique
Acide (3R,4R)-4-[3-(R,S)-fluoro-3-(6-méthoxyquinolin-4-yl)propyl]-1-[2-(3,5-difluorophénylthio)éthyl]pipéridine-3-acétique
Acide (3R,4R)-4-[3-(R,S)-fluoro-3-(6-méthoxyquinolin-4-yl)propyl]-1-[3-(3,5-difluorophénylthio)propyl]pipéridine-3-acétique
Acide (3R,4R)-4-[3-(R,S)-fluoro-3-(6-méthoxyquinolin-4-yl)propyl]-1-[2-(2,5-difluorophénylthio)éthyl]pipéridine-3-acétique
Acide (3R,4R)-4-[3-(R,S)-fluoro-3-(6-méthoxyquinolin-4-yl)propyl]-1-[3-(2,5-difluorophénylthio)propyl]pipéridine-3-acétique
Acide (3R,4R)-4-[3-(R,S)-fluoro-3-(6-méthoxyquinolin-4-yl)propyl]-1-[2-(2,3,4,6-tétrafluorophénylthio)éthyl]pipéridine-3-acétique
Acide (3R,4R)-4-[3-(R,S)-fluoro-3-(6-méthoxyquinolin-4-yl)propyl]-1-[3-(2,3,4,6-tétrafluorophénylthio)propyl]pipéridine-3-acétique
Acide (3R,4R)-4-[3-(R,S)-fluoro-3-(6-méthoxyquinolin-4-yl)propyl]-1-[2-(3-fluoro-5-chlorophénylthio)éthyl]pipéridine-3-acétique
Acide (3R,4R)-4-[3-(R,S)-fluoro-3-(6-méthoxyquinolin-4-yl)propyl]-1-[3-(3-fluoro-5-chlorophénylthio)propyl]pipéridine-3-acétique
Acide (3R,4R)-4-[3-(R,S)-fluoro-3-(6-méthoxyquinolin-4-yl)propyl]-1-[2-(3-trifluorométhoxyphénylthio)éthyl]pipéridine-3-acétique
Acide (3R,4R)-4-[3-(R,S)-fluoro-3-(6-méthoxyquinolin-4-yl)propyl]-1-[3-(3-trifluorométhoxyphénylthio)propyl]pipéridine-3-acétique
Acide (3R,4R)-4-[3-(R,S)-fluoro-3-(6-méthoxyquinolin-4-yl)propyl]-1-[2-(3-cyanophénylthio)éthyl]pipéridine-3-acétique
Acide (3R,4R)-4-[3-(R,S)-fluoro-3-(6-méthoxyquinolin-4-yl)propyl]-1-[3-(3-cyanophénylthio)propyl]pipéridine-3-acétique
Acide (3R,4R)-4-[3-(R,S)-fluoro-3-(6-méthoxyquinolin-4-yl)propyl]-1-[2-(pyridin-2-yl)thioéthyl]pipéridine-3-acétique
Acide (3R,4R)-4-[3-(R,S)-fluoro-3-(6-méthoxyquinolin-4-yl)propyl]-1-[3-(pyridin-2-yl)thiopropyl]pipéridine-3-acétique
Acide (3R,4R)-4-[3-(R,S)-fluoro-3-(6-méthoxyquinolin-4-yl)propyl]-1-[2-(3-fluoropyridin-2-yl)thioéthyl]pipéridine-3-acétique
Acide (3R,4R)-4-[3-(R,S)-fluoro-3-(6-méthoxyquinolin-4-yl)propyl]-1-[3-(3-fluoropyridin-2-yl)thiopropyl]pipéridine-3-acétique
Acide (3R,4R)-4-[3-(R,S)-fluoro-3-(6-méthoxyquinolin-4-yl)propyl]-1-[2-(cycloheptylthio)éthyl]pipéridine-3-acétique
Acide (3R,4R)-4-[3-(R,S)-fluoro-3-(6-méthoxyquinolin-4-yl)propyl]-1-[3-(cycloheptylthio)propyl]pipéridine-3-acétique
(3R,4R)-3-hydroxyméthyl-4-[3-(6-méthoxyquinolin-4-yl)propyl]-1-[2-(3,5-difluorophénylthio)éthyl]pipéridine
(3R,4R)-3-hydroxyméthyl-4-[3-(6-méthoxyquinolin-4-yl)propyl]-1-[3-(3,5-difluorophénylthio)propyl]pipéridine
(3R,4R)-3-hydroxyméthyl-4-[3-(6-méthoxyquinolin-4-yl)propyl]-1-[2-(2,5-difluorophénylthio)éthyl]pipéridine
(3R,4R)-3-hydroxyméthyl-4-[3-(6-méthoxyquinolin-4-yl)propyl]-1-[3-(2,5-difluorophénylthio)propyl]pipéridine
(3R,4R)-3-hydroxyméthyl-4-[3-(6-méthoxyquinolin-4-yl)propyl]-1-[2-(2,3,4,6-tétrafluorophénylthio)éthyl]pipéridine
(3R,4R)-3-hydroxyméthyl-4-[3-(6-méthoxyquinolin-4-yl)propyl]-1-[3-(2,3,4,6-tétrafluorophénylthio)propyl]pipéridine
(3R,4R)-3-hydroxyméthyl-4-[3-(6-méthoxyquinolin-4-yl)propyl]-1-[2-(3-fluoro-5-chlorophénylthio)éthyl]pipéridine
(3R,4R)-3-hydroxyméthyl-4-[3-(6-méthoxyquinolin-4-yl)propyl]-1-[3-(3-fluoro-5-chlorophénylthio)propyl]pipéridine
(3R,4R)-3-hydroxyméthyl-4-[3-(6-méthoxyquinolin-4-yl)propyl]-1-[2-(3-trifluorométhoxyphénylthio)éthyl]pipéridine
(3R,4R)-3-hydroxyméthyl-4-[3-(6-méthoxyquinolin-4-yl)propyl]-1-[3-(3-trifluorométhoxyphénylthio)propyl]pipéridine
(3R,4R)-3-hydroxyméthyl-4-[3-(6-méthoxyquinolin-4-yl)propyl]-1-[2-(3-cyanophénylthio)éthyl]pipéridine
(3R,4R)-3-hydroxyméthyl-4-[3-(6-méthoxyquinolin-4-yl)propyl]-1-[3-(3-cyanophénylthio)propyl]pipéridine
(3R,4R)-3-hydroxyméthyl-4-[3-(6-méthoxyquinolin-4-yl)propyl]-1-[2-(pyridin-2-yl) thioéthyl]pipéridine
(3R,4R)-3-hydroxyméthyl-4-[3-(6-méthoxyquinolin-4-yl)propyl]-1-[3-(pyridin-2-yl) thiopropyl]pipéridine
(3R,4R)-3-hydroxyméthyl-4-[3-(6-méthoxyquinolin-4-yl)propyl]-1-[2-(3-fluoropyridin-2-yl)thioéthyl]pipéridine
(3R,4R)-3-hydroxyméthyl-4-[3-(6-méthoxyquinolin-4-yl)propyl]-1-[3-(3-fluoropyridin-2-yl)thiopropyl]pipéridine
(3R,4R)-3-hydroxyméthyl-4-[3-(6-méthoxyquinolin-4-yl)propyl]-1-[2-(cycloheptylthio)éthyl]pipéridine
(3R,4R)-3-hydroxyméthyl-4-[3-(6-méthoxyquinolin-4-yl)propyl]-1-[3-(cycloheptylthio)propyl]pipéridine
(3R,4R)-3-hydroxyméthyl-4-[3-(R,S)-hydroxy-3-(6-méthoxyquinolin-4-yl)propyl]-1-[2-(3,5-difluorophénylthio)éthyl]pipéridine
(3R,4R)-3-hydroxyméthyl-4-[3-(R,S)-hydroxy-3-(6-méthoxyquinolin-4-yl)propyl]-1-[3-(3,5-difluorophénylthio)propyl]pipéridine
(3R,4R)-3-hydroxyméthyl-4-[3-(R,S)-hydroxy-3-(6-méthoxyquinolin-4-yl)propyl]-1-[2-(2,5-difluorophénylthio)éthyl]pipéridine
(3R,4R)-3-hydroxyméthyl-4-[3-(R,S)-hydroxy-3-(6-méthoxyquinolin-4-yl)propyl]-1-[3-(2,5-difluorophénylthio)propyl]pipéridine
(3R,4R)-3-hydroxyméthyl-4-[3-(R,S)-hydroxy-3-(6-méthoxyquinolin-4-yl)propyl]-1-[2-(2,3,4,6-tétrafluorophénylthio)éthyl]pipéridine
(3R,4R)-3-hydroxyméthyl-4-[3-(R,S)-hydroxy-3-(6-méthoxyquinolin-4-yl)propyl]-1-[3-(2,3,4,6-tétrafluorophénylthio)propyl]pipéridine
(3R,4R)-3-hydroxyméthyl-4-[3-(R,S)-hydroxy-3-(6-méthoxyquinolin-4-yl)propyl]-1-[2-(3-fluoro-5-chlorophénylthio)éthyl]pipéridine
(3R,4R)-3-hydroxyméthyl-4-[3-(R,S)-hydroxy-3-(6-méthoxyquinolin-4-yl)propyl]-1-[3-(3-fluoro-5-chlorophénylthio)propyl]pipéridine
(3R,4R)-3-hydroxyméthyl-4-[3-(R,S)-hydroxy-3-(6-méthoxyquinolin-4-yl)propyl]-1-[2-(3-trifluorométhoxyphénylthio)éthyl]pipéridine
(3R,4R)-3-hydroxyméthyl-4-[3-(R,S)-hydroxy-3-(6-méthoxyquinolin-4-yl)propyl]-1-[3-(3-trifluorométhoxyphénylthio)propyl]pipéridine
(3R,4R)-3-hydroxyméthyl-4-[3-(R,S)-hydroxy-3-(6-méthoxyquinolin-4-yl)propyl]-1-[2-(3-cyanophénylthio)éthyl]pipéridine
(3R,4R)-3-hydroxyméthyl-4-[3-(R,S)-hydroxy-3-(6-méthoxyquinolin-4-yl)propyl]-1-[3-(3-cyanophénylthio)propyl]pipéridine
(3R,4R)-3-hydroxyméthyl-4-[3-(R,S)-hydroxy-3-(6-méthoxyquinolin-4-yl)propyl]-1-[2-(pyridin-2-yl)thioéthyl]pipéridine
(3R,4R)-3-hydroxyméthyl-4-[3-(R,S)-hydroxy-3-(6-méthoxyquinolin-4-yl)propyl]-1-[3-(pyridin-2-yl)thiopropyl]pipéridine
(3R,4R)-3-hydroxyméthyl-4-[3-(R,S)-hydroxy-3-(6-méthoxyquinolin-4-yl)propyl]-1-[2-(3-fluoropyridin-2-yl)thioéthyl]pipéridine
(3R,4R)-3-hydroxyméthyl-4-[3-(R,S)-hydroxy-3-(6-méthoxyquinolin-4-yl)propyl]-1-[3-(3-fluoropyridin-2-yl)thiopropyl]pipéridine
(3R,4R)-3-hydroxyméthyl-4-[3-(R,S)-hydroxy-3-(6-méthoxyquinolin-4-yl)propyl]-1-[2-(cycloheptylthio)éthyl]pipéridine
(3R,4R)-3-hydroxyméthyl-4-[3-(R,S)-hydroxy-3-(6-méthoxyquinolin-4-yl)propyl]-1-[3-(cycloheptylthio)propyl]pipéridine
(3R,4R)-3-hydroxyméthyl-4-[3-(R,S)-fluoro-3-(6-méthoxyquinolin-4-yl)propyl]-1-[2-(3,5-difluorophénylthio)éthyl]pipéridine
(3R,4R)-3-hydroxyméthyl-4-[3-(R,S)-fluoro-3-(6-méthoxyquinolin-4-yl)propyl]-1-[3-(3,5-difluorophénylthio)propyl]pipéridine
(3R,4R)-3-hydroxyméthyl-4-[3-(R,S)-fluoro-3-(6-méthoxyquinolin-4-yl)propyl]-1-[2-(2,5-difluorophénylthio)éthyl]pipéridine
(3R,4R)-3-hydroxyméthyl-4-[3-(R,S)-fluoro-3-(6-méthoxyquinolin-4-yl)propyl]-1-[3-(2,5-difluorophénylthio)propyl]pipéridine
(3R,4R)-3-hydroxyméthyl-4-[3-(R,S)-fluoro-3-(6-méthoxyquinolin-4-yl)propyl]-1-[2-(2,3,4,6-tétrafluorophénylthio)éthyl]pipéridine
(3R,4R)-3-hydroxyméthyl-4-[3-(R,S)-fluoro-3-(6-méthoxyquinolin-4-yl)propyl]-1-[3-(2,3,4,6-tétrafluorophénylthio)propyl]pipéridine
(3R,4R)-3-hydroxyméthyl-4-[3-(R,S)-fluoro-3-(6-méthoxyquinolin-4-yl)propyl]-1-[2-(3-fluoro-5-chlorophénylthio)éthyl]pipéridine
(3R,4R)-3-hydroxyméthyl-4-[3-(R,S)-fluoro-3-(6-méthoxyquinolin-4-yl)propyl]-1-[3-(3-fluoro-5-chlorophénylthio)propyl]pipéridine
(3R,4R)-3-hydroxyméthyl-4-[3-(R,S)-fluoro-3-(6-méthoxyquinolin-4-yl)propyl]-1-[2-(3-trifluorométhoxyphénylthio)éthyl]pipéridine
(3R,4R)-3-hydroxyméthyl-4-[3-(R,S)-fluoro-3-(6-méthoxyquinolin-4-yl)propyl]-1-[3-(3-trifluorométhoxyphénylthio)propyl]pipéridine
(3R,4R)-3-hydroxyméthyl-4-[3-(R,S)-fluoro-3-(6-méthoxyquinolin-4-yl)propyl]-1-[2-(3-cyanophénylthio)éthyl]pipéridine
(3R,4R)-3-hydroxyméthyl-4-[3-(R,S)-fluoro-3-(6-méthoxyquinolin-4-yl)propyl]-1-[3-(3-cyanophénylthio)propyl]pipéridine
(3R,4R)-3-hydroxyméthyl-4-[3-(R,S)-fluoro-3-(6-méthoxyquinolin-4-yl)propyl]-1-[2-(pyridin-2-yl)thioéthyl]pipéridine
(3R,4R)-3-hydroxyméthyl-4-[3-(R,S)-fluoro-3-(6-méthoxyquinolin-4-yl)propyl]-1-[3-(pyridin-2-yl)thiopropyl]pipéridine
(3R,4R)-3-hydroxyméthyl-4-[3-(R,S)-fluoro-3-(6-méthoxyquinolin-4-yl)propyl]-1-[2-(3-fluoropyridin-2-yl)thioéthyl]pipéridine
(3R,4R)-3-hydroxyméthyl-4-[3-(R,S)-fluoro-3-(6-méthoxyquinolin-4-yl)propyl]-1-[3-(3-fluoropyridin-2-yl)thiopropyl]pipéridine
(3R,4R)-3-hydroxyméthyl-4-[3-(R,S)-fluoro-3-(6-méthoxyquinolin-4-yl)propyl]-1-[2-(cycloheptylthio)éthyl]pipéridine
(3R,4R)-3-hydroxyméthyl-4-[3-(R,S)-fluoro-3-(6-méthoxyquinolin-4-yl)propyl]-1-[3-(cycloheptylthio)propyl]pipéridine
Acide (3R,4R)-4-[3-(6-méthoxyquinolin-4-yl)propyl]-1-[2-(6-fluoropyridin-2-yl)thioéthyl]pipéridine-3-carboxylique
Acide (3R,4R)-4-[3-(6-méthoxyquinolin-4-yl)propyl]-1-[3-(6-fluoropyridin-2-yl)thiopropyl]pipéridine-3-carboxylique
Acide (3R,4R)-4-[3-(R,S)-hydroxy-3-(6-méthoxyquinolin-4-yl)propyl]-1-[2-(6-fluoropyridin-2-yl)thioéthyl]pipéridine-3-carboxylique
Acide (3R,4R)-4-[3-(R,S)-hydroxy-3-(6-méthoxyquinolin-4-yl)propyl]-1-[3-(6-fluoropyridin-2-yl)thiopropyl]pipéridine-3-carboxylique
Acide (3R,4R)-4-[3-(R,S)-fluoro-3-(6-méthoxyquinolin-4-yl)propyl]-1-[2-(6-fluoropyridin-2-yl)thioéthyl]pipéridine-3-carboxylique
Acide (3R,4R)-4-[3-(R,S)-fluoro-3-(6-méthoxyquinolin-4-yl)propyl]-1-[3-(6-fluoropyridin-2-yl)thiopropyl]pipéridine-3-carboxylique
Acide (3R,4R)-4-[3-(6-méthoxyquinolin-4-yl)propyl]-1-[2-(6-fluoropyridin-2-yl)thioéthyl]pipéridine-3-acétique
Acide (3R,4R)-4-[3-(6-méthoxyquinolin-4-yl)propyl]-1-[3-(6-fluoropyridin-2-yl)thiopropyl]pipéridine-3-acétique
Acide (3R,4R)-4-[3-(R,S)-hydroxy-3-(6-méthoxyquinolin-4-yl)propyl]-1-[2-(6-fluoropyridin-2-yl)thioéthyl]pipéridine-3-acétique
Acide (3R,4R)-4-[3-(R,S)-hydroxy-3-(6-méthoxyquinolin-4-yl)propyl]-1-[3-(6-fluoropyridin-2-yl)thiopropyl]pipéridine-3-acétique
Acide (3R,4R)-4-[3-(R,S)-fluoro-3-(6-méthoxyquinolin-4-yl)propyl]-1-[2-(6-fluoropyridin-2-yl)thioéthyl]pipéridine-3-acétique
Acide (3R,4R)-4-[3-(R,S)-fluoro-3-(6-méthoxyquinolin-4-yl)propyl]-1-[3-(6-fluoropyridin-2-yl)thiopropyl]pipéridine-3-acétique

Les exemples suivants donnés à titre non limitatif illustrent la présente invention.

### Exemple de référence 1

Le chlorhydrate de l'acide (3R,4R)-4-[3-(6-méthoxyquinolin-4-yl)propyl]pipéridine-3-carboxylique peut être préparé de la manière suivante :

8,8 g de d'acide (3R,4R)-1-benzoyl-4-[3-(6-méthoxyquinolin-4-yl)propyl]pipéridine-3-carboxylique sont chauffés, sous agitation, dans 200 cm³ d'acide chlorhydrique aqueux 5N, à une température voisine de 100°C pendant 48 heures. Le mélange réactionnel est concentré sous pression réduite (5 kPa) à une température voisine de 50°C. Le résidu est repris par 100 cm³ d'acétone. Le mélange est concentré sous pression réduite (5 kPa), à une température voisine de 60°C. Cette opération est répétée deux fois supplémentaires. Le résidu est enfin trituré dans 100 cm³ d'acétone, jusqu'à cristallisation. Après filtration des cristaux, et séchage au dessicateur sous pression réduite (10 kPa), on obtient 7,2 g de chlorhydrate d'acide (3R,4R)-4-[3-(6-méthoxyquinolin-4-yl)propyl]pipéridine-3-carboxylique, sous forme d'un solide beige fondant aux environs de 270°C (fusion pâteuse).

L'acide (3R,4R)-1-benzoyl-4-[3-(6-méthoxyquinolin-4-yl)propyl]pipéridine-3-carboxylique peut être préparé de la manière suivante :

25 g de (3R,4R)-1-benzoyl-4-[3-(6-méthoxyquinolin-4-yl)propyl]-3-vinyl pipéridine sont dissous dans un mélange de 250 cm³ de tétrachlorure de carbone et 250 cm³ d'acétonitrile. 51,3 g de métapériodate de sodium en solution dans 325 cm³ d'eau sont ajoutés à une température voisine de 20°C, sous bonne agitation, puis 0,27 g de trichlorure de ruthénium hydrate. La réaction, légèrement exothermique, est maintenue au voisinage de 30°C pendant 15 minutes après l'addition des réactifs. Le mélange est agité 2 heures à température ambiante. La suspension obtenue est filtrée, l'insoluble lavé par 5 fois 80 cm³ de dichlorométhane. Après agitation du filtrat, la phase organique est décantée, la phase aqueuse saturée par du chlorure de sodium, puis extraite par deux portions supplémentaires de 300 cm³ de dichlorométhane. Les extraits organiques réunis sont lavés à l'eau (3 fois 200 cm³), séchés sur sulfate de magnésium, filtrés s ur p apier, puis concentrés sous pression réduite (5 k Pa), à une température voisine de 40°C. On obtient 23,2 g d'une huile que l'on purifie par chromatographie, à pression atmosphérique, sur gel de silice (granulométrie 20-45 µ ; diamètre 6,5 cm ; hauteur 30 cm), en éluant par un mélange de dichlorométhane-méthanol (97/3 en volumes), et en recueillant des fractions de 400 cm³. Les fractions 4 à 8 sont réunies, puis concentrées sous pression réduite (5 kPa). On obtient 11,8 g d'une huile brune. Celle-ci est dissoute d ans 60 cm³ d'acétonitrile portés au reflux pendant quelques minutes en présence de 0,5 g de charbon animal. Après filtration, la solution obtenue est refroidie. Le produit qui a cristallisé est essoré, lavé par 2 fois 10 cm³ d'acétonitrile. Le solide est séché au dessiccateur sous vide potassique (10 kPa). On obtient 8,8 g d'acide (3R,4R)-1-benzoyl-4-[3-(6-méthoxyquinolin-4-yl)propyl]pipéridine-3-carboxylique sous forme d'un solide beige fondant à 160°C.

La (3R,4R)-1-benzoyl-4-[3-(6-méthoxyquinolin-4-yl)propyl]-3-vinylpipéridine peut être préparée de la manière suivante :

A une solution agitée de 20,8 g de (3R,4R)-4-[3-(6-méthoxyquinolin-4-yl)propyl]-3-vinylpipéridine dans 270 cm³ de chloroforme, on ajoute 18,4 cm³ de triéthylamine, puis, en 1 heure, une solution de 7,2 cm³ de chlorure de benzoyle dans 50 cm³ de chloroforme. Après 1 heure 30 minutes d'agitation du mélange à une température voisine de 20°C, 100 cm³ d'eau distillée sont ajoutés au mélange réactionnel. La phase chloroformique est décantée, lavée par 2 fois 100 cm³ d'eau, puis séchée sur sulfate de magnésium. Après filtration sur papier, la solution chloroformique est concentrée sous pression réduite (5 kPa), à une température voisine de 40°C. On obtient 25 g de (3R,4R)-1-benzoyl-4-[3-(6-méthoxyquinolin-4-yl)propyl]-3-vinylpipéridine, sous forme d'huile brune.

La (3R,4R)-4-[3-(6-méthoxyquinolin-4-yl)propyl]-3-vinylpipéridine peut être obtenue par application de la méthode décrite dans la demande de brevet FR 2354771.

### Exemple 2

### Dihlorhydrate de l'acide (3R,4R)-4-[3-(6-méthoxyquinolin-4-yl)propyl]-1-[2-(3-fluorophénylthio)-2-éthyl]pipéridine-3-carboxylique

Un mélange de 0,54 g de (3R,4R)-4-[3-(6-méthoxyquinolin-4-yl)propyl]-1-[2-(3-fluorophénylthio)-2-éthyl]pipéridine-3-carboxylate de méthyle dans 4 cm³ de méthanol et 0,8 cm³ de soude aqueuse 5N est chauffé sous agitation à 60°C pendant 20 heures. Après mélange des solvants sous pression réduite (5 kPa), à une température voisine de 40°C, le résidu obtenu est repris dans 10 cm³ d'eau, puis acidifié par 0,4 cm³ d'acide chlorhydrique concentré. La solution est mélangée dans les mêmes conditions, puis le résidu obtenu est trituré dans un mélange de dichlorométhane-méthanol (90/10 en volumes). L'insoluble est filtré, lavé par 5 cm³ de d ichlorométhane. Le filtrat est séché sur sulfate de sodium, puis concentré sous pression réduite (5 kPa), à une température voisine de 40°C. Le résidu est trituré dans 10 cm³ d'éther diisopropylique, puis additionné, sous agitation, de 1 cm³ d'éther diisopropylique chlorhydrique 5N. Les cristaux sont séparés par filtration, puis lavés par 2 fois 5 cm³ d'éther diisopropylique. Après séchage à l'air, on obtient 0,45 g de dichlorhydrate d'acide (3R,4R)-4-[3-(6-méthoxyquinolin-4-yl)propyl]-1-[2-(3-fluoro phénylthio)-2-éthyl]pipéridine-3-carboxylique,_sous forme d'un solide amorphe de couleur jaune-pâle fondant au voisinage de 140°C en devenant pâteux.
Spectre infra rouge (KBr) : 3058 et 3012 cm⁻¹ (ν CH aromatiques); 2935 et 2862 cm⁻ ¹ (ν CH₂) ; 3000 et 2750 cm⁻¹ (ν OH acide); 2800 et 1900 cm⁻¹ (ν N⁺H ( sel d'amine tertiaire + sel de quinoline)) ; 1719 cm⁻¹ (ν C=O acide) ; 1618 ;1600 ;1578 ;1541 et1496 cm⁻¹ (ν C=C noyaux aromatiques); 1274 cm⁻¹ (ν C-O acide ); 1251 et 1216 cm⁻¹ (νₐₛ C-O éther) ; 1021 cm⁻¹ (νₛ C-O éther + n C-O alcool) ; 847 cm⁻¹ (γ CH quinoline 4-6 disubstituée); 781 et 729 cm⁻¹ (γ CH phényl 1-3 disubstitué).
Spectre de masse (IE - m/z) : =482 (M⁺); 438 (M-CO₂)⁺; 341 (M-C₇H₆SF)⁺ pic de base ; 297 ; 341 (M- CO₂)⁺; 186 (C₁₂H₁₂NO⁺); 128 (C₆H₅SF⁺); 36 (HCl⁺)

Le (3R,4R)-4-[3-(6-méthoxyquinolin-4-yl)propyl]-1-[2-(3-fluorophénylthio)-2-éthyl] pipéridine-3-carboxylate de méthyle peut être préparé de la manière suivante :

En opérant par analogie avec l'exemple 3 ci-après, mais à partir de chlorhydrate du (3R,4R)-4-[3-(6-méthoxyquinolin-4-yl)propyl]pipéridine-3-carboxylate de méthyle et de 2-bromo-1-(3-fluorophényl)thioéthane, on obtient 0,55 g de (3R,4R)-4-[3-(6-méthoxyquinolin-4-yl)propyl]-1-[2-(3-fluorophénylthio)-2-éthyl]pipéridine-3-carboxylate de méthyle, sous forme d'une huile visqueuse de couleur jaune.
Spectre infra rouge (CCl₄)2949 cm⁻¹ νCH aliphatiques ;1737 cm⁻¹ νC=O;1227 cm⁻¹ ν C-O éther ;845 cm⁻¹ γCH quinoline.

Le chlorhydrate de (3R,4R)-4-[3-(6-méthoxyquinolin-4-yl)propyl]pipéridine-3-carboxylate de méthyle a été préparé dans les conditions de l'exemple 4.

### Exemple 3

### Acide (3R,4R)-4-(3-(6-méthoxyquinolin-4-yl)propyl]-1-[2-(phénylthioéthyl)] pipéridine-3-carboxylique

Une suspension de 0,7 g de (3R,4R)-4-[3-(6-méthoxyquinolin-4-yl)propyl]-1-[2-(phénylthioéthyl)]pipéridine-3-carboxylate de méthyle dans 5 cm³ de méthanol additionnés de 2,9 cm³ de soude aqueuse N est agitée pendant 2 heures à une température voisine de 80°C. La solution obtenue est neutralisée par 0,18 cm³ d'acide acétique, puis mélangée sous pression réduite (5 kPa), à une température voisine de 40°C. Le résidu obtenu est purifié par chromatographie à pression atmosphérique sur une colonne de gel de silice (granulométrie 40-63 µ ; diamètre 3 cm ; hauteur 20 cm), en éluant par un mélange de dichlorométhane-éthanol (90/10 en volumes), et en recueillant des fractions de 20 cm³. Les fractions 21 à 52 sont réunies, concentrées sous pression réduite (5 kPa), à une température voisine de 40°C. On obtient 0,53 g d'acide (3R,4R)-4-[3-(6-méthoxyquinolin-4-yl)propyl]-1-[2-(phénylthioéthyl)] pipéridine-3-carboxylique, sous forme d'une huile de couleur beige.
Spectre de R.M.N. ¹H (400 MHz, (CD₃)₂SO d6, δ en ppm) : de 1,35 à 1,95 (mt : 7H); 2,28 (t large, J = 10,5 Hz : 1H) ; 2,43 (d large, J = 10,5 Hz : 1H) ; 2,59 (mt : 1H) ; 2,64 (t, J = 7 Hz : 2H) ; 2,77 (mf : 1H) ; 2,93 (mf : 1H) ; 3,03 (mt : 2H) ; 3,13 (mt : 2H) ; 3,95 (s : 3H) ; 7,21 (tt, J = 7,5 et 2 Hz : 1H) ; de 7,25 à 7,45 (mt : 7H) ; 7,93 (d, J = 9 Hz : 1H) ; 8,63 (d, J = 4,5 Hz : 1H).

Le (3R,4R)-4-[3-(6-méthoxyquinolin-4-yl)propyl]-1-(2-phénylthioéthyl)]pipéridine-3-carboxylate de méthyle peut être préparé de la manière suivante :
1 g de chlorhydrate de (3R,4R)-4-[3-(6-méthoxyquinolin-4-yl)propyl]pipéridine-3-carboxylate de méthyle 1 g de carbonate de potassium sont agités à une température voisine de 20°C dans 100 cm³ d'acétonitrile pendant 20 minutes. Après addition de 0,61 g de 2-bromo-1-phénylthioéthane préalablement dissous dans 5 cm³ d'acétonitrile, le mélange est chauffé à une température voisine de 60°C pendant 5 heures. Après addition de 20 cm³ de diméthylformamide et 0,61 g supplémentaire de 2-bromo-1-phénylthioéthane, le chauffage est maintenu encore 8 heures 30 minutes. Après refroidissement, le mélange réactionnel est filtré; la solution obtenue est concentrée sous pression réduite (5 kPa), à une température voisine de 70°C. Le résidu est repris dans 50 cm³ d'éthanol, puis concentré à nouveau dans les mêmes conditions que ci-dessus. Le résidu est dilué par 30 cm³ d'eau, puis extrait par 3 fois 20 cm³ de dichlorométhane. Les extraits réunis sont séchés sur sulfate de magnésium, concentrés sous pression réduite (5 kPa), à une température voisine de 40°C. On obtient 1 g d'une huile que l'on purifie par chromatographie à pression atmosphérique sur une colonne de gel de silice (granulométrie 40-63 µ ; diamètre 3,5 cm ; hauteur 20 cm), en éluant par un mélange de dichlorométhane-éthanol (90/10 en volumes), et en recueillant des fractions de 25 cm³. Les fractions 15 à 26 sont réunies, puis concentrées sous pression réduite (5 kPa), à une température voisine de 40°C. On obtient 0,79 g de (3R,4R)-4-[3-(6-méthoxyquinolin-4-yl)propyl]-1-(2-phénylthioéthyl)pipéridine-3-carboxylate de méthyle, sous forme d'une huile de couleur jaune.

Le chlorhydrate de (3R,4R)-4-[3-(6-méthoxyquinolin-4-yl)propyl]pipéridine-3-carboxylate de méthyle peut être préparé dans les conditions de l'exemple 4.

### Exemple de référence 4

Le chlorhydrate de (3R,4R)-4-[3-(6-méthoxyquinolin-4-yl)propyl]pipéridine-3-carboxylate de méthyle peut être préparé de la manière suivante :

A une suspension agitée de 4,29 g d'acide (3R,4R)-4-[3-(6-méthoxyquinolin-4-yl)propyl]-1-(tert-butyloxycarbonyl)pipéridine-3-carboxylique dans 50 cm³ de méthanol et refroidie à une température voisine de -30°C par un bain réfrigérant d'acétone et de carboglace, on ajoute goutte à goutte 2 cm³ de chlorure de thionyle. La solution obtenue est ramenée à une température voisine de 20°C, et le mélange réactionnel est agité pendant 16 heures à cette température. Après mélange de la solution sous pression réduite (5 kPa), à une température voisine de 40°C, le résidu obtenu est trituré dans 30 cm³ environ d'éther diisopropylique. Les cristaux obtenus sont essorés, lavés par 2 fois 10 cm³ d'éther diisopropylique, puis séchés à l'air. On obtient 4,20 g de chlorhydrate de (3R,4R)-4-[3-(6-méthoxyquinolin-4-yl)propyl] pipéridine-3-carboxylate de méthyle, sous forme d'un solide de couleur jaune clair, fondant en se ramollissant à une température voisine de 140°C.

L'acide (3R,4R)-4-[3-(6-méthoxyquinolin-4-yl)propyl]-1-(tert-butyloxycarbonyl) pipéridine-3-carboxylique peut être préparé de la manière suivante :

3 g de (3R,4R)-4-[3-(6-méthoxyquinolin-4-yl)propyl]-3-vinylpipéridine sont dissous dans 3 cm³ d'acétone. A cette solution, refroidie à une température voisine de 5°C par un bain de glace et d'acétone, on ajoute sous agitation 14,5 cm³ d'acide sulfurique 3M préalablement refroidis à cette même température. A la solution obtenue, on ajoute en 30 minutes une solution de 4,64 g de permanganate de sodium monohydraté dans 25 cm³ d'eau, en maintenant la température entre 0 et 7°C. Le mélange réactionnel est agité pendant 4 heures à une température comprise entre 10 et 17°C. La masse réactionnelle est filtrée ; l'insoluble est lavé par 2 fois 10 cm³ d'eau. On obtient d'une part une solution de couleur orangée, et d'autre part une masse minérale de couleur noire. La solution orangée est amenée à pH 10 par addition de 4,6 g de carbonate de sodium. Le mélange est filtré : on obtient une solution (1) et un insoluble minéral (2). La masse minérale de couleur noire est agitée pendant 30 minutes dans 20 cm³ d'eau après que le pH ait été amené à 12 par addition de 2 cm³ de lessive de potasse. Après filtration du mélange, on obtient une solution (3) et un insoluble minéral (4). Les insolubles (2) et (4) sont agités pendant 15 minutes dans 15 cm³ d'eau additionnés de 3 cm³ de lessive de potasse. La suspension est filtrée. On obtient une solution (5). Les solutions aqueuses (1), (3) et (5) sont réunies, additionnées de 2,31 g de di-tertiobutyl dicarbonate, et agitées pendant 15 heures à une température voisine de 20°C. Le mélange est extrait par 6 fois 10 cm³ d'acétate d'éthyle. Les phases organiques sont réunies, lavées par 20 cm³ d'eau, puis par 20 cm³ d'une solution aqueuse saturée de chlorure de sodium. Après séchage sur sulfate de sodium, filtration, puis concentration sous pression réduite (5 kPa), à une température voisine de 35°C, on obtient 2,86 g d'acide (3R,4R)-4-[3-(6-méthoxyquinolin-4-yl)propyl]-1-(tert-butyloxycarbonyl) pipéridine-3-carboxylique, sous forme d'un solide de couleur beige, devenant pâteux à 154°C.

La (3R,4R)-4-[3-(6-méthoxyquinolin-4-yl)propyl]-3-vinylpipéridine peut être obtenue par application de la méthode décrite dans la demande de brevet FR 2354771.

### Exemple 5

### Trichlorhydrate de l'acide (3R,4R)-4-[3-(6-méthoxyquinolin-4-yl)propyl]-1-[2-(1,3-thiazol-2-ylthioéthyl)]pipéridine-3-carboxylique

Un mélange de 0,3 g de (3R,4R)-4-[3-(6-méthoxyquinolin-4-yl)propyl]-1-[2-(1,3-thiazol-2-ylthioéthyl)]pipéridine-3-carboxylate de méthyle et de 0,44 cm³ de soude aqueuse 5N dans 2,5 cm³ de méthanol est chauffé à une température voisine de 60°C, sous agitation, pendant 20 heures. Après refroidissement, le mélange réactionnel est mélangé sous pression réduite (5 kPa), à une température voisine de 40°C ; le résidu est repris dans 5 cm³ d'eau, puis acidifié par addition de 1 cm³ d'acide chlorhydrique à 35 %. Le mélange est mélangé dans les mêmes conditions que ci-dessus, puis le résidu obtenu est trituré dans 10 cm³ d'un mélange de dichlorométhane-méthanol (90/10 en volumes). Après filtration de l'insoluble, le filtrat est mélangé sous pression réduite (5 kPa), à une température voisine de 40°C. On obtient 0,34 g de trichlorhydrate de l'acide (3R,4R)-4-[3-(6-méthoxyquinolin-4-yl)propyl]-1-[2-(1,3-thiazol-2-ylthioéthyl)]pipéridine-3-carboxylique, sous forme d'une meringue de couleur jaune-pâle.
Spectre infra rouge (KBr) : 3097 cm⁻¹ (ν CH thiazole) ; 3058 et 3012 cm⁻¹ (ν CH aromatiques) ; 2929 et 28615 cm⁻¹ (ν CH₂) ; 3000 et 2750 cm⁻¹ (ν OH acide) ; 2800 et 1900 cm⁻¹ (ν N⁺H ( sel d'amine tertiaire + sel de quinoline)) ; 1715 cm⁻¹ (ν C=O acide) ; 1617 ;1600 ;1543 et 1496 cm⁻¹ (ν C=C noyaux aromatiques) ; 1274 cm⁻¹ (ν C-O acide); 1250 et 1219 cm⁻¹ (νₐₛ C-O éther) ; 1020 cm⁻¹ (νₛ C-O éther + ν C O alcool) ; 846 cm⁻¹ (γ CH quinoline 4-6 disubstituée) ; 740 cm⁻¹ (γ CH thiazole) .
Spectre de masse (IE - m/z) : 471 (M⁺) ; 355 (M-C₃H₂NS₂)⁺ ; 341 (M-C₄H₄NS₂)⁺ pic de base ; 297 ; 341 (M- CO₂)⁺; 186 (C₁₂H₁₂NO⁺) ; 117 (C₃H₃NS₂⁺) ; 44 (CO₂⁺).

### (3R,4R)-4-[3-(6-méthoxyquinolin-4-yl)propyl]-1-[2-(1,3-thiazol-2-ylthioéthyl)] pipéridine-3-carboxylate de méthyle

En opérant par analogie avec l'exemple 3, mais à partir de (3R,4R)-4-[3-(6-méthoxyquinolin-4-yl)propyl]pipéridine-3-carboxylate de méthyle et de 2-bromo-1-[(1,3-thiazol-2-yl)thio]éthane, on obtient 0,31 g de (3R,4R)-4-[3-(6-méthoxyquinolin-4-yl)propyl]-1-[2-(1,3-thiazol-2-ylthioéthyl)]pipéridine-3-carboxylate de méthyle, sous forme d'une laque de couleur orangée.
Spectre de R.M.N. ¹H (300 MHz, (CD₃)₂SO d6, δ en ppm) : de 1,30 à 1,85 (mt : 7H) ; de 2,20 à 2,35 (mt : 1H) ; 2,35 (dd, J = 11 et 3 Hz : 1H) ; de 2,50 à 2,85 (mt : 5H) ; 3,03 (t, J = 7 Hz : 2H) ; de 3,25 à 3,40 (mt : 2H) ; 3,53 (s : 3H) ; 3,94 (s : 3H) ; 7,31 (d, J = 5 Hz : 1H) ; 7,35 (d, J = 2,5 Hz : 1H) ; 7,40 (dd, J = 9 et 2,5 Hz : 1H) ; 7,63 (d, J = 3,5 Hz : 1H) ; 7,72 (d, J = 3,5 Hz : 1H) ; 7,93 (d, J = 9 Hz : 1H) ; 8,63 (d, J = 5 Hz : 1H).

### Exemple 6

### Chlorhydrate de l'acide (3R,4R)-4-[3-(6-méthoxyquinolin-4-yl)propyl]-1-[2-(2-fluorophénylthio)éthyl]pipéridine-3-carboxylique

On agite pendant 20 heures à une température voisine de 60°C un mélange de 0,12 g (3R,4R)-4-[3-(6-méthoxyquinolin-4-yl)propyl]-1-[2-(2-fluorophénylthio)éthyl] pipéridine-3-carboxylate de méthyle, 0,6 cm³ de soude aqueuse N dans 1,8 cm³ de méthanol. Le mélange réactionnel est mélangée sous pression réduite (5 kPa), à une température voisine de 40°C. Le résidu obtenu est repris par 5 cm³ d'eau, et acidifié par 1 cm³ d'acide chlorhydrique aqueux 2N. Le mélange est à nouveau mélangé dans les mêmes conditions q ue ci-dessus, puis, le nouveau résidu e st trituré dans 5 cm³ d'un mélange de dichlorométhane-méthanol (90/10 en volumes). Après filtration et mélange du filtrat sous pression réduite (5 kPa), à une température voisine de 40°C, le résidu obtenu est trituré dans 3 cm³ d'éther diisopropylique. L'insoluble est filtré, lavé par 2 fois 1 cm³ d'éther diisopropylique, séché à l'air. On obtient 0,14 g de chlorhydrate de l'acide (3R,4R)-4-[3-(6-méthoxyquinolin-4-yl)propyl]-1-[2-(2-fluorophényl thio)éthyl]pipéridine-3-carboxylique, sous forme d'un solide de couleur beige fondant, en se ramollissant, vers 148°C.
Spectre infra rouge (KBr) : 3058 et 3013 cm⁻¹ (ν CH aromatiques) ; 2934 et 2862 cm⁻¹ (ν CH₂); 3000 et 2750 cm⁻¹ (ν OH acide) ; 2800 et 1900 cm⁻¹ (ν N⁺H ( sel d'amine tertiaire + sel de quinoline)) ; 1719 cm⁻¹ (ν C=O acide) ; 1618 ;1600 ;1541 et 1497 cm⁻¹ (ν C=C noyaux aromatiques) ; 1276 cm⁻¹ (ν C-O acide) ; 1251 et 1219 cm⁻¹ (νₐₛ C-O éther) ; 1022 cm⁻¹ (νₛ C-O éther + n C-O alcool) ; 847 cm⁻¹ (γ CH quinoline 4-6 disubstituée) ; 760 cm⁻¹ (γ CH phényl 1-2 disubstitué).
Spectre de masse (IE - m/z) : 438 (M-CO₂)⁺; 355 (M-C₆H₄SF)⁺; 341 (M-C₇H₆SF)⁺ pic de base ; 297 ; 341 (M-CO₂)⁺; 186 (C₁₂H₁₂NO⁺) ; 128 (C₆H₅SF⁺); 36 (HCl⁺.)
Spectre de masse (DCI) : m/z=483 (M+H)⁺

### (3R,4R)-4-[3-(6-méthoxyquinolin-4-yl)propyl]-1-[2-(2-fluorophénylthio)éthyl]pipéridine-3-carboxylate de méthyle

En opérant par analogie avec l'exemple 3, mais à partir de (3R,4R)-4-[3-(6-méthoxyquinolin-4-yl)propyl]pipéridine-3-carboxylate de méthyle et de 2-bromo-1-(2-fluorophénylthio)éthane, on obtient 0,17 g de (3R,4R)-4-[3-(6-méthoxyquinolin-4-yl)propyl]-1-[2-(2-fluorophénylthio)éthyl]pipéridine-3-carboxylate de méthyle, sous forme d'une laque de couleur orangée.
Spectre infra rouge (CH₂Cl₂) : 2942 cm⁻¹ nCH aliphatiques ;1727 cm⁻¹ nC=O ;1227 cm⁻¹ n C-O éther ;848 cm⁻¹ gCH quinoline.

### Exemple 7

### Dichlorhydrate de l'acide (3R,4R)-4-[3-(6-méthoxyquinolin-4-yl)propyl]-1-[2-(thién-2-ylthio)éthyl]pipéridine-3-carboxylique

A une solution agitée de 0,34 g de (3R,4R)-4-[3-(6-méthoxyquinolin-4-yl)propyl]-1-[2-(thièn-2-ylthio)éthyl]pipéridine-3-carboxylate de méthyle dans 3 cm³ de méthanol, on ajoute 0,5 cm³ de soude aqueuse 5N, puis le mélange est chauffé à une température voisine de 60°C pendant 20 heures. Après mélange des solvants sous pression réduite (5 kPa), à une température voisine de 40°C, le résidu obtenu est repris par 5 cm³ d'eau, puis acidifié par addition de 1 cm³ d'acide chlorhydrique à 35 %. Le mélange est à nouveau mélangé sous pression réduite dans les mêmes conditions que ci-dessus ; le résidu obtenu est repris par 5 cm³ d'un mélange de dichlorométhane-méthanol (90/10 en volumes). L'insoluble est filtré, puis le solvant est mélangé sous pression r éduite (5 k Pa), à u ne t empérature voisine d e 40°C. On obtient 0,35 g de dichlorhydrate de l'acide (3R,4R)-4-[3-(6-méthoxyquinolin-4-yl)propyl)-1-[2-(thièn-2-ylthio)éthyl]pipéridine-3-carboxylique, sous forme d'un solide de couleur beige fondant en se ramollissant au voisinage de 150°C.
Spectre infra rouge (KBr) : 3102 cm⁻¹ (ν CH thiophène) ; 3058 et 3012 cm⁻¹ (ν CH aromatiques) ; 2932 et 2865 cm⁻¹ (ν CH₂) ; 3000 et 2750 cm⁻¹ (ν OH acide) ; 2800 et 1900 cm⁻¹ (ν N⁺H ( sel d'amine tertiaire + sel de quinoline)) ; 1717 cm⁻¹ (ν C=O acide) ; 1618 ;1600 ;1541 et 1496 cm⁻¹ (ν C=C noyaux aromatiques) ; 1276 cm⁻¹ (ν C-O acide) ; 1250 et 1218 cm⁻¹ (νₐₛ C-O éther) ; 1020 cm⁻¹ (νₛ C-O éther + ν C-O alcool) ; 846 cm⁻¹ (γ CH quinoline 4-6 disubstituée) ; 725 cm⁻¹ (γ CH thiophène).
Spectre de masse (IE - m/z) : 355 (M-C₄H₃S₂)⁺; 341 (M-C₅H₅S₂)⁺ pic de base ; 297 ; 341- M-CO₂)⁺/; 186 (C₁₂H₁₂NO⁺) ; 115 (C₄H₃S₂⁺)
Spectre de masse (DCI) m/z=471 (M+H)⁺

### (3R,4R)-4-[3-(6-méthoxyquinolin-4-yl)propyl]-1-[2-(thién-2-ylthio)éthyl]pipéridine-3-carboxylate de méthyle

En opérant par analogie avec l'exemple 3, mais à partir de (3R,4R)-4-[3-(6-méthoxyquinolin-4-yl)propyl]pipéridine-3-carboxylate de méthyle et de 2-bromo-1-(thién-2-ylthio)éthane, on obtient 0,34 g de (3R,4R)-4-[3-(6-méthoxyquinolin-4-yl)propyl]-1-[2-(thién-2-ylthio)éthyl] pipéridine-3-carboxylate de méthyle, sous forme d'une laque de couleur verte.
Spectre de R.M.N. ¹H (300 MHz, (CD₃)₂SO d6, δ en ppm) : de 1,30 à 1,90 (mt : 7H); de 2,15 à 2 ,30 (mt : 1H); de 2,35 à 2,60 (mt : 4H); de 2,65 à 2,80 (mt : 2H) ; 3,02 (t, J = 7 Hz : 2H) ; 3,03 (t large, J = 7,5 Hz : 2H) ; 3,54 (s : 3H) ; 3,95 (s : 3H) ; 7,06 (dd, J = 5,5 et 3,5 Hz : 1H) ; 7,19 (dd, J = 3,5 et 1,5 Hz : 1H) ; 7,32 (d, J = 4,5 Hz : 1H) ; 7 ,36 (d, J = 3 Hz : 1H) ; 7,42 (dd, J = 9 et 3 Hz : 1H) ; 7,62 (dd, J = 5,5 et 1,5 Hz : 1H) ; 7,95 (d, J = 9 Hz : 1H) ; 8,64 (d, J = 4,5 Hz : 1H).

### Exemple 8

### Trichlorhydrate de l'acide (3R,4R)-4-[3-(6-méthoxyquinolin-4-yl)propyl]-1-[2-(pyridin-2-ylthio)éthyl]pipéridine-3-carboxylique

Une solution de 0,5 g de (3R,4R)-4-[3-(6-méthoxyquinolin-4-yl)propyl]-1-[2-(pyridin-2-ylthio)éthyl]pipéridine-3-carboxylate de méthyle dans 7,8 cm³ d'acide chlorhydrique 6N est chauffée sous agitation à une température voisine de 100°C pendant 2 heures. Après concentration à sec du mélange réactionnel, sous pression réduite (5 kPa), à une température voisine de 80°C, le résidu obtenu est trituré dans 10 cm³ d'éther diisopropylique. L'insoluble est essoré, puis séché sous pression réduite (13 kPa), à une température voisine de 60°C. On obtient 0,55 g de trichlorhydrate de l'acide (3R,4R)-4-[3-(6-méthoxyquinolin-4-yl)propyl]-1-[2-(pyridin-2-ylthio)éthyl]pipéridine-3-carboxylique, sous forme d'un solide de couleur crème, fondant en se ramollissant au voisinage de 165°C.
Spectre de R.M.N. 1H (400 MHz, (CD₃)₂SO d6, δ en ppm) : de 1,20 à 2,40 et de 3,00 à 3,60 (mts : 16H) ; 3,62 (t large, J = 7,5 Hz : 2H) ; 4,05 (s : 3H) ; 7,21 (dd, J = 8 et 5 Hz : 1H) ; 7,43 (d, J = 8 Hz : 1H) ; 7,67 (s large : 1H) ; 7,73 (t dédoublé, J = 8 et 1,5 Hz : 1H) ; 7,84 (dd, J = 9 et 2,5 Hz : 1H) ; 7,95 (d, J = 5 Hz : 1H) ; 8,45 (d, J = 9 Hz : 1H) ; 8,49 (d large, J = 5 Hz : 1H) ; 9,05 (d, J = 5 Hz : 1H) ; 11,25 (mf : 1H).

### (3R,4R)-4-[3-(6-méthoxyqainolin-4-yl)propyl]-1-[2-(pyridin-2-ylthio)éthyl] pipéridine-3-carboxylate de méthyle

En opérant par analogie avec l'exemple 3, mais à partir de (3R,4R)-4-[3-(6-méthoxyquinolin-4-yl)propyl]pipéridine-3-carboxylate de méthyle et de 2-bromo-1-(pyridin-2-ylthio)éthane, on obtient 0,52 g de (3R,4R)-4-[3-(6-méthoxyquinolin-4-yl)propyl]-1-[2-(pyridin-2-ylthio) éthyl]pipéridine-3-carboxylate de méthyle, sous forme d'une huile de couleur jaune.
Spectre infra rouge (CH₂Cl₂) : 2949 cm⁻¹ nCH aliphatiques ;1737 cm⁻¹ nC=O ;1227 cm⁻¹ n C-O éther ;845 cm⁻¹ gCH quinoline.

### Exemple 9

### Dichlorhydrate de l'acide (3R,4R)-4-[3-(6-méthoxyquinolin-4-yl)propyl]-1-[2-(cyclohexylthio)-2-éthyl]pipéridine-3-carboxylique

Une solution de 0,55 g de (3R,4R)-4-[3-(6-méthoxyquinolin-4-yl)propyl]-1-[2-(cyclohexylthio)-2-éthyl]pipéridine-3-carboxylate de méthyle dans 8,5 cm³ d'acide chlorhydrique 6 N est chauffée sous agitation, à une température voisine de 100°C, pendant 2 heures. Le mélange réactionnel est refroidi, puis concentré à sec sous pression réduite (5 kPa), à une température voisine de 80°C. Le résidu obtenu est trituré dans 15 cm³ d'éther diisopropylique. L'insoluble est filtré, puis le gâteau est lavé par 10 cm³ d'éther diisopropylique. Le solide obtenu est séché sous pression réduite (13 Pa), à une température voisine de 60°C, pendant 2 heures. On obtient 0,51 g dichlorhydrate de l'acide (3R,4R)-4-[3-(6-méthoxyquinolin-4-yl)propyl]-1-[2-(cyclohexylthio)-2-éthyl]pipéridine-3-carboxylique, sous forme d'un solide de couleur beige, fondant en se ramollissant vers 150°C.
Spectre de R.M.N.1H (400 MHz, (CD3)2SO d6, avec ajout de quelques gouttes de CD3COOD d4, à une température de 373K, δ en ppm) : de 1,20 à 2,35 et de 2,75 à 3,50 (mts : 29H) ; 4,03 (s : 3H) ; 7,57 (mt : 1H) ; de 7,60 à 7,75 (mt : 2H) ; 8,24 (d, J = 9 Hz : 1H) ; 8,85 (d, J = 5 Hz : 1H).

### (3R,4R)-4-[3-(6-méthoxyquinolin-4-yl)propyl]-1-[2-(cyclobexylthio)-2-éthyl]pipéridine-3-carboxylate de méthyle

En opérant par analogie avec l'exemple 3, mais à partir de (3R,4R)-4-[3-(6-méthoxyquinolin-4-yl)propyl]pipéridine-3-carboxylate de méthyle et de 2-bromo-1-(cyclohexylthio)éthane, on obtient 0,57 g de (3R,4R)-4-[3-(6-méthoxyquinolin-4-yl)propyl]-1-[2-(cyclohexylthio)-2-éthyl]pipéridine-3-carboxylate de méthyle, sous forme d'une huile de couleur orange.
Spectre infra rouge (CH₂Cl₂) : 2934 cm⁻¹ νCH aliphatiques ;1732 cm⁻¹ νC=O ;1227 cm⁻¹ ν C-O éther ;848 cm⁻¹ γCH quinoline.

### Exemple de référence 10

### Di-trifluoroacétate de l'acide (3R, 4R)-4-[3-(6-méthoxyquinolin-4-yl)propyl]-1-[4-(2-thiényl)butan-4-one]-pipéridine-3-carboxylique

A une solution de 1,55 g de di-trifluoroacétate de l'acide (3R, 4R)-4-[3-(6-méthoxy-4-quinolin-4-yl)propyl]-pipéridine-3-carboxylique dans 40 cm³ d'acétone anhydre, on ajoute à une température voisine de 20°C, sous agitation et sous atmosphère inerte, 1,76 cm³ de 4-chloro-2'-butyrothiénone, puis 3,86 g de carbonate de potassium. Le mélange est chauffé à une température voisine de 57°C pendant 20 heures. Après refroidissement du mélange réactionnel, puis filtration de l'insoluble, le gâteau est lavé par 10 cm³ d'acétone, puis le filtrat est mélangé sous pression réduite (5 kPa), à une température voisine de 40°C. Le résidu obtenu est purifié par chromatographie à pression atmosphérique, sur une colonne de gel de silice (granulométrie 20-45 µ; diamètre 4 cm ; hauteur 35 cm), en éluant par un mélange de dichlorométhane-méthanol (90/10 en volumes), et en recueillant des fractions de 100 cm³. Les fractions 43 à 122 sont réunies, puis mélangées sous pression réduite (5 kPa), à une température voisine de 40°C. On obtient 1,09 g d'une huile que l'on soumet à une nouvelle purification par chromatographie à pression atmosphérique sur une colonne de gel de silice (granulométrie 20-45 µ ; diamètre 4 cm ; hauteur 35 cm), en éluant par un mélange de dichlorométhane-méthanol (90/10 en volumes), et en recueillant des fractions de 100 cm³. Les fractions 125 à 216 sont réunies, puis mélangées sous pression réduite (5 kPa), à une température voisine de 40°C. On obtient 0,48 g d'une huile que l'on purifie sous forme de di-trifluoroacétate, préparé à partir de 0,1 cm³ d'acide trifluoroacétique dans un mélange de 10 cm³ de dichlorométhane et 5 cm³ de méthanol. On obtient, après mélange du mélange réactionnel (sous une pression partielle de 5 kPa et à une température voisine de 40°C), puis reprise du résidu obtenu par 10 cm³ d'éther diéthylique et filtration du solide, 0,35 g de di-trifluoroacétate de l'acide (3R, 4R)-4-[3-(6-méthoxyquinolin-4-yl)propyl]-1-[4-(2-thiényl) butan-4-one]-pipéridine-3-carboxylique, sous forme d'un solide de couleur beige, à 90 % de pureté.

Spectre de R.M.N. 1H (400 MHz, (CD3)2SO d6, avec ajout de quelques gouttes de CD3COOD d4, à une température de 383K, δ en ppm) : de 1,50 à 2,25 et de 2,95 à 3,55 (mts : 20H) ; 3,96 (s : 3H) ; 7,23 (mt : 1H) ; 7,36 (d, J = 5 Hz : 1H) ; de 7,40 à 7,50 (mt : 2H) ; 7,88 (d, J = 4 Hz : 1H) ; 7,92 (d, J = 5 Hz : 1H) ; 7,99 (d, J = 9 Hz : 1H) ; 8,66 (d, J = 5 Hz : 1H).

### Di-trifluoroacétate de l'acide (3R, 4R)-4-[3-(6-méthoxyquinolin-4-yl)propyl]-pipéridine-3-carboxylique

A une suspension agitée de 1,5 g de l'acide (3R,4R)-1-(*tert*-butyloxycarbonyl)-4-[3-(6-methoxy-4-quinolin-4-yl)propyl]-pipéridine-3-carboxylique dans 15 cm³ de dichlorométhane, on ajoute à une température voisine de 20°C, 1,75 cm³ d'acide trifluoroacétique pur. La solution obtenue est agitée pendant 20 heures à la même température, puis mélangée sous pression réduite (5 kPa), à une température voisine de 40°C. On obtient 2,46 g de di-trifluoroacétate de l'acide (2R,4R)-4-[3-(6-méthoxyquinolin-4-yl)propyl]-pipéridine-3-carboxylique, sous forme d'une huile de couleur brune.

L'acide (3R, 4R)-1-(t-butyloxycarbonyl)-4-[3-(6-méthoxyquinolin-4-yl)propyl]-pipéridine-3-carboxylique a été préparé comme dans l'exemple 4.

### Exemple 11

### Dichlorhydrate de l'acide (3R, 4R)-1-[2-(3-fluorophénylthio)éthyl]-4-[3-(R, S)-hydroxy-3-(6- méthoxyquinolin-4-yl)propyl]-pipéridine-3-carboxylique

0,8 g de (3R, 4R)-1-[2-(3-fluorophénylthio)éthyl]-4-[3-(R, S)-hydroxy-3-(6-méthoxyquinolin-4-yl)propyl]-pipéridine-3-carboxylate de méthyle dans 10 cm³ de méthanol et 1,25 cm³ de soude 5N sont chauffés, sous agitation, à une température voisine de 60°C pendant 4 heures. Le mélange réactionnel est mélangé sous pression réduite (5 kPa), à une température voisine de 50°C. Au résidu obtenu, on ajoute 15 cm³ d'eau puis on ajoute 2 cm³ d'acide chlorhydrique aqueux 5N. Le mélange réactionnel est de nouveau mélangé à sec. Le résidu obtenu est trituré avec un mélange de dichlorométhane/méthanol (90/10 en volumes). Le chlorure de sodium est filtré, puis le filtrat est concentré sous pression réduite (5 kPa), à une température voisine de 30°C. La meringue obtenue est triturée avec de l'éther éthylique. Le solide formé est filtré. On obtient 0,75 g de solide beige. Ce solide est dissous dans un mélange de 50 cm³ de chloroforme et de 50 cm³ d'acétonitrile. L'insoluble est filtré, puis le filtrat est acidifié avec 20 cm³ d'éther chlorhydrique 1N. Le mélange réactionnel est mélangé sous pression réduite (5 kPa), à une température voisine de 30°C. Le résidu obtenu est trituré avec de l'éther éthylique. Le solide formé est filtré puis séché sous vide. On obtient 0,7 g de dichlorhydrate de l'acide (3R,4R)-1-[2-(3-fluorophénylthio)éthyl]-4-[3-(R,S)-hydroxy-3-(6-méthoxyquinolin-4-yl)propyl]-pipéridine-3-carboxylique, sous forme de solide de couleur beige.
Spectre de R.M.N. ¹H (400 MHz, (CD₃)₂SO d6, δ en ppm) : de 1,25 à 1,90 (mt: 8H); 2,22 (mt : 2H) ; de 2,65 à 2,90 (mt : 2H) ; de 3,35 à 3,60 (mt : 4H) ; 3,91 et 3,93 (2s : 3H); 4,29 (mf: 1H) ; 5,28 (mt : 1H) ; 5,50 et 5,52 (2d, J = 4,5 Hz : 1H) ; 7,07 (mt : 1H); 7,28 (dd, J = 4 et 1 Hz : 1H) ; de 7,35 à 7,45 (mt : 2H) ; de 7,50 à 7,60 (mt : 2H); 7,95 (d, J = 9 Hz : 1H) ; 8,71 (d, J = 5 Hz : 1H).

### (3R,4R)-1-[2-(3-Fluorophénylthio)éthyl]-4-[3-(R,S)-hydroxy-3-(6-méthoxyquinolin-4-yl)propyl]-pipéridine-3-carboxylate de méthyl

A une solution de 1,8 g de (3R, 4R)-1-[2-(3-fluorophénylthio)éthyl]-4-[3-(6-méthoxyquinolin-4-yl)-propan-3-one]-pipéridine-3-carboxylate de méthyle dans 20 cm³ de méthanol, on ajoute sous agitation 0,16 g de borohydrure de sodium à une température inférieure à 25°C. Le mélange réactionnel est agité à la température ambiante pendant 2 heures. Après mélange du méthanol sous pression réduite (5 kPa), le mélange est agité avec 50 cm³ de dichlorométhane et 50 cm³ d'une solution saturée de chlorure d'ammonium. La phase organique est décantée, puis séchée sur sulfate de magnésium. Après filtration sur papier, concentration sous pression réduite (5 kPa), à une température voisine de 40°C, on obtient 1,6 g d'un produit que l'on purifie par chromatographie, à pression atmosphérique, sur une colonne de gel de silice (granulométrie 20-45 µ ; diamètre 3 cm ; 60 g), en éluant par un mélange de dichlorométhane-méthanol (96/4 en volumes), et en recueillant des fractions de 10 cm³. On recueille les fractions de 30 à 45. Ces fractions sont réunies, puis concentrées sous pression réduite (5 kPa) à environ 40°C. On obtient 1,05 g de (3R,4R)-1-[2-(3-fluorophénylthio)éthyl]-4-[3-hydroxy-3-(6-méthoxyquinolin-4-yl]-pipéridine-3-carboxylate de méthyle sous forme d'une huile mobile, brune.

Le (3R,4R)-1-[2-(3-Fluorophénylthio)éthyl]-4-[3-(6-méthoxyquinolin-4-yl)-propan-3-one]-pipéridine-3-carboxylate de méthyle peut être préparé de la manière suivante :

Un mélange de 6,44 g de (3R,4R)-4-[3-(6-méthoxyquinolin-4-yl)-propan-3-one]-pipéridine-3-carboxylate de méthyle dans 100 cm³ d'acétonitrile, de 3,43 g de 2-(3-fluoro phenylthio)éthyl-1-chlorure, de 8,85 g de carbonate de potassium et de 1,24 g de l'iodure de potassium est chauffé à une température voisine de 65°C pendant 48 heures. A près refroidissement, l'insoluble est filtré. Le filtrat est concentré sous pression réduite (5 kPa), à une t empérature voisine de 40°C. Le résidu huileux est purifié par chromatographie, à pression atmosphérique, sur une colonne de gel de silice (granulométrie 20-45 µ ; diamètre 6 cm ; 250 g), en éluant par un mélange de dichlorométhane-acétate d'éthyle-méthanol (50/50/3 en volumes), et en recueillant des fractions de 50 cm³. On recueille les fractions de 19 à 25. Ces fractions sont réunies, puis concentrées sous pression réduite (5 kPa) à environ 40°C. On obtient 2,1 g de (3R,4R)-1-[2-(3-fluorophénylthio)éthyl]-4-[3-(6-méthoxyquinolin-4-yl) propan-3-one]-pipéridine-3-carboxylate de méthyle sous forme d'une huile mobile, brune.

Le 2-(3-fluorophénylthio)éthyl-1-chlorure peut être obtenu de la manière suivante :

A une solution de 10 g de 3-fluorothiophénol, de 0,1 cm³ d'aliquat 336 dans 125 cm³ de 1,2-dichloroéthane, on ajoute, goutte à goutte, une solution de 3,75 g de pastille de soude dans 50 cm³ d'eau distillée. La température monte à 33°C. Le mélange réactionnel est agité à la température ambiante pendant 5 heures. Le mélange réactionnel est décanté. La phase organique est lavée avec 50 cm³ d'HCl 0,1N, avec 50 cm³ d'eau distillée, puis séchée sur sulfate de magnésium. Après filtration sur papier, concentration sous pression réduite (5 kPa), à une température voisine de 40°C, on obtient 15 g d'un résidu que l'on purifie par chromatographie, à pression atmosphérique, sur une colonne de gel de silice (granulométrie 20-45 µ ; diamètre 6 cm ; 400 g), en éluant avec du cyclohexane et en recueillant des fractions de 100 cm³. On recueille les fractions de 15 à 40. Ces fractions sont réunies, puis concentrées sous pression réduite (5 kPa) à environ 40°C. On obtient 13,6 g de 2-(3-fluorophényl thio)éthyl-1-chlorure sous forme d'une huile mobile, incolore.

### (3R,4R)-4-[3-(R,S)-hydroxy-3-(6-méthoxyquinolin-4-yl)propyl]-1-(3-phénylpropyl)pipéridine-3-carboxylate de méthyle

A une solution agitée de 1,59 g de (3R,4R)-4-[3-oxo-3-(6-méthoxyquinolin-4-yl) propyl]-1-(3-phénylpropyl)pipéridine-3-carboxylate de méthyle dans 25 cm³ de méthanol, on ajoute par petites fractions, à une température voisine de 20°C et sous atmosphère inerte, 0,15 g de borohydrure de sodium. Le mélange est ensuite agité pendant 75 minutes à une température voisine de 20°C. Puis, on ajoute 15 cm³ d'eau distillée en maintenant la même température. Le mélange, d'aspect laiteux, est concentré sous pression réduite (5 kPa), à une température voisine de 30°C. Le résidu obtenu est repris dans 40 cm³ d'eau distillée additionnés de 80 cm³ de dichlorométhane, agité, puis décanté. La phase organique est soutirée, puis lavée par une fois 40 cm³ d'eau, séchée sur sulfate de magnésium. Après filtration sur papier, puis mélange du solvant sous pression réduite (5 kPa), à une température voisine de 40°C, on obtient 1,39 g de (3R,4R)-4-[3-(R,S)-hydroxy-3-(6-méthoxyquinolin-4-yl)propyl]-1-(3-phénylpropyl)pipéridine-3-carboxylate de méthyle, sous forme d'un solide d'aspect meringué, et collant, de couleur orange.

Le (3R,4R)-4-[3-oxo-3-(6-méthoxyquinolin-4-yl)propyl]-1-(3-phénylpropyl) pipéridine-3-carboxylate de méthyle peut être préparé de la manière suivante :

Une suspension de 4,51 g de (3R,4R)-4-[3-oxo-3-(6-méthoxyquinolin-4-yl)propyl] pipéridine-3-carboxylate de méthyle et de 2,3 g de carbonate de potassium dans 75 cm³ d'acétone est chauffée sous agitation à une température voisine de 58°C. A cette température, on ajoute goutte à goutte une solution de 2,5 cm³ de 1-bromo-3-phénylpropane dans 7,5 cm³ d'acétone. Le chauffage est prolongé pendant 19 heures. Après refroidissement, la masse réactionnelle est filtrée ; le gâteau est lavé par 2 fois 30 cm³ d'acétone. Le filtrat et les eaux de lavage sont réunis, concentrés sous pression réduite (5 kPa), à une température voisine de 30°C. On obtient 7,12 g d'un produit sous forme d'huile que l'on purifie par chromatographie à pression atmosphérique sur une colonne de gel de silice (granulométrie 20-45 µ ; diamètre 7 cm ; masse 712 g), en éluant par un mélange chloroforme-méthanol-ammoniaque (12/2,25/0,38 en volumes), et en recueillant des fractions de 65 cm³. Les fractions 9 à 14 sont réunies, puis concentrées sous pression réduite (5 kPa), à une température voisine de 30°C. On obtient 6,7 g d'une huile que l'on soumet à une seconde purification par chromatographie à pression atmosphérique sur colonne de gel de silice (granulométrie 20-45 µ ; diamètre 4,8 cm ; masse 336 g), en éluant par un mélange d'acétate d'éthyle et de méthanol (9/1 en volumes), et en recueillant des fractions de 20 cm³. Les fractions 71 à 122 sont réunies puis concentrées sous pression réduite (5 kPa), à une température voisine de 30°C. On obtient 1,66 g de (3R,4R)-4-[3-oxo-3-(6-méthoxyquinolin-4-yl)propyl]-1-(3-phénylpropyl)pipéridine-3-carboxylate de méthyle, sous forme d'une huile de couleur brune.

Le (3R,4R)-4-[3-oxo-3-(6-méthoxyquinolin-4-yl)propyl]pipéridine-3-carboxylate de méthyle peut être préparé de la manière suivante :

Une solution de 19,4 g d'acide (3R,4R)-4-[3-oxo-3-(6-méthoxyquinolin-4-yl)propyl]-1-(t-butyloxocarbonyl)pipéridine-3-carboxylique (teneur à 80 %) dans 355 cm³ de méthanol est refroidie à une température voisine de -30°C. On ajoute sous agitation 7,7 cm³ de chlorure de thionyle en maintenant la température entre -25 et -30°C. Après l'addition, on maintient le mélange aux environs de -30°C pendant 30 minutes, puis on laisse revenir la température aux environs de 20°C. Après agitation à température ambiante pendant 19 heures, le mélange réactionnel est concentré sous pression réduite (5 kPa), à une température voisine de 30°C. Le résidu obtenu est repris par 300 cm³ d'eau additionnés de 200 cm³ de dichlorométhane, puis agité. La phase organique est décantée ; la phase aqueuse est à nouveau extraite par 200 cm³ de dichlorométhane. La solution aqueuse est amenée à pH 8 par addition progressive d'hydrogénocarbonate de sodium solide. Après extraction de la solution alcaline obtenue par 3 fois 200 cm³ de dichlorométhane, les extraits organiques réunis sont lavés par 2 fois 200 cm³ d'eau, puis séchés sur sulfate de magnésium. Après filtration sur papier, la solution organique est concentrée sous pression réduite (5 kPa), à une température voisine de 40°C. On obtient 4,51 g de (3R,4R)-4-[3-oxo-3-(6-méthoxyquinolin-4-yl)propyl]pipéridine-3-carboxylate de méthyle sous forme d'une laque de couleur brune.

L'acide (3R,4R)-4-[3-oxo-3-(6-méthoxyquinolin-4-yl)propyl]-1-(tert-butoxycarbonyl)pipéridine-3-carboxylique peut être préparé de la manière suivante :

Une solution de 36 g de (3R,4R)-4-[3-oxo-3-(6-méthoxyquinolin-4-yl)propyl]-3-vinylpipéridine dans 54 cm³ d'acétone est refroidie à une température voisine de 0°C. On ajoute en 15 minutes, sous agitation, 150 cm³ d'acide sulfurique 3 M, en maintenant la température entre 0 et 5°C. On abaisse la température au voisinage de 0°C et l'on ajoute goutte à goutte au mélange une solution de 32 g de permanganate de sodium dans 200 cm³ d'eau distillée. Le mélange réactionnel est agité 45 minutes supplémentaires à une température comprise entre 10 et 15°C, puis on laisse remonter la température au voisinage de 20°C. Après agitation 3 heures à cette température, la masse réactionnelle est refroidie à une température voisine de 0°C, puis on ajoute lentement 160 cm³ de lessive de potasse à 38 % à une température inférieure à 10°C. Après 30 minutes d'agitation à une température voisine de 10°C, le mélange est filtré. Le gâteau est repris dans 300 cm³ d'eau additionnés de 15 cm³ de lessive de potasse à 38 %, et agité pendant 20 minutes. Après filtration, puis lavage du gâteau par 2 fois 200 cm³ d'eau distillée, les filtrats sont réunis puis additionnés de 24 g de di-tertiobutyldicarbonate. La solution est agitée à une température voisine de 2 0°C pendant 15 heures. Après addition d'un litre d'acétate d'éthyle, et agitation, le mélange est décanté, la phase aqueuse séparée puis amenée à pH 5 par addition de 38 cm³ d'acide chlorhydrique aqueux concentré à 37 %. Le mélange est extrait à nouveau par 5 fois 1 litre d'acétate d'éthyle. Les extraits sont réunis puis lavés par 2 fois 1 litre d'eau saturée en chlorure de sodium. La solution organique est séchée sur sulfate de magnésium, filtrée sur papier, concentrée sous pression réduite (5 kPa), à une température voisine de 40°C. On obtient 21,2 g d'acide (3R,4R)-4-[3-oxo-3-(6-méthoxyquinolin-4-yl)propyl]-1-(t-butyloxycarbonyl)pipéridine-3-carboxylique, sous forme d'un solide brun fondant à 114°C en devenant pâteux.

La (3R,4R)-4-[3-oxo-3-(6-méthoxyquinolin-4-yl)propyl]-3-vinylpipéridine peut être obtenue par application de la méthode décrite dans la demande de brevet FR 2354771.

### Exemple 12

### Dichlorhydrate de l'acide (3R, 4R)-1-[2-(3-fluorophénylthio)éthyl]-4-[3-(R, S)-fluoro-3-(6-méthoxyquinolin-4-yl)propyl]-pipéridine-3-carboxylique

0,9 g de (3R, 4R)-1-[2-(3-fluorophénylthio)éthyl]-4-[3-(R, S)-fluoro-3-(6-méthoxyquinolin-4-yl)propyl]-pipéridine-3-carboxylate de méthyle dans 12 cm³ de méthanol et 1,4 cm³ de soude 5N sont chauffés, sous agitation, à une température voisine de 60°C pendant 4 heures. Le mélange réactionnel est mélangé sous pression réduite (5 kPa), à une température voisine de 50°C. Au résidu obtenu, on ajoute 15 cm³ d'eau puis on ajoute 2,1 cm³ d'acide chlorhydrique aqueux 5N. Le mélange réactionnel est de nouveau mélangé à sec. Le résidu obtenu est repris avec un mélange de dichlorométhane/méthanol (90/10 en volumes). Le chlorure de sodium est filtré, puis le filtrat est concentré sous pression réduite (5 kPa), à une température voisine de 30°C. La meringue obtenue est triturée avec de l'éther éthylique. Le solide formé est filtré. On obtient 0,9 g de solide beige. Ce solide est dissous dans un mélange de 50 cm³ de chloroforme et de 50 cm³ d'acétonitrile. L'insoluble est filtré, puis le filtrat est acidifié avec 20 cm³ d'éther chlorhydrique 1N. Le mélange réactionnel est mélangé sous pression réduite (5 kPa), à une température voisine de 30°C. Le résidu obtenu est trituré avec de l'éther éthylique. Le solide formé est filtré puis séché sous vide. On obtient 0,9 g de dichlorhydrate de l'acide (3R,4R)-1-[2-(3-fluorophényl thio)éthyl]-4-[3-(R,S)-fluoro-3-(6-méthoxyquinolin-4-yl)propyl]-pipéridine-3-carboxylique, sous forme de solide de couleur beige.
Spectre de R.M.N. ¹H (600 MHz, (CD₃)₂SO d6 avec ajout de quelques gouttes de CD3COOD d4, à une température de 383K, δ en ppm) : de 1,40 à 2,25 et de 2,65 à 3,65 (mts : 16H); 3,96 (s : 3H); 6,31 (mt, J_{HF} = 47 Hz : 1H) ; 7,03 (mt : 1H) ; 7,25 (mt : 2H) ; de 7,30 à 7,45 (mt : 2H) ; 7,53 (mt : 1H) ; 7,57 (mt : 1H) ; 8,09 (d, J = 9 Hz : 1H) ; 8,82 (d, J = 5 Hz : 1H).

### (3R,4R)-1-[2-(3-Fluorophénylthio)éthyl]-4-[3-(R,S)-fluoro-3-(6-méthoxyquinolin-4-yl)propyl]-pipéridine-3-carboxylate de méthyle

1,7 g de (3R, 4R)-1-[2-(3-fluorophénylthio)éthyl]-4-[3-(R, S)-hydroxy-3-(6-méthoxyquinolin-4-yl)propyl]-pipéridine-3-carboxylate de méthyle sont dissous dans 17 cm³ de dichlorométhane, sous atmosphère d'argon. On ajoute, goutte à goutte, sous agitation à 20°C, 0,53 cm³ de diéthylaminosulfure trifluorure. Après 2 heures d'agitation à la température ambiante, le mélange réactionnel est refroidi à 15°C, puis 20 cm³ d'une solution saturée d'hydrogénocarbonate de sodium sont ajoutés goutte à goutte. La phase organique est décantée, puis la phase aqueuse est extraite 2 fois par 50 cm³ de dichlorométhane. Les extraits organiques réunis sont lavés par 2 fois 50 cm³ d'eau distillée, puis séchés sur sulfate de magnésium. Après filtration sur papier, la solution est mélangée sous pression réduite (5 kPa) à une température voisine de 30°C. On obtient 1,6 g d'une huile brune que l'on purifie en deux fois par chromatographie à pression atmosphérique sur une colonne de gel de silice (granulométrie 20-45 µ ; diamètre 3 cm ; 60 g), en éluant par un mélange de dichlorométhane/méthanol (98/2 en volumes) et en recueillant des fractions de 10 cm³. Pour la première fois, on recueille les fractions de 15 à 25. Pour la seconde fois, on recueille les fractions de 18 à 30. Ces fractions sont réunies, puis concentrées sous pression réduite (5 kPa). On obtient 0,92 g de (3R,4R)-1-[2-(3-fluorophényl thio)éthyl]-4-[3-(R,S)-fluoro-3-(6-méthoxyquinolin-4-yl)propyl]-pipéridine-3-carboxylate de méthyle, sous forme d'une huile brune.

Le (3R,4R)-1-[2-(3-fluorophénylthio)éthyl]-4-[3-(R,S)-hydroxy-3-(6-méthoxy quinolin-4-yl) propyl]-pipéridine-3-carboxylate de méthyle est obtenu comme décrit à l'exemple 11.

### Exemple 13

### Dichlorhydrate de l'acide (3R,4R)-4-[3-(R,S)-hydroxy-3-(6-méthoxyquinolin-4-yl)propyl]-1-[2-(1,3-thiazol-2-ylthio)éthyl]pipéridine-3-carboxylique

Un mélange de 0,21 g de (3R,4R)-4-[3(R,S)-hydroxy-3-(6-methoxyquinolin-4-yl)propyl]-1-[2-(1,3-thiazol-2-ylthio)éthyl] pipéridine-3-carboxylate de méthyle, et de 0,33 cm³ de soude aqueuse 5N dans 3 cm³ de méthanol est chauffé à une température voisine de 60°C, sous agitation, pendant 18 heures. Après refroidissement, le mélange réactionnel est mélangé sous pression réduite (5 kPa), à une température voisine de 40°C ; le résidu est repris dans 6 cm³ d'eau, puis lavé avec 6 cm³ d'acétate d'éthyle. La phase aqueuse est mélangée à sec sous pression réduite (5 kPa), à une température voisine de 80°C. Le résidu obtenu est trituré dans 5 cm³ de dichlorométhane, puis acidifié par addition de 1 cm³ d'éther diisopropylique chlorhydrique 3,3 N. L'insoluble est filtré, lavé par 2 fois 3 cm³ d'un mélange de dichlorométhane-méthanol (90/10 en volumes). Le filtrat est mélangé sous pression réduite (5 kPa), à une température voisine de 40°C. On obtient 0,19 g de dichlorhydrate de l'acide (3R,4R)-4-[3-(R,S)-hydroxy-3-(6-méthoxyquinolin-4-yl)propyl]-1-[2-(1,3-thiazol-2-ylthio) éthyl]pipéridine-3-carboxylique, sous forme d'un solide amorphe de couleur beige, fondant vers 75°C en devenant pâteux.
Spectre de R.M.N. ¹H (400 MHz, (CD₃)₂SO d6 avec ajout de quelques gouttes de CD₃COOD d4, à une température de 373K, δ en ppm) : de 1,35 à 2,15 et de 2,50 à 3,70 (mt : 16H) ; 3,94 (s : 3H) ; 5,28 (mt : 1H) ; de 7,40 à 7,75 (mt : 5H) ; 7,99 (d, J = 9 Hz : 1H) ; 8,72 (d, J = 5 Hz : 1H).

### (3R,4R)- 4-[3-(R,S)-Hydroxy-3-(6-méthoxyquinolin-4-yl)propyl]-1-[2-(1,3-thiazol-2-ylthio)éthyl] pipéridine-3-carboxylate de méthyle

En opérant par analogie avec l'exemple 11, mais à partir de (3R,4R)-4-[3-oxo-3-(6-méthoxyquinolin-4-yl)propyl]-1-[2-(1,3-thiazol-2-ylthio)éthyl]pipéridine-3-carboxylate de méthyle et de borohydrure de sodium, on obtient 0,17 g de (3R,4R)-4-[3(R,S)-hydroxy-3-(6-méthoxyquinolin-4-yl)propyl]-1-[2-(1,3-thiazol-2-ylthio)éthyl] pipéridine-3-carboxylate de méthyle, sous forme d'une huile visqueuse de couleur jaune.
Spectre infra rouge (CCl₄): 3550-3150 cm⁻¹ ν OH alcool; 2949 cm⁻¹ νCH aliphatiques ;1736 cm⁻¹ νC=O ;1228 cm⁻¹ ν C-O éther ;1031cm⁻¹ ν C-O alcool; 854 cm⁻¹ γCH quinoline.

Le (3R,4R)-4-[3-oxo-3-(6-méthoxyquinolin-4-yl)propyl]-1-[2-(1,3-thiazol-2-ylthio) éthyl]pipéridine-3-carboxylate de méthyle peut être préparé par analogie avec l'exemple 4, à partir de (3R,4R)-4-[3-oxo-3-(6-méthoxyquinolin-4-yl)propyl] pipéridine-3-carboxylate de méthyle et de 2-(2-chloro-éthylthio)-thiazole.

Le 2-(2-chloro-éthylthio)-thiazole peut être préparé de la manière suivante :

Dans une solution agitée de 1,47 g de 2-mercaptothiazole et de 1,95 g de carbonate de potassium dans 12,5 cm³ de diméthylformamide, on coule à une température voisine de 20°C, 1,2 cm³ de 1-bromo-2-chloroéthane. Le mélange est ensuite agité pendant 2 heures à une température voisine de 20°C. L'insoluble est filtré, lavé par 2 fois 5 cm³ de diméthylformamide. L e filtrat e st coulé s ur u n mélange de 50 g de glace pilée et 50 cm³ d'eau distillée, puis on ajoute 50 cm³ d'éther éthylique, le mélange est agité, puis décanté. La phase aqueuse est décantée, puis extraite par 2 fois 25 cm³ d'éther éthylique. Les phases éthérées réunies, sont lavées par 2 fois 25 cm³ d'eau, puis séchées sur sulfate de magnésium. Après filtration sur papier, la solution organique est mélangée sous pression réduite (5 kPa), à une température voisine de 40°C. On obtient 2,11 g de 2-(2-chloro-éthylthio)-thiazole, sous forme d'une huile mobile de couleur jaune.

### Exemple 14

### Dichlorhydrate de l'acide (3R,4R)- 4-[3-(R,S)-hydroxy 3-(6-méthoxyquinolin-4-yl)propyl]-1-[2-(2-thiénylthio)éthyl] pipéridine-3-carboxylique

Un mélange de 0,45 g de (3R,4R)- 4-[3-(R,S)-hydroxy 3-(6-méthoxyquinolin-4-yl)propyl]-1-[2-(2-thiényllthio)éthyl] pipéridine-3-carboxylate de méthyle, 3,5 cm³ de méthanol, et 0,54 cm³ de soude aqueuse 5N est chauffé sous agitation à une température voisine de 60°C pendant 20 heures. Après mélange des solvants sous pression réduite (5 kPa), à une température voisine de 40°C, le résidu obtenu est repris dans 3 cm³ d'une solution aqueuse 6N d'acide chlorhydrique. La solution est mélangée dans les mêmes conditions, puis le résidu obtenu est trituré dans un mélange de dichlorométhane-méthanol (90/10 en volumes). L'insoluble est filtré, lavé 2 fois par 1 cm³ de ce mélange. Le filtrat est séché sur sulfate de sodium, puis concentré sous pression réduite (5 kPa), à une température voisine de 40°C. Après séchage à l'air, on obtient 0,16 g de dichlorhydrate de l'acide (3R,4R)-4-[3-(R,S)-hydroxy 3-(6-méthoxyquinolin-4-yl)propyl]-1-[2-(2-thiényllthio)éthyl] pipéridine-3-carboxylique, sous la forme d'un solide beige fondant, en se ramollissant, vers 148°C.
Spectre de R.M.N. ¹H (300 MHz, (CD₃)₂SO d6 avec ajout de quelques gouttes de CD₃COOD d4, δ en ppm) : de 1,35 à 2,30 et de 2,80 à 3,75 (mts : 16H) ; 3,98 et 4,00 (2s : 3H) ; de 5,40 à 5,63 (mt : 1H) ; de 7,05 à 7,15 (mt : 1H) ; de 7,25 à 7,40 (mt : 1H) ; de 7,50 à 7,80 (mt : 3H) ; 8,00 (mt : 1H) ; 8,24 (d large, J = 9 Hz : 1H) ; 9,05 (d, J = 5 Hz : 1H).

### (3R,4R)- 4-[3-(R,S)-hydroxy 3-(6-méthoxyquinolin-4-yl)propyl]-1-[2-(2-thiénylthio)éthyl] pipéridine-3-carboxylate de méthyle

En opérant par analogie avec l'exemple 11, mais à partir de (3R,4R)-4-[3-oxo-3-(6-méthoxyquinolin-4-yl)propyl]-1-[2-(2-thiénylthio)éthyl]pipéridine-3-carboxylate de méthyle et de borohydrure de sodium, on obtient 0,95,g de (3R,4R)-4-[3-(R,S)-hydroxy 3-(6-méthoxyquinolin-4-yl)propyl]-1-[2-(2-thiénylthio)éthyl]pipéridine-3-carboxylate de méthyle sous la forme d'une huile orange.
Spectre infra rouge (CH₂Cl₂) : 3600-3150 cm⁻¹ ν OH alcool ; 2951 cm⁻¹ νCH aliphatiques ;1732 cm⁻¹ νC=O ;1228 cm⁻¹ ν C-O éther ;1031cm⁻¹ ν C-O alcool; 847 cm⁻¹ γCH quinoline.

Le (3R,4R)-4-[3-oxo-3-(6-méthoxyquinolin-4-yl)propyl]-1-[2-(2-thiénylthio)éthyl] pipéridine-3-carboxylate de méthyle peut être préparé par analogie avec l'exemple 3, à partir de (3R,4R)-4-[3-oxo-3-(6-méthoxyquinolin-4-yl)propyl]pipéridine-3-carboxylate de méthyle et de 2-(2-chloro-éthylthio)-thiophène.

Le 2-(2-chloro-éthylthio)-thiophène peut être préparé de la manière suivante :

Dans une solution agitée 8,25 cm3 de solution aqueuse de soude à 20 % et de 14,6 cm³ de 1-bromo-2-chloro-éthane on coule sous agitation à une température voisine de 20°C 4,72 cm³ de thiophène-2-thiol. Le mélange est ensuite agité pendant 6 heures à une température voisine de 20°C. On ajoute ensuite 40 cm³ d'éther éthylique, la phase organique est lavée par de l'eau, puis séchée sur sulfate de magnésium. Après filtration sur papier, la solution organique est mélangée sous pression réduite (5 kPa), à une température voisine de 40°C. Le résidu obtenu est purifié par chromatographie sous pression réduite à 50 kPa d'azote sur une colonne de gel de silice (granulométrie 20-45 µ ; diamètre 4,5 cm; poids de silice 250 g), en éluant par un mélange de cyclohexane-acétate d'éthyle (95/5 en volumes). On obtient 7,27 g de 2-(2-chloroéthylthio)-thiophène, sous forme d'une huile mobile de couleur jaune.

### Exemple 15

### (3R,4R)-4-[3-(R,S)-Hydroxy-3-(6-méthoxyquinolin-4-yl)propyl]-3-hydroxy méthyl 1-[2-(1,3-thiazol-2-ylthio)éthyl]-3-pipéridine

En opérant par analogie avec l'exemple 11, mais à partir de (3R,4R)-4-[3-oxo-3-(6-méthoxyquinolin-4-yl)propyl]-1-[2-(1,3-thiazol-2-ylthio)éthyl]pipéridine-3-carboxylate de méthyle et de borohydrure de sodium, on obtient 0,33 g de (3R,4R)-4-[3-(R,S)-hydroxy-3-(6-méthoxyquinolin-4-yl)propyl]-3-hydroxyméthyl-1-[2-(1,3-thiazol-2-ylthio)éthyl]-3-pipéridine, sous la forme d'une huile.
Spectre de R.M.N. ¹H (300 MHz, (CD₃)₂SO d6, δ en ppm) : de 1,10 à 2,20 - de 2,55 à 2,90 et de 3,30 à 3,60 (mts : 16H) ; 2,60 (t, J = 6,5 Hz : 2H) ; 3,93 et 3,94 (2s : 3H) ; 4,26 (mf: 1H) ; 5,27 (mt : 1H) ; de 5,50 à 5,60 (mt : 1H); de 7,35 à 7,45 (mt : 2H) ; 7,56 (mt : 1H) ; 7,63 (d, J = 3 Hz : 1H); 7,71 (d, J = 3 Hz : 1H) ; 7,95 (d, J = 9 Hz : 1H) ; 8,72 (d, J = 5 Hz : 1H).

Le (3R,4R)-4-[3-oxo-3-(6-méthoxyquinolin-4-yl)propyl]-1-[2-(1,3-thiazol-2-ylthio) éthyl] pipéridine-3-carboxylate de méthyle est préparé par analogie avec l'exemple 4 à partir de (3R,4R)-4-[3-oxo-3-(6-méthoxyquinolin-4-yl)propyl]pipéridine-3-carboxylate de méthyle et de 2-bromo-1-(1,3-thiazol-2-ylthio)éthane.

Le (3R,4R)- 4-[3-oxo 3-(6-méthoxyquinolin-4-yl)propyl] pipéridine-3-carboxylate de méthyle est préparé comme décrit dans l'exemple 11.

### Exemple 16

### Acide (3R,4R)-4-[3-hydroxy-3-(6-méthoxyquinolin-4-yl)propyl]-1-[2-(2-thiénylthio) éthyl]pipéridine-3-carboxylique, diastéréoisomère A et acide (3R,4R)-4-[3-hydroxy-3-(6-méthoxyquinolin-4-yl)propyl]-1-[2-(2-thiénylthio)éthyl]pipéridine-3-carboxylique, diastéréoisomère B.

1,36 g d'acide (3R,4R)-4-(3-(R,S)-hydroxy-3-(6-méthoxyquinolin-4-yl)propyl]-1-[2-(2-thiénylthio)éthyl]pipéridine-3-carboxylique dissous dans 100 cm³ de dichlorométhane sont chromatographiés sur une colonne de 35 cm et de 6 cm de diamètre conditionnée avec 700 g de silice (granulométrie 5-15 µ) de DAISO. L'élution est effectuée à l'aide d'un mélange dichlorométhane-méthanol (92/8 en volumes) contenant 0,05 % de triéthylamine. Le débit est de 90 cm³ par minute et la détection effectuée en ultra violet à 280 nm. Cette opération conduit à l'obtention des deux diastéréoisomères. Les fractions correspondant au premier sont concentrées à sec sous pression réduite (5 kPa) à une température voisine de 40°C, puis le résidu obtenu est séché à l'étuve sous pression réduite (13 Pa) à une température voisine de 40°C. On obtient 0,28 g d'acide (3R,4R)-4-[3-hydroxy-3-(6-méthoxyquinolin-4-yl)propyl]-1-[2-(2-thiénylthio)éthyl]pipéridine-3-carboxylique, diastéréoisomère A. (α_{D}²⁰=-73,8°+/-1,4, dans le dichlorométhane à 0,5 %), sous forme d'une meringue de couleur jaune. Les fractions correspondant au second diastéréoisomère sont traitées comme précédemment. On obtient 0,46 g d'acide (3R,4R)-4-[3-hydroxy-3-(6-méthoxyquinolin-4-yl)propyl]-1-[2-(2-thiénylthio)éthyl]pipéridine-3-carboxylique, diastéréoisomère B. (α_{D}²⁰=+71,2°+/-1,2, dans le dichlorométhane à 0,5 %), sous forme d'une meringue jaune.
diastéréoisomère A : Spectre de R.M.N. ¹H (300 MHz, (CD₃)₂SO, δ en ppm) : de 1,35 à 1,75 et 1,94 (mts : 7H) ; 2,22 (mt : 1H) ; 2,36 (d large, J = 10,5 Hz : 1H) ; de 2,50 à 2,65 (mt : 1H) ; 2,61 (t, J = 7 Hz : 2H) ; 2,76 (mt : 1H) ; de 2,85 à 3,05 (mt : 1H) ; 2,98 (mt : 2H) ; 3,95 (s : 3H) ; 5,22 (mt : 1H) ; 5,51 (d large, J = 4,5 Hz : 1H) ; 7,07 (dd, J = 5 et 4 Hz : 1H) ; 7,22 (d large, J = 4 Hz : 1H) ; 7,36 (mt : 1H) ; de 7,35 à 7,45 (mt : 2H) ; 7,64 (d large, J = 5 Hz : 1H) ; 7,95 (d, J = 9,5 Hz : 1H) ; 8,72 (d, J = 5 Hz : 1H).
diastéréoisomère B : Spectre de R.M.N. ¹H (300 MHz, (CD₃)₂SO, δ en ppm) : de 1,20 à 1,95 (mts : 7H) ; 2,22 (mt : 1H) ; 2,39 (d large, J = 10,5 Hz : 1H) ; de 2,50 à 2,95 (mt : 3H) ; 2,60 (t, J = 7 Hz : 2H) ; 2,97 (mt : 2H) ; 3,92 (s : 3H) ; 5,25 (mt : 1H) ; 5,51 (mf : 1H) ; 7,06 (dd, J = 5 et 3,5 Hz : 1 H) ; 7,20 (dd, J = 3,5 et 1,5 Hz : 1H) ; 7,40 (mt : 2H) ; 7,53 (d, J = 5 Hz : 1H) ; 7,63 (dd, J = 5 et 1,5 Hz : 1H) ; 7,93 (d, J = 10 Hz : 1H) ; 8,70 (d, J = 5 Hz : 1H).

### Exemple de référence 17

### Dichlorhydrate de l'acide (3R,4R)-4-[3-(R,S)-hydroxy-3-(6-méthoxyquinolin-4-yl)propyl]-1-[2-(tertiobutylthio)éthyl]pipéridine-3-carboxylique

A une solution de 0,26 g de (3R,4R)-4-[3-(R,S)-hydroxy-3-(6-méthoxyquinolin-4-yl) propyl]-1-[2-(tertiobutylthio)éthyl]pipéridine-3-carboxylate de méthyle dans 5 cm³ de méthanol, on ajoute sous agitation et sous atmosphère inerte, 1 cm³ de soude aqueuse 5 N. Après chauffage de la solution à une température voisine de 60°C, puis refroidissement à température ambiante, la masse réactionnelle est évaporée sous pression réduite (2,8 kPa) à une température voisine de 60°C. Le résidu obtenu est repris par 5 cm³ d'eau distillée, puis on ajoute 5 cm³ d'acide chlorhydrique concentré (d=1,18). Le mélange est évaporé sous pression réduite (2,8 kPa) à une température voisine de 60°C. Le résidu obtenu est repris dans 4 cm³ d'un mélange de dichlorométhane-méthanol (90/10 en volumes). Le précipité blanc obtenu est filtré, lavé par 2 fois 2 cm³ de ce même mélange. Le filtrat est concentré sous pression réduite (2,8 kPa) à une température voisine de 40°C. Le produit obtenu est séché sous pression réduite (16 Pa) à une température voisine de 60°C. On obtient 0,29 g de dichlorhydrate de l'acide (3R,4R)-4-[3-(R,S)-hydroxy-3-(6-méthoxyquinolin-4-yl) propyl]-1-[2-(tertiobutylthio)éthyl]pipéridine-3-carboxylique, sous forme d'un solide de couleur beige fondant en se ramollissant au voisinage de 169°C.
Spectre de R.M.N. ¹H (400 MHz, (CD₃)₂SO d6 à une température de 383K, δ en ppm). On observe un mélange de deux diastéréoisomères :
1,34 et 1,36 (2s : 9H en totalité) ; de 1,40 à 2,35 et de 2,90 à 3,70 (mts : 12H) ; 3,00 (t large, J = 8 Hz : 2H) ; 3,26 (t, J = 8 Hz : 2H) ; 4,01 (s : 3H) ; 5,40 (mt : 1H) ; de 7,55 à 7,70 (mt : 2H) ; 7,82 (mt : 1H) ; 8,22 (d, J = 9 Hz : 1H) ; 8,88 (d, J = 5 Hz : 1H) ; de 10,90 à 11,45 (mf étalé: 1H).

### (3R,4R)-4-[3-(R,S)-Hydroxy-3-(6-méthoxyquinolin-4-yl)propyl]-1-[2-(tertiobutylthio)éthyl]pipéridine-3-carboxylate de méthyle

On chauffe pendant 16 heures à une température voisine du reflux, sous agitation et sous atmosphère inerte, 0,717 g de (3R,4R)-4-[3-(R,S)-hydroxy-3-(6-méthoxyquinolin-4-yl)propyl]pipéridine-3-carboxylate de méthyle dans 15 cm³ d'acétonitrile et 15 cm³ de méthanol avec 0,43 g de 2-chloroéthyltertiobutyl sulfure en présence de 0,33 g de carbonate de potassium et 0,4 g d'iodure de potassium. Après refroidissement du mélange réactionnel à une température voisine de 20°C, l'insoluble est filtré: Le filtrat est évaporé sous pression réduite (5 kPa) à une température voisine de 40°C. Le résidu obtenu est purifié par chromatographie sous une pression d'azote de 100 kPa sur une colonne de gel de silice (granulométrie 40-63 µ ; diamètre 3,5 cm ; hauteur 35 cm) en éluant par un mélange de dichlorométhane-méthanol (97/3 en volumes) et en recueillant des fractions de 35 cm³. Les fractions 54 à 70 sont réunies puis évaporées sous pression réduite (5 kPa) à une température voisine de 40°C. On obtient 0,265 g de (3R,4R)-4-[3-(R,S)-hydroxy-3-(6-méthoxyquinolin-4-yl)propyl]-1-[2-(tertiobutylthio)éthyl]pipéridine-3-carboxylate de méthyle, sous forme d'une laque de couleur brune.
Spectre infra rouge (CCl₄) 3550-3100 cm⁻¹ ν OH alcool ; 2959 cm⁻¹ ν CH aliphatiques ;1736 cm⁻¹ νC=O ;1242 ;1228 cm⁻¹ ν C-O éther ;1034cm⁻¹ ν C-O alcool ; 853 cm⁻¹ γCH quinoline

Le 2-chloroéthyltertiobutyl sulfure peut être préparé par application de la méthode décrite dans le brevet EP 136878.

### Exemple 18

### Dichlorhydrate de l'acide (3R,4R)-4-[3-(R,S)-hydroxy-3-(6-méthoxyquinolin-4-yl)propyl]-1-[2-(cyclopentylthio)éthyl]pipéridine-3-carboxylique

A une solution de 0,48 g de (3R,4R)-4-[3-(R,S)-hydroxy-3-(6-méthoxyquinolin-4-yl) propyl]-1-[2-(cyclopentylthio)éthyl]pipéridine-3-carboxylate de méthyle dans 10 cm³ de méthanol on ajoute sous agitation et sous atmosphère inerte 2 cm³ de soude aqueuse 5 N. Après 16 heures de chauffage du mélange à une température voisine de 60°C, la masse réactionnelle est évaporée sous pression réduite (2,9 kPa) à une température voisine de 60°C, le résidu obtenu est repris par 4,3 cm³ d'eau distillée additionnés de 4,3 cm³ d'acide chlorhydrique à 28%. L'insoluble persistant est filtré, puis le filtrat est évaporé dans les mêmes conditions que ci-dessus. Le résidu obtenu est agité dans un mélange de dichlorométhane-méthanol (90/10 en volumes). Le précipité résultant est filtré, lavé par 3 fois 2,5 cm³ du même mélange. Le filtrat est évaporé sous pression réduite (5 kPa) à une température voisine de 40°C. On obtient 0,48 g de dichlorhydrate de l'acide (3R,4R)-4-[3-(R,S)-hydroxy-3-(6-méthoxyquinolin-4-yl)propyl]-1-[2-(cyclopentylthio)éthyl]pipéridine-3-carboxylique, sous forme d'une meringue de couleur verte fondant en se ramollissant à une température voisine de 156°C.
Spectre de R.M.N. ¹H (400 MHz, (CD₃)₂SO d6 à une température de 373K, δ en ppm). On observe un mélange de deux diastéréoisomères :
de 1,45 à 2,30 et de 2,90 à 3,60 (mts : 20H) ; 2,98 (t large, J = 7,5 Hz : 2H) ; 3,22 (mt : 1H) ; 3,30 (t, J = 7,5 Hz : 2H) ; 4,00 (s : 3H) ; 5,37 (mt : 1H) ; de 7,50 à 7,65 (mt : 2H) ; 7,77 (mt : 1H) ; 8,17 (d, J = 9 Hz : 1H) ; 8,85 (d, J = 5 Hz : 1H).

### (3R,4R)-4-[3-(R,S)-Hydroxy-3-(6-méthoxyquinolin-4-yl)propyl]-1-[2-(cyclopentylthio)éthyl]pipéridine-3-carboxylate de méthyle

On chauffe pendant 3 h ½ à une température voisine du reflux, sous agitation et sous atmosphère inerte, 0,717 g de (3R,4R)-4-[3-(R,S)-hydroxy-3-(6-méthoxyquinolin-4-yl)propyl]pipéridine-3-carboxylate de méthyle dans 15 cm³ d'acétonitrile et 15 cm³ de méthanol avec 0,439 g de 2-chloroéthylcyclopentyl sulfure à 90 % en présence de 0,332 g de carbonate de potassium et 0,4 g d'iodure de potassium. Après refroidissement du mélange réactionnel à une température voisine de 20°C, l'insoluble est filtré puis lavé par de l'acétonitrile. Le filtrat est évaporé sous pression réduite (5 kPa) à une température voisine de 40°C. Le résidu obtenu est purifié par chromatographie sous une pression d'azote de 100 kPa sur une colonne de gel de silice (granulométrie 40-63µ ; diamètre 3,5 cm ; hauteur 45 cm), en éluant par un mélange de dichlorométhane-méthanol (95/5 en volumes) et en recueillant des fractions de 35 cm³. Les fractions 25 à 45 sont réunies puis évaporées sous pression réduite (5 kPa) à une température voisine de 40°C. On obtient 0,48 g de (3R,4R)-4-[3-(R,S)-hydroxy-3-(6-méthoxyquinolin-4-yl)propyl]-1-[2-(cyclopentylthio)éthyl] pipéridine-3-carboxylate de méthyle, sous forme d'une laque de couleur vert-foncé.
Spectre infra rouge (CCl₄) 3550-3100 cm⁻¹ ν OH alcool ; 2951 cm⁻¹ ν CH aliphatiques ;1736 cm⁻¹ νC=O ;1242 ;1228 cm⁻¹ ν C-O éther ;1034cm⁻¹ ν C-O alcool ; 853 cm⁻¹ γCH quinoline

Le 2-chloroéthylcyclopentyl sulfure peut être préparé par application de la méthode décrite dans la demande de brevet FR 2 395 260.

### Exemple 19

### Dichlorhydrate de l'acide (3R,4R)-1-[2-(3-fluorophénylthio)éthyl]-4-[3-(6-méthoxyquinolin-4-yl)propyl]pipéridine-3-acétique

Un mélange de 0,48 g de (3R,4R)-1-[2-(3-fluorophénylthio)éthyl]-4-[3-(6-méthoxyquinolin-4-yl)propyl]pipéridine-3-acétate de méthyle dans 10 cm³ de dioxanne additionnés de 0,78 cm³ de soude aqueuse 5N est agité pendant 20 heures à une température voisine de 60°C. Après refroidissement à une température voisine de 20°C, la masse réactionnelle est évaporée sous pression réduite (5 kPa) à une température voisine de 40°C, puis diluée par 10 cm³ d'eau. Le pH est amené à 4 par addition d'une quantité suffisante d'une solution aqueuse d'acide citrique. Le mélange est évaporé sous pression réduite (5 kPa) à une température voisine de 40°C, puis le résidu obtenu est repris par 70 cm³ d'eau et 20 cm³ d'éthanol. Le mélange est ramené à pH 9, puis évaporé sous pression réduite (5 kPa) à une température voisine de 50°C. Après reprise du résidu obtenu par 50 cm³ d'un mélange de chloroforme-méthanol-ammoniaque à 28 % (12/3/0,5 en volumes), les sels minéraux sont filtrés. Le filtrat est évaporé sous pression réduite (5 kPa) à une température voisine de 40°C. On obtient 1,2 g d'un produit que l'on purifie par chromatographie sous pression atmosphérique sur une colonne de gel de silice (granulométrie 20-45 µ ; diamètre 2 cm ; hauteur 25 cm) en éluant par un mélange de chloroforme-méthanol-ammoniaque à 28 % (12/3/0,5 en volumes), et en recueillant d'abord une fraction de 1 00 cm³, puis des fractions d'environ 15 cm³. Les fractions 8 à 18 sont réunies puis concentrées sous pression réduite (5 kPa) à une température voisine de 40°C. On obtient 0,31 g d'un produit sous forme d'une meringue de couleur jaune-pâle que l'on agite avec 13 cm³ d'acide chlorhydrique aqueux 0,1N pendant 2 heures à une température voisine de 20°C. Après addition de 5 cm³ de dioxanne et agitation 2 heures supplémentaires à une température voisine de 20°C, on évapore le dioxanne sous pression réduite (5 kPa) à une température voisine de 40°C. La solution obtenue est congelée puis lyophilisée. On obtient 0,35 g de dichlorhydrate de l'acide (3R,4R)-1-[2-(3-fluorophénylthio) éthyl]-4-[3-(6-méthoxyquinolin-4-yl)propyl]pipéridine-3-acétique, sous forme de lyophilisat de couleur blanche.
Spectre de R.M.N. ¹H (400 MHz, (CD₃)₂SO d6 à une température de 383K, δ en ppm) : de 1,45 à 1,95 (mt : 8H) ; 2,33 (dd, J = 16 et 5,5 Hz : 1H) ; de 2,45 à 2,60 (mt : 1H) ; de 2,90 à 3,55 (mt : 10H) ; 4,00 (s : 3H) ; 7,06 (t large, J = 8 Hz : 1H) ; 7,28 (d, J = 8 Hz : 2H) ; 7,41 (mt : 1H) ; 7,46 (d, J = 5 Hz : 1H) ; 7,48 (d, J = 2,5 Hz : 1H) ; 7,51 (dd, J = 9,5 et 2,5 Hz : 1H) ; 8,07 (d, J = 9,5 Hz : 1H) ; 8,72 (d, J = 5 Hz : 1H).

### (3R,4R)-1-[2-(3-Fluorophénylthio)éthyl]-4-[3-(6-méthoxyquinolin-4-yl)propyl]pipéridine-3-acétate de méthyle

Un mélange de 0,76 g de (3R,4R)]-4-[3-(6-méthoxyquinolin-4-yl)propyl]pipéridine-3-acétate de méthyle et 0,42 g de 2-(3-fluorophénylthio)éthyl-1-chlorure dans 15 cm³ d'acétonitrile additionnés de 1,4 g de carbonate de potassium et 0,15 g d'iodure de potassium est chauffé sous agitation et sous atmosphère inerte à une température voisine de 70°C pendant 20 heures. Après addition de 0,15 g d'iodure de potassium et 5 heures supplémentaires de chauffage, le mélange réactionnel est refroidi à une température voisine de 20°C, dilué par 15 cm³ d'eau puis extrait par 2 fois 20 cm³ d'acétate d'éthyle. Les extraits réunis sont lavés par 30 cm³ d'acide chlorhydrique aqueux N. La solution acide est séparée puis rendue alcaline (pH 8-9) par la quantité suffisante d'une solution saturée aqueuse d'hydrogénocarbonate de sodium. Le mélange est extrait par 2 fois 30 cm³ d'acétate d'éthyle. Les extraits réunis sont séchés sur sulfate de magnésium, filtrés puis évaporés sous pression réduite (5 kPa) à une température voisine de 40°C. On obtient 0,71 g d'une huile que l'on purifie par chromatographie sous pression atmosphérique sur une colonne de gel de silice (granulométrie 20-45µ ; diamètre 2,5 cm ; volume silice 120 cm³) en éluant par un mélange de dichlorométhane-méthanol (99/1 en volumes). On recueille d'abord une fraction de 500 cm³ puis on recueille des fractions de 15 cm³ environ. Les fractions 10 à 20 sont réunies puis évaporées sous pression réduite (5 kPa) à une température voisine de 40°C. On obtient 0,48 g de (3R,4R)-1-[2-(3-fluorophénylthio)éthyl]-4-[3-(6-méthoxyquinolin-4-yl)propyl]pipéridine-3-acétate de méthyle, sous forme d'une huile de couleur jaune.
Spectre infra rouge (CH₂Cl₂) 2934,2861 cm⁻¹ ν CH aliphatiques ; 2807,2767 cm⁻¹ ν CH₂ N(CH₂)₃;1730 cm⁻¹ νC=O ester;1242,1227 cm⁻¹ ν C-O éther; 848 cm⁻¹ γCH quinoline

### (3R,4R)]-4-[3-(6-Méthoxyquinolin-4-yl)propyl]pipéridine-3-acétate de méthyle

Un mélange de 2,8 g d'acide (3R,4R)-4-[3-(6-méthoxyquinolin-4-yl)propyl]-1-(tert.butyloxycarbonyl)pipéridine-3-acétique dans 100 cm³ de méthanol anhydre additionnés d'1 cm³ d'acide sulfurique à 95 % est chauffé, sous agitation, à une température voisine de l'ébullition pendant 2 heures. Après refroidissement à une température voisine de 20°C la masse réactionnelle est évaporée sous pression réduite (5 kPa) à une température voisine de 40°C, puis au résidu obtenu on ajoute 20 cm³ d'une solution aqueuse saturée d'hydrogénocarbonate de sodium. Le mélange est extrait par 4 fois 20 cm³ de dichlorométhane. Les extraits réunis sont séchés sur sulfate de magnésium, filtrés puis évaporés sous pression réduite (5 kPa) à une température voisine de 40°C. On obtient 2,25 g de (3R,4R)]-4-[3-(6-méthoxyquinolin-4-yl) propyl]pipéridine-3-acétate de méthyle, sous forme d'une huile de couleur brune.
Spectre infra rouge (CH₂Cl₂) 2954,2865 cm⁻¹ ν CH aliphatiques ; 2788 cm⁻¹ ν CH₂ N(CH₂)₂ ;1736 cm⁻¹ νC=O ester;1242,1227 cm⁻¹ ν C-O éther; 848 cm⁻¹ γCH quinoline

### Acide (3R,4R)-4-[3-(6-méthoxyquinolin-4-yl)propyl]-1-(tert.butyloxycarbonyl)pipéridine-3-acétique

A une solution de 2 g de (3R,4R)-4-[3-(6-méthoxyquinolin-4-yl)propyl]-1-(tert.butyloxycarbonyl)pipéridine-3-acétaldéhyde dans 100 cm³ d'acétone, on ajoute sous agitation et sous atmosphère inerte à une température voisine de 20°C 1,48 g de permanganate de potassium préalablement dissous dans 45 cm³ d'eau distillée, puis 220 cm³ d'acétone. Le mélange obtenu est agité pendant 2 heures à une température voisine de 20°C, puis après refroidissement à une température comprise entre 0 et 5°C, on ajoute une solution de 5 g de sulfite de sodium dans 150 cm³ d'eau. Le précipité brun de dioxyde de manganèse est filtré sur célite, puis l'acétone est évaporée sous pression réduite (5 kPa) à une température voisine de 40°C. On ajoute au mélange réactionnel la quantité suffisante d'acide citrique pour obtenir un pH de 4-5. Le mélange est extrait par 2 fois 100 cm³ d'acétate d'éthyle. Les extraits réunis sont séchés sur sulfate de magnésium, filtrés puis évaporés sous pression réduite (5 kPa) à une température voisine de 40°C. On obtient 2,8 g d'acide (3R,4R)-4-[3-(6-méthoxyquinolin-4-yl)propyl]-1-(tert.butyloxycarbonyl)pipéridine-3-acétique, sous forme d'une meringue de couleur blanche.
Spectre infra rouge (KBr) 2977, 2932 ,2868 cm⁻¹ νCH aliphatiques; 3000-2200 cm⁻¹ ν OH acide 1734 cm⁻¹ νC=O acide; 1689 cm⁻¹ νC=O carbamate ; 1391,1365 cm⁻¹ δₐₛ CH₃ ;1246 cm⁻¹ ν C-O éther;1170 cm⁻¹ ν C-O carbamate ; 848 cm⁻¹ γCH quinoline

### (3R,4R)-4-[3-(6-Méthoxyquinolin-4-yl)propyl]-1-(tert.butyloxycarbonyl)pipéridine-3-acétaldéhyde

A une solution de 3,5 cm³ de chlorure d'oxalyle dans 80 cm³ de dichlorométhane refroidie à une température voisine de -60°C, on ajoute successivement sous agitation et sous atmosphère d'azote une solution de 5,6 cm³ de diméthylsulfoxyde dissous dans 80 cm³ de dichlorométhane, puis 13,5 g de (3R,4R)-3-(2-hydroxyéthyl)-4-[3-(6-méthoxyquinolin-4-yl)propyl]-1-(tert.butyloxycarbonyl)pipéridine dissous dans 80 cm³ de dichlorométhane, enfin 26,5 cm³ de triéthylamine dissous dans 80 cm³ de dichlorométhane. La solution obtenue est maintenue pendant 1 heure au voisinage de -60°C, puis 3 h ½ à une température voisine de 20°C. Après dilution par 150 cm³ de dichlorométhane, le mélange réactionnel est lavé par 2 fois 300 cm³ d'eau. La solution organique décantée est séchée sur sulfate de magnésium, filtrée, puis évaporée sous pression réduite (5 kPa) à une température voisine de 40°C. On obtient 12,7 g d'une huile brune que l'on reprend par 400 cm³ d'éther diéthylique. La solution résultante est lavée par 2 fois 300 cm³ d'eau, puis 1 fois par 300 cm³ d'une solution aqueuse d'acide citrique à 5 %, enfin par 2 fois 300 cm³ d'eau. La solution organique est séchée sur sulfate de magnésium, puis concentrée sous pression réduite (5 kPa) à une température voisine de 40°C. On obtient 7,73 g de (3R,4R)-4-[3-(6-méthoxyquinolin-4-yl)propyl]-1-(tert.butyloxycarbonyl)pipéridine-3-acétaldéhyde, sous forme d'une gomme collante de couleur jaune.
Spectre infra rouge (CCl₄) 2978, 2932 ,2864 cm⁻¹ ν CH aliphatiques ;2717 cm⁻¹ ν CH aldéhyde ; 1729 cm⁻¹ νC=O aldéhyde; 1694 cm⁻¹ νC=O carbamate ; 1391,1366 cm⁻¹ δₐₛ CH₃ ;1242 cm⁻¹ ν C-O éther;1158 cm⁻¹ ν C-O carbamate ; 844 cm⁻¹ γCH quinoline

### (3R,4R)-3-(2-Hydroxyéthyl)-4-[3-(6-méthoxyquinolin-4-yl)propyl]-1-(tert.butyloxycarbonyl)pipéridine

Une solution de 2 g de (3R,4R)-4-[3-(6-méthoxyquinolin-4-yl)propyl]-1-(tert.butyloxycarbonyl)-3-vinylpipéridine et de 0,72 cm³ de triéthylamine borane dans 10 cm³ de toluène est agitée sous atmosphère inerte à une température voisine de 110°C pendant 10 heures. La masse réactionnelle est concentrée à sec sous pression réduite (5 kPa) à une température voisine de 60°C. On obtient 2,1 g d'une meringue de couleur orangée que l'on solubilise dans 9 cm³ d'acétone et auxquels on ajoute 1,9 cm³ d'acide chlorhydrique aqueux à 5 %. Après agitation du mélange pendant 20 minutes à une température voisine de 20°C, la masse réactionnelle est concentrée sous pression réduite (5 kPa) à une température voisine de 40°C. Le résidu obtenu est repris par 7,5 cm³ de tétrahydrofuranne, 6,2 cm³ de soude aqueuse à 30 % et 7,5 cm³ de peroxyde d'hydrogène en solution aqueuse à 30 %. Le mélange est chauffé pendant 3 heures à une température voisine du reflux. Après refroidissement, le mélange réactionnel est agité avec 30 cm³ de chloroforme à une température voisine de 20°C. La phase aqueuse est décantée ; la phase organique est lavée par 3 fois 30 cm³ d'eau, puis 1 fois 20 cm³ d'une solution saturée de chlorure de sodium. Après séchage sur sulfate de magnésium la solution organique est concentrée sous pression réduite (5 kPa) à une température voisine de 40°C. On obtient 1,7 g d'une huile de couleur jaune-brun que l'on purifie par chromatographie à pression atmosphérique sur une colonne de gel de silice (granulométrie 40-63 µ ; diamètre 3,5 cm ; masse 60 g) en éluant par un mélange de dichlorométhane-méthanol (95/5 en volumes) et en recueillant des fractions de 20 cm³. Les fractions 4 et 5 sont réunies, évaporées sous pression réduite (5 kPa) à une température voisine de 40°C. On obtient 0,95 g de (3R,4R)-3-(2-hydroxyéthyl)-4-[3-(6-méthoxyquinolin-4-yl)propyl)-1-(tert.butyloxycarbonyl)pipéridine, sous forme d'une meringue de couleur jaune-orangé.
Spectre infra rouge (KBr) 3550-3100 cm⁻¹ ν OH alcool ; 2972, 2931, 2865 cm⁻¹ ν CH aliphatiques ;1690 cm⁻¹ νC=O carbamate ;1391,1365 cm⁻¹ δₐₛ CH₃ ;1244,1228 cm⁻¹ ν C-O éther ;1158 cm⁻¹ ν C-O carbamate;1031cm⁻¹ ν C-O alcool; 845 cm⁻¹ γCH quinoline

### (3R,4R)-4-[3-(6-Méthoxyquinolin-4-yl)propyl]-1-(tert.butyloxycarbonyl)-3-vinylpipéridine

A une suspension de 5 g de chlorhydrate de (3R,4R)-4-[3-(6-méthoxyquinolin-4-yl)propyl]-3-vinylpipéridine dans 50 cm³ de dichlorométhane, on ajoute sous agitation à une température voisine de 20°C 4 cm³ de triéthylamine, puis 3,15 g de di-tertiobutyl dicarbonate. Après 45 minutes la solution obtenue est lavée par 2 fois 30 cm³ d'eau, séchée sur sulfate de magnésium puis concentrée sous pression réduite (5 kPa) à une température voisine de 40°C. On obtient 6 g de (3R,4R)-4-[3-(6-méthoxyquinolin-4-yl)propyl)-1-(tert.butyloxycarbonyl)-3-vinylpipéridine, sous forme d'une huile de couleur brune.
Spectre infra rouge (CH₂Cl₂) 2972, 2933, 2860 cm⁻¹ ν CH aliphatiques ;1680 cm⁻¹ νC=O carbamate ;1391,1365 cm⁻¹ δₐₛ CH₃ ;1244,1228 cm⁻¹ ν C-O éther ;1165 cm⁻¹ ν C-O carbamate ; 845 cm⁻¹ γCH quinoline

Le chlorhydrate de (3R,4R)-4-[3-(6-méthoxyquinolin-4-yl)propyl]-3-vinylpipéridine peut être obtenu par application de la méthode décrite dans la demande de brevet FR 2354771.

### Exemple 20

### Dichlorhydrate de l'acide (3R,4R)-4-[3-(6-méthoxyquinolin-4-yl)propyl]-1-[2-(cyclohexylthio)éthyl]pipéridine-3-acétique

Un mélange de 0,3 g de (3R,4R)-4-[3-(6-méthoxyquinolin-4-yl)propyl]-1-[2-(cyclohexylthio)éthyl]pipéridine-3-acétate de méthyle dans 8 cm³ de dioxanne en présence de 0,5 cm³ de soude aqueuse 5N est chauffée sous agitation à une température voisine de 60°C pendant 20 heures. Après refroidissement à une température voisine de 20°C, le mélange réactionnel est évaporé sous pression réduite (5 kPa) à une température voisine de 40°C. On obtient une meringue de couleur jaune que l'on purifie par chromatographie à pression atmosphérique sur une colonne de gel de silice (granulométrie 20-45µ ; diamètre 1,6 cm ; volume silice 40 cm³) en éluant par un mélange de chloroforme-méthanol-ammoniaque à 28 % (12/3/0,5 en volumes) et en recueillant d'abord une fraction de 125 cm³, puis des fractions d'environ 10 cm³. Les fractions 3 à 7 sont réunies puis évaporées sous pression réduite (5 kPa) à une température voisine de 40°C. On obtient 0,27 g d'une laque que l'on reprend par 5 cm³ de dioxanne, 11 cm³ d'acide chlorhydrique aqueux 0,1N et 14 cm³ d'eau distillée. Le dioxanne est évaporé sous pression réduite (5 kPa) à une température voisine de 40°C, puis la solution résiduelle est lyophilisée. On obtient 0,29 g de dichlorhydrate de l'acide (3R,4R)-4-[3-(6-méthoxyquinolin-4-yl)propyl]-1-[2-(cyclohexylthio)éthyl]pipéridine-3-acétique, sous forme d'un lyophilisat de couleur blanche.
Spectre de R.M.N. ¹H (400 MHz, (CD₃)₂SO d6 à une température de 383K, δ en ppm) : de 1,25 à 2,05 (mt : 18H) ; 2,33 (dd, J = 16 et 5,5 Hz : 1H) ; de 2,50 à 2,60 (mt : 1H) ; 2,83 (mt : 1H) ; de 2,95 à 3,30 (mt : 10H) ; 4,00 (s : 3H) ; 7,45 (d, J = 5 Hz : 1H) ; 7,48 (d, J = 2 Hz : 1H) ; 7,51 (dd, J = 9,5 et 2 Hz : 1H) ; 8,08 (d, J = 9,5 Hz : 1H) ; 8,72 (d, J = 5 Hz : 1H).

### (3R,4R)-4-[3-(6-Méthoxyquinolin-4-yl)propyl]-1-[2-(cyclohexylthio)éthyl]pipéridine-3-acétate de méthyle

Un mélange de 1,2 g de (3R,4R)]-4-[3-(6-méthoxyquinolin-4-yl)propyl]pipéridine-3-acétate de méthyle, 0,65 g de 2-chloroéthylcyclohexyl sulfure dans 25 cm³ d'acétonitrile additionnés de 2,3 g de carbonate de potassium et 0,55 g d'iodure de potassium est chauffé sous agitation et sous atmosphère inerte à une température voisine de 80°C pendant 4 heures. Après refroidissement à une température voisine de 20°C, le mélange réactionnel est additionné de 30 cm³ d'eau puis extrait par 2 fois 200 cm³ d'acétate d'éthyle. Les extraits organiques réunis sont lavés par 2 fois 40 cm³ d'acide chlorhydrique aqueux N. Après décantation les extraits acides réunis sont alcalinisés par une solution aqueuse saturée d 'hydrogénocarbonate d e sodium, puis extraits par 2 fois 100 cm³d'acétate d'éthyle. Les extraits organiques sont réunis, séchés sur sulfate de magnésium, filtrés, évaporés sous pression réduite (5 kPa) à une température voisine de 40°C. On obtient 0,80 g d'une huile brune que l'on purifie par chromatographie sous pression atmosphérique sur une colonne de gel de silice (granulométrie 20-45µ ; diamètre 2,5 cm ; volume silice 120 cm³) en éluant d'abord par un mélange de dichlorométhane-méthanol (99/1 en volumes) et en recueillant d'abord une fraction de 250 cm³, puis en éluant par un mélange de dichlorométhane-méthanol (90/l en volumes) et en recueillant une fraction de 200 cm³. On élue à nouveau par un mélange de dichlorométhane-méthanol (99/l en volumes) et l'on recueille des fractions d'environ 10 cm³. Les fractions 10 à 33 sont réunies puis concentrées sous pression réduite (5 kPa) à une température voisine de 40°C. On obtient 0,30 g de (3R,4R)-4-[3-(6-méthoxyquinolin-4-yl)propyl]-1-[2-(cyclohexylthio)éthyl]pipéridine-3-acétate de méthyle, sous forme d'une huile incolore.
Spectre infra rouge (CCl₄) 2932,2855 cm⁻¹ ν CH aliphatiques ;2800,2763 cm⁻¹ ν CH₂ N(CH₂)₃ ;1737 cm⁻¹ ν C=O ester ;1241,1227 cm⁻¹ ν C-O éther; 844 cm⁻¹ γCH quinoline

Le (3R,4R)]-4-[3-(6-méthoxyquinolin-4-yl)propyl]pipéridine-3-acétate de méthyle peut être préparé comme décrit à l'exemple 19.

### Exemple 21

### Dichlorhydrate de l'acide (3R,4R)-4-[3-(R,S)-fluoro-3-(6-méthoxyquinolin-4-yl)propyl]-1-[2-(2-thiénylthio)éthyl]pipéridine-3-carboxylique.

Une solution de 0,2 g de (3R,4R)-4-[3-(R,S)-fluoro-3-(6-méthoxyquinolin-4-yl)propyl]-1-[2-(2-thiénylthio)éthyl]pipéridine-3-carboxylate de méthyle dans 3 cm³ d'acide chlorhydrique aqueux 6N est chauffée sous agitation à une température voisine de 100°C pendant 7 heures, puis évaporée sous pression réduite (5 kPa) à une température voisine de 50°C. Le résidu obtenu est agité dans 10 cm³ d'éther diisopropylique. Les cristaux qui en résultent sont filtrés, lavés par 2 fois 5 cm³ d'éther diisopropylique, séchés sous pression réduite (13 Pa) à une température voisine de 60°C. On obtient 0,22 g de dichlorhydrate de l'acide (3R,4R)-4-[3-(R,S)-fluoro-3-(6-méthoxyquinolin-4-yl)propyl]-1-[2-(2-thiénylthio)éthyl]pipéridine-3-carboxylique, sous forme d'un solide de couleur beige fondant en se ramollissant à une température voisine de 140°C.
Spectre de R.M.N. ¹H (400 MHz, (CD₃)₂SO d6 avec ajout de quelques gouttes de CD₃COOD d4, à une température de 373K, δ en ppm). On observe un mélange de deux diastéréoisomères :
de 1,55 à 2,30 et de 3,10 à 3,50 (mts : 15H); 3,10 (s large : 1H) ; 3,98 (s : 3H); 6,31 (mt, J_{HF} = 48 Hz : 1H) ; 7,10 (mt : 1H) ; 7,29 (d large, J = 4 Hz : 1H) ; de 7,35 à 7,45 (mt : 1H) ; de 7,45 à 7,60 (mt : 2H) ; 7,64 (d large, J = 5,5 Hz : 1H) ; 8,06 (d, J = 9 Hz : 1H) ; 8,82 (d,J = 5 Hz : 1H).

### (3R,4R)-4-[3-(R,S)-Fluoro-3-(6-méthoxyquinolin-4-yl)propyl]-1-[2-(2-thiénylthio)éthyl]pipéridine-3-carboxylate de méthyle.

A une solution agitée de 0,5 g de (3R,4R)-4-[3-(R,S)-hydroxy-3-(6-méthoxyquinolin-4-yl)propyl]-1-[2-(2-thiénylthio)éthyl]pipéridine-3-carboxylate de méthyle dans 8 cm³ de dichlorométhane, refroidie à une température voisine de 10°C, on ajoute sous atmosphère inerte 0,16 cm³ de diéthylaminosulfure trifluorure dissous dans 2 cm³ de dichlorométhane. Après 10 minutes d'agitation à cette température, on laisse revenir à une température voisine de 20°C et l'on poursuit l'agitation pendant 18 heures. Après addition de 8 cm³ d'une solution aqueuse saturée d'hydrogénocarbonate de sodium et agitation pendant 15 minutes, la phase organique est décantée. La phase aqueuse est extraite 1 fois par 5 cm³ de dichlorométhane, puis les extraits organiques sont réunis, séchés sur sulfate de magnésium, filtrés, évaporés sous pression réduite (5 kPa) à une température voisine de 40°C. On obtient 0,5 g d'une huile rouge que l'on purifie par chromatographie sous une pression de 100 kPa d'azote sur une colonne de gel de silice (granulométrie 40-63µ ; diamètre 3,5 cm; hauteur silice 35 cm), en éluant par un mélange de dichlorométhane-méthanol (97,5/2,5 en volumes), et en recueillant des fractions de 30 cm³. On réunit les fractions 25 à 29 que l'on évapore sous pression réduite (5 kPa) à une température voisine de 40°C. On obtient 0,2 g de (3R,4R)-4-[3-(R,S)-fluoro-3-(6-méthoxyquinolin-4-yl)propyl]-1-[2-(2-thiénylthio)éthyl]pipéridine-3-carboxylate de méthyle, sous forme d'une huile de couleur orangée.
Spectre infra rouge (CCl₄) 2950 cm⁻¹ ν CH aliphatiques ;2804,2767 cm⁻¹ ν CH₂ N(CH₂)₃ ;1737 cm⁻¹ ν C=O ester; 1243,1229 cm⁻¹ ν C-O éther; 852 cm⁻¹ γCH quinoline

### Exemple 22

### Dichlorhydrate de l'acide (3R,4R)-4-[3-hydroxy-3-(6-méthoxyquinolin-4-yl)propyl]-1-[2-(cycloheptylthio)éthyl]pipéridine-3-carboxylique, diastéréoisomère A et .dichlorhydrate de l'acide (3R,4R)-4-[3-hydroxy-3-(6-méthoxyquinolin-4-yl)propyl]-1-[2-(cycloheptylthio)éthyl]pipéridine-3-carboxylique, diastéréoisomère B.

Un mélange de 1,8 g de (3R,4R)-4-[3-(R,S)-hydroxy-3-(6-méthoxyquinolin-4-yl)propyl]-1-[2-(cycloheptylthio)éthyl]pipéridine-3-carboxylate de méthyle dans 36 cm³ de dioxanne avec 2,8 cm³ de soude aqueuse 5N est chauffé pendant 24 heures à une température voisine de 60°C. Après refroidissement du mélange réactionnel à une température voisine de 20°C, puis concentration sous pression réduite (5 kPa) à une température voisine de 40°C, le résidu obtenu est purifié par c hromatographie sous pression atmosphérique sur une colonne de gel de silice (granulométrie 20-45µ ; diamètre 4 cm ; masse 180 g) en éluant par un mélange de dichlorométhane-méthanol-ammoniaque à 28 % (40/5/0,5 en volumes) et en recueillant des fractions de 50 cm³. On obtient 3 lots que l'on évapore sous pression réduite (5 kPa) à une température voisine de 40°C : le lot A (0,48 g) correspondant au diastéréoisomère A ; le lot B (0,6 g) correspondant au diastéréoisomère B ; le lot C (0,5 g) correspondant à un mélange des deux diastéréoisomères. Le lot A est purifié de la façon suivante: après dissolution dans 15 cm³ de dichlorométhane et addition à 10 cm³ d'éther diéthylique chlorhydrique N, le mélange gommeux obtenu est évaporé sous pression réduite (5 kPa) à une température voisine de 40°C. Après reprise du résidu par 40 cm³ d'acétonitrile et 2 cm³ d'acide chlorhydrique 0,5 N, la solution obtenue est évaporée dans les mêmes conditions que ci-dessus. On obtient un solide blanc que l'on sèche pendant 16 heures sous pression réduite (vide phosphorique, 5 kPa). Après agitation dans 100 cm³ d'éther diéthylique puis filtration, on obtient 0,514 g de dichlorhydrate de l'acide (3R,4R)-4-[3-hydroxy-3-(6-méthoxyquinolin-4-yl)propyl]-1-[2-(cycloheptylthio)éthyl]pipéridine-3-carboxylique, diastéréoisomère A. (α_{D}²⁰=-58,3°+/-1,00, dans le méthanol à 0,5%), sous forme d'un solide de couleur blanche. Les lots B et C sont traités de la même façon. On obtient, en particulier avec le lot B, 0,650 g de dichlorhydrate de l'acide (3R,4R)-4-[3-hydroxy-3-(6-méthoxyquinolin-4-yl)propyl]-1-[2-(cycloheptylthio)éthyl]pipéridine-3-carboxylique, diastéréoisomère B. (α_{D}²⁰=+120,4°+/-1,7, dans le méthanol à 0,5 %), sous forme d'un solide de couleur blanche.
diastéréoisomère A : Spectre de R.M.N. ¹H (400 MHz, (CD₃)₂SO d6, δ en ppm).
de 1,40 à 2,35 et de 2,70 à 3,85 (mts : 29H) ; 4,01 (s : 3H) ; 5,50 (mt : 1H) ; de 5,60 à 6,40 (mf étalé : 1H) ; 7,55 (mt : 1H) ; 7,75 (d large, J = 9 Hz : 1H) ; 7,99 (d, J = 5 Hz : 1H) ; 8,30 (d, J = 9 Hz : 1H) ; 9,04 (d, J = 5 Hz : 1H) ; 10,80 (mf : 1H).
diastéréoisomère B : Spectre de R.M.N. ¹H (400 MHz, (CD₃)₂SO d6, δ en ppm).
de 1,35 à 2,25 et de 2,70 à 3,80 (mts : 29H); 4,00 (s : 3H) ; 5,49 et 5,55 (2mts : 1H en totalité) ; de 5,75 à 6,20 (mf étalé : 1H) ; 7,56 et 7,62 (2s larges : 1H en totalité) ; 7,71 (d large, J = 9 Hz : 1H) ; 7,92 (mt : 1H) ; 8,24 (d, J = 9 Hz : 1H); 9,00 (d, J = 4 Hz : 1H) ; 10,56 (mf : 1H) .

### (3R,4R)-4-[3-(R,S)-Hydroxy-3-(6-méthoxyquinolin-4-yl)propyl]-1-[2-(cycloheptylthio)éthyl]pipéridine-3-carboxylate de méthyle.

A un mélange de 2,5 g de (3R,4R)-4-[3-oxo-3-(6-méthoxyquinolin-4-yl)propyl]-1-[2-(cycloheptylthio)éthyl]pipéridine-3-carboxylate de méthyle dans 20 cm³ de méthanol, on ajoute par petites fractions à une température voisine de 20°C, sous agitation et sous atmosphère inerte, 0,221 g de borohydrure de sodium. Après addition, le mélange est agité pendant 2 heures à une température voisine de 20°C. Le mélange réactionnel est évaporé sous pression réduite (5 kPa), puis le résidu obtenu est repris par 50 cm³ de dichlorométhane et 30 cm³ d'une solution aqueuse saturée de chlorure d'ammonium. La phase organique décantée est séchée sur sulfate de magnésium, filtrée puis concentrée sous pression réduite (5 kPa) à une température voisine de 40°C. On obtient 2,5 g d'un produit que l'on purifie par chromatographie à pression atmosphérique sur une colonne de gel de silice (granulométrie 20-45µ ; diamètre 4 cm ; masse 150 g), en éluant par un mélange de dichlorométhane-méthanol (95/5 en volumes) et en recueillant des fractions de 50 cm³. Les fractions contenant le produit recherché sont réunies puis évaporées sous pression réduite (5 kPa) à une température voisine de 40°C. On obtient 1,95 g de (3R,4R)-4-[3-(R,S)-hydroxy-3-(6-méthoxyquinolin-4-yl)propyl]-1-[2-(cycloheptylthio)éthyl]pipéridine-3-carboxylate de méthyle, sous forme d'une huile visqueuse.
Spectre infra rouge (CCl₄) : 3500-3100 cm⁻¹ ν OH alcool; 2930 cm⁻¹ ν CH aliphatiques; 2805,2772 cm⁻¹ ν CH₂ N(CH₂)₃ ;1736 cm⁻¹ ν C=O ester ; 1242; 1228 cm⁻¹ ν C-O éther ;1034cm⁻¹ ν C-O alcool ; 854 cm⁻¹ γCH quinoline

### (3R,4R)-4-[3-Oxo-3-(6-méthoxyquinolin-4-yl)propyl]-1-[2-(cycloheptylthio)éthyl]pipéridine-3-carboxylate de méthyle

Un mélange constitué par 3,06 g de (3R,4R)-4-[3-oxo-3-(6-méthoxyquinolin-4-yl) propyl]pipéridine-3-carboxylate de méthyle, 1,79 g de 2-chloroéthylcycloheptyl sulfure, 5,39 g de carbonate de potassium, 1,29 g d'iodure de potassium dans 75 cm³ d'acétonitrile est chauffé sous agitation et sous atmosphère inerte à une température voisine de 72°C pendant 24 heures. Après refroidissement à environ 20°C, le mélange réactionnel est filtré sur célite, puis le filtrat est concentré sous pression réduite (5 kPa) à une température voisine de 40°C. Le résidu obtenu est purifié par chromatographie à pression atmosphérique sur une colonne de gel de silice (granulométrie 20-45µ ; diamètre 4 cm ; masse 150 g), en éluant par un mélange acétate d'éthyle-méthanol (95/5 en volumes) et en recueillant des fractions de 50 cm³. Les fractions contenant le produit recherché sont réunies puis concentrées sous pression réduite (5 kPa) à une température voisine de 40°C. On obtient 2,6 g de (3R,4R)-4-[3-oxo-3-(6-méthoxyquinolin-4-yl)propyl]-1-[2-(cycloheptylthio)éthyl] pipéridine-3-carboxylate de méthyle, sous forme d'une huile visqueuse.
Spectre infra rouge (CCl₄) : 2930 cm⁻¹ ν CH aliphatiques ;2805,2767 cm⁻¹ ν CH₂ N(CH₂)₃ ;1740 cm⁻¹ ν C=O ester; 1693 cm⁻¹ ν C=O cétone ; 1241,1228 cm⁻¹ ν C-O éther ; 850 cm⁻¹ γCH quinoline

### 2-Chloroéthylcycloheptyl sulfure

A une solution de 1,74 g de 2-hydroxyéthylcycloheptylsulfure dans 30 cm³ de chloroforme, on ajoute goutte à goutte sous agitation et sous atmosphère inerte 5,11 cm³ de chlorure de thionyle à une température voisine de 20°C. Après l'addition le mélange réactionnel est agité d'abord pendant 15 minutes à cette même température, puis pendant 1 heure à une température voisine de 60°C. Le mélange est évaporé à sec sous pression réduite (5 kPa) à une température voisine de 40°C, puis le résidu obtenu est repris par 2 fois 50 cm³ d'eau puis 1 fois par 50 cm³ d'une solution saturée d'hydrogénocarbonate de sodium. La solution éthérée décantée est séchée sur sulfate de magnésium, filtrée puis concentrée sous pression réduite (5 kPa) à une température voisine de 40°C. On obtient 1,89 g de 2-chloroéthylcycloheptyl sulfure sous forme d'huile.
Spectre infra rouge (CCl₄) 2930,2855 cm⁻¹ ν CH₂ ; 1459,1445 cm⁻¹ δ CH₂;1210 cm⁻¹ ω C-Cl ; 702 cm⁻¹ ν C-Cl

### 2-Hydroxyéthylcycloheptyl sulfure

A une suspension agitée de 0,91 g d'hydrure de sodium à 60 % dans 10 cm³ de diméthylformamide anhydre on ajoute lentement, sous atmosphère inerte et à une température voisine de 20°C, 2,32 g de 2-mercaptoéthanol préalablement solubilisés dans 10 cm³ de diméthylformamide. Après 20 minutes d'agitation, on ajoute 3,5 g de bromocycloheptane dissous dans 10 cm³ de diméthylformamide. La réaction est achevée par agitation à une température voisine de 20°C pendant 1 heure 30 minutes. Le mélange réactionnel est versé sur 150 cm³ d'eau additionnés de 100 cm³ d'éther diéthylique. La phase éthérée est décantée, la phase aqueuse extraite une fois par 50 cm³ d'éther diéthylique. Les phases organiques sont réunies puis lavées par 2 fois 100 cm³ d'eau, séchées sur sulfate de magnésium, filtrées, concentrées sous pression réduite (5 kPa) à une température voisine de 40°C. On obtient 3,2 g de 2-hydroxyéthylcycloheptyl sulfure, sous forme d'huile de couleur jaune.
Spectre infra rouge (CH₂Cl₂) 3608,3457 cm⁻¹ ν OH (libre et lié) ;2927,2855 cm⁻¹ ν CH aliphatiques ;1057 cm⁻¹ ν CO

### Exemple 23

### Dichlorhydrate de l'acide (3R,4R)-4-[3-(6-méthoxyquinolin-4-yl)propyl]-1-[2-(2-thiénylthio)éthyl]pipéridine-3-acétique

Un mélange agité de 0,598 g de (3R,4R)-4-[3-(6-méthoxyquinolin-4-yl)propyl]-1-[2-(2-thiénylthio)éthyl]pipéridine-3-acétate de méthyle dans 13 cm³ de dioxanne avec 1 cm³ de soude aqueuse 5N, en atmosphère inerte, est chauffé à 60°C pendant 20 heures. Après évaporation du mélange réactionnel sous pression réduite (5 kPa) à une température voisine de 50°C, on obtient 1 g d'une meringue jaune que l'on purifie par chromatographie à pression atmosphérique sur une colonne de gel de silice (granulométrie 20-45µ ; diamètre 3 cm ; hauteur silice 21 cm) en éluant par un mélange chloroforme-méthanol-ammoniaque à 28% (12/3/0,5 en volumes) et en recueillant des fractions de 50 cm³. Les fractions 4 à 7 sont réunies puis évaporées sous pression réduite (5 kPa) à une température voisine de 40°C. On obtient 0,495 g d'un produit que l'on dissout dans 5 cm³ de dichlorométhane. Après addition de 10 cm³ d'éther chlorhydrique 1N, on obtient un précipité pâteux que l'on dilue par 100 cm³ d'éther diéthylique. Le mélange est agité à une température voisine de 20°C. La suspension blanche obtenue est filtrée, lavée par 3 fois 30 cm³ d'éther diéthylique. Le solide blanc résultant est séché sous pression réduite (10 Pa) à une température voisine de 40°C jusqu'à obtention d'un poids constant. On obtient 0,55 g de dichlorhydrate de l'acide (3R,4R)-4-[3-(6-méthoxyquinolin-4-yl)propyl]-1-[2-(2-thiénylthio)éthyl]pipéridine-3-acétique, sous forme d'un solide blanc fondant en se ramollissant au voisinage de 200°C.
Spectre de R.M.N. ¹H (400 MHz, (CD₃)₂SO d6 à une température de 383K, δ en ppm) : de 1,40 à 1,95 (mt : 8H) ; 2,30 (dd, J = 16 et 5,5 Hz : 1H) ; de 2,45 à 2,60 (mt : 1H) ; de 3,00 à 3,35 (mt : 10H); 4,00 (s : 3H) ; 7,08 (dd, J = 5 et 3,5 Hz : 1H) ; 7,28 (d large, J = 3,5 Hz : 1H) ; de 7,45 à 7,55 (mt : 2H) ; 7,56 (dd, J = 9,5 et 3 Hz : 1H) ; 7,62 (d large, J = 5 Hz : 1H) ; 8,15 (d, J = 9,5 Hz : 1H) ; 8,75 (d, J = 5 Hz : 1H).

### Exemple 24

### (3R,4R)-4-[3-(6-Méthoxyquinolin-4-yl)propyl]-1-[2-(2-thiénylthio)éthyl]pipéridine-3-acétate de méthyle

A une solution agitée de 2,1 g de (3R,4R)-4-[3-(6-méthoxyquinolin-4-yl)propyl] pipéridine-3-acétate de méthyle et de 1,15 g de 2-(2-chloroéthylthio) thiophène dans 50 cm³ d'acétonitrile, on ajoute sous atmosphère inerte 4,06 g de carbonate de potassium puis 1 g d'iodure de potassium. Le mélange est chauffé à 70°C pendant 20 heures. Après refroidissement à une température voisine de 20°C, le mélange réactionnel est dilué par 100 cm³ d'acétate d'éthyle et 100 cm³ d'eau. Après agitation du mélange et décantation de la phase organique, la phase aqueuse est extraite par 2 fois 50 cm³ d'acétate d'éthyle. Les phases organiques sont réunies, extraites par 3 fois 50 cm³ d'acide chlorhydrique aqueux N. Les solutions acides sont réunies, amenées à pH 8 par la quantité suffisante d 'hydrogénocarbonate de sodium. L'huile relarguée résultante est extraite par 3 fois 100 cm³ d'acétate d'éthyle. Les extraits réunis sont séchés sur sulfate de magnésium, filtrés, évaporés sous pression réduite (5 kPa) à une température voisine de 40°C. On obtient 1,5 g d'une huile de couleur orangée que l'on purifie par chromatographie à pression atmosphérique sur une colonne de gel de silice (granulométrie 20-45µ ; diamètre 3 cm ; hauteur silice 55 cm) en éluant par de l'acétate d'éthyle pur et en recueillant des fractions de 50 cm³. Les fractions 12 à 30 sont réunies puis évaporées dans les mêmes conditions que ci-dessus. On obtient 0,60 g de (3R,4R)-4-[3-(6-méthoxyquinolin-4-yl)propyl]-1-[2-(2-thiénylthio)éthyl] pipéridine-3-acétate de méthyle, sous forme d'une huile visqueuse incolore.
Spectre infrarouge (CH₂Cl₂) 2933,2861 cm⁻¹ ν CH aliphatiques ;2803,2766 cm⁻¹ ν CH₂ N(CH₂)₃ ;1731 cm⁻¹ ν C=O ester ;1242,1227cm⁻¹ ν C-O éther; 847 cm⁻¹ γCH quinoline

Le (3R,4R)-4-[3-(6-méthoxyquinolin-4-yl)propyl]pipéridine-3-acétate de méthyle a été préparé selon le mode opératoire décrit dans l'exemple 19.

### Exemple 25

### Acide (3R,4R)-4-[3-hydroxy-3-(6-méthoxyquinolin-4-yl)propyl]-1-[2-(cyclohexylthio)éthyl]pipéridine-3-carboxylique, diastéréoisomère A et acide (3R,4R)-4-[3-hydroxy-3-(6-méthoxyquinolin-4-yl)propyl]-1-[2-(cyclohexylthio)éthyl]pipéridine-3-carboxylique, diastéréoisomère B.

5 g d'acide (3R,4R)-4-[3-(R,S)-hydroxy-3-(6-méthoxyquinolin-4-yl)propyl]-1-[2-(cyclohexylthio)éthyl]pipéridine-3-carboxylique dissous dans 100 cm³ d'un mélange de dichlorométhane-éthanol-tétrahydrofuranne (65/15/20 en volumes) sont chromatographiés sur une colonne de 35 cm de long et 8 cm de diamètre, conditionnée avec 1,200 kg de silice KROMASIL® (granulométrie 10 µ). L'élution est effectuée à l'aide du même mélange que ci-dessus. Le débit est de 150 cm³ par minute pendant les 30 premières minutes, puis 200 cm³ par minute au-delà. La détection est effectuée en ultra violet 280 nm. Cette opération conduit à l'obtention des deux diastéréoisomères. Les fractions correspondantes au premier sont concentrées à sec sous pression réduite (5 kPa) à une température voisine de 40°C. On obtient un résidu solide que l'on reprend dans l'éther diéthylique, filtre, sèche à l'air à une température voisine de 20°C. On obtient 1,5 g d'acide (3R,4R)-4-[3-hydroxy-3-(6-méthoxyquinolin-4-yl)propyl]-1-[2-(cyclohexylthio)éthyl]pipéridine-3-carboxylique, diastéréoisomère A. (α_{D}²⁰ =-47,1° +/-0,9 dans le dichlorométhane à 0,5 %), sous forme d'un solide de couleur beige. Les fractions correspondant au second diastéréoisomère sont traitées comme précédemment. On obtient 1,7 g d'acide (3R,4R)-4-[3-hydroxy-3-(6-méthoxyquinolin-4-yl)propyl]-1-[2-(cyclohexylthio) éthyl]pipéridine-3-carboxylique, diastéréoisomère B. (α_{D}²⁰ =+98,7° +/-1,6 dans le dichlorométhane à 0,5 %), sous forme d'un solide de couleur beige.
diastéréoisomère A : Spectre de R.M.N. ¹H (300 MHz, (CD₃)₂SO d6, δ en ppm) : de 1,10 à 2,05 (mts : 17H) ; 2,33 (t très large, J = 10 Hz : 1H) ; 2,45 (d large, J = 10 Hz : 1H) ; 2,59 (mf : 1H) ; de 2,60 à 2,80 (mt : 5H) ; 2,90 (d large, J = 10 Hz : 1H) ; 3,06 (d large, J = 10 Hz : 1H) ; 3,96 (s : 3H) ; 5,22 (d très large, J = 7 Hz : 1H) ; de 5,40 à 5,70 (mf étalé : 1H) ; de 7,30 à 7,45 (mt : 2H) ; 7,57 (d, J = 4,5 Hz : 1H) ; 7,94 (d, J = 9 Hz : 1H) ; 8,72 (d, J = 4,5 Hz : 1H) ; de 12,50 à 13,40 (mftrés étalé : 1H).
diastéréoisomère B : Spectre de R.M.N. ¹H (300 MHz, (CD₃)₂SO d6, δ en ppm) : de 1,10 à 2,00 (mts : 17H) ; 2,33 (mt : 1H) ; de 2,40 à 2,55 (mt : 1H) ; 2,57 (s large : 1H) ; de 2,60 à 2,80 (mt : 5H) ; 2,82 (mt : 1H) ; 2,99 (mt : 1H) ; 3,92 (s : 3H) ; 5,25 (mt : 1H) ; de 5,40 à 5,70 (mf étalé : 1H) ; de 7,35 à 7,45 (mt : 2H) ; 7,53 (d, J = 4,5 Hz : 1H) ; 7,94 (d, J = 10 Hz : 1H) ; 8,71 (d, J = 4,5 Hz : 1H) ; de 12,40 à 13,50 (mf très étalé : 1H).

L'acide (3R,4R)-4-[3-(R,S)-hydroxy-3-(6-méthoxyquinolin-4-yl)propyl]-1-[2-(cyclohexylthio)éthyl]pipéridine-3-carboxylique a été préparé à partir de son chlorhydrate selon le procédé suivant.

### Dichlorhydrate de l'acide (3R,4R)-4-[3-(R,S)-hydroxy-3-(6-méthoxyquinolin-4-yl)propyl]-1-[2-(cyclohexylthio)éthyl]pipéridine-3-carboxylique

On chauffe 0,4 g de (3R,4R)-4-[3-(R,S)-hydroxy-3-(6-méthoxyquinolin-4-yl)propyl]-1-[2-(cyclohexylthio)éthyl]pipéridine-3-carboxylate de méthyle dans 3 cm³ de méthanol additionnés de 0,48 cm³ de soude aqueuse 5N en atmosphère inerte pendant 16 heures. Après concentration de la masse réactionnelle sous pression réduite (5 kPa) à une température voisine de 40°C, on reprend le résidu obtenu dans 5 cm³ d'acide chlorhydrique 6N, puis 2,5 cm³ de méthanol. La solution brune obtenue est évaporée dans les mêmes conditions que ci-dessus. Le résidu qui en résulte est repris dans 5 cm³ d'éther diisopropylique, filtré, lavé, par 2 fois 3 cm³ du même solvant. Le solide obtenu est séché sous pression réduite (13 Pa) à une température voisine de 60°C. On obtient 0,37 g de dichlorhydrate de l'acide (3R,4R)-4-[3-(R,S)-hydroxy-3-(6-méthoxyquinolin-4-yl)propyl]-1-[2-(cyclohexylthio)éthyl]pipéridine-3-carboxylique, sous forme d'un solide de couleur brune fondant en se ramollissant au voisinage de 170°C.
Spectre de R.M.N.¹H (400 MHz, (CD₃)₂SO d6, à une température de 383K, δ en ppm). On observe un mélange de deux diastéréoisomères :
de 1,20 à 2,25 et de 2,80 à 3,40 (mts : 25H) ; 3,28 (t, J = 8 Hz : 2H) ; 4,00 (s : 3H) ; 5,36 (mt : 1H) ; de 7,50 à 7,65 (mt : 2H) ; 7,70 (mt : 1H) ; 8,13 (d large, J = 9 Hz : 1H) ; 8,81 (d, J = 5 Hz : 1H).

### (3R,4R)-4-[3-(R,S)-Hydroxy-3-(6-méthoxyquinolin-4-yl)propyl]-1-[2-(cyclohexylthio)éthyl]pipéridine-3-carboxylate de méthyle

Un mélange agité de 0,54 g de (3R,4R)-4-[3-(R,S)-hydroxy-3-(6-méthoxyquinolin-4-yl)propyl]pipéridine-3-carboxylate de méthyle, 0,295 g de 2-chloroéthylcyclohexyl sulfure, 0,23 g de carbonate de potassium, 0,27 g d'iodure de potassium dans 9 cm³ d'acétonitrile et 1 cm³ de méthanol est porté à une température voisine de l'ébullition sous atmosphère inerte pendant 20 heures. Le mélange réactionnel est concentré sous pression réduite (5 kPa) à une température voisine de 40°C. Le résidu obtenu est purifié par chromatographie sous une pression d'argon de 80 kPa, sur une colonne de gel de silice (granulométrie 40-63µ ; diamètre 3,5 cm ; hauteur 35 cm) en éluant par un mélange de dichlorométhane-méthanol (95/5 en volumes), et en recueillant des fractions de 35 cm³. Les fractions 23 à 40 sont réunies puis concentrées sous pression réduite (5 kPa) à une température voisine de 40°C. On obtient 0,4 g de (3R,4R)-4-[3-(R,S)-hydroxy-3-(6-méthoxyquinolin-4-yl)propyl]-1-[2-(cyclohexylthio)éthyl] pipéridine-3-carboxylate de méthyle, sous forme d'une laque de couleur brune.
Spectre infra rouge (CCl₄) 3600-3200 cm⁻¹ νOH; 2932,2854 cm⁻¹ νCH aliphatiques; 1736 cm⁻¹ ν C=O; 1242 cm⁻¹ ν C-O éther

### Exemple de référence 26

### Acide (3R,4R)-4-[3-(R,S)-hydroxy-3-(6-méthoxyquinolin-4-yl)propyl]-1-[2-(5-chlorothién-2-yl)]pipéridine-3-carboxylique

Une solution de 0,460 g de (3R,4R)-4-[3-(R,S)-hydroxy-3-(6-méthoxyquinolin-4-yl) propyl]-1-[2-(5-chlorothién-2-yl)]pipéridine-3-carboxylate de méthyle dans 5 cm³ de dioxanne additionnés de 0,51 cm³ d'une solution aqueuse de soude 5N est agitée à une température voisine de 60°C pendant 48 heures. Après une addition supplémentaire de 1 cm³ de soude 5N, le mélange est de nouveau chauffé à une température voisine de 70°C pendant 72 heures. Le mélange réactionnel est évaporé sous pression réduite (5 kPa) à une température voisine de 45°C, puis le résidu obtenu est purifié par chromatographie sous une pression d'azote de 40 kPa, sur une colonne de gel de silice (granulométrie 40-63µ; diamètre 3 cm ; hauteur de silice 27 cm), en éluant par un mélange de dichlorométhane-méthanol-ammoniaque à 28% (14/4/0,6 en volumes) et en recueillant des fractions de 15 cm³. Les fractions 7 à 16 sont évaporées sous pression réduite (5 kPa) à une température voisine de 40°C. On obtient 0,30 g d' acide (3R,4R)-4-[3-(R,S)-hydraxy-3-(6-méthoxyquinolin-4-yl)propyl]-1-[2-(5-chlorothién-2-yl)]pipéridine-3-carboxylique, sous forme d'une meringue de couleur blanche.
Spectre de R.M.N. ¹H (300 MHz, (CD₃)₂SO d6, δ en ppm). On observe un mélange de deux diastéréoisomères :
de 1,40 à 3,10 (mts : 16H) ; 3,92 et 3,95 (2 s : 3H en totalité) ; 5,23 (mt : 1H) ; 5,50 (mt : 1H) ; de 7,05 à 7,15 (mt : 2H) ; de 7,30 à 7,45 (mt : 2H) ; 7,52 et 7,54 (2 d, J = 5 Hz : 1H en totalité) ; 7,94 (mt : 1H) ; 8,70 (d, J = 5 Hz : 1H).

### (3R,4R)-4-[3-(R,S)-Hydroxy-3-(6-méthoxyquinolin-4-yl)propyl]-1-[2-(5-chlorothién-2-yl)]pipéridine-3-carboxylate de méthyle

Une solution de 1 g de (3R,4R)-4-[3-(R,S)-hydroxy-3-(6-méthoxyquinolin-4-yl)propyl]pipéridine-3-carboxylate de méthyle dans 40 cm³ d'acétonitrile et 10 cm³ de méthanol est agitée à une température voisine de 20°C, puis additionnée de 1,16 g de carbonate de potassium et 0,5 g d'iodure de potassium. A la suspension obtenue, on ajoute 1 g de 2-chloro-5-(2-chloroéthylthio)thiophène et 10 cm³ d'acétonitrile. Le mélange e st a gité pendant 72 heures à une température voisine de 80°C. La masse réactionnelle est versée sur 75 cm³ d'acétate d'éthyle, puis lavée par 3 fois 70 cm³ d'eau. La phase organique est séchée sur sulfate de sodium, filtrée, évaporée sous pression réduite (5 kPa) à une température voisine de 45°C. Le résidu obtenu est purifié par chromatographie sous une pression d'azote de 40 kPa sur une colonne de gel de silice (granulométrie 20-40µ ; diamètre 3,5 cm ; hauteur silice 31 cm), en éluant par de l'acétate d'éthyle et en recueillant des fractions de 30 cm³. Les fractions 24 à 52 sont réunies et concentrées sous pression réduite (5 kPa) à une température voisine de 40°C. On obtient 0,48 g de (3R,4R)-4-[3-(R,S)-hydroxy-3-(6-méthoxyquinolin-4-yl)propyl]-1-[2-(5-chlorothién-2-yl)] pipéridine-3-carboxylate de méthyle, sous forme d'une huile de couleur brune.
Spectre de masse : (IC) m/z : 535 MH⁺

### 2-Chloro-5-(2-chloroéthylthio)thiophène

A une solution de 17,9 g de 2-chloro-5-thiophènethiol dans 30 cm³ de 1-chloro-2-bromoéthane refroidi à une température voisine de 5°C, on ajoute sous agitation 28 cm³ d'une solution aqueuse de soude 5N, puis on laisse revenir la température au voisinage de 20°C, tandis que l'agitation est poursuivie pendant 16 heures. Le mélange réactionnel est dilué par 300 cm³ d'acétate d'éthyle, lavé par 3 fois 150 cm³ d'eau. La solution organique est séchée sur sulfate de sodium, filtrée, évaporée sous pression réduite (5 kPa) à une température voisine de 45°C. On obtient 20,6 g de 2-chloro-5-(2-chloroéthylthio)thiophène, sous forme d'une huile de couleur brune.
Spectre de masse : DCI m/z = 535 MH⁺

Le 2-chloro-5-thiophénethiol peut être préparé selon E. JONES et M. MOODIE, Tetrahedron, 1965, vol. 21, 1333-1336.

### Exemple de référence 27

### Acide (3R,4R)-4-[3-(6-méthoxyquinolin-4-yl)propyl]-1-[3-fluoro-3-phénylpropyl]pipéridine-3-carboxylique.

Un mélange de 0,4 g de (3R,4R)-4-[3-(6-méthoxyquinolin-4-yl)propyl]-1-[3-(R,S)-fluoro-3-phénylpropyl]pipéridine-3-carboxylate de méthyle et 0,18 g de monohydrate hydroxyde de lithium dans 2 cm³ d'eau et 10 cm³ d'acétone est agité pendant 3 jours à une température voisine de 20°C, puis évaporé sous pression réduite (5 kPa) à une température voisine de 40°C. Le résidu obtenu est purifié par chromatographie sous une pression d'argon de 50 kPa, sur une colonne de gel de silice (granulométrie 20-45µ ; diamètre 3 cm ; 50 g), en éluant par un mélange de dichlorométhane-méthanol (95/5 en volumes) et en recueillant d'abord une fraction de 910 cm³. Puis on élue par un mélange de dichlorométhane-méthanol (90/10 en volumes) et en recueillant des fractions de 13 cm³. Les fractions 47 à 69 sont réunies puis évaporées sous pression réduite (5 kPa) à une température voisine de 40°C. Le résidu d'évaporation est repris par du dichlorométhane, filtré, concentré dans les mêmes conditions que ci-dessus. Le produit obtenu est agité pendant 10 minutes dans 10 cm³ d'éther diisopropylique. Les cristaux sont filtrés, lavés par 1 fois 5 cm³ d'éther diisopropylique et 3 fois 5 cm³ de pentane. On obtient 0,102 g d'acide (3R,4R)-4-[3-(6-méthoxyquinolin-4-yl)propyl]-1-[3-fluoro-3-phénylpropyl]pipéridine-3-carboxylique, sous forme d'un solide de couleur grise fondant à 60°C, et correspondant à l'un des diastéréoisomères.
α_{D}²⁰ =+37,5 +/-0,9 dans le méthanol à 0,5%.
Spectre de R.M.N. ¹H (300 MHz, (CD₃)₂SO d6, δ en ppm) : de 1,45 à 3,10 (mts : 16H) ; 3,02 (t large, J = 7 Hz : 2H) ; 4,04 (s : 3H) ; 5,59 (mt, J_{HF} = 48 Hz : 1H) ; 7,32 (d, J = 4,5 Hz : 1H) ; de 7,35 à 7,50 (mt : 7H) ; 7,42 (d, J = 9 Hz : 1H) ; 8,62 (d, J = 4,5 Hz : 1H).

### (3R,4R)-4-[3-(6-Méthoxyquinolin-4-yl)propyl]-1-[3-(RS)-fluoro-3-phénylpropyl]pipéridine-3-carboxylate de méthyle

A une solution agitée de 1,18 g de (3R,4R)-4-[3-(6-méthoxyquinolin-4-yl)propyl]-1-[3-(R,S)-hydroxy-3-phénylpropyl]pipéridine-3-carboxylate de méthyle dans 17,7 cm³ de dichlorométhane, on ajoute sous atmosphère inerte à une température voisine de 20°C 0,425 cm³ de diéthylaminosulfure trifluorure. Après 3 heures d'agitation, le mélange est versé sur 32 cm³ d'une solution aqueuse saturée d'hydrogénocarbonate de sodium. On ajoute 15 cm³ de dichlorométhane, puis on agite le mélange pendant encore 10 minutes. Après décantation, la phase organique est séparée, tandis que la phase aqueuse est extraite par 3 fois 10 cm³ de dichlorométhane. Les extraits organiques sont réunis, lavés par 3 fois 20 cm³ d'eau, séchés sur sulfate de sodium, filtrés, concentrés sous pression réduite (5 kPa) à une température voisine de 40°C. On obtient 1,11 g d'un résidu que l'on purifie par chromatographie sous une pression d'argon de 50 kPa, sur une colonne de silice (granulométrie 20-45µ ; diamètre 2,8 cm ; 45 g silice), en éluant par un mélange d'acétate d'éthyle-méthanol (98/2 en volumes), et en recueillant des fractions de 15 cm³. Les fractions 10 à 20 sont réunies puis évaporées sous pression réduite (5 kPa) à une température voisine de 40°C. On obtient 0,6 g de (3R,4R)-4-[3-(6-méthoxyquinolin-4-yl)propyl]-1-[3-(RS)-fluoro-3-phénylpropyl]pipéridine-3-carboxylate de méthyle, sous forme d'une huile épaisse de couleur jaune clair.
Spectre infra rouge (CH₂Cl₂) : 2951 ; 1732 ; 1621 ; 1509 ; 1473 ; 1227 ; 1167 ; 1031 et 848 cm⁻¹

Le (3R,4R)-4-[3-(6-méthoxyquinolin-4-yl)propyl]-1-[3-(R,S)-hydroxy-3-phénylpropyl]pipéridine-3-carboxylate de méthyle peut être préparé par analogie avec la méthode décrite dans l'exemple 1.

### Exemple 28

### Trichlorhydrate d e l'acide (3R,4R)-4-[3-(R,S)-hydroxy-3-(6-méthoxyquinolin-4-yl)propyl]-1-[2-(pyridin-2-ylthio)éthyl]pipéridine-3-carboxylique

A une solution de 0,33 g de (3R,4R)-4-[3-(R,S)-hydroxy-3-(6-méthoxyquinolin-4-yl)propyl]-1-[2-(pyridin-2-ylthio)éthyl]pipéridine-3-carboxylate de méthyle dans 6,66 cm³ de méthanol maintenue à une température voisine de 20°C, on ajoute sous agitation et sous atmosphère d'azote 1,33 cm³ d'une solution aqueuse de soude 5N. La solution obtenue est chauffée au voisinage de 60°C pendant 16 heures. La masse réactionnelle est évaporée à sec sous pression réduite (5 kPa) à une température voisine de 60°C. Le résidu obtenu est repris dans 3,4 cm³ d'eau distillée, puis additionné de 3,43 cm³ d'acide chlorhydrique aqueux concentré à 36 %. On obtient une solution de couleur jaune que l'on évapore dans les mêmes conditions que précédemment. Le résidu d'évaporation est repris dans 10 cm³ d'un mélange de dichlorométhane-méthanol (80/20 en volumes). L'insoluble qui en résulte est filtré, lavé par 2 fois 2,5 cm³ d'un mélange de dichlorométhane-méthanol (90/10 en volumes). Le filtrat est évaporé sous pression réduite (5 kPa) à une température voisine de 40°C. On obtient 0,40 g de trichlorhydrate de l'acide (3R,4R)-4-[3-(R,S)-hydroxy-3-(6-méthoxyquinolin-4-yl)propyl]-1-[2-(pyridin-2-ylthio)éthyl]pipéridine-3-carboxylique, sous forme d'un solide de couleur beige fondant à 155°C.
Spectre de R.M.N. ¹H (400 M Hz, (CD₃)₂SO d 6, à une température de 383K, δ en ppm). On observe un mélange de deux diastéréoisomères :
de 1,50 à 2,30 et de 3,15 à 3,65 (mts : 16H); 4,00 (s : 3H) ; 5,38 (mt : 1H) ; 7,18 (dd large, J = 8 et 5 Hz : 1H) ; 7,38 (d, J = 8 Hz : 1H) ; de 7,55 à 7,65 (mt : 2H) ; 7,69 (t, J = 8 et 2 Hz : 1H); 7,80 (mt : 1H) ; 8,20 (d large, J = 10 Hz : 1H) ; 8,48 (dmt, J = 5 Hz : 1H) ; 8,35 (d large, J = 5 Hz : 1H).

### (3R,4R)-4-[3-(R,S)-Hydroxy-3-(6-méthoxyquinolin-4-yl)propyl]-1-[2-(pyridin-2-ylthio)éthyl]pipéridine-3-carboxylate de méthyle

Une solution agitée de 0,77 g de (3R,4R)-4-[3-oxo-3-(6-méthoxyquinolin-4-yl)propyl]-1-[2-(pyridin-2-ylthio)éthyl]pipéridine-3-carboxylate de méthyle dans 15 cm³ de méthanol est refroidie à une température voisine de 0°C sous atmosphère inerte. On ajoute 0,063 g de borohydrure de sodium, puis après 15 minutes la température est ramenée au voisinage de 20°C pendant 16 heures. Après addition de 5 cm³ d'une solution aqueuse saturée de chlorure d'ammonium, le mélange est agité pendant 10 minutes, puis évaporé sous pression réduite (5 kPa) à une température voisine de 40°C. Le résidu obtenu est repris dans 10 cm³ d'un mélange de dichlorométhane-méthanol (95/5 en volumes), puis filtré. L'insoluble est lavé par 2 fois 5 cm³ du même mélange. Le filtrat est évaporé dans les mêmes conditions que ci-dessus. On obtient une meringue que l'on purifie par chromatographie sous une pression d'azote de 100 kPa, sur une colonne de gel de silice (granulométrie 40-63µ ; diamètre 3,5 cm ; hauteur silice 35 cm) en éluant d'abord par un mélange de dichlorométhane-méthanol (96/4 en volumes), et en recueillant des fractions de 35 cm³. Après les 50 premières fractions, on élue par un mélange de dichlorométhane-méthanol (90/10 en volumes). Les fractions 61 à 90 sont réunies puis évaporées sous pression réduite (5 kPa) à une température voisine de 40°C. On obtient 0,33 g de (3R,4R)-4-[3-(R,S)-hydroxy-3-(6-méthoxyquinolin-4-yl)propyl]-1-[2-(pyridin-2-ylthio)éthyl]pipéridine-3-carboxylate de méthyle, sous forme d'une laque de couleur beige.
Spectre infra rouge (CH₂Cl₂) : 2596 ; 2951 ; 1622 ; 1579 ; 1508 ; 1455 ; 1415 ; 1242 ; 1228 ; 1125 ; 1031 ; 856 et 831 cm⁻¹

### (3R,4R)-4-[3-Oxo-3-(6-méthoxyquinolin-4-yl)propyl]-1-[2-(pyridin-2-ylthio)éthyl]pipéridine-3-carboxylate de méthyle

Un mélange de 1,35 g de dichlorhydrate de (3R,4R)-4-[3-oxo-3-(6-méthoxyquinolin-4-yl)propyl]-1-(2-chloroéthyl)pipéridine-3-carboxylate de méthyle dans 20 cm³ d'acétonitrile est agité à une température voisine de 20°C sous atmosphère inerte. On ajoute 1,37 g de carbonate de potassium et 0,456 g d'iodure de potassium, puis 0,367 g de 2-mercaptopyridine et 1 cm³ de méthanol. On obtient une suspension rouge que l'on chauffe à une température voisine de 80°C pendant 1 heure 30 minutes. Après refroidissement de la masse réactionnelle à une température voisine de 20°C, l'insoluble est filtré, lavé par de l'acétonitrile. Le filtrat est évaporé sous pression réduite (5 kPa) à une température voisine de 40°C. Le résidu d'évaporation est purifié par chromatographie sous une pression d'azote de 100 kPa, sur une colonne de gel de silice (granulométrie 40-63µ ; diamètre 3,5 cm ; hauteur silice 35 cm), en éluant par un mélange d'acétate d'éthyle-méthanol (95/5 en volumes) et en recueillant des fractions de 35 cm³. Les fractions 19 à 40 sont réunies puis évaporées sous pression réduite (5 kPa) à une température voisine de 40°C. On obtient 0,77 g de (3R,4R)-4-[3-oxo-3-(6-méthoxyquinolin-4-yl)propyl]-1-[2-(pyridin-2-ylthio)éthyl]pipéridine-3-carboxylate de méthyle, sous forme d'une huile visqueuse de couleur rouge.
Spectre infra rouge (CH₂Cl₂) : 2952 ; 2809 ; 1733 ; 1692 ; 1620 ; 1580 ; 1455 ; 1415 ; 1243 ; 1125 ; 1029 et 853 cm⁻¹

Le (3R,4R)-4-[3-oxo-3-(6-méthoxyquinolin-4-yl)propyl]-1-[2-(pyridin-2-ylthio) éthyl]pipéridine-3-carboxylate de m éthyle est préparé par analogie avec la méthode décrite dans l'exemple 11.

### Exemple 29

### Dichlorhydrate de l'acide (3R,4R)-4-[3-hydroxy-3-(6-méthoxyquinolin-4-yl)propyl]-1-[2-(2-thiénylthio)éthyl]pipéridine-3-acétique, diastéréoisomère A et acide (3R,4R)-4-[3-hydroxy-3-(6-méthoxyquinolin-4-yl)propyl]-1-[2-(2-thiénylthio)éthyl]pipéridine-3-acétique, diastéréoisomère B.

1,2 g d'acide (3R,4R)-4-[3-(R,S)-hydroxy-3-(6-méthoxyquinolin-4-yl)propyl]-1-[2-(2-thiénylthio)éthyl]pipéridine-3-acétique est chromatographié sur une colonne de 35 cm de long et 6 cm de diamètre, conditionnée avec 700 g de silice Kromasil®-CN (granulométrie 10µ). L'élution est effectuée à l'aide d'un mélange de dichlorométhane-éthanol-triétylamine (98/2/0,1 en volumes) Le d ébit e st de 70 cm³/minute. La détection est effectuée en ultra violet à 265 nm. Après plusieurs injections préparatives, on recueille les fractions correspondant au diastéréoisomère A. Celles-ci sont concentrées sous pression réduite (5 kPa) à une température voisine de 40°C. On obtient 0,34 g d'un produit que l'on salifie de la façon suivante : 0,3 g de ce produit est repris dans 30 cm³ d'éther, filtré puis dissous dans 25 cm³ d'acétone. La solution obtenue est versée sur 5 cm³ d'éther chlorhydrique 5N. Après concentration du mélange sous pression réduite dans les mêmes conditions que ci-dessus, le résidu obtenu est repris par 20 cm³ d'eau, la solution lyophilisée. On obtient 0,190 g de dichlorhydrate de l'acide (3R,4R)-4-[3-hydroxy-3-(6-méthoxyquinolin-4-yl)propyl]-1-[2-(2-thiénylthio)éthyl]pipéridine-3-acétique, diastéréoisomère A, sous forme d'un lyophilisat de couleur orangée. Au cours de la séparation préparative des deux diastéréoisomères, les fractions-mélange sont concentrées comme ci-dessus pour un retraitement. Les conditions de séparation sont les suivantes : colonne de 35 cm de long et 6 cm de diamètre, conditionnée avec 700 g de silice Kromasil® (granulométrie 10µ). L'élution est effectuée à l'aide d'un mélange de dichlorométhane-acétonitrile-méhanol-triétylamine (60/40/4/0,1 en volumes) Le débit est de 80 cm³/minute. Après 3 injections préparatives on recueille les fractions correspondant au diastéréoisomère B. Celles-ci sont concentrées dans les mêmes conditions que ci-dessus. On obtient 0,34 g d'acide (3R,4R)-4-[3-hydroxy-3-(6-méthoxyquinolin-4-yl)propyl]-1-[2-(2-thiénylthio) éthyl]pipéridine-3-acétique, diastéréoisomère B, sous forme d'une meringue de couleur jaune. (α_{D}²⁰ =+55,4 +/-1,1 dans le dichlorométhane à 0,5 %).
diastéréoisomère A : Spectre de R.M.N. ¹H (400 MHz, (CD₃)₂SO d6 avec ajout de quelques gouttes de CD₃COOD d4 à une température de 373K, δ en ppm) : de 1,40 à 1,90 et de 2,20 à 2,55 (mts : 10H) ; de 2,95 à 3,35 (mt : 8H) ; 3,99 (s : 3H) ; 5,40 (mt : 1H) ; 7,07 (dd, J = 5,5 et 3,5 Hz : 1H) ; 7,27 (d large, J = 3,5 Hz : 1H) ; de 7,55 à 7,65 (mt : 3H) ; 7,85 (d, J = 4,5 Hz : 1H) ; 8,17 (d, J = 9 Hz : 1H) ; 8,88 (d, J = 4,5 Hz : 1H).
diastéréoisomère B : Spectre de R.M.N. ¹H (250 MHz, (CD₃)₂SO d6, δ en ppm) : de 1,20 à 2,80 (mts : 16H) ; 2,88 (t large, J = 7 Hz : 2H) ; 3,92 (s : 3H) ; 5,27 (mt : 1H) ; 7,04 (dd, J = 5,5 et 3,5 Hz : 1H) ; 7,17 (dd, J = 3,5 et 1,5 Hz : 1H) ; de 7,30 à 7,45 (mt : 2H) ; 7,54 (d, J = 4,5 Hz : 1H) ; 7,60 (dd, J = 5,5 et 1,5 Hz : 1H); 7,94 (d, J = 9,5 Hz : 1H) ; 8,71 (d, J = 4,5 Hz : 1H).

### Acide (3R,4R)-4-[3-(R,S)-hydroxy-3-(6-méthoxyquinolin-4-yl)propyl]-1-[2-(2-thiénylthio)éthyl]pipéridine-3-acétique

Un mélange de 0,22 g de dichlorhydrate de (3R,4R)-4-[3-(R,S)-hydroxy-3-(6-méthoxyquinolin-4-yl)propyl]-1-[2-(2-thiénylthio)éthyl]pipéridine-3-acétate de méthyle dans 5 cm³ de dioxanne, additionné de 0,683 cm³ de soude aqueuse 5N, est agité pendant 20 heures à une température voisine de 60°C. Après évaporation sous pression réduite (5 kPa) à une température voisine de 40°C, le résidu obtenu est purifié par chromatographie à pression atmosphérique sur une colonne de gel de silice (granulométrie 20-45µ ; diamètre 1 cm ; volume de silice 20 cm³) en éluant par un mélange de chloroforme-méthanol-ammoniaque (12/3/1 en volumes). Les fractions 1 à 3 sont réunies, concentrées dans les conditions de ci-dessus. On obtient un produit que l'on sèche à l'étuve à poids constant sous pression réduite (10 Pa) à une température voisine de 40°C. On obtient 0,179 g d'acide (3R,4R)-4-[3-(R,S)-hydroxy-3-(6-méthoxyquinolin-4-yl)propyl]-1-[2-(2-thiénylthio)éthyl]pipéridine-3-acétique, sous forme d'une meringue blanche qui est un mélange de deux diastéréoisomères.
Spectre de R.M.N. ¹H (300 MHz, (CD₃)₂SO d6, δ en ppm). On observe un mélange de deux diastéréoisomères :
de 1,15 à 2,80 (mts : 16H) ; 2,88 (t large, J = 7 Hz : 2H) ; 3,91 et 3,92 (2s : 3H en totalité) ; 5,26 (mf: 1H) ; 5,53 (mt : 1H) ; 7,04 (dd, J = 5,5 et 3,5 Hz : 1H) ; 7,16 (dd, J = 3,5 et 1 Hz : 1H) ; de 7,30 à 7,45 (mt : 2H) ; de 7,54 (mt : 1H) ; 7,60 (d large, J = 5,5 Hz : 1H) ; 7,94 (d, J = 9 Hz : 1H) ; 8,70 (d, J = 4,5 Hz : 1H).

### Dichlorhydrate de (3R,4R)-4-[3-(R,S)-hydroxy-3-(6-méthoxyquinolin-4-yl)propyl]-1-[2-(2-thiénylthio)éthyl]pipéridine-3-acétate de méthyle

A une solution de 0,84 g de (3R,4R)-4-[3-oxo-3-(6-méthoxyquinolin-4-yl)propyl]-1-[2-(2-thiénylthio)éthyl]pipéridine-3-acétate de méthyle dans 12 cm³ de méthanol maintenue à une température voisine de 20°C, on ajoute sous agitation 1 goutte de soude aqueuse 5N, puis 0,17 g de borohydrure de sodium par petites fractions. Le mélange est agité pendant 3 heures à une température voisine de 20°C. Après addition de 10 cm³ d'eau, on évapore le méthanol sous pression réduite (5 kPa) à une température voisine de 40°C. Le résidu obtenu est extrait par 2 fois 20 cm³ de dichlorométhane, puis les extraits réunis sont lavés par 2 fois 30 cm³ d'eau, séchés sur sulfate de magnésium, filtrés, concentrés sous pression réduite (5 kPa) à une température voisine de 40°C. On obtient une huile que l'on purifie par chromatographie à pression atmosphérique sur une colonne de gel de silice (granulométrie 20-45µ ; diamètre 2,5 cm ; volume silice 150 cm³), en éluant par un mélange de dichlorométhane-méthanol (99/1 en volumes). Les fractions 56 à 115 sont réunies puis concentrées comme ci-dessus. On obtient une huile dont on fait le chlorhydrate de la façon suivante : l'huile est solubilisée dans 20 cm³ d'éther diéthylique, puis versée sur 2 cm³ d'éther chlorhydrique 1N. Le précipité formé est essoré, séché à l'étuve sous pression réduite (10 Pa) à une température voisine de 40°C. On obtient 0,3 g de dichlorhydrate de (3R,4R)-4-[3-(R,S)-hydroxy-3-(6-méthoxyquinolin-4-yl)propyl]-1-[2-(2-thiénylthio)éthyl]pipéridine-3-acétate de méthyle, sous forme d'un solide blanc.
Spectre de R.M.N. ¹H (400 MHz, (CD₃)₂SO d6 à une température de 383K, δ en ppm). On observe un mélange de deux diastéréoisomères :
de 1,40 à 2,00 et de 2,25 à 2,70 (mts : 10H en totalité) ; de 2,90 à 3,40 (mf : 4H) ; 3,27 (mt : 4H) ; 3,61 et 3,63 (2s : 3H en totalité) ; 4,00 (s : 3H) ; 5,38 (mt : 1H) ; 7,10 (dd, J = 5 et 3,5 Hz : 1H) ; 7,29 (d large, J = 3,5 Hz : 1H) ; de 7,55 à 7,60 (mt : 2H) ; 7,65 (d, J = 5 Hz : 1H) ; 7,76 (mt : 1H) ; 8,16 (d, J = 9,5 Hz : 1H) ; 8,85 (d, J = 4,5 Hz : 1H).

### (3R,4R)-4-[3-Oxo-3-(6-méthoxyquinolin-4-yl)propyl]-1-[2-(2-thiénylthio)éthyl]pipéridine-3-acétate de méthyle

A une solution de 2,8 g de (3R,4R)-4-[3-oxo-3-(6-méthoxyquinolin-4-yl)propyl] pipéridine-3-acétate de méthyle dans 60 cm³ d'acétonitrile on ajoute à une température voisine de 20°C, sous agitation, 1,5 g de 2-(2-chloroéthylthio)thiophène, puis 5,2 g de carbonate de potassium et 2,5 g d'iodure de potassium. Le mélange est chauffé pendant 20 heures à une température voisine du reflux, puis refroidi aux environs de 20°C. On ajoute 130 cm³ d'eau et 50 cm³ d'acétate d'éthyle au mélange réactionnel. Après décantation du mélange, la phase organique est lavée par 150 cm³ d'eau, séchée sur sulfate de magnésium, filtrée puis concentrée sous pression réduite (5 kPa) à une température voisine de 40°C. On obtient une huile que l'on purifie par chromatographie à pression atmosphérique sur une colonne de gel de silice (granulométrie 20-45µ ; diamètre 2,5 cm ; volume silice100 cm³) en éluant par un mélange de dichlorométhane-méthanol (95/5 en volumes). On recueille d'abord une fraction de 30 cm³, puis des fractions d'environ 10 cm³. Les fractions 1 à 4 sont réunies, puis évaporées comme précédemment. On obtient 2,3 g d'une huile brune que l'on soumet une seconde fois à une purification par chromatographie à pression atmosphérique sur une colonne de gel de silice (granulométrie 20-45µ ; diamètre 2,5 cm ; volume silice 300 cm³), en éluant par un mélange de dichlorométhane-méthanol ( 99/1 en volumes) et en recueillant des fractions de 10 cm³ environ. Les fractions 21 à 30 sont réunies puis concentrées dans les conditions de ci-dessus. On obtient 0,84g de (3R,4R)-4-[3-oxo-3-(6-méthoxyquinolin-4-yl)propyl]-1-[2-(2-thiénylthio)éthyl]pipéridine-3-acétate de méthyle, sous forme d'une huile jaune pâle.
Spectre infra rouge (CH₂Cl₂) : 2937 ; 2806 ; 2765 ; 1731 ; 1693 ; 1620 ; 1505 ; 1243 et 849 cm⁻¹

### (3R,4R)-4-[3-Oxo-3-(6-méthoxyquinolin-4-yl)propyl]pipéridine-3-acétate de méthyle

Une solution de 10,8 g d'acide (3R,4R)-4-[3-oxo-3-(6-méthoxyquinolin-4-yl)propyl]-1-(tert-butyloxycarbonyl)pipéridine-3-acétique dans 460 cm³ de méthanol anhydre additionnés de 4,3 cm³ d'acide sulfurique concentré (d=1,83) est chauffée à une température voisine de 65 °C sous agitation pendant 2 heures. Après refroidissement aux environs de 20°C, le mélange réactionnel est évaporé sous pression réduite (5 kPa) à une température voisine de 40°C, puis le résidu obtenu est repris par 200 cm³ d'eau, rendu alcalin par addition d'hydrogénocarbonate de sodium j usqu'à l'obtention d'un pH voisin de 8-9. Le mélange est extrait par 4 fois 200 cm³ d'acétate d'éthyle. La phase aqueuse est alcalinisée jusqu'à un pH voisin de 11 par addition de la quantité nécessaire de carbonate de sodium. Le mélange est extrait par 2 fois 200 cm³ d'acétate d'éthyle. Les extraits organiques sont réunis, séchés sur sulfate de magnésium, filtrés, évaporés sous pression réduite (5 kPa) à une température voisine de 40°C. On obtient 6,84 g de (3R,4R)-4-[3-oxo-3-(6-méthoxyquinolin-4-yl) propyl]pipéridine-3-acétate de méthyle, sous forme d'une huile de couleur brune.
Spectre infra rouge (CCl₄) : 2935 ; 2812 ; 1738 ; 1692 ; 1620 ; 1504 ; 1242 ; 1032 et 851 cm⁻¹

### Acide (3R,4R)-4-[3-oxo-3-(6-méthoxyquinolin-4-yl)propyl]-1-(tert-butyloxycarbonyl)pipéridine-3-acétique

A une solution de 1,2 g de (3R,4R)-4-[3-oxo-3-(6-méthoxyquinolin-4-yl)propyl]-1-(tert-butyloxycarbonyl)pipéridine-3-acétaldéhyde dans 60 cm³ d'acétone, on ajoute en 1 heure environ, sous agitation et à une température voisine de 25°C, une solution de 0,85 g de permanganate de potassium dans 25 cm³ d'eau et 120 cm³ d'acétone. Le mélange est agité pendant 3 heures à cette même température, puis refroidi à environ 10°C. Une solution de 5 g de sulfite de sodium dans 200 cm³ d'eau est ajoutée au mélange réactionnel, puis le mélange obtenu est filtré sur célite. L'acétone du filtrat est évaporée sous pression réduite (5 kPa) à une température voisine de 40°C, puis le résidu d'évaporation est repris par 200 cm³ d'eau, lavé par 200 cm³ d'éther diéthylique. La phase aqueuse est décantée, acidifiée par de l'acide citrique à l'état solide à un pH voisin de 3-4, extraite par 200 cm³ d'éther diéthylique. La solution éthérée décantée est séchée sur sulfate de magnésium, filtrée, évaporée dans les conditions de ci-dessus. On obtient 0,74 g d'acide (3R,4R)-4-[3-oxo-3-(6-méthoxyquinolin-4-yl)propyl]-1-(*tert*-butyloxycarbonyl)pipéridine-3-acétique, sous forme d'un solide jaune.
Spectre infra rouge (KBr) : 2932 ; 2588 ; 1730 ; 1690 ; 1620 ; 1431 ; 1246 ; 1165 et 857 cm⁻¹

### (3R,4R)-4-[3-Oxo-3-(6-méthoxyquinolin-4-yl)propyl]-1-(tert-butyloxycarbonyl)pipéridine-3-acétaldéhyde

A une solution de 8,3 cm³ de chlorure d'oxalyle dans 65 cm³ de dichlorométhane refroidie à une température voisine de -60°C, on ajoute lentement, sous agitation et sous atmosphère inerte, un mélange de 13,7 g de diméthylsulfoxyde dans 65 cm³ de dichlorométhane. Après 15 minutes d'agitation du mélange, on ajoute lentement 10 g de (3R,4R)-3-(2-hydroxyéthyl)-4-[3-(R,S)-hydroxy-3-(6-méthoxyquinolin-4-yl) propyl]-1-(*tert*-butyloxycarbonyl)pipéridine solubilisés dans 65 cm³ de dichlorométhane. Après 30 minutes d'agitation du mélange, on ajoute finalement, goutte à goutte, 61,7 cm³ de triéthylamine dissous dans 65 cm³ de dichlorométhane. Le mélange est encore agité pendant 3 heures aux environs de -60°C, puis versé sur 400 cm³ d'eau glacée. Après décantation du mélange, la phase organique est lavée par 400 cm³ d'une solution aqueuse d'acide citrique à 10 % (en masse), puis séchée sur sulfate de magnésium, filtrée, évaporée sous pression réduite (5 kPa) à une température voisine de 40°C. On obtient 9,95 g de (3R,4R)-4-[3-oxo-3-(6-méthoxy-quinolin-4-yl)propyl]-1-(*tert*-butyloxycarbonyl)pipéridine-3-acétaldéhyde, sous forme d'une huile de couleur brune.
Spectre infra rouge (CCl₄) : 2932 ; 2720 ; 1729 ; 1694 ; 1430 ; 1244 ; 1164 et 850 cm⁻¹

### (3R,4R)-3-(2-Hydroxyéthyl)-4-[3-(R,S)-hydroxy-3-(6-méthoxyquinolin-4-yl)propyl]-1-(tert-butyloxycarbonyl)pipéridine

A une solution de 52,6 g de (3R,4R)-4-[3-oxo-3-(6-méthoxyquinolin-4-yl)propyl]-1-(*tert*-butyloxycarbonyl)-3-vinylpipéridine dans 500 cm³ de toluène, on ajoute 33,4 cm³ de complexe de triéthylamine borane sous agitation, à une température voisine de 20°C, puis le mélange est chauffé pendant 18 heures à une température voisine de 110°C. Après avoir concentré le mélange réactionnel sous pression réduite (5 kPa) à une température voisine de 45°C, le résidu obtenu est repris par 500 cm³ de-tétrahydrofuranne. La solution qui en résulte est additionnée en 20 minutes environ de 63 cm³ d'eau, puis on ajoute en 1 heure environ et par petites fractions, 47,5 g de perborate de sodium. Le mélange est agité pendant 4 heures à une température voisine de 20°C, puis on ajoute 300 cm³ d'une solution saturée de chlorure d'ammonium. La solution organique est décantée, séchée sur sulfate de magnésium, concentrée dans les mêmes conditions que ci-dessus. Le résidu obtenu est purifié par chromatographie sur une colonne de gel de silice (granulométrie 20-45µ ; diamètre 9 cm ; volume silice 2500 cm³) en éluant d'abord par un mélange de dichlorométhane-méthanol (97,5/2,5 en volumes) et en recueillant des fractions d'1 litre. Les fractions 1 à 17 sont séparées, puis on élue par un mélange de dichlorométhane-méthanol (95/5 en volumes) en recueillant des fractions d'1 litre. Les fractions 30 à 35 sont réunies et l'on élue finalement par un mélange de dichlorométhane-méthanol (90/10 en volumes) en recueillant des fractions d'1 litre. Les fractions 36 à 41 sont réunies, tandis que l'ensemble des fractions 30 à 41 est évaporé sous pression réduite (5 kPa) à une température voisine de 40°C. On obtient 20 g de (3R,4R)-3-(2-hydroxyéthyl)-4-[3-(R,S)-hydroxy-3-(6-méthoxyquinolin-4-yl)propyl]-1-(*tert*-butyloxycarbonyl)pipéridine, sous forme d'une huile.
Spectre infra rouge (CH₂Cl₂) : 3612 ;2480 ;2937 ; 1680 ;1432 ;1243 ;1163 et 859 cm⁻¹

### (3R,4R)-4-[3-Oxo-3-(6-méthoxyquinolin-4-yl)propyl]-1-(tert-butyloxycarbonyl)-3-vinylpipéridine

A une solution agitée de 126 g de (3R,4R)-4-[3-oxo-3-(6-méthoxyquinolin-4-yl) propyl]-3-vinylpipéridine dans 1700 cm³ de dichlorométhane, on ajoute en 20 minutes environ, à une température voisine de 20°C, 162 cm³ de triéthylamine, puis en 2 heures 85 g de di-tertiobutyl dicarbonate solubilisés dans 300 cm³ de dichlorométhane. Le mélange est agité pendant 16 heures à une température voisine de 20°C, puis on y ajoute 400 cm³ d'eau. La phase organique est décantée, séchée sur sulfate de magnésium, filtrée, évaporée sous pression réduite (5 kPa) à une température voisine de 40°C. On obtient un résidu huileux que l'on reprend par 1000 cm³ d'acétate d'éthyle et que l'on lave par 2 fois 200 cm³d'eau, 1 fois par 250 cm³ d'une solution aqueuse saturée d'acide citrique, 2 fois par 200 cm³ d'eau. La solution organique est séchée sur sulfate de magnésium, filtrée, concentrée dans les conditions de ci-dessus. On obtient 148 g de (3R,4R)-4-[3-oxo-3-(6-méthoxyquinolin-4-yl)propyl]-1-(*tert*-butyloxycarbonyl)-3-vinylpipéridine, sous forme d'une huile de couleur brune.
Spectre infra rouge (CH₂Cl₂) : 2979 ;1683 ;1431 ;1246 ;1164 et 853 cm⁻¹

La (3R,4R)-4-[3-oxo-3-(6-méthoxyquinolin-4-yl)propyl]-3-vinylpipéridine peut être obtenue par application de la méthode décrite dans la demande de brevet FR 2 354 771.

### Exemple 30

### Acide (3R,4R)-1-[2-(3-fluorophénylthio)éthyl]-4-[3-hydroxy-3-(6-méthoxyquinolin-4-yl)propyl]pipéridine-3-acétique, diastéréoisomère A et acide (3R,4R)- 1-[2-(3-fluorophénylthio)éthyl]-4-[3-hydroxy-3-(6-méthoxyquinolin-4-yl)propyl]pipéridine-3-acétique, diastéréoisomère B.

On chromatographie 0,7 g d'acide (3R,4R)-1-[2-(3-fluorophénylthio)éthyl]-4-[3-(R,S)-hydroxy-3-(6-méthoxyquinolin-4-yl)propyl]pipéridine-3-acétique sur une colonne de 35 cm de long et 6 cm de diamètre, conditionnée avec 700 g de silice KROMASIL® (granulométrie 10µ). L'élution est effectuée à l'aide d'un mélange de dichlorométhane-acétonitrile-méthanol-triéthylamine (56/40/4/0,5 en volumes). Le débit est de 70 cm³/mn. La détection est effectuée en ultra violet à 265 nm. Plusieurs injections préparatives ont conduit à la séparation des 2 diastéréoisomères. Les fractions correspondant au premier, le diastéréoisomère A, sont concentrées sous pression réduite (5 kPa) à une température voisine de 40°C. La masse cristalline obtenue est séchée à l'étuve sous pression réduite (10 Pa) à une température voisine de 20°C. On obtient 0,185 g d'acide (3R,4R)-1-[2-(3-fluorophénylthio)éthyl]-4-[3-hydroxy-3-(6-méthoxyquinolin-4-yl)propyl]pipéridine-3-acétique, diastéréoisomère A, sous forme d'un solide de couleur blanc cassé. (α_{D}²⁰ = -55,9 +/-1,2 dans le méthanol à 0,5 %). Les fractions mélange sont concentrées dans les mêmes conditions que ci-dessus pour retraitement. De nouvelles injections sont réalisées pour obtenir le second diastéréoisomère. Trois injections préparatives permettent d'obtenir les fractions correspondant au diastéréoisomère B. Celles-ci sont concentrées sous pression réduite (5 kPa) à une température voisine de 40°C. La masse cristalline obtenue est séchée à l'étuve sous pression réduite (10 Pa) à une température voisine de 20°C. On obtient 0,200 g d'acide(3R,4R)-1-[2-(3-fluorophénylthio)éthyl]-4-[3-hydroxy-3-(6-méthoxy-quinolin-4-yl)propyl]pipéridine-3-acétique, diastéréoisomère B, sous forme d'un solide de couleur blanc cassé. (α_{D}²⁰ = +41,0 +/-1,0 dans le méthanol à 0,5 %).
diastéréoisomère A : Spectre de R.M.N.¹H (400 MHz, (CD₃)₂SO d6, δ en ppm) : de 1,15 à 1,85 et de 1,95 à 2,20 (2 séries de mts : 11H en totalité) ; de 2,40 à 2,60 (mt : 3H) ; 2,65 à 2,80 (mt : 2H) ; 3,08 (t, J = 7 Hz : 2H) ; 3,92 (s : 3H) ; 5,27 (mt : 1H) ; 5,52 (mf : 1H) ; 6,98 (t dédoublé, J = 8,5 et 2,5 Hz : 1H) ; de 7,10 à 7,20 (mt : 2H) ; de 7,25 à 7,45 (mt : 3H) ; 7,55 (d, J = 4,5 Hz : 1H) ; 7,94 (d, J = 9 Hz : 1H) ; 8,71 (d, J = 4,5 Hz : 1H).
diastéréoisomère B : Spectre de R.M.N.¹H (400 MHz, (CD₃)₂SO d6, δ en ppm) : de 1,30 à 1,90 et de 1,95 à 2,15 (2 séries de mts : 10H en totalité) ; de 2,40 à 2,60 (mt : 3H) ; 2,75 (mt : 1H) ; de 2,95 à 3,15 (mf : 2H) ; 3,10 (t, J = 7 Hz : 2H) ; 3,93 (s : 3H) ; 5,28 (mt : 1H) ; 5,53 (d, J = 5 Hz : 1H) ; 7,00 (t dédoublé, J = 8,5 et 2,5 Hz : 1H) ; de 7,10 à 7,20 (mt : 2H) ; 7,35 (mt : 1H) ; de 7,35 à 7,45 (mt : 2H) ; 7,55 (d, J = 4,5 Hz : 1H) ; 7,95 (d, J = 9,5 Hz : 1H) ; 8,72 (d, J = 4,5 Hz : 1H) ; de 11,00 à 12,5 (mf très étalé : 1H).

### Acide (3R,4R)-1-[2-(3-fluorophénylthio)éthyl]-4-[3-(R,S)-hydroxy-3-(6-méthoxyquinolin-4-yl)propyl] pipéridine-3-acétique

Une solution de 1,3 g de (3R,4R)-1-[2-(3-fluorophénylthio)éthyl]-4-[3-(R,S)-hydroxy-3-(6-méthoxyquinolin-4-yl)propyl]pipéridine-3-acétate de méthyle dans 20 cm³ de dioxanne, additionnée de 2 cm³ de soude aqueuse 5N, est chauffée sous agitation pendant 20 heures à une température voisine de 60°C. Le mélange réactionnel est évaporé sous pression réduite (5 kPa) à une température voisine de 40°C, puis le résidu obtenu est repris par 30 cm³ d'eau et 5 cm³ de méthanol, acidifié par de l'acide citrique. Les solvants sont évaporés dans les mêmes conditions que précédemment, puis le résidu obtenu est repris par 70 cm³ d'un mélange de dichlorométhane-méthanol (80/20 en volumes). Les cristaux d'acide citrique sont filtrés ; le filtrat est évaporé comme ci-dessus, et le résidu d'évaporation est repris par 30 cm³d'éther diéthylique, filtré, séché à l'air. On obtient 1,1 g d'acide (3R,4R)-1-[2-(3-fluorophénylthio)éthyl]-4-[3-(R,S)-hydroxy-3-(6-méthoxyquinolin-4-yl)propyl]pipéridine-3-acétique, sous forme d'un solide de couleur crème.
Spectre de R.M.N. ¹H (300 MHz, (CD₃)₂SO d6, δ en ppm). On observe un mélange de deux diastéréoisomères :
de 1,15 à 1,90 - de 2,00 à 2,25 et de 2,35 à 2,90 (mts : 16H en totalité) ; 3,13 (t large, J = 7 Hz : 2H); 3,92 (s : 3H); 5,27 (mt : 1H) ; 5,54 (mt : 1H) ; 7,00 (t dédoublé, J = 8,5 Hz : 1H) ; de 7,10 à 7,25 (mt : 2H) ; de 7,30 à 7,50 (mt : 3H) ; 7,55 (mt : 1H) ; 7,94 (d, J = 9 Hz : 1H) ; 8,71 (d, J = 4,5 Hz : 1H) ; de 11,50 à 13,50 (mf très étalé : 1H).

### Exemple 31

### Dichlorhydrate de (3R,4R)-1-[2-(3-fluorophénylthio)éthyl]-4-[3-(R,S)-hydroxy-3-(6-méthoxyquinolin-4-yl)propyl]pipéridine-3-acétate de méthyle

A une solution de 3,2 g de (3R,4R)-1-[2-(3-fluorophénylthio)éthyl]-4-[3-oxo-3-(6-méthoxyquinolin-4-yl)propyl]pipéridine-3-acétate de méthyle dans 50 cm³ de méthanol, additionnée de 2 gouttes de soude aqueuse 5N et maintenue à une température voisine de 20°C, on ajoute sous agitation et par petites fractions 0,28 g de borohydrure de sodium. Le mélange est agité pendant 4 heures à une température voisine de 20°C. Après addition de 30 cm³ d'eau, le mélange est extrait par 2 fois 30 cm³ de dichlorométhane. Les phases organiques réunies sont lavées par 6 0 cm³ d'eau, séchées sur sulfate de magnésium, filtrées, évaporées sous pression réduite (5 kPa) à une température voisine de 40°C. L'huile résiduelle est purifiée par chromatographie à pression atmosphérique sur une colonne de gel de silice (granulométrie 20-45µ ; diamètre 4 cm ; volume silice 520 cm³), en éluant par un mélange de dichlorométhane-méthanol (99/l) et en recueillant des fractions d'environ 60 cm³. Les fractions 40 à 72 sont réunies puis évaporées comme dans les conditions précédentes. On obtient 1,7 g d'une huile dont on fait le chlorhydrate de la façon suivante : une solution de 0,30 g d'huile dans 5 cm³ d'éther diéthylique est versée sur 5 cm³ d'éther chlorhydrique 5N. Le gel obtenu est dilué par 10 cm³ d'éther, agité pendant 15 minutes à une température voisine de 20°C, puis évaporé sous pression réduite (5 k Pa) à u ne t empérature voisine d e 3 0°C. L e résidu obtenu est solubilisé dans 30 cm³ d'eau, puis lyophilisé. On obtient 0,26 g de dichlorhydrate de (3R,4R)-1-[2-(3-fluorophénylthio)éthyl]-4-[3-(R,S)-hydroxy-3-(6-méthoxyquinolin-4-yl)propyl] pipéridine-3-acétate de méthyle, sous forme d'un lyophilisat de couleur crème.
Spectre de R.M.N. ¹H (400 MHz, (CD₃)₂SO d6 avec ajout de quelques gouttes de CD3COOD d4 à une température de 373K, δ en ppm). On observe un mélange de deux diastéréoisomères :
de 1,45 à 2,00 et de 2,25 à 2,60 (mts : 10H en totalité) ; de 3,05 à 3,45 (mts : 8H) ; 3,60 et 3,63 (2s : 3H en totalité) ; 3,97 et 3,98 (2s : 3H en totalité) ; 5,39 (mt : 1H) ; 7,03 (mt : 1H) ; 7,23 (mt : 2H) ; 7,37 (mt : 1H) ; de 7,50 à 7,65 (mt : 2H) ; 7,80 (mt : 1H) ; 8,12 (d, J = 9,5 Hz : 1H) ; 8,84 (d, J = 5 Hz : 1H).

### (3R,4R)-1-[2-(3-Fluorophénylthio)éthyl]-4-[3-oxo-3-(6-méthoxyquinolin-4-yl)propyl]pipéridine-3-acétate de méthyle

A une solution de 4 g de 1-[(2-chloroéthyl)thio]-3-fluorobenzène dans 50 cm³ d'acétonitrile, on ajoute goutte à goutte, sous agitation et à une température voisine de 20°C, 2,26 g de (3R,4R)-4-[3-oxo-3-(6-méthoxyquinolin-4-yl)propyl]pipéridine-3-acétate de méthyle préalablement solubilisés dans 50 cm³ d'acétonitrile, puis 6,91 g de carbonate de potassium et 2 g d'iodure de potassium. Le mélange est chauffé pendant 18 heures à une température voisine de 70°C. Après une addition supplémentaire de 0,3 g d'iodure de potassium et un chauffage complémentaire à une température voisine de 70°C pendant 4 heures, la masse réactionnelle est refroidie aux environs de 20°C, additionnée de 200 cm³ d'eau, extraite par 2 fois 150 cm³ d'acétate d'éthyle. Les extraits réunis sont lavés par 300 cm³ d'eau, séchés sur sulfate de magnésium, filtrés, évaporés sous pression réduite (5 kPa) à une température voisine de 40°C. Le résidu obtenu est purifié par chromatographie à pression atmosphérique sur une colonne de gel de silice ( granulométrie 20-45µ ; diamètre 5 ,2 cm ; volume silice 950 cm³) en éluant par un mélange de dichlorométhane-méthanol (99/l en volumes), et en recueillant des fractions d'environ 60 cm³. Les fractions 24 à 36 sont réunies, concentrées dans les conditions de ci-dessus. On obtient 3,3 g de (3R,4R)-1-[2-(3-fluorophénylthio)éthyl]-4-[3-oxo-3-(6-méthoxyquinolin-4-yl)propyl]pipéridine-3-acétate de méthyle, sous forme d'une huile de couleur jaune.
Spectre infra rouge (CH₂Cl₂) : 2936 ; 2806 ; 1731 ; 1692 ; 1620 ; 1505 ; 1474 ; 1243 ; 881 et 853 cm⁻¹

Le (3R,4R)-4-[3-oxo-3-(6-méthoxyquinolin-4-yl)propyl]pipéridine-3-acétate de méthyle a été obtenu dans l'exemple 29.

### Exemple 32

### Acide (3R,4R)-1-[2-(cyclohexylthio)éthyl]-4-[3-hydroxy-3-(6-méthoxyquinolin-4-yl)propyl]pipéridine-3-acétique, diastéréoisomère A et acide (3R,4R)-1-[2-(cyclohexylthio)éthyl]-4-[3-hydroxy-3-(6-méthoxyquinolin-4-yl)propyl]pipéridine-3-acétique, diastéréoisomère B.

1,3 g d'acide (3R,4R)-1-[2-(cyclohexylthio)éthyl]-4-[3-(R,S)-hydroxy-3-(6-méthoxyquinolin-4-yl)propyl]pipéridine-3-acétique est chromatographié sur une colonne de 35 cm de long et 6 cm de diamètre, conditionnée avec 700 g de silice KROMASIL® (granulométrie 10µ). L'élution est effectuée à l'aide d'un mélange de dichlorométhane-acétonitrile-méthanol-triéthylamine (56/40/4/0,5 en volumes). Le débit est de 70 cm³/mn. La détection est effectuée en ultra violet à 265 nm. Deux injections préparatives ont conduit à la séparation des 2 diastéréoisomères. Les fractions correspondant au premier, le dastéréoisomère A, sont concentrées sous pression réduite (5 kPa) à une température voisine de 40°C. Le résidu d'évaporation obtenu est séché à l'étuve, sous pression réduite (10 Pa) à une température voisine de 20°C. On obtient 0,310 g d'acide (3R,4R)-1-[2-(cyclohexylthio)éthyl]-4-[3-hydroxy-3-(6-méthoxyquinolin-4-yl)propyl]pipéridine-3-acétique, diastéréoisomère A, sous forme d'une meringue de couleur beige. (α_{D}²⁰ = -43,6+/-1,0 dans le dichlorométhane à 0,5 %). Les fractions correspondant au second, le diastéréoisomère B, sont concentrées sous pression réduite (5 kPa) à une température voisine de 40°C. Le résidu d'évaporation obtenu est séché à l'étuve, sous pression réduite (10 Pa) à une température voisine de 20°C. On obtient 0,260 g d'acide (3R,4R)-1-[2-(cyclohexylthio)éthyl]-4-[3-hydroxy-3-(6-méthoxyquinolin-4-yl)propyl]pipéridine-3-acétique, diastéréoisomère B, sous forme d'une meringue de couleur beige. (α_{D}²⁰ = +55,4 +/-1,1 dans le dichlorométhane à 0,5%)
diastéréoisomère A : Spectre de R.M.N.¹H (300 MHz, (CD₃)₂SO d6, δ en ppm) : de 1,10 à 2,20 et de 2,30 à 2,80 (2 séries de mts : 29H en totalité) ; 3,93 (s : 3H) ; 5,28 (dd large, J = 7,5 et 3 Hz : 1H) ; de 5,30 à 5,70 (mf étalé : 1H) ; de 7,35 à 7,45 (mt : 2H) ; 7,56 (d, J = 4,5 Hz : 1H) ; 7,94 (d, J = 9,5 Hz : 1H) ; 8,72 (d, J = 4,5 Hz : 1H).
diastéréoisomère B : Spectre de R.M.N.¹H (300 MHz, (CD₃)₂SO d6, δ en ppm) : de 1,10 à 2,10 et de 2,30 à 2,80 (2 séries de mts : 29H en totalité) ; 3,92 (s : 3H) ; 5,27 (dd large, J = 7,5 et 3 Hz : 1H) ; de 5,30 à 5,75 (mf étalé : 1H) ; de 7,35 à 7,45 (mt : 2H) ; 7,54 (d, J = 4,5 Hz : 1H) ; 7,94 (d, J = 9,5 Hz : 1H) ; 8,71 (d, J = 4,5 Hz : 1H).

### Acide (3R,4R)-1-[2-(cyclohexylthio)éthyl]-4-[3-(R,S)-hydrooy-3-(6-méthoxyquinolin-4-yl)propyl]pipéridine-3-acétique

A une solution de 1,9 g de (3R,4R)-1-[2-(cyclohexylthio)éthyl]-4-[3-(R,S)-hydroxy-3-(6-méthoxyquinolin-4-yl)propyl]pipéridine-3-acétate de méthyle dans 35 cm³ de dioxanne, on ajoute sous agitation à une température voisine de 20°C, 2,96 cm³ de soude aqueuse 5N. La solution est chauffée pendant 16 heures à une température voisine de 60°C. Le mélange réactionnel est évaporé sous pression réduite (5 kPa) à une température voisine de 50°C, puis le résidu obtenu est repris par 50 cm³ d'acétone, évaporé à nouveau comme ci-dessus. Après avoir repris le résidu obtenu par 50 cm³ d'éther diéthylique et concentré comme dans les conditions précédentes, le solide jaune obtenu est additionné de 20 cm³ d'eau, acidifié par la quantité suffisante d'acide citrique pour obtenir un pH voisin de 4-5. Le mélange est extrait par 50 cm³ de dichlorométhane. L'extrait organique est concentré sous pression réduite (5 kPa) à une température voisine de 50°C. Le résidu d'évaporation est repris par 2 fois 100 cm³ d'un mélange de dichlorométhane-méthanol (90/10 en volumes), l'insoluble étant filtré après chaque reprise. Les filtrats réunis sont concentrés comme ci-dessus, puis le résidu obtenu est repris par 50 cm³ d'éther diéthylique, évaporé comme dans les conditions précédentes. Les cristaux obtenus sont repris dans 50 cm³ d'éther diéthylique, filtrés, lavés par 2 fois 50 cm³ d'éther. Le produit est enfin séché sous pression réduite, d'abord sous 5 kPa, puis à l'étuve sous 10 Pa, à une température voisine de 60°C. On obtient 1,9 g d'acide (3R,4R)-1-[2-(cyclohexylthio)éthyl]-4-[3-(R,S)-hydroxy-3-(6-méthoxyquinolin-4-yl)propyl]pipéridine-3-acétique, sous forme d'un solide de couleur jaune.
Spectre de R.M.N. ¹H (300 MHz, (CD₃)₂SO d6, δ en ppm). On observe un mélange de deux diastéréoisomères :
de 1,00 à 2,95 (mts : 29H) ; 3,92 (s : 3H) ; 5,27 (mt : 1H) ; 5,54 (mf : 1H) ; de 7,30 à 7,45 (mt : 2H) ; 7,55 (mt : 1H) ; 7,95 (d, J = 9,5 Hz : 1H) ; 8,72 (d, J = 4,5 Hz : 1H) ; de 10,80 à 11,90 (mf très étalé : 1H).

### Exemple 33

### Dichlorhydrate du (3R,4R)-1-[2-(cyclohexylthio)éthyl]-4-[3-(R,S)-hydroxy-3-(6-méthoxyquinolin-4-yl)propyl]pipéridine-3-acétate de méthyle

A une solution de 3,5 g de (3R,4R)-1-[2-(cyclohexylthio)éthyl]-4-[3-oxo-3-(6-méthoxyquinolin-4-yl)propyl]pipéridine-3-acétate de méthyle dans 50 cm³ de méthanol additionnés d'1 goutte de soude 5N, refroidie à une température voisine de 15°C, on ajoute par petites fractions, en 1 heure environ, sous agitation et sous atmosphère inerte, 0,42 g de borohydrure de sodium. Le mélange est agité pendant 2 heures à cette température, puis refroidi aux environs de 10°C. On ajoute alors goutte à goutte 10 cm³ d'eau. Le mélange est concentré sous pression réduite (5 kPa) à une température voisine de 45°C. Après addition de 100 cm³ d'eau au résidu obtenu, on extrait le mélange par 2 fois 100 cm³ d'acétate d'éthyle. Les extraits réunis sont séchés sur sulfate de sodium, filtrés, concentrés comme ci-dessus. On obtient une huile que l'on purifie par chromatographie sous une pression de 50 kPa d'azote, sur une colonne de gel de silice (granulométrie 20-45µ ; diamètre 3 cm ; hauteur 35 cm), en éluant par de l'acétate d'éthyle, et en recueillant des fractions de 30 cm³. Les fractions 19 à 42 sont réunies, concentrées comme dans les conditions précédentes. On obtient 2,44 g d'un produit dont on fait le chlorhydrate de la façon suivante : On solubilise 0,5 g de produit dans 10 cm³ d'éther diéthylique, puis on verse la solution dans 5 cm³ d'éther chlorhydrique 1N. On ajoute 10 cm³ d'éther, puis on laisse agiter le mélange pendant 1 heure à une température voisine de 20°C. Le mélange est filtré, le gâteau lavé par 2 fois 10 cm³ d'éther diéthylique, séché sous pression réduite (5 kPa) à une température voisine de 20°C, puis à l'étuve sous pression réduite (10 Pa) à une température voisine de 60°C. On obtient 0,46 g de dichlorhydrate du (3R,4R)-1-[2-(cyclohexylthio)éthyl]-4-[3-(R,S)-hydroxy-3-(6-méthoxyquinolin-4-yl) propyl]pipéridine-3-acétate de méthyle, sous forme d'un solide de couleur rose pâle, fondant à 80°C en se ramollissant.
Spectre de R.M.N. ¹H (400 MHz, (CD₃)₂SO d6 à une température de 383K, δ en ppm). On observe un mélange de deux diastéréoisomères :
de 1,20 à 2,05 et de 2,30 à 2,65 (mts : 20H en totalité) ; 2,82 (mt : 1H) ; de 2,85 à 3,50 (mf étalé : 4H) ; 2,96 (mt : 2H) ; 3,19 (mt : 2H) ; 3,62 et 3,63 (2s : 3H en totalité) ; 4,00 (s : 3H) ; 5,37 (mt : 1H) ; de 7,50 à 7,65 (mt : 2H) ; 7,73 (mf : 1H) ; 8,15 (d large, J = 9 Hz : 1H) ; 8,83 (d, J = 4,5 Hz : 1H).

### (3R,4R)-1-[2-(Cyclohexylthio)éthyl]-4-[3-oxo-3-(6-méthoxyquinolin-4-yl)propyl]pipéridine-3-acétate de méthyle

A une solution de 3,9 g de (3R,4R)-4-[3-oxo-3-(6-méthoxyquinolin-4-yl)propyl] pipéridine-3-acétate de méthyle dans 50 cm³ d'acétonitrile, on ajoute sous agitation à une température voisine de 20°C, et sous atmosphère inerte, 2,06 g de 2-chloroéthylcyclohexyl sulfure, 50 cm³ d'acétonitrile, puis 1,78 g d'iodure de potassium et 7,25 g de carbonate de potassium. Le mélange est chauffé pendant 18 heures à une température voisine de 80°C. Après refroidissement à environ 20°C, le mélange est additionné de 100 cm³ d'eau et 100 cm³ d'acétate d'éthyle. La phase organique est décantée tandis que la phase aqueuse est extraite par 200 cm³ d'acétate d'éthyle. Les extraits organiques sont réunis, séchés sur sulfate de sodium, filtrés, concentrés sous pression réduite (5 kPa) à une température voisine de 45°C. On obtient une huile que l'on purifie par chromatographie sous une pression de 50 kPa d'azote, sur une colonne de gel de silice (granulométrie 20-45µ ; diamètre 3,5 cm ; hauteur 46 cm) en éluant par un mélange de dichlorométhane-méthanol (95/5 en volumes) et en recueillant des fractions de 50 cm³. Les fractions 18 à 37 sont réunies, concentrées comme ci-dessus. On obtient un produit que l'on purifie une seconde fois par chromatographie sous une pression de 50 kPa d'azote, sur une colonne de gel de silice (granulométrie 20-45µ ; diamètre 4 cm ; hauteur 40 cm) en éluant par un mélange d'acétate d'éthyle-cyclohexane (7/3 en volumes) et en recueillant des fractions de 100 cm³. Les fractions 24 à 54 sont réunies puis concentrées dans les mêmes conditions que précédemment. On obtient 3,7 g de (3R,4R)-1-[2-(cyclohexylthio)éthyl]-4-[3-oxo-3-(6-méthoxyquinolin-4-yl)propyl]pipéridine-3-acétate de méthyle, sous forme d'une huile de couleur brune.
Spectre infra rouge (CH₂Cl₂) : 2933 ; 2855 ; 1732 ; 1693 ; 1620 ; 105 ; 1244 ; 1029 et 853 cm⁻¹

Le (3R,4R)-4-[3-oxo-3-(6-méthoxyquinolin-4-yl)propyl]pipéridine-3-acétate de méthyle a été obtenu dans l'exemple 29.

### Exemple 34

### Acide (3R,4R)-4-[3-hydroxy-3-(6-méthoxyquinolin-4-yl)propyl]-1-[2-(cyclopentylthio)éthyl]pipéridine-3-acétique, diastéréoisomère A et dichlorhydrate de l'acide (3R,4R)-4-[3-hydroxy-3-(6-méthoxyquinolin-4-yl)propyl]-1-[2-(cyclopentylthio)éthyl]pipéridine-3-acétique, diastéréoisomère B.

En opérant comme décrit dans l'exemple 52, les deux diastéréoisomères de l'acide (3R,4R)-4-[3-(R,S)-hydroxy-3-(6-méthoxyquinolin-4-yl)propyl]-1-[2-cyclopentylthio) éthyl]pipéridine-3-acétique ont été séparés. On isole ainsi l'acide (3R,4R)-4-[3-hydroxy-3-(6-méthoxyquinolin-4-yl)propyl]-1-[2-(cyclopentylthio)éthyl]pipéridine-3-acétique, diastéréoisomère B, sous forme d'un solide de couleur blanche dont on fait le dichlorhydrate. (α_{D}²⁰ = -89,6 +/-1,6 dans le méthanol à 0,5 %), et l'acide (3R,4R)-4-[3-hydroxy-3-(6-méthoxyquinolin-4-yl) propyl]-1-[2-(cyclopentylthio)éthyl]pipéridine-3-acétique, diastéréoisomère A, sous forme d'une huile épaisse de couleur jaune. (α_{D}²⁰ = +57,4 +/-0,9 dans le méthanol à 0,5%).
diastéréoisomère A : Spectre de R.M.N.¹H (300 MHz, (CD₃)₂SO d6, δ en ppm) : de 1,15 à 2,10 et de 2,25 à 2,60 (mts : 24H en totalité) ; 2,70 (mt : 2H) ; 3,11 (mt : 1H) ; 3,92 (s : 3H) ; 5,26 (dd, J = 8 et 4 Hz : 1H) ; de 7,35 à 7,45 (mt : 2H) ; 7,54 (d, J = 4,5 Hz : 1H) ; 7,94 (d, J = 10 Hz : 1H) ; 8,71 (d, J = 4,5 Hz : 1H).
diastéréoisomère B : Spectre de R.M.N.¹H (300 MHz, (CD₃)₂SO d6, δ en ppm).de 1,30 à 2,10 - de 2,15 à 2,40 et de 2,65 à 3,50 (mts : 27H en totalité) ; 4,00 et 4,01 (2s : 3H en totalité) ; 5,50 (mt : 1H) ; 7,54 et 7,58 (2d, J = 2,5 Hz : 1H en totalité) ; 7,71 (d très large, J = 9 Hz : 1H) ; 7,96 (mt : 1H) ; 8,23 (d très large, J = 9 Hz ; 1H) ; 9,00 (d très large, J = 4,5 Hz : 1H) ; de 9,70 à 9,85 et de 10,15 à 10,35 (2mfs : 1H en totalité).

### Acide (3R,4R)-4-[3-(R,S)-hydroxy-3-(6-méthoxyquinolin-4-yl)propyl]-1-[2-(cyclopentylthio)éthyl]pipéridine-3-acétique

Une solution de 1,6 g de dichlorhydrate de (3R,4R)-4-[3-(R,S)-hydroxy-3-(6-méthoxyquinolin-4-yl)propyl]-1-[2-(cyclopentylthio)éthyl]pipéridine-3-acétate de méthyle dans 30 cm³ de dioxanne, additionnée de 2,6 cm³ de soude aqueuse 5N, est agitée pendant 6 heures à une température voisine de 65°C. Après concentration sous pression réduite (5 kPa) à une température voisine de 40°C du mélange réactionnel, on obtient un résidu que l'on reprend par 2 fois 30 cm³ de chloroforme et que l'on concentre comme dans les conditions précédentes. Le résidu est purifié par chromatographie à pression atmosphérique sur une colonne de gel de silice (granulométrie 20-45µ ; diamètre 3 cm ; volume de silice 100 cm³), en éluant par un mélange de chloroforme-méthanol-ammoniaque à 28 % (12/3/0,5 en volumes), et en recueillant d es fractions de 3 0 cm³. Les fractions contenant le produit attendu sont réunies et concentrées comme ci-dessus. On obtient 1,5 g d'une laque incolore que l'on reprend par 20 cm³ d'éther diéthylique, essore et lave par 2 fois 10 cm³ d'éther diéthylique. Le produit est séché sous pression partielle (5 kPa), puis à poids constant, à l'étuve sous pression réduite (10 Pa) à une température voisine de 40°C. On obtient 1,35 g d'acide (3R,4R)-4-[3-(R,S)-hydroxy-3-(6-méthoxyquinolin-4-yl)propyl]-1-[2-(cyclopentylthio)éthyl]pipéridine-3-acétique, sous forme d'un solide blanc.
Spectre de R.M.N.¹H (300 MHz, (CD₃)₂SO d6, δ en ppm). On observe un mélange de deux diastéréoisomères :
de 1,00 à 2,20 et de 2,25 à 2,80 (mts : 25H en totalité) ; 3,10 (mt : 2H) ; 3,92 (s large: 3H) ; 5,26 (mf : 1H) ; de 7,20 à 7,60 (mt : 3H) ; 7,93 (d large, J = 9 Hz : 1H) ; 8,71 (mt : 1H).

### Exemple 35

### Dichlorhydrate de (3R,4R)-4-[3-(R,S)-hydroxy-3-(6-méthoxyquinolin-4-yl)propyl]-1-[2-(cyclopentylthio)éthyl]pipéridine-3-acétate de méthyle

A un mélange de 2,5 g de (3R,4R)-4-[3-oxo-3-(6-méthoxyquinolin-4-yl)propyl]-1-[2-(cyclopentylthio)éthyl]pipéridine-3-acétate de méthyle dans 40 cm³ de méthanol additionnés d'une goutte de soude aqueuse 5N, on ajoute par petites fractions, en 30 minutes environ, à une température inférieure à 30°C, sous agitation et sous atmosphère inerte, 0,227 g de borohydrure de sodium. Après 3 heures d'agitation du mélange réactionnel à une température voisine de 20°C, on ajoute 100 cm³ d'eau, puis l'on extrait par 4 fois 50 cm³ d'acétate d'éthyle. Les extraits réunis sont lavés par 3 fois 50 cm³ d'eau, séchés sur sulfate de magnésium, filtrés, concentrés sous pression réduite (5 kPa) à une température voisine de 40°C. L'huile résiduelle obtenue est purifiée par chromatographie à pression atmosphérique, sur une colonne de gel de silice (granulométrie 20-45µ ; diamètre 3,2 cm ; volume de silice 100 cm³), en éluant par de l'acétate d'éthyle pur et en recueillant des fractions de 25 cm³. Les fractions contenant le produit attendu sont réunies, puis concentrées comme ci-dessus. On obtient 2,1 g de (3R,4R)-4-[3-(R,S)-hydroxy-3-(6-méthoxyquinolin-4-yl)propyl]-1-[2-(cyclopentylthio)éthyl]pipéridine-3-acétate de méthyle, sous forme d'une huile de couleur jaune pâle. Le chlorhydrate a été préparé de la façon suivante : une solution de 0,5 g de l'huile obtenue ci-dessus, solubilisée dans 15 cm³ de dichlorométhane, est versée s ous a gitation d ans 4 c m³ d'éther d iéthylique c hlorhydrique 1 N. Un produit précipite, puis cristallise par addition de 25 cm³ d'éther diéthylique. Le solide est filtré, lavé par 2 fois 10 cm³ d'éther, séché sous vide potassique (5 kPa), puis à poids constant, à l'étuve, sous pression réduite (10 Pa) à une température voisine de 40°C. On obtient 0,35 g de dichlorhydrate de (3R,4R)-4-[3-(R,S)-hydroxy-3-(6-méthoxyquinolin-4-yl)propyl]-1-[2-(cyclopentylthio)éthyl]pipéridine-3-acétate de méthyle, sous forme d'un solide de couleur blanc cassé.
Spectre infra rouge (KBr) : 3355 ; 2950 ; 2560 ; 2051 ; 1982 ; 1731 ; 1619 ; 1601 ; 1428 ; 1248 ; 1206 ; 1020 ; 849 et 714 cm⁻¹

### (3R,4R)-4-[3-Oxo-3-(6-méthoxyquinolin-4-yl)propyl]-1-[2-(cyclopentylthio)éthyl]pipéridine-3-acétate de méthyle

Un mélange de 4,9 g de (3R,4R)-4-[3-oxo-3-(6-méthoxyquinolin-4-yl)propyl] pipéridine-3-acétate de m éthyle, 2 ,9 g de (2-chloroéthyl)cyclopentyl sulfure, 9 g de carbonate de potassium et 2,7 g d'iodure de potassium dans 130 cm³ d'acétonitrile est agité pendant 17 heures à une température voisine de 65°C. Après refroidissement à une température voisine de 20°C, le mélange réactionnel est additionné de 150 cm³ d'eau, extrait par 3 fois 60 cm³ d'acétate d'éthyle. Les extraits organiques sont réunis, lavés par 2 fois 50 cm³, séchés sur sulfate de magnésium, filtrés, concentrés sous pression réduite (5 kPa) à une température voisine de 40°C. On obtient une huile que l'on purifie par chromatographie à pression atmosphérique sur une colonne de gel de silice (granulométrie 20-45µ ; diamètre 4 cm ; volume de silice 300 cm³), en éluant par de l'acétate d'éthyle pur et en recueillant des fractions de 70 cm³ environ. Les fractions contenant le produit attendu sont réunies, puis concentrées dans les conditions de ci-dessus. On obtient 2,6 g de (3R,4R)-4-[3-oxo-3-(6-méthoxyquinolin-4-yl)propyl]-1-[2-(cyclopentylthio)éthyl]pipéridine-3-acétate de méthyle, sous forme d'une huile de couleur jaune.
Spectre infra rouge (CCl4) : 2952 ; 2802 ; 1738 ; 1692 ; 1620 ; 1504 ; 1242 ; 1165 ; 1032 et 850 cm⁻¹

Le (3R,4R)-4-[3-oxo-3-(6-méthoxyquinolin-4-yl)propyl]pipéridine-3-acétate de méthyle a été obtenu dans l'exemple 29.

Le 2-chloroéthylcyclopentyl sulfure peut être préparé par application de la méthode décrite dans la demande de brevet FR 2 395 260.

### Exemple 36

### Dichlorhydrate de l'acide (3R,4R)-1-[2-(3,5-difluorophénylthio)éthyl]-4-[3-(R,S)-hydroxy-3-(6-méthoxyquinolin-4-yl)propyl]pipéridine-3-carboxylique

A une solution de 0,42 g de (3R,4R)-1-[2-(3,5-difluorophénylthio)éthyl]-4-[3-(R,S)-hydroxy-3-(6-méthoxyquinolin-4-yl)propyl]pipéridine-3-carboxylate de méthyle dans 5,5 cm³ de méthanol, on ajoute sous agitation et sous atmosphère inerte, à une température voisine de 20°C, 1,6 cm³ d'une solution aqueuse de soude 5N. Le mélange est chauffé pendant 16 heures à une température voisine de 60°C. La solution obtenue est évaporée sous pression réduite (5 kPa) à une température voisine de 40°C, puis au résidu obtenu on ajoute 5 cm³ d'eau distillée et 2,66 cm³ d'acide chlorhydrique aqueux 6N. Le mélange est alors chauffé au voisinage de 60°C jusqu'à l'obtention d'une solution homogène que l'on évapore ensuite dans les conditions de ci-dessus. Le résidu obtenu est trituré dans 10 cm³ d'un mélange de dichlorométhane-méthanol (90/10 en volumes), puis l'insoluble résultant est filtré, lavé par 2 fois 2,5 cm³ de ce même mélange. Le filtrat est concentré dans les mêmes conditions que précédemment. On obtient 0,465 g de dichlorhydrate de l'acide (3R,4R)-1-[2-(3,5-difluorophénylthio)éthyl]-4-[3-(R,S)-hydroxy-3-(6-méthoxyquinolin-4-yl)propyl] pipéridine-3-carboxylique, sous forme d'une meringue de couleur jaune fondant à 160°C en se décomposant.
Spectre de R.M.N.¹H (300 MHz, (CD₃)₂SO d6, δ en ppm). On observe un mélange de deux diastéréoisomères :
de 1,35 à 2,35 et de 2,60 à 4,00 (mts : 16H en t otalité) ; 4,01 et 4,02 ( 2s : 3H en totalité) ; de 5,45 à 5,65 (mt : 1H) ; 7,10 (mt : 1H) ; 7,21 (mt : 2H) ; de 7,50 à 7,70 (mt : 1H) ; 7,76 (mt : 1H) ; 8,00 (mt : 1H) ; 8,33 (mt : 1H) ; 9,04 (d, J = 5,5 Hz : 1H) ; de 11,10 à 11,55 (2mfs : 1H en totalité).

### (3R,4R)-1-[2-(3,5-Difluorophénylthio)éthyl]-4-[3-(R,S)-hydroxy-3-(6-méthoxyquinolin-4-yl)propyl]pipéridine-3-carboxylate de méthyle

A une solution de 0,717 g de (3R,4R)-4-[3-(R,S)-hydroxy-3-(6-méthoxyquinolin-4-yl)propyl]pipéridine-3-carboxylate de méthyle dans 15 cm³ d'acétonitrile et 1 cm³ de méthanol, on ajoute sous agitation et sous atmosphère inerte, à une température voisine de 20°C, 0,332 g de carbonate de potassium puis 0,4 g d'iodure de potassium et enfin 0,675 g de 1-[(2-bromoéthyl)thio]-3,5-difluorobenzène préalablement solubilisé dans 5 cm³ d'acétonitrile. Le mélange est chauffé pendant 3 heures à une température voisine de 80°C. Après refroidissement à une température voisine de 20°C, le mélange réactionnel est filtré, le gâteau lavé par 2 fois 5 cm³ d'acétonitrile. Le filtrat est évaporé sous pression réduite (5 kPa) à une température voisine de 40°C. Le résidu obtenu est purifié par chromatographie sous une pression de 50 kPa d'azote, sur une colonne de gel de silice (granulométrie 40-63µ ; diamètre 3,5 cm ; hauteur 35 cm), en éluant par un mélange de dichlorométhane-méthanol (95/5 en volumes) et en recueillant des fractions de 35 cm³. Les fractions 18 à 21 sont réunies puis évaporées comme ci-dessus. On obtient 0,47 g de (3R,4R)-1-[2-(3,5-difluorophénylthio)éthyl]-4-[3-(R,S)-hydroxy-3-(6-méthoxyquinolin-4-yl)propyl] pipéridine-3-carboxylate de méthyle, sous forme d'une huile visqueuse de couleur jaune orangé.
Spectre infra rouge (CH₂Cl₂) : 3597 ; 2951 ; 2814 ; 1733 ; 1611 ; 1586 ; 1242 ; 1119 ; 985 ; 877 ; 840 et 667 cm-1

### 1-[(2-Bromoéthyl)thio]-3,5-difluorobenzène

A un mélange de 7,5 g de 3,5-difluorothiophénol dans 9,01 cm³ de 1,2-dibromoéthane maintenu à une température voisine de 23°C, on ajoute sous agitation et sous atmosphère inerte, une solution de 2,59 g de soude en pastille dans 27 cm³ d'eau distillée, puis 0,27 cm³ d'aliquat 336 (tricaprylylméthylammonium chlorure). Après 15 minutes d'agitation à une température voisine de 20°C, on ajoute au mélange 50 cm³ de dichlorométhane, puis au bout de quelques minutes la phase organique est décantée, lavée par 25 cm³ d'eau, 25 cm³ d'une solution saturée de chlorure de sodium à 10 %, séchée sur sulfate de magnésium, filtrée, évaporée sous pression réduite (5 kPa) à une température voisine de 40°C. Le résidu d'évaporation est purifié par chromatographie sous une pression d'azote de 50 kPa, sur une colonne de gel de silice (granulométrie 40-63µ ; diamètre 5 cm ; hauteur 30 cm) en éluant par du cyclohexane et en recueillant d'abord une fraction de 500 cm³, puis des fractions de 50 cm³. Les fractions 9 à 26 sont réunies puis concentrées sous pression réduite (5 kPa) à une température voisine de 40°C. On obtient 5,9 g de 1-[(2-bromoéthyl) thio]-3,5-difluorobenzène, sous forme d'un liquide incolore.
Spectre infra rouge (CCl₄) : 3094 ; 1607 ; 1587 ; 1429 ; 1192 ; 1122 ; 988 ; 876 ; 841 et 667 cm⁻¹

Le 3,5-difluorothiophénol peut être préparé selon DAE-KEE KIM; JONGSIK GAM et col. J. Med. Chem., 1997 p. 2371

### Exemple 37

### Acide (3R,4R)-4-[3-(R,S)-fluoro-3-(6-méthoxyquinolin-4-yl)propyl]-1-[2-(2-thiénylthio)éthyl]pipéridine-3-acétique

Un mélange de 1,28 g de (3R,4R)-4-[3-(R,S)-fluoro-3-(6-méthoxyquinolin-4-yl) propyl]-1-[2-(2-thiénylthio)éthyl]pipéridine-3-acétate de méthyle dans 25 cm³ de dioxanne, additionné de 2 cm³ de soude aqueuse 5N, est agité pendant 18 heures à une température voisine de 60°C. Après évaporation sous pression réduite (5 kPa) à une température voisine de 50°C, le résidu est repris par 50 cm³ d'acétone. L'acétone est évaporée sous pression réduite (5 kPa) à une température voisine de 50°C. Le résidu est repris par 75 cm³ d'eau et 100 cm³ de dichlorométhane. La phase organique est séparée. La phase aqueuse est extraite 2 fois par 100 cm³ de dichlorométhane, puis les extraits organiques s ont réunis, séchés sur sulfate de sodium, filtrés, évaporés sous pression réduite (5 kPa) à une température voisine de 40°C. Le solide obtenu est repris par 50 cm³ d'éther de diéthyle et la suspension obtenue est agitée durant 48 heures à une température voisine de 20°C. Le solide est filtré, rincé par 75 cm³ au total d'éther de diéthyle puis séché sous pression réduite (5 kPa) à une température voisine de 40°C. On obtient 1 g de solide que l'on purifie par chromatographie sous une pression de 100 kPa d'azote sur une colonne de gel de silice (granulométrie 60-200µ ; diamètre 2 cm ; hauteur silice 15 cm), en éluant par un mélange chloroforme-méthanol-ammoniaque (24/6/1 en volumes), et en recueillant des fractions de 20 cm³. Les fractions contenant le produit sont réunies et évaporées sous pression réduite (5 kPa) à une température voisine de 40°C. On obtient 0,97 g d'acide (3R,4R)-4-[3-(R,S)-fluoro-3-(6-méthoxyquinolin-4-yl)propyl]-1-[2-(2-thiénylthio)éthyl] pipéridine-3-acétique, sous forme d'une meringue blanche qui est un mélange de deux diastéréoisomères.
Spectre de R.M.N.¹H (400 MHz, (CD₃)₂SO d6 avec ajout de quelques gouttes de CD₃COOD d4, δ en ppm). On observe un mélange de deux diastéréoisomères dans les proportions 50/50 : de 1,15 à 1,65 (mt : 5H) ; 1,90 à 2,60 (mt : 7H) ; 2,61 (mt : 2H) ; 2,78 (mt : 2H) ; 2,93 (t large, J = 7 Hz : 2H) ; 3,92 et 3,93 (2 s : 3H en totalité) ; 6,36 (mt, J _{HF} = 48 Hz : 1H) ; 7,04 (dd, J = 5,5 et 3,5 Hz : 1H) ; 7,18 (dd, J = 3,5 et 1 Hz : 1H) ; 7,29 (mt : 1H) ; 7,44 (mt : 1H) ; 7,49 (d large, J = 4,5 Hz : 1H) ; 7,59 (dd, J = 5,5 et 1 Hz : 1H) ; 7,98 (d large, J = 9 Hz : 1H) ; 8,77 (d, J = 4,5 Hz : 1H).

### (3R,4R)-4-[3-(R,S)-Fluoro-3-(6-méthoxyquinolin-4-yl)propyl]-1-[2-(2-thiénylthio)éthyl]pipéridine-3-acétate de méthyle

A une solution agitée de 1,96 g de (3R,4R)-4-[3-(R,S)-hydroxy-3-(6-méthoxyquinolin-4-yl)propyl]-1-[2-(2-thiénylthio)éthyl]pipéridine-3-acétate de méthyle dans 15 cm³ de dichlorométhane, on ajoute sous atmosphère inerte et à une température voisine de 20°C, 0,62 cm³ de diéthylaminosulfure trifluorure en approximativement 15 minutes L'agitation est maintenue durant 18 heures. Après addition de 30 cm³ d'une solution aqueuse saturée d'hydrogénocarbonate de sodium, la phase organique est décantée. La phase aqueuse est extraite 2 fois par 50 cm³ de dichlorométhane, puis les extraits organiques sont réunis, lavés 2 fois par 50 cm³ d'eau distillée, séchés sur sulfate de sodium, filtrés, évaporés sous pression réduite (5 kPa) à une température voisine de 40°C. On obtient 1,95 g d'une huile jaune que l'on purifie par chromatographie sous une pression de 100 kPa d'azote sur une colonne de gel de silice (granulométrie 20-45µ ; diamètre 3 cm ; hauteur silice 40 cm), en éluant par un mélange cyclohexane-acétate d'éthyle (3/2 en volumes), et en recueillant des fractions de 80 cm³. On réunit les fractions 32 à 64 que l'on évapore sous pression réduite (5 kPa) à une température voisine de 40°C. On obtient 1,28 g de (3R,4R)-4-[3-(R,S)-fluoro-3-(6-méthoxy-quinolin-4-yl)propyl]-1-[2-(2-thiénylthio)éthyl]pipéridine-3-acétate de méthyle sous forme d'une huile incolore.
Spectre de R.M.N.¹H (300 MHz, (CD₃)₂SO d6, δ en ppm). On observe un mélange de deux diastéréoisomères dans les proportions 60/40 : de 1,10 à 1,60 (mt : 5H) ; 1,85 à 2,15 (mt : 6H) ; de 2,20 à 2,80 (mt : 5H) ; 2,88 (t large, J = 7 Hz : 2H) ; 3,53 et 3,55 (2 s : 3H en totalité) ; 3,93 et 3,94 (2 s : 3H en totalité) ; 6,40 (mt, J _{HF} = 48 Hz : 1H) ; 7,03 (dd, J = 5,5 et 3,5 Hz : 1H) ; 7,15 (d large, J = 3,5 Hz : 1H) ; 7,30 (d large, J = 1,5 Hz : 1H) ; 7,46 (dd, J = 9 et 1,5 Hz : 1H) ; 7,49 (d large, J = 4,5 Hz : 1H) ; 7,60 (dd, J = 5,5 et 1 Hz : 1H) ; 7,99 (d large, J = 9 Hz : 1H) ; 8,79 (d, J = 4,5 Hz : 1H).

Le (3R,4R)-4-[3-(R,S)-hydroxy-3-(6-méthoxyquinolin-4-yl)propyl]-1-[2-(2-thiénylthio)éthyl]pipéridine-3-acétate de méthyle est préparé comme décrit à l'exemple 29.

### Exemple 38

### Acide (3R,4R)-4-[3-fluoro-3-(6-méthoxyquinolin-4-yl)propyl]-1-[2-(2-thiénylthio)éthyl] pipéridine-3-acétique, diastéréoisomère A

0,5 g de (3R,4R)-4-[3-fluoro-3-(6-méthoxyquinolin-4-yl) propyl]-1-[2-(2-thiénylthio)éthyl] pipéridine-3-acétate de méthyle, diastéréoisomère A (α_{D}²⁰= -48 ±3 dans le méthanol) dans 10 cm³ de dioxanne, additionné de 0.77 cm³ de soude aqueuse 5N, est agité pendant 18 heures à une température voisine de 60°C. Après évaporation sous pression réduite (5 kPa) à une température voisine de 50°C, on obtient 0,6 g de solide que l'on purifie par chromatographie sous une pression de 100 kPa d'azote sur une colonne de gel de silice (granulométrie 20-45µ ; diamètre 2 cm ; hauteur silice 30 cm), en éluant par un mélange chloroforme-méthanol-ammoniaque (24/6/1 en volumes), et en recueillant des fractions de 20 cm³. Les fractions contenant le produit sont réunies et évaporées sous pression réduite (5 kPa) à une température voisine de 40°C. On obtient 0,27 g d'acide (3R,4R)-4-[3-fluoro-3-(6-méthoxyquinolin-4-yl) propyl]-1-[2-(2-thiénylthio)éthyl] pipéridine-3-acétique, diastéréoisomère A, sous forme d'une meringue blanche. (α_{D}²⁰= -66_±1.2 dans le méthanol).
Spectre de R.M.N. ¹H (300 MHz, (CD₃)₂SO d6, δ en ppm) : de 1,20 à 1,55 (mt : 5H) ; de 1,85 à 2,20 et de 2,35 à 2,60 (2 séries de mts : 9H en totalité) ; 2,68 (mt : 2H) ; 2,89 (t large, J = 7 Hz : 2H) ; 3,94 (s : 3H) ; 6,38 (mt, J_{HF} = 48 Hz : 1H) ; 7,05 (dd, J = 5,5 et 3,5 Hz : 1H) ; 7,17 (dd, J = 3,5 et 1 Hz : 1H) ; 7,32 (d, J = 2,5 Hz : 1H) ; 7,45 (dd, J = 9 et 2,5 Hz : 1H) ; 7,51 (d, J = 4,5 Hz : 1H) ; 7,60 (dd, J = 5,5 et 1 Hz : 1H) ; 7,99 (d, J = 9 Hz : 1H) ; 8,78 (d, J = 4,5 Hz : 1H).

### (3R,4R)-4-[3-fluoro-3-(6-méthoxyquinolin-4-yl)propyl]-1-[2-(2-thiénylthio)éthyl] pipéridine-3-acétate de méthyle, diastéréoisomère A

### (3R,4R)-4-[3-fluoro-3-(6-méthoxyquinolin-4-yl)propyl]-1-[2-(2-thiénylthio)éthyl] pipéridine-3-acétate de méthyle, diastéréoisomère B

3,2 g du (3R,4R)-4-[3-(R,S)-fluoro-3-(6-méthoxyquinolin-4-yl)propyl]-1-[2-(2-thiénylthio) éthyl]pipéridine-3-acétate de méthyle est chromatographié sur une colonne de 30 cm de long et 8 cm de diamètre, conditionnée avec 1200 g de silice Chiralcel OD (granulométrie 20µ). L'élution est effectuée à l'aide d'un mélange de heptane-isopropanol (90/10 en volumes) Le débit est de 140 cm³/minute. La détection est effectuée en ultra violet à 265nm. Après plusieurs injections préparatives, on recueille les fractions correspondant aux diastéréoisomères A et B. Les fractions contenant la diastéréoisomères A sont concentrées sous pression réduite (5 kPa) à une température voisine de 40°C. On obtient 1,6 g de (3R,4R)-4-[3-fluoro-3-(6-méthoxyquinolin-4-yl)propyl]-1-[2-(2-thiénylthio)éthyl]pipéridine-3-acétate de méthyle, diastéréoisomère A sous forme d'une huile épaisse. (α_{D}²⁰ = -48 +/-3 dans le méthanol à 0,1%). L es fractions contenant la diastéréoisomères B sont concentrées dans les mêmes conditions que ci-dessus. On obtient 1,17 g (3R,4R)-4-[3-fluoro-3-(6-méthoxyquinolin-4-yl)propyl]-1-[2-(2-thiénylthio)éthyl]pipéridine-3-acétate de méthyle, diastéréoisomère B, sous forme d'une huile épaisse. (α_{D}²⁰ =+82 +/-3 dans le méthanol à 0,1 %).
diastéréoisomère A :
Spectre infra rouge (KBr) 2936 ; 2861 ; 2805 ; 2768 ; 1731 ; 1623 ; 1594 ; 1508 ; 1475 ; 1435 ; 1359 ; 1229 ; 1217 ; 1167 ; 1108 ; 1084 ; 1030 ; 855 ; 847 et 830 cm⁻¹.
diastéréoisomère B :
Spectre infra rouge (KBr) 2932 ; 2861 ; 2805 ; 2768 ; 1731 ; 1623 ; 1594 ; 1509 ; 1475 ; 1435 ; 1359 ; 1229 ; 1217; 1168 ; 1108 ; 1083 ; 1030; 855 ; 847 et 830 cm⁻¹.

Le (3R,4R)-4-[3(R,S)-fluoro-3-(6-méthoxyquinolin-4-yl)propyl]-1-[2-(2-thiénylthio) éthyl] pipéridine-3-acétate de méthyle est préparé comme décrit précédemment à l'exemple 37.

### Exemple 39

### Acide (3R,4R)-4-[3-fluoro-3-(6-méthoxyquinolin-4-yl)propyl]-1-[2-(2-thiénylthio)éthyl] pipéridine-3-acétique, diastéréoisomère B

En opérant comme décrit à l'exemple 60, mais à partir de 0,5 de (3R,4R)-4-[3-fluoro-3-(6-méthoxyquinolin-4-yl) propyl]-1-[2-(2-thiénylthio)éthyl]pipéridine-3-acétate de méthyle, diastéréoisomère B (α_{D}²⁰= +82) on obtient 0,29 g d'acide (3R,4R)-4-[3-fluoro-3-(6-méthoxyquinolin-4-yl)propyl]-1-[2-(2-thiénylthio)éthyl]pipéridine-3-acétique, diastéréoisomère B, sous forme d'une meringue blanche (α_{D}²⁰= +66.3±1.1)
Spectre de R.M.N. ¹H (300 MHz, (CD₃)₂SO d6, δ en ppm) : de 1,20 à 1,60 (mt : 5H) ; de 1,90 à 2,15 et de 2,30 à 2,60 (2 séries de mts : 9H en totalité) ; 2,68 (mt : 2H) ; 2,89 (t large, J = 7 Hz : 2H) ; 3,95 (s : 3H) ; 6,38 (mt, J_{HF} = 48 Hz : 1H) ; 7,05 (dd, J = 5,5 et 3,5 Hz : 1H) ; 7,18 (dd, J = 3,5 et 1 Hz : 1H) ; 7,32 (d, J = 2,5 Hz : 1H) ; 7,46 (dd, J = 9 et 2,5 Hz : 1H) ; 7,51 (d, J = 4,5 Hz : 1H) ; 7,61 (dd, J = 5,5 et 1 Hz : 1H) ; 7,99 (d, J = 9 Hz : 1H) ; 8,78 (d, J = 4,5 Hz : 1H).

Le (3R,4R)-4-[3-fluoro-3-(6-méthoxyquinolin-4-yl)propyl]-1-[2-(2-thiénylthio) éthyl] pipéridine-3-acétate de méthyle, diastéréoisomère B, est préparé comme décrit précédemment à l'exemple 38.

La présente invention concerne également les compositions pharmaceutiques contenant au moins un dérivé de quinolyl propyl pipéridine selon l'invention, le cas échéant sous forme de sel, à l'état pur ou sous forme d'une association avec un ou plusieurs diluants ou adjuvants compatibles et pharmaceutiquement acceptables.

Les compositions selon l'invention peuvent être utilisées par voie orale, parentérale, topique, rectale ou en aérosols.

Comme compositions solides pour administration orale peuvent être utilisés des comprimés, des pilules, des gélules, des poudres ou des granulés. Dans ces compositions, le produit actif selon l'invention est mélangé à un ou plusieurs diluants ou adjuvants inertes, tels que saccharose, lactose ou amidon. Ces compositions peuvent comprendre des substances autres que les diluants, par exemple un lubrifiant tel que le stéarate de magnésium ou un enrobage destiné à une libération contrôlée.

Comme compositions liquides pour administration orale, on peut utiliser des solutions pharmaceutiquement acceptables, des suspensions, des émulsions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration parentérale, peuvent être des solutions stériles ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle. Ces compositions peuvent également contenir des adjuvants, en particulier des agents mouillants, isotonisants, émulsifiants, dispersants et stabilisants.

La stérilisation peut se faire de plusieurs façons, par exemple à l'aide d'un filtre bactériologique, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration topique peuvent être par exemple des crèmes, des pommades, des lotions ou des aérosols.

Les compositions par administration rectale sont les suppositoires ou les capsules rectales, qui contiennent outre le principe actif, des excipients tels que le beurre de cacao, des glycérides semi-synthétiques ou des polyéthylèneglycols.

Les compositions peuvent également être des aérosols. Pour l'usage sous forme d'aérosols liquides, les compositions peuvent être des solutions stériles stables ou des compositions solides dissoutes au moment de l'emploi dans de l'eau stérile apyrogène, dans du sérum ou tout autre véhicule pharmaceutiquement acceptable. Pour l'usage sous forme d'aérosols secs destinés à être directement inhalés, le principe actif est finement divisé et associé à un diluant ou véhicule solide hydrosoluble d'une granulométrie de 30 à 80 µm, par exemple le dextrane, le mannitol ou le lactose.

En thérapeutique humaine, les nouveaux dérivés de quinolyl propyl pipéridine selon l'invention sont particulièrement utiles dans le traitement des infections d'origine bactérienne. Les doses dépendent de l'effet recherché et de la durée du traitement. Le médecin déterminera la posologie qu'il estime la plus appropriée en fonction du traitement, en fonction de l'âge, du poids, du degré de l'infection et des autres facteurs propres au sujet à traiter. Généralement, les doses sont comprises entre 750 mg et 3 g de produit actif en 2 ou 3 prises par jour par voie orale ou entre 400 mg et 1,2 g par voie intraveineuse pour un adulte.

L'exemple suivant illustre une composition selon l'invention.

### EXEMPLE 1

On prépare selon la technique habituelle une composition liquide destinée à l'usage parentéral comprenant :
- acide (3R,4R)-4-[3-(R,S)-hydroxy-3-(6-méthoxyquinolin-4-yl)propyl]-1-[2-(2-thiénylthio)éthyl] pipéridine-3-carboxylique 125 mg
- glucose qsp 2,5%
- hydroxyde de sodium qsp pH = 4-4,5
- eau ppi qsp 20 ml

### EXEMPLE 2

On prépare selon la technique habituelle une composition liquide destinée à l'usage parentéral comprenant :
- acide (3R,4R)-4-[3-(R,S)-hydroxy-3-(6-méthoxyquinolin-4-yl)propyl]-1-[2-(2-thiénylthio)éthyl]pipéridine-3-acétique 125 mg
- glucose qsp 2,5 %
- hydroxyde de sodium qsp pH = 4-4,5
- eau ppi qsp 20 ml

## Revendications

1. Un dérivé de quinolyl propyl pipéridine de formule générale : 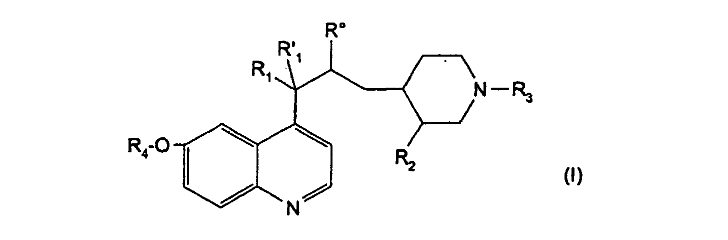 dans laquelle :
R₁ est un atome d'hydrogène ou d'halogène, ou un radical hydroxy,
R'₁ est un atome d'hydrogène, ou peut représenter un atome d'halogène lorsque R₁ est également un atome d'halogène, et
R° est un atome d'hydrogène, ou bien
R₁ et R° forment ensemble une liaison et
R'₁ est un atome d'hydrogène,
R₂ représente un radical carboxy, carboxyméthyle, carboxy-2 éthyle, hydroxyméthyle, alcoyloxycarbonyle, alcoyloxycarbonylméthyle ou alcoyloxycarbonyl-2-éthyle (dont les parties alcoyle contiennent de 1 à 6 C),
R₃ représente un radical alcoylène (contenant 1 à 6 atomes de carbone) substitué par un atome de soufre portant un substituant phényle pouvant lui-même porter 1 à 4 substituants [choisis parmi halogène, hydroxy, alcoyle (C₁₋₄), alcoyloxy (C₁₋₄), trifluorométhyle, trifluorométhoxy, carboxy, alcoyl (C₁₋₄) oxycarbonyle, cyano et amino], cycloalcoyle de 3 à 7 chaînons, ou hétérocyclyle aromatique de 5 à 6 chaînons, comprenant 1 à 4 hétéroatomes choisis parmi l'azote, l'oxygène et le soufre et étant lui-même éventuellement substitué [par halogène, hydroxy, alcoyle (C₁₋₄), alcoyloxy (C₁₋₄), trifluorométhyle, trifluorométhoxy, oxo, carboxy, alcoyl (C1-4) oxycarbonyle, cyano ou amino],
et R₄ représente un radical alcoyle (contenant 1 à 6 atomes de carbone), alcényl-CH₂- ou alcynyl-CH₂- dont les parties alcényle ou alcynyle contiennent 2 à 6 atomes de carbone,
étant entendu que les radicaux alcoyle et alcoylène sont en en chaîne droite ou ramifiée,
sous ses formes diastéréoisomères ou leurs mélanges, ainsi que ses sels.

2. Un dérivé de quinolyl propyl pipéridine selon la revendication 1, **caractérisé en ce que**
R₁ est un atome d'hydrogène ou d'halogène, ou un radical hydroxy,
R'₁ est un atome d'hydrogène, et
R° est un atome d'hydrogène, ou bien
R₁ et R° forment ensemble une liaison et
R'₁ est un atome d'hydrogène,
R₂ représente un radical carboxy ou carboxyméthyle, et
R₃ représente un radical alcoylène (1 à 6 atomes de carbone) substitué par un atome de soufre portant un substituant phényle pouvant lui-même porter 1 à 4 atomes d'halogène, cycloalcoyle dont la partie cyclique contient 3 à 7 chaînons, ou hétérocyclyle aromatique de 5 à 6 chaînons comprenant 1 à 4 hétéroatomes choisis parmi l'azote, l'oxygène et le soufre et éventuellement lui-même substitué par halogène ou bien
lorsque R₂ représente un radical hydroxyméthyle, alcoyloxycarbonyle ou alcoyloxycarbonylméthyle (dont les portions alcoyle contiennent 1 à 6 atomes de carbone) alors
R₃ représente un radical alcoylène (1 à 6 atomes de carbone) substitué par un atome de soufre portant un substituant hétérocyclyle aromatique de 5 à 6 chaînons comprenant 1 à 4 hétéroatomes choisis parmi l'azote, l'oxygène ou le soufre et éventuellement lui-même substitué par halogène
et R₄ représente un radical alcoyle (contenant 1 à 6 atomes de carbone),
les radicaux et portions alcoyle étant en en chaîne droite ou ramifiée,
sous ses formes diastéréoisomères ou leurs mélanges, ainsi que ses sels.

3. Un dérivé de quinolyl propyl pipéridine selon l'une des revendications 1 ou 2, **caractérisé en ce qu'**il s'agit de l'acide (3R,4R)-4-[3-hydroxy-3-(6-méthoxyquinolin-4-yl)propyl]-1-[2-(2-thiénylthio)éthyl]pipéridine-3-carboxylique, sous ses formes diastéréoisomères ou leurs mélanges, ainsi que ses sels.

4. Un dérivé de quinolyl propyl pipéridine selon l'une des revendications 1 ou 2, **caractérisé en ce qu'**il s'agit de l'acide (3R,4R)-4-[3-hydroxy-3-(6-méthoxyquinolin-4-yl)propyl]-1-[2-(2-thiénylthio)éthyl]pipéridine-3-acétique, sous ses formes diastéréoisomères ou leurs mélanges, ainsi que ses sels.

5. Un dérivé de quinolyl propyl pipéridine selon l'une des revendications 1 ou 2, **caractérisé en ce qu'**il s'agit de l'acide (3R,4R)-4-[3-(6-méthoxyquinolin-4-yl)propyl]-1-[2-(2-thiénylthio)éthyl] pipéridine-3-acétique, ainsi que ses sels.

6. Un dérivé de quinolyl propyl pipéridine selon l'une des revendications 1 ou 2, **caractérisé en ce qu'**il s'agit de l'acide (3R,4R)-4-[3-fluoro-3-(6-méthoxyquinolin-4-yl)propyl]-1-[2-(2-thiénylthio)éthyl]pipéridine-3-acétique, sous ses formes diastéréoisomères ou leurs mélanges, ainsi que ses sels.

7. Un dérivé de quinolyl propyl pipéridine selon l'une des revendications 1 ou 2, **caractérisé en ce qu'**il s'agit de l'acide (3R,4R)-1-[2-(3-fluorophénylthio)éthyl]-4-[3-hydroxy-3-(6-méthoxyquinolin-4-yl)propyl]pipéridine-3-acétique, sous ses formes diastéréoisomères ou leurs mélanges, ainsi que ses sels.

8. Un procédé de préparation de dérivé de quinolyl propyl pipéridine selon la revendication 1, **caractérisé en ce que** l'on condense la chaîne R₃ définie dans la revendication 1, sur le dérivé de quinolyl propyl pipéridine de formule générale : 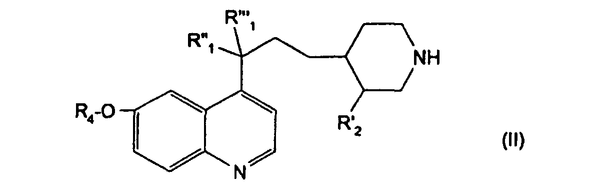 dans laquelle R₄ est défini comme dans la revendication 1, R"₁ et R"'₁ représentent des atomes d'hydrogène ou forment ensemble un radical oxo et R'₂ représente un radical carboxy, carboxyméthyle ou carboxy-2-éthyle protégés, ou un radical alcoyloxycarbonyle, alcoyloxycarbonylméthyle ou alcoyloxycarbonyl-2-éthyle, pour obtenir un dérivé de quinolyl propyl pipéridine de formule générale : 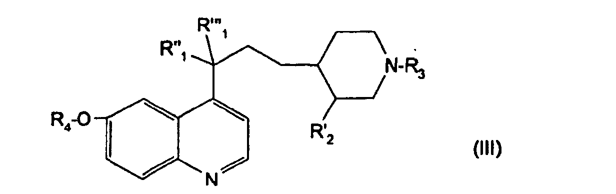 pour lequel R"₁, R"'₁, R'₂ et R₄ sont définis comme ci-dessus et R₃ est défini comme dans la revendication 1,
puis élimine le cas échéant le radical protecteur d'acide,
ou bien, le cas échéant réduit le radical oxo représenté par R"₁ et R"'₁ en un alcool pour lequel R₁ représente hydroxy puis éventuellement effectue l'halogénation si l'on veut obtenir un dérivé de quinolyl propyl pipéridine pour lequel R₁ est un atome d'halogène, et éventuellement effectue la déhydrohalogénation du dérivé halogéné correspondant, pour obtenir un dérivé de quinolyl propyl pipéridine pour lequel R₁ et R° forment ensemble une liaison, ou bien met en oeuvre la dihalogénation du produit de formule générale (III) pour lequel R"₁ et R"'₁ forment ensemble un radical oxo pour obtenir un dérivé de quinolyl propyl pipéridine pour lequel R₁ et R'₁ sont des atomes d'halogène,
et/ou le cas échéant réduit l'acide protégé sous forme d'un radical R'₂ en position -3 de la pipéridine, en un radical hydroxyméthyle et éventuellement transforme en un radical carboxyméthyle ou carboxy-2 éthyle selon les méthodes habituelles,
puis éventuellement élimine le radical protecteur d'acide et/ou sépare, le cas échéant, les diastéréoisomères et éventuellement transforme le produit obtenu en un sel.

9. Un procédé selon la revendication 8, **caractérisé en ce que** la condensation de la chaîne R₃ sur la pipéridine s'effectue par action d'un dérivé de formule générale :
R₃-X
dans laquelle R₃ est défini comme dans la revendication 1 et X représente un atome d'halogène, un radical méthylsulfonyle, un radical trifluorométhylsulfonyle ou p.toluènesulfonyle.

10. Composition pharmaceutique **caractérisée en ce qu'**elle contient au moins un dérivé selon la revendication 1, à l'état pur ou en association avec un ou plusieurs diluants ou adjuvants compatibles et pharmaceutiquement acceptables.

## Patentansprüche

1. Chinolyl-propyl-piperidin-derivat der allgemeinen Formel: 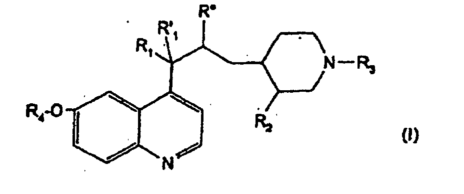 worin:
R₁ ein Wasserstoff- oder Halogenatom oder ein Hydroxyrest ist,
R'₁ ein Wasserstoffatom ist, oder ein Halogenatom bedeuten kann, wenn R₁ ebenfalls ein Halogenatom ist und
R⁰ ein Wasserstoffatom ist, oder
R₁ und R⁰ gemeinsam eine Bindung bilden und
R'₁ ein Wasserstoffatom ist,
R₂ einen Carboxy-, Carboxymethyl-, 2-Carboxyethyl-, Hydroxymethyl-, Alkyloxycarbonyl-, Alkyloxycarbonylmethyl- oder 2-Alkyloxycarbonylethylrest bedeutet (deren Alkylteile 1 bis 6 C enthalten),
R₃ einen Alkylenrest (mit 1 bis 6 Kohlenstoffatomen) bedeutet, der substituiert ist durch ein Schwefelatom, welches einen Substituenten Phenyl, der seinerseits 1 bis 4 Substituenten aufweisen kann [ausgewählt unter Halogen, Hydroxy, C₁₋₄-Alkyl, C₁₋₄-Alkoxy, Trifluormethyl, Trifluormethoxy, Carboxy, C₁₋₄-Alkyloxycarbonyl, Cyano und Amino], Cycloalkyl mit 3 bis 7 Gliedern oder einen aromatischen heterocyclischen Substituenten mit 5 bis 6 Gliedern trägt, der 1 bis 4 Heteroatome, ausgewählt unter Stickstoff, Sauerstoff und Schwefel, umfaßt, und seinerseits gegebenenfalls substituiert ist [durch Halogen, Hydroxy, C₁₋₄-Alkyl, C₁₋₄-Alkoxy, Trifluormethyl, Trifluormethoxy, Oxo, Carboxy, C₁₋₄-Alkyloxycarbonyl, Cyano oder Amino] und
R₄ einen Alkylrest (mit 1 bis 6 Kohlenstoffatomen), Alkenyl-CH₂- oder Alkinyl-CH₂-Rest, deren Alkenyl- oder Alkinylteile 2 bis 6 Kohlenstoffatome aufweisen, bedeutet, mit der Maßgabe, dass die Alkyl- und Alkylenreste in gerader oder verzweigter Kette vorliegen,
in seinen diastereomeren Formen oder deren Gemische, sowie seine Salze.

2. Chinolylpropylpiperidinderivat gemäß Anspruch 1, **dadurch gekennzeichnet, dass**
R₁ ein Wasserstoff- oder Halogenatom oder ein Hydroxyrest ist,
R'₁ ein Wasserstoffatom ist und
R⁰ ein Wasserstoffatom ist, oder
R₁ und R⁰ gemeinsam eine Bindung bilden und
R'₁ ein Wasserstoffatom ist,
R₂ einen Carboxy- oder Carboxymethylrest bedeutet, und
R₃ einen Alkylenrest (1 bis 6 Kohlenstoffatome) bedeutet, der substituiert ist durch ein Schwefelatom, welches einen Substituenten Phenyl, der seinerseits 1 bis 4 Halogenatome aufweisen kann, Cycloalkyl, dessen cyclischer Teil 3 bis 7 Glieder enthält oder einen aromatischen heterocyclischen Substituenten mit 5 bis 6 Gliedern trägt, der 1 bis 4 Heteroatome, ausgewählt unter Stickstoff, Sauerstoff und Schwefel, umfaßt, und seinerseits gegebenenfalls durch Halogen substituiert ist, oder
wenn R₂ einen Hydroxymethyl-, Alkoxycarbonyl- oder Alkoxycarbonylmethylrest (deren Alkylteile 1 bis 6 Kohlenstoffatome enthalten) bedeutet, dann
R₃ einen Alkylenrest (1 bis 6 Kohlenstoffatome) bedeutet, der substituiert ist durch ein Schwefelatom, das einen aromatischen heterocyclischen Substituenten mit 5 bis 6 Gliedern trägt, der 1 bis 4 Heteroatome, ausgewählt unter Stickstoff, Sauerstoff oder Schwefel, umfasst, und gegebenenfalls seinerseits durch Halogen substituiert ist,
und R₄ einen Alkylrest (mit 1 bis 6 Kohlenstoffatomen) bedeutet, wobei die Alkylreste und -teile in gerader oder verzweigter Kette vorliegen, in seinen diastereomeren Formen oder deren Gemische, sowie seine Salze.

3. Chinolylpropylpiperidinderivat gemäß einem der Ansprüche 1 oder 2, **dadurch gekennzeichnet, dass** es sich um die (3R,4R)-4-[3-Hydroxy-3-(6-methoxychinolin-4-yl)-propyl]-1-[2-(2-thienylthio)-ethyl]-piperidin-3-carbonsäure in ihren diastereomeren Formen oder deren Gemische, sowie ihre Salze, handelt.

4. Chinolylpropylpiperidinderivat gemäß einem der Ansprüche 1 oder 2, **dadurch gekennzeichnet, dass** es sich um die (3R,4R)-4-[3-Hydroxy-3-(6-methoxychinolin-4-yl)-propyl]-1-[2-(2-thienylthio)-ethyl]-piperidin-3-essigsäure in ihren diastereomeren Formen oder ihre Gemische, sowie ihre Salze, handelt.

5. Chinolylpropylpiperidinderivat gemäß einem der Ansprüche 1 oder 2, **dadurch gekennzeichnet, dass** es sich um die (3R,4R)-4-[3-(6-Methoxychinolin-4-yl)-propyl]-1-[2-(2-thienylthio)-ethyl]-piperidin-3-essigsäure, sowie ihre Salze, handelt.

6. Chinolylpropylpiperidinderivat gemäß einem der Ansprüche 1 oder 2, **dadurch gekennzeichnet, dass** es sich um die (3R,4R)-4-[3-Fluor-3-(6-methoxychinolin-4-yl)-propyl]-1-[2-(2-thienylthio)-ethyl]-piperidin-3-essigsäure in ihren diastereomeren Formen oder ihre Gemische, sowie ihre Salze, handelt.

7. Chinolylpropylpiperidinderivat gemäß einem der Ansprüche 1 oder 2, **dadurch gekennzeichnet, dass** es sich um die (3R,4R)-1-[2-(3-Fluorphenylthio)-ethyl]-4-[3-hydroxy-3-(6-methoxychinolin-4-yl)-propyl]-piperidin-3-essigsäure in ihren diastereomeren Formen oder ihre Gemische, sowie ihre Salze, handelt.

8. Verfahren zur Herstellung des Chinolylpropylpiperidinderivats gemäß Anspruch 1, **dadurch gekennzeichnet, dass** man die Kette R₃, wie in Anspruch 1 definiert, mit dem Chinolylpropylpiperidinderivat der allgemeinen Formel 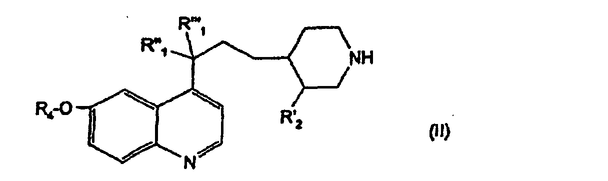 kondensiert, worin R₄ wie in Anspruch 1 definiert ist, R"₁ und R'''₁ Wasserstoffatome bedeuten oder gemeinsam einen Oxorest bilden und R'₂ einen Carboxy-, Carboxymethyloder 2-Carboxyethylrest in geschützter Form oder einen Alkoxycarbonyl-, Alkoxycarbonylmethyl- oder 2-Alkoxycarbonylethylrest bedeutet, um zu einem Chinolylpropylpiperidinderivat der allgemeinen Formel 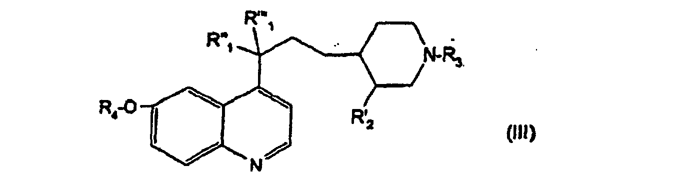 zu gelangen, worin R"₁, R"'₁, R'₂ und R₄ wie vorstehend definiert sind und R₃ die in Anspruch 1 angegebene Bedeutung besitzt,
anschließend gegebenenfalls die Schutzgruppe der Säure entfernt,
oder gegebenenfalls den durch R"₁ und R'''₁ wiedergegebenen Oxorest in einen Alkohol überführt, worin R₁ Hydroxy bedeutet, danach gegebenenfalls die Halogenierung durchführt, wenn man ein Chinolylpropylpiperidinderivat erhalten möchte, bei dem R₁ ein Halogenatom bedeutet und gegebenenfalls die Halogenwasserstoffabspaltung von dem entsprechenden Halogenderivat durchführt, um zu einem Chinolylpropylpiperidinderivat zu gelangen, bei dem R₁ und R⁰ gemeinsam eine Bindung bilden, oder die Dihalogenierung des Produkts der allgemeinen Formel (III), worin R"₁ und R"'₁ gemeinsam einen Oxorest bilden, durchführt, um zu einem Chinolylpropylpiperidinderivat zu gelagen, bei dem R₁ und R'₁ Halogenatome sind, und/oder gegebenenfalls die in Form eines Restes R'₂ in 3-Stellung des Piperidins geschützte Säure in einen Hydroxymethylrest reduziert, und gegebenenfalls in einen Carboxymethyl- oder 2-Carboxyethylrest nach üblichen Methoden überführt, anschließend gegebenenfalls die Schutzgruppe der Säure entfernt und/oder gegebenenfalls die Diastereomeren trennt und gegebenenfalls das erhaltene Produkt in ein Salz überführt.

9. Verfahren gemäß Anspruch 8, **dadurch gekennzeichnet, dass** die Kondensation der Kette R₃ mit dem Piperidin durch Einwirken eines Derivats der allgemeinen Formel:
R₃-X
erfolgt, worin R₃ wie in Anspruch 1 definiert ist, und X ein Halogenatom, einen Methylsulfonylrest, einen Trifluormethylsulfonyl- oder p-Toluolsulfonylrest bedeutet.

10. Pharmazeutische Zusammensetzung, **dadurch gekennzeichnet, dass** sie zumindest ein Derivat gemäß Anspruch 1 in reiner Form oder in Assoziation mit ein oder mehreren kompatiblen und pharmazeutisch verträglichen Verdünnungsmitteln oder Adjuvantien enthält.

## Claims

1. A quinolylpropylpiperidine derivative of general formula: 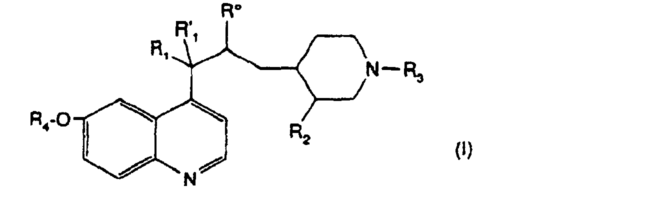 in which:
R₁ is a hydrogen or halogen atom or a hydroxyl radical,
R'₁ is a hydrogen atom or can represent a halogen atom when R₁ is also a halogen atom, and
R^{o} is a hydrogen atom, or else
R₁ and R^{o} together form a bond and
R'₁ is a hydrogen atom,
R₂ represents a carboxyl, carboxymethyl, 2-carboxyethyl, hydroxymethyl, alkyloxycarbonyl, alkyloxycarbonylmethyl or 2-(alkyloxycarbonyl)ethyl radical (the alkyl parts of which comprise from 1 to 6 C),
R₃ represents an alkylene radical (comprising 1 to 6 carbon atoms) substituted by a sulphur atom carrying a phenyl substituent which can itself carry 1 to 4 substituents [chosen from halogen, hydroxyl, (C₁₋₄)alkyl, (C₁₋₄)alkyloxy, trifluoromethyl, trifluoromethoxy, carboxyl, (C₁₋₄)alkyloxycarbonyl, cyano and amino], a 3- to 7-membered cycloalkyl substituent or a 5- to 6-membered aromatic heterocyclyl substituent comprising 1 to 4 heteroatoms chosen from nitrogen, oxygen and sulphur and being itself optionally substituted [by halogen, hydroxyl, (C₁₋₄)alkyl, (C₁₋₄)alkyloxy, trifluoromethyl, trifluoromethoxy, oxo, carboxyl, (C₁₋₄)alkyloxycarbonyl, cyano or amino],
and R₄ represents an alkyl radical (comprising 1 to 6 carbon atoms), an alkenyl-CH₂- radical or an alkynyl-CH₂- radical, the alkenyl or alkynyl parts of which comprise 2 to 6 carbon atoms,
it being understood that the alkyl and alkylene radicals are straight- or branched-chain radicals,
in its diastereoisomeric forms or their mixtures, as well as its salts.

2. A quinolylpropylpiperidine derivative according to Claim 1, **characterized in that**
R₁ is a hydrogen or halogen atom or a hydroxyl radical,
R'₁ is a hydrogen atom, and
R^{o} is a hydrogen atom, or else
R₁ and R^{o} together form a bond and
R'₁ is a hydrogen atom,
R₂ represents a carboxyl or carboxymethyl radical, and
R₃ represents an alkylene radical (1 to 6 carbon atoms) substituted by a sulphur atom carrying a phenyl substituent which can itself carry 1 to 4 halogen atoms, a cycloalkyl substituent, the cyclic part of which comprises 3 to 7 members, or a 5- to 6-membered aromatic heterocyclyl substituent comprising 1 to 4 heteoatoms chosen from nitrogen, oxygen and sulphur and optionally itself substituted by halogen, or else
when R² represents a hydroxymethyl, alkyloxycarbonyl or alkyloxycarbonylmethyl radical (the alkyl portions of which comprise 1 to 6 carbon atoms), then
R₃ represents an alkylene radical (1 to 6 carbon atoms) substituted by a sulphur atom carrying a 5- to 6-membered aromatic heterocyclyl substituent comprising 1 to 4 heteroatoms chosen from nitrogen, oxygen or sulphur and optionally itself substituted by halogen,
and R₄ represents an alkyl radical (comprising 1 to 6 carbon atoms),
the alkyl radicals and portions being straight- or branched-chain radicals and portions,
in its diastereoisomeric forms or their mixtures, as well as its salts.

3. A quinolylpropylpiperidine derivative according to either of Claims 1 and 2, **characterized in that** it is (3R,4R)-4-[3-hydroxy-3-(6-methoxyquinoline-4-yl)propyl]-1-[2-(2-thienylthio)ethyl]piperidine-3-carboxylic acid, in its diastereoisomeric forms or their mixtures, as well as its salts.

4. A quinolylpropylpiperidine derivative according to either of Claims 1 and 2, **characterized in that** it is (3R,4R)-4-[3-hydroxy-3-(6-methoxyquinoline-4-yl)propyl]-1-[2-(2-thienylthio)ethyl]piperidine-3-acetic acid, in its diastereoisomeric forms or their mixtures, as well as its salts.

5. A quinolylpropylpiperidine derivative according to either of Claims 1 and 2, **characterized in that** it is (3R,4R)-4-[3-(6-methoxyquinoline-4-yl)propyl]-1-[2-(2-thienylthio)ethyl]piperidine-3-acetic acid, as well as its salts.

6. A quinolylpropylpiperidine derivative according to either of Claims 1 and 2, **characterized in that** it is (3R,4R)-4-[3-fluoro-3-(6-methoxyquinoline-4-yl)propyl]-1-[2-(2-thienylthio)ethyl]piperidine-3-acetic acid, in its diastereoisomeric forms or their mixtures, as well as its salts.

7. A quinolylpropylpiperidine derivative according to either of Claims 1 and 2, **characterized in that** it is (3R,4R)-1-[2-(3-fluorophenylthio)ethyl]-4-[3-hydroxy-3-(6-methoxyquinolin-4-yl)propyl]piperidine-3-acetic acid, in its diastereoisomeric forms or their mixtures, as well as its salts.

8. A process for the preparation of quinolylpropylpiperidine derivative according to Claim 1, **characterized in that** the R₃ chain defined in Claim 1 is condensed onto the quinolylpropylpiperidine derivative of general formula: 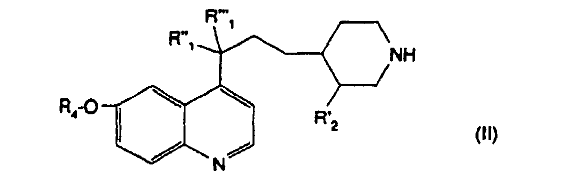 in which R₄ is defined as in Claim 1, R''₁ and R'''₁ represent hydrogen atoms or together form an oxo radical and R'₂ represents a protected carboxyl, carboxymethyl or 2-carboxyethyl radical or an alkyloxycarbonyl, alkyloxycarbonylmethyl or 2-(alkyloxycarbonyl)ethyl radical, in order to obtain a quinolylpropylpiperidine derivative of general formula: 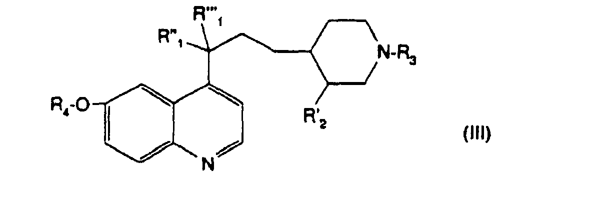 in which R''₁, R'''₁, R'₂ and R₄ are defined as above and R₃ is defined as in Claim 1,
then, if appropriate, the acid-protecting radical is removed,
or else, if appropriate, the oxo radical represented by R''₁ and R'''₁ is reduced to an alcohol in which R₁ represents hydroxyl, then, optionally, halogenation is carried out, if it is desired to obtain a quinolylpropylpiperidine derivative in which R₁ is a halogen atom, and, optionally, dehydrohalogenation of the corresponding halogenated derivative is carried out, in order to obtain a quinolylpropylpiperidine derivative in which R₁ and R^{o} together form a bond, or else dihalogenation of the product of general formula (III) in which R''₁ and R'''₁ together form an oxo radical is carried out, in order to obtain a quinolylpropylpiperidine derivative in which R₁ and R'₁ are halogen atoms,
and/or, if appropriate, the acid, protected in the form of an R'₂ radical, in the 3-position of the piperidine is reduced to a hydroxymethyl radical and optionally converted to a carboxymethyl or 2-carboxyethyl radical according to the usual methods,
then, optionally, the acid-protecting radical is removed and/or, if appropriate, the diastereoisomers are separated and the product obtained is optionally converted to a salt.

9. The process according to Claim 8, **characterized in that** the condensation of the R₃ chain onto the piperidine is carried out by the action of a derivative of general formula:
R₃-X
in which R₃ is defined as in Claim 1 and X represents a halogen atom, a methylsulphonyl radical, a trifluoromethylsulphonyl radical or a p-toluenesulphonyl radical.

10. A pharmaceutical composition, **characterized in that** it comprises at least one derivative according to Claim 1 in the pure state or in combination with one or more compatible and pharmaceutically acceptable diluents or adjuvants.
